# EUROPEAN PATENT APPLICATION

(11) **EP 4 385 520 A2**
(43) Date of publication of application: **19.06.2024**
(21) Application number: 24160015.4
(22) Date of filing: 16.09.2016
(51) Int. Cl.: A61K 39/35

(54) **GLYCOTARGETING THERAPEUTICS**

(30) Priority: 19.09.2015 US 201514859292; 17.06.2016 US 201615185564
(62) Divisional of application: 16778870.2
(71) Applicant: Ecole Polytechnique Fédérale de Lausanne, 1015 Lausanne (CH)
(72) Inventor: HUBBELL, Jeffrey A., 1015 Lausanne (CH); WILSON, David Scott, 1015 Lausanne (CH)
(74) Representative: Zwicker, Jörk

(57) **Abstract**

Several embodiments of the present disclosure relate to therapeutic compositions configured to target the liver of a subject and that are useful in the treatment or prevention of one or more of transplant rejection, autoimmune disease, food allergy, and immune response against a therapeutic agent. In several embodiments, the compositions are configured to target the liver and deliver antigens to which tolerance is desired. In several embodiments, the compositions are configured for clearance of a circulating protein or peptide or antibody associated with one or more of the above-mentioned maladies. Methods and uses of the compositions for induction of immune tolerance are also disclosed herein.

## Description

### CROSS-REFERENCE TO RELATED APPLICATION

This application claims the benefit of US Patent Application No. 14/859,292 filed September 19, 2015, and US Patent Application 15/185,564, filed June 17, 2016, each entitled "GLYCOTARGETING THERAPEUTICS," the entirety of each of which is hereby incorporated by reference.

### REFERENCE TO SEQUENCE LISTING

A Sequence Listing submitted as an ASCII text file via EFS-Web is hereby incorporated by reference in accordance with 35 U.S.C. § 1.52(e). The name of the ASCII text file for the Sequence Listing is ANOK001P1WO_ST25.TXT, the date of creation of the ASCII text file is September 14, 2016, and the size of the ASCII text file is 47.4 KB.

### BACKGROUND

### Field

Several embodiments of the invention disclosed herein relate to pharmaceutically acceptable compositions that are useful in the treatment of transplant rejection, autoimmune disease, allergy (e.g., food allergy), and immune response against a therapeutic agent.

### Description of Related Art

Various approaches have been used to induce tolerance to antigens that elicit an unwanted immune response. Some approaches employ targeting of the antigens to specific cells. Applications US 2012/0039989, US 2012/0178139 and WO 2013/121296 describe the targeting of antigens to erythrocytes to take advantage of the erythrocytes' role in antigen presentation for tolerization.

### SUMMARY

Notwithstanding the positive results generated to date using cell-targeting approaches, the possibility of alternative approaches has remained of interest. In particular, several embodiments disclosed herein relate to compositions configured to target one or more cell types in the liver and, as a result, deliver an antigen to which tolerance is desired to the one or more cell types targeted, and thereby induce a processing of the antigen and induce immune tolerance to the antigen. The antigen, as disclosed in more detail below, can comprise a therapeutic agent, a protein, a protein fragment, an antigenic mimic of a protein or protein fragment (e.g., a mimotope). Additional types of antigens are discussed in more detail below. Methods and uses of such compositions are also provided for, in several embodiments.

In several embodiments, there is provided a compound comprising Formula 1: 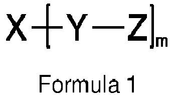 wherein m is an integer from about 1 to 10, X comprises molecule comprising an antigenic region, Y is of a linker moiety having a formula selected from the group consisting of: 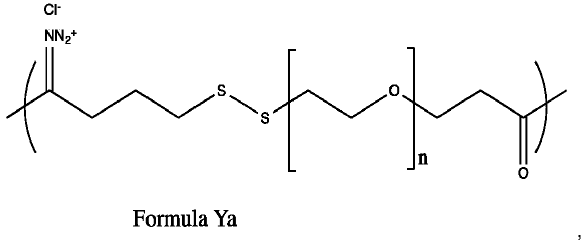 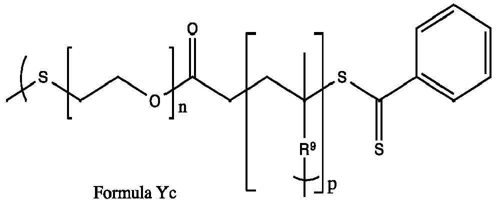 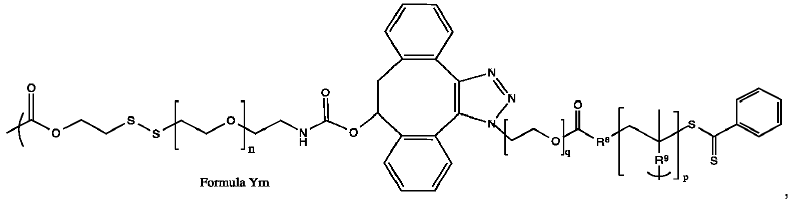 and 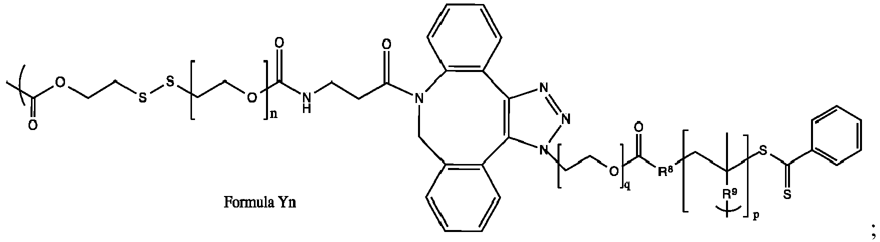 wherein the left bracket "(" indicates a bond to X, the right or bottom bracket and ")" indicates the bond between Y and Z, n is an integer from about 1 to 100, where present p is an integer from about 2 to 150, where present q is an integer from about 1 to 44, where present R⁸ is -CH₂- or -CH₂-CH₂-C(CH₃)(CN)-; and where present R⁹ is a direct bond or -CH₂-CH₂--NH-C(O)-, and Z comprises a liver-targeting moiety. In several embodiments, X is a protein or protein fragment comprising an antigenic region. In several embodiments, Z is galactose, while in some embodiments Z is glucose. In several embodiments, Z is galactosamine, while in some embodiments Z is glucosamine. In several embodiments, the alpha anomer of glucose or galactose is used in the composition. In several embodiments, the beta anomer of glucose or galactose is used in the composition. In several embodiments, mixtures of the alpha and beta anomers are used, including optionally mixtures of glucose and galactose. In several embodiments, Z is N-acetylgalactosamine, while in some embodiments Z is N-acetylglucosamine. In several embodiments, the alpha anomer of glucosamine or galactosamine is used in the composition. In several embodiments, the alpha anomer of glucosamine or galactosamine is used in the composition. In several embodiments, mixtures of the alpha and beta anomers are used, including optionally mixtures of glucosamine and galactosamine. Likewise, in several embodiments the alpha anomer, the beta anomer, or combinations of the alpha and beta anomers of N-acetylgalactosamine or N-acetylglucosamine. Combinations of any of the alpha or beta anomeric forms of any of the liver targeting sugars, and any combinations of the sugars can be employed in various embodiments. In several embodiments Y comprises Yₘ or Yₙ, m is between 1 and 5, n is between 75 and 85, p is between 85 and 95, and q is between 2 and 6. In several embodiments m is between 1 and 3, n is 79, p is 90, and q is 4. In several embodiments, X is selected from the group consisting of insulin, proinsulin, preproinsulin, gluten, gliadin, myelin basic protein, myelin oligodendrocyte glycoprotein and proteolipid protein, Factor VIII, Factor IX, asparaginase, uricase and fragments of any of the preceding. In several embodiments, the antigen X is not a full length protein. For example, in some embodiments, the antigen is not full length gliadin, insulin, or proinsulin. In several embodiments, the antigen X is not a fragment of a protein. In several embodiments, m is not greater than 3, n is not greater than 80, p is not greater than 100 and q is not more than 5.

In several embodiments, Y is a linker moiety having a formula of:

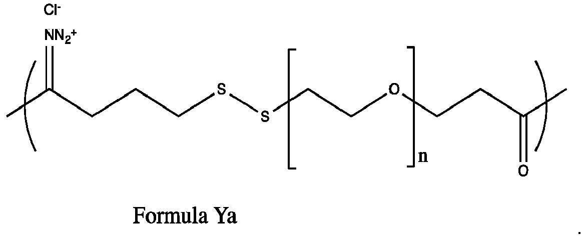

In several embodiments, Y is a linker moiety having a formula of:

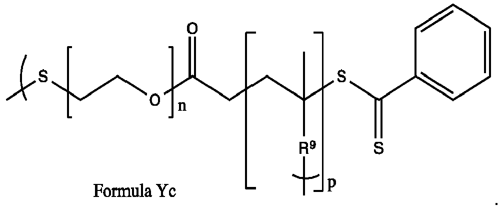

In several embodiments, Y is a linker moiety having a formula of:

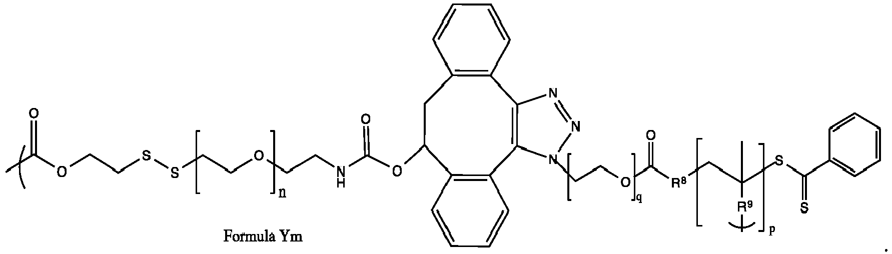

In several embodiments, Y is a linker moiety having a formula of:

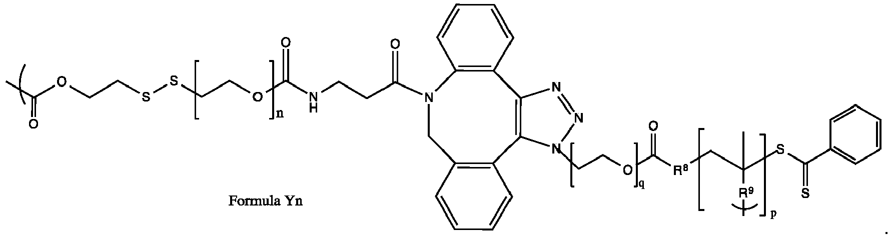

In some embodiments, combinations of the linkers disclosed herein may be used, just as combinations of the liver targeting moieties can be employed.

As discussed in more detail below, there exist a variety of antigens to which tolerance may be desired. These may include, but are not limited to, exogenous antigens that result in an adverse immune response when a subject is exposed to the antigen. In several embodiments, the adverse immune response could be a result of ingestion of the antigen, e.g., orally or nasally, or via some other mucosal route. These routes could be the case, for example, with food antigens. In some embodiments, the antigen may be purposefully administered to a subject, for example, with the administration of a therapeutic composition to treat a disease or condition that the subject is affected by. In still additional embodiments, the antigen may be produced by the subject, e.g., an autoimmune antigen. For example, in several embodiments, X comprises a foreign transplant antigen against which transplant recipients develop an unwanted immune response or a tolerogenic portion thereof. In several embodiments, X comprises a foreign food, animal, plant or environmental antigen against which patients develop an unwanted immune response or a tolerogenic portion thereof. In several embodiments, X comprises a foreign therapeutic agent against which patients develop an unwanted immune response or a tolerogenic portion thereof. In several embodiments, X comprises a synthetic self-antigen against the endogenous version of which patients develop an unwanted immune response or a tolerogenic portion thereof.

In further detail to the above, there are provided in several embodiments compounds where X is a food antigen. In some such embodiments, X is one or more of conarachin (Ara h 1), allergen II (Ara h 2), arachis agglutinin, conglutin (Ara h 6), a-lactalbumin (ALA), lactotransferrin, Pen a 1 allergen (Pen a 1), allergen Pen m 2 (Pen m 2), tropomyosin fast isoform, high molecular weight glutenin, low molecular weight glutenin, alpha- gliadin, gamma-gliadin, omega-gliadin, hordein, seclain, and avenin. Fragment of any of these antigens and/or mimotopes of any of these antigens are also used, in several embodiments. In several embodiments, X is selected from the group consisting of gluten, high molecular weight glutenin, low molecular weight glutenin, alpha- gliadin, gamma-gliadin, omega-gliadin, hordein, seclain, and avenin and fragments thereof. In several embodiments, X is selected from the group consisting of gluten, high molecular weight glutenin, low molecular weight glutenin, alpha- gliadin, gamma-gliadin, and omega-gliadin and fragments thereof. In several embodiments, X is gluten or fragment thereof. In several embodiments, X is gliadin or fragment thereof.

In several embodiments, there are provided compounds where X is a therapeutic agent. In several embodiments, X is selected from the group consisting of Factor VII, Factor IX, asparaginase, and uricase and fragments thereof. In several embodiments, X is a therapeutic agent selected from the group consisting of Factor VII and Factor IX and fragments thereof. In several embodiments, X is a therapeutic agent selected from the group consisting of Factor VIII or fragment thereof. In several embodiments, when X is a therapeutic agent, the compound can be used in the treatment, prevention, reduction, or otherwise amelioration of an immune response developed against a therapeutic agent for hemophilia. As discussed herein, mimotopes of any antigenic portion of the antigens above can be used in several embodiments.

In several embodiments, X comprises asparaginase or a fragment thereof. In several embodiments, X comprises uricase or a fragment thereof. In several such embodiments, the compound can be used in the treatment, prevention, reduction, or otherwise amelioration of an immune response developed against an anti-neoplastic agent. As discussed herein, mimotopes of any antigenic portion of the antigens above can be used in several embodiments.

In several embodiments, X is associated with an autoimmune disease. For example, in several embodiments, the associated autoimmune disease is one or more of Type I diabetes, multiple sclerosis, rheumatoid arthritis, vitiligo, uveitis, pemphis vulgaris and neuromyelitis optica.

In several embodiments, the autoimmune disease is Type I diabetes and X comprises insulin or a fragment thereof. In several embodiments, the autoimmune disease is Type I diabetes and X comprises proinsulin or a fragment thereof. In several embodiments, the autoimmune disease is Type I diabetes and X comprises preproinsulin or a fragment thereof. As discussed herein, mimotopes of any antigenic portion of the antigens above can be used in several embodiments. In several embodiments, combinations of these antigens can be incorporated into the tolerogenic compound which may aid in reducing immune responses to self-antigens at multiple points along the insulin pathway.

In several embodiments, the autoimmune disease is multiple sclerosis and X comprises myelin basic protein or a fragment thereof. In several embodiments, the autoimmune disease is multiple sclerosis and X comprises myelin oligodendrocyte glycoprotein or a fragment thereof. In several embodiments, the autoimmune disease is multiple sclerosis and X comprises myelin proteolipid protein or a fragment thereof. As discussed herein, mimotopes of any antigenic portion of the antigens above can be used in several embodiments. In several embodiments, combinations of these antigens can be incorporated into the tolerogenic compound which may aid in reducing immune responses to self-antigens at multiple points along the enzymatic pathways that control myelination or myelin repair.

In several embodiments, the autoimmune disease is rheumatoid arthritis and X is selected from the group consisting of fibrinogen, vimentin, collagen type II, alpha enolase and fragments thereof.

In several embodiments, the autoimmune disease is vitiligo and X is selected from the group consisting of Pmel17, tyrosinase and fragments thereof.

In several embodiments, the autoimmune disease is uveitis and X is selected from the group consisting of retinal arrestin and interphotoreceptor retinoid-binding protein (IRBP) and fragments thereof.

In several embodiments, the autoimmune disease is pemphigus vulgaris and X is selected from the group consisting of desmoglein 3, 1 and 4, pemphaxin, desmocollins, plakoglobin, perplakin, desmoplakins, acetylcholine receptor and fragments thereof.

In several embodiments, the autoimmune disease is neuromyelitis optica and X is aquaporin-4 or a fragment thereof.

As discussed herein, mimotopes of any antigenic portion of the self-antigens above (or otherwise disclosed herein) can be used in several embodiments.

Also provided for in several embodiments is the use of the compounds disclosed above (or otherwise disclosed herein) for use in inducing tolerance to X.

There are also provided for in several embodiments herein pharmaceutically acceptable compositions comprising a compound disclosed above (or otherwise disclosed herein). There is also provided for the use of such compositions in inducing tolerance to X. In several embodiments, the pharmaceutically acceptable composition consists of, or consists essentially of a compound wherein X is a food antigen, therapeutic agent, a self antigen, or fragment thereof, a linker Y, and a liver targeting moiety Z selected from glucose, galactose, glucosamine, galactosamine, N-acetylglucosamine, and N-acetylgalactosamine.

Also provided for herein are methods of inducing tolerance to an antigen to which a subject is capable of developing an unwanted immune response, comprising administering a compounds disclosed above (or otherwise disclosed herein). In several embodiments, the compound is administered prior to the subject being exposed to the antigen. However, in several embodiments, the compound is administered after the subject has been exposed to the antigen. In several embodiments, the administration comprises at least one intravenous administration of the compound (e.g., a bolus dose followed by a series of optional maintenance doses).

In several embodiments, there is provided for the use of compounds disclosed above (or otherwise disclosed herein) in the preparation of a medicament for inducing tolerance to an antigen to which a subject develops an unwanted immune response or a tolerogenic portion thereof.

In several embodiments disclosed herein, there are provided compositions for inducing immune tolerance in a subject and methods and uses of the compositions for achieving the same. In several embodiments, immune tolerance is desired because a subject develops an unwanted immune response to an antigen. Depending on the embodiment, the antigen may be one or more of a variety of antigens, for example a foreign antigen such as a food antigen that is ingested, or an antigenic portion of a therapeutic drug given to a subject. In additional embodiments, the antigen may be a self-antigen that the subject's immune system fails to recognize (or only recognizes as self to a limited degree) and therefore mounts an immune response against, leading to autoimmune disorders.

In several embodiments, there is provided a composition comprising Formula 1: 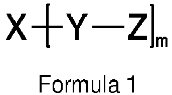 wherein m is an integer from about 1 to 10, X comprises a food antigen, a therapeutic agent, a self-antigen, a fragment of any of such antigens, or a mimotope of any of such antigens, Y is of a linker moiety having the following formula: 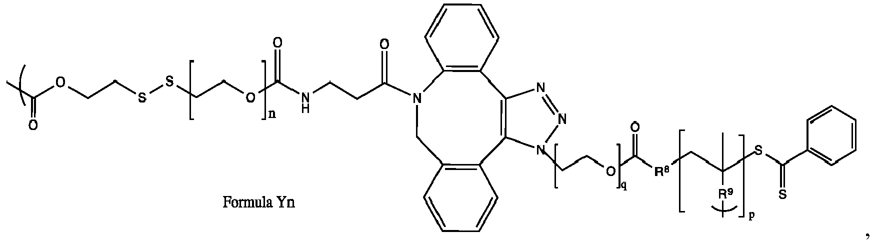 wherein:, the left bracket "(" indicates a bond to X, the right or bottom bracket and ")" indicates the bond between Y and Z, n is an integer from about 70 to 85, where present p is an integer from about 85 to 95, where present q is an integer from about 1 to 10, where present R⁸ is -CH₂- or -CH₂-CH₂-C(CH₃)(CN)-; and where present R⁹ is a direct bond or -CH₂-CH₂--NH-C(O)-; and Z comprises a liver-targeting moiety comprising glucose or galactose. In several embodiments, m is between 1 and 3, n is 79, p is 90, and q is 4. In several embodiments, X is selected from the group consisting of insulin, proinsulin, preproinsulin, gluten, gliadin, myelin basic protein, myelin oligodendrocyte glycoprotein and proteolipid protein, Factor VIII, Factor IX, asparaginase, uricase and fragments of any of the preceding. In several embodiments, the composition comprises, consists of, or consists essentially of the antigen X, the linker Y and the liver targeting moiety Z.

In several embodiments, there is provided a compound comprising Formula 1: 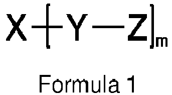 wherein m is an integer from about 1 to 10, X is selected from the group consisting of insulin, proinsulin, preproinsulin, gluten, gliadin, myelin basic protein, myelin oligodendrocyte glycoprotein and proteolipid protein, Factor VIII, Factor IX, asparaginase, uricase and fragments of any of the preceding, Y is of a linker moiety having the following formula: 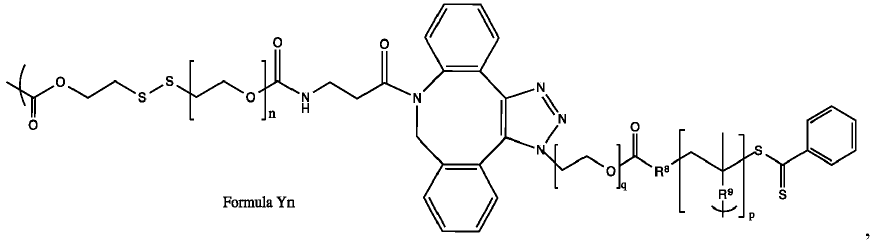 wherein, the left bracket "(" indicates a bond to X, the right or bottom bracket and ")" indicates the bond between Y and Z, n is an integer from about 70 to 85, where present p is an integer from about 85 to 95, where present q is an integer from about 1 to 10, where present R⁸ is -CH₂- or -CH₂-CH₂-C(CH₃)(CN)-; and where present R⁹ is a direct bond or -CH₂-CH₂--NH-C(O)-, and Z comprises a liver-targeting moiety comprising a sugar moiety. In several embodiments, m is between 1 and 3, n is 79, p is 90, and q is 4. In several embodiments, Z is selected from the group consisting of glucose, glucosamine, galactose, galactosamine, N-acetylgalactosamine and N-acetylglucosamine.

In several embodiments, 2,5-dioxopyrrolidin-1-yl propyl carbonate-linkers and/or 2-(ethyldisulfanyl)ethyl ethylcarbamate-linkers can be used.

In several embodiments, there is provided a composition comprising a compound of Formula 1: 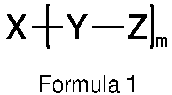 wherein:
m is an integer from about 1 to 10;
X comprises an antigen to which patients develop an unwanted immune response, wherein the antigen is a food antigen, a therapeutic agent, a self-antigen, or a fragment of any of such antigens;
Y is of a linker moiety having a formula selected from the group consisting of: 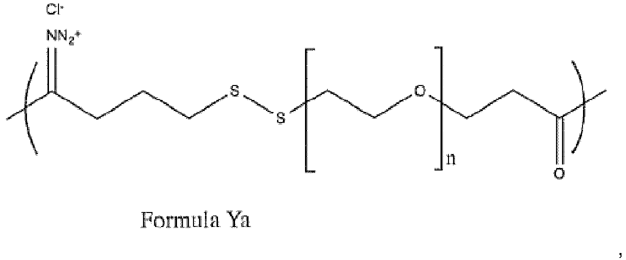 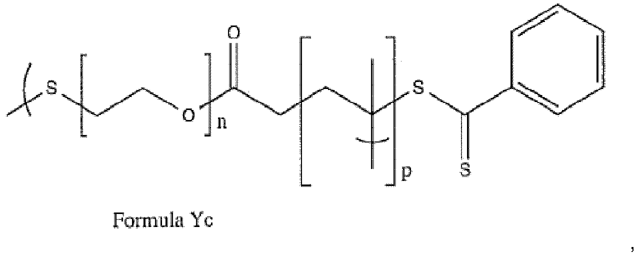 and 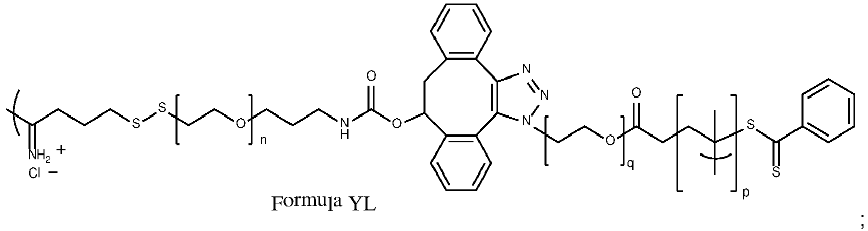
wherein the left bracket "(" indicates a bond to X, where present the right ")" indicates a bond to Z, where present the bottom ")" indicates a bond to Z, where present n is an integer from about 1 to about 80, where present q is an integer from about 1 to about 4, where present p is an integer from about 1 to about 90, where present R₈ is -CH₂- or -CH₂-CH₂-C(CH₃)(CN)-, and Z comprises one or more liver-targeting moieties that specifically target liver cells expressing asialoglycoprotein receptors.

In several embodiments of the composition, m is 1 to 4, Y is of a linker moiety having a formula of: 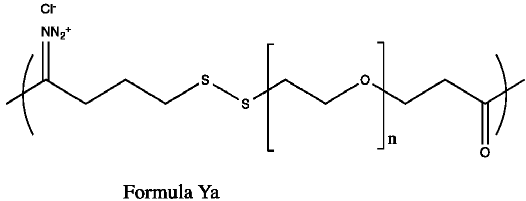 and Z comprises a liver-targeting moiety comprising one or more of galactose, galactosamine, or N-acetyl galactosamine.

In several embodiments, m is resolved to an integer from 1 to 4, Y is of a linker moiety having a formula of: 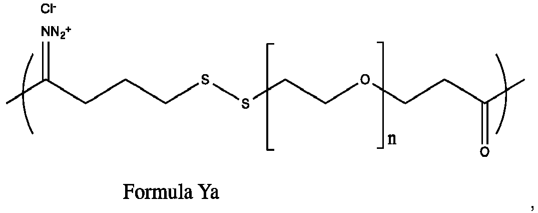 and Z comprises a liver-targeting moiety comprising one or more of glucose, glucosamine, or N-acetyl glucosamine.

In several embodiments, there is provided compositions of Formula 1 (X-⁅-Y-Z]ₘ), where m is an integer from about 1 to 100, X comprises an antigen against which a patient develops an unwanted immune response, or a tolerogenic portion thereof or X comprises an antibody, antibody fragment or ligand that specifically binds a circulating protein or peptide or antibody, which circulating protein or peptide or antibody is causatively involved in transplant rejection, immune response against a therapeutic agent, autoimmune disease, hypersensitivity and/or allergy, Y comprises a linker moiety, and Z comprises a liver-targeting moiety. In several embodiments, Z comprises galactose, galactosamine, N-acetylgalactosamine, glucose, glucosamine or N-acetylglucosamine.

In several embodiments, Y is selected from N-hydroxysuccinamidyl linkers, malaemide linkers, vinylsulfone linkers, pyridyl di-thiol-poly(ethylene glycol) linkers, pyridyl di-thiol linkers, n-nitrophenyl carbonate linkers, NHS-ester linkers, and nitrophenoxy polyethylene glycol)ester linkers. In some embodiments, Y comprises an antibody, antibody fragment, peptide or other ligand that specifically binds X, a disulfanyl ethyl ester, a structure represented by one of Formulae Ya to Yp: 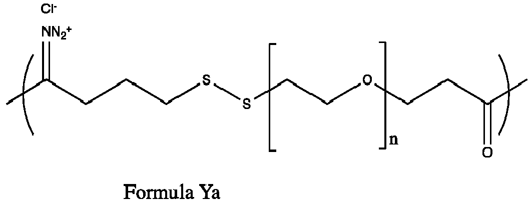 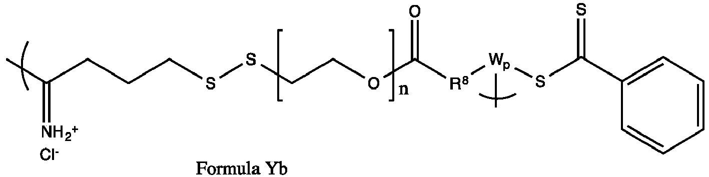 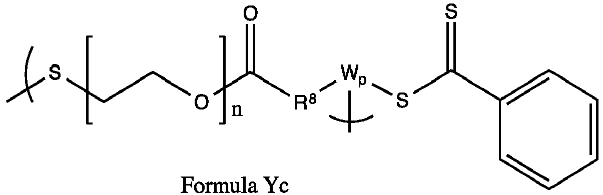 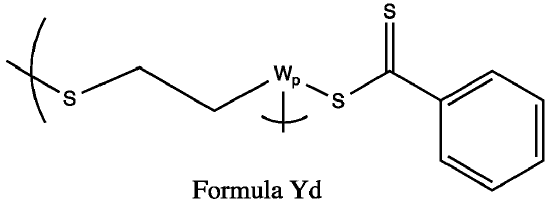 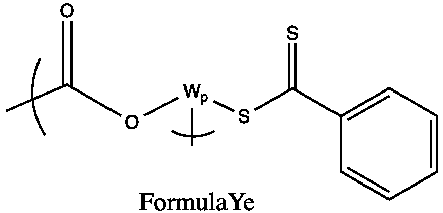 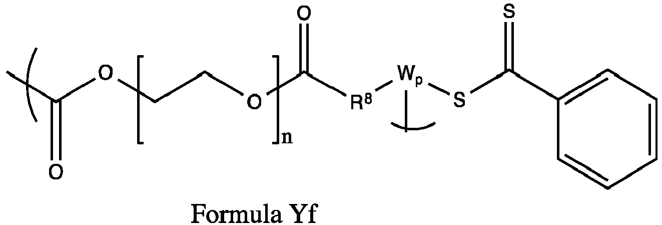 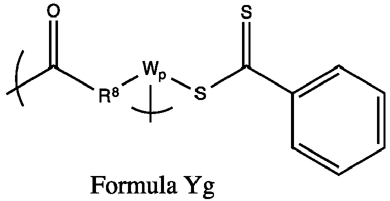 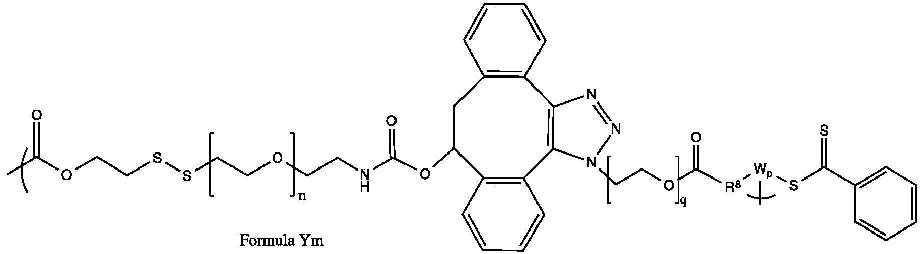 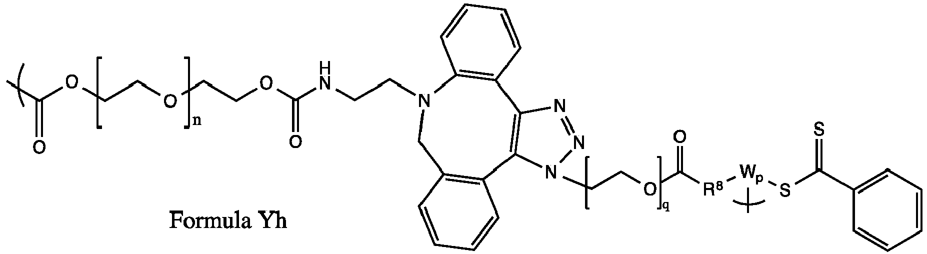 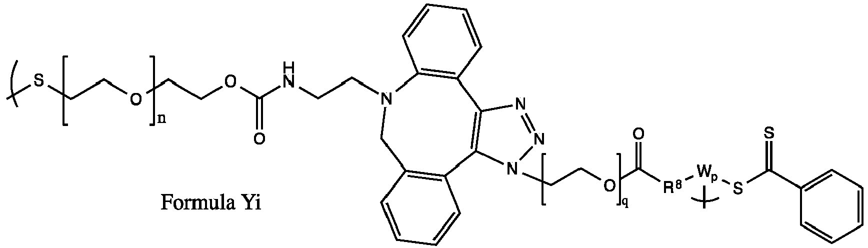 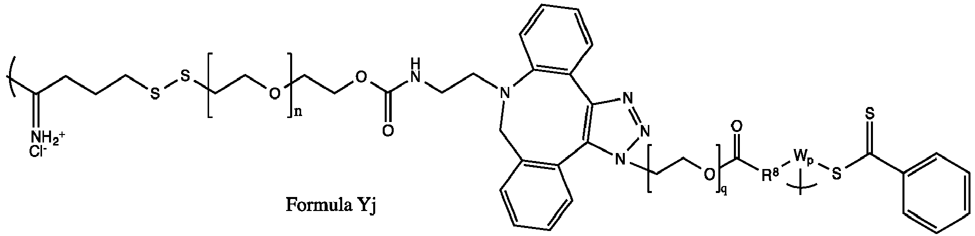 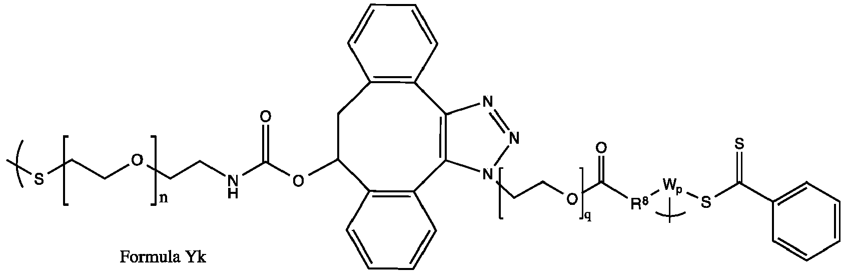 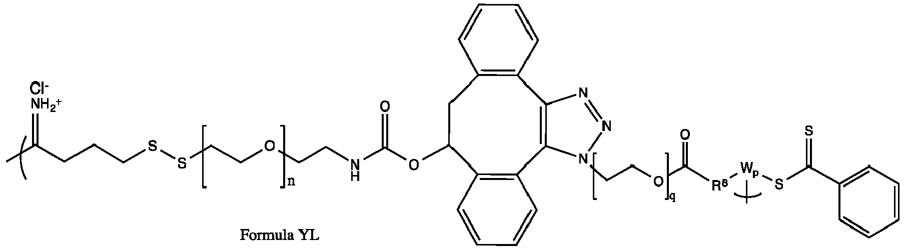 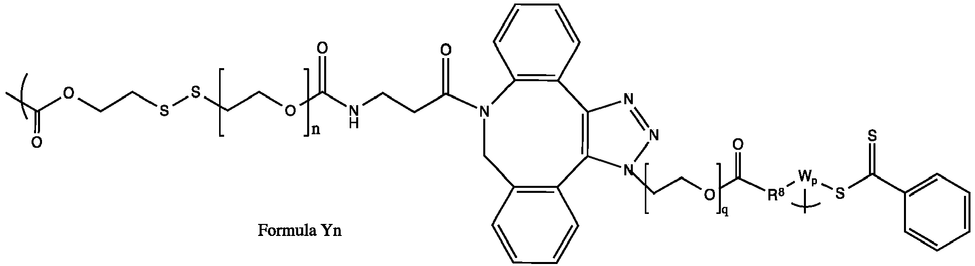 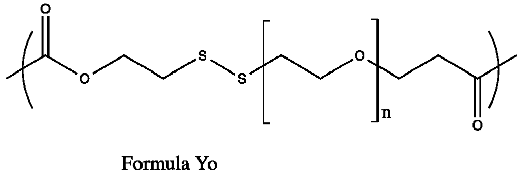 and 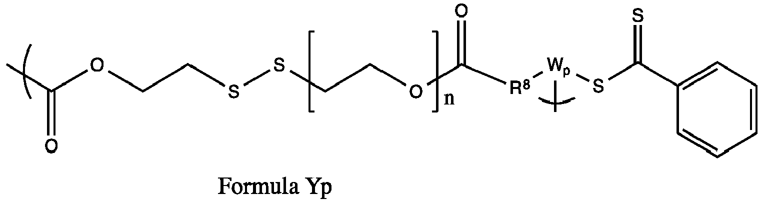 or Y has a portion represented by Formula Y'-CMP:

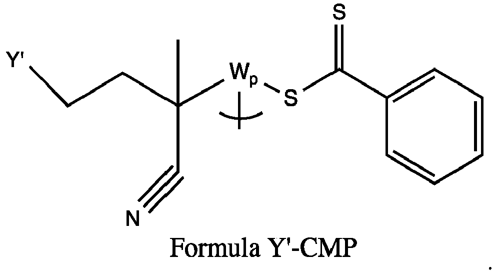

In such embodiments, the left bracket "(" indicates the bond between X and Y, the right or bottom bracket and ")" indicates the bond between Y and Z, n is an integer from about 1 to 100, q is an integer from about 1 to 44, R⁸ is -CH₂- or -CH₂-CH₂-C(CH₃)(CN)-, Y' represents the remaining portion of Y; and W represents a polymer of the same W¹ group, or W is a copolymer or a random copolymer of the same or different W¹ and W² groups, where: 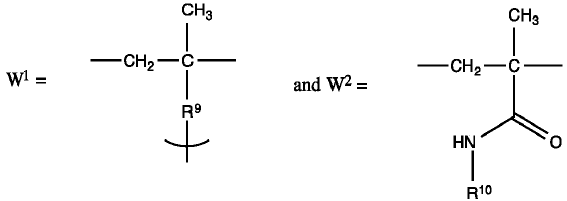 and where p is an integer from 2 to about 150, R⁹ is a direct bond, -CH₂-CH₂--NH-C(O)- or -CH₂-CH₂-(O-CH₂-CH₂)ₜ-NH-C(O)-, t is an integer from 1 to 5; and R¹⁰ is an aliphatic group, an alcohol or an aliphatic alcohol. In one such embodiment, m is 1 to 3, Y is represented by Formula Ym, wherein R⁸ is -CH₂-CH₂-C(CH₃)(CN)-, and W is represented by a block copolymer of W¹ and W² where R⁹ is -CH₂-CH₂-(O-CH₂-CH₂)ₜ-NH-C(O)-, t is 1, and R¹⁰ is 2-hydroxypropyl; and Z comprises a liver-targeting moiety comprising one or more of galactose, galactosamine, N-acetylgalactosamine, glucose, glucosamine, N-acetylglucosamine. In several embodiments, Z is the β-anomer of the corresponding sugar.

In several additional embodiments compositions are provided for inducing tolerance to an antigen to which a subject develops an unwanted immune response, the compositions comprising a compound of Formula 1 (Formula 1 (X-⁅-Y-Z]ₘ), where m is an integer from about 1 to 10, X comprises an antigen to which patients develop an unwanted immune response, wherein the antigen is a food antigen, a therapeutic agent, a self-antigen, or a fragment of any of such antigens, Y is of a linker moiety having a formula selected from the group consisting of: 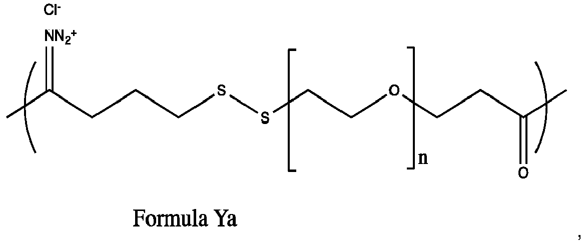 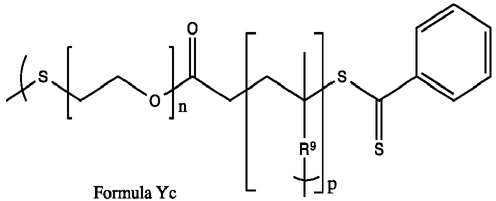 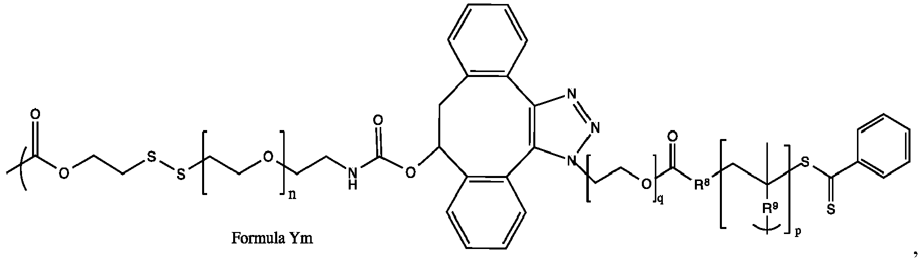 and 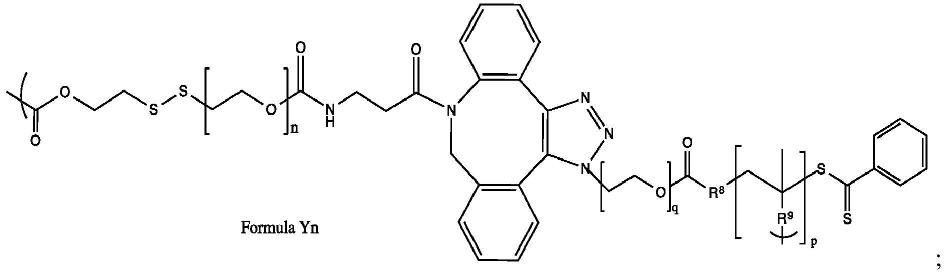 wherein the left bracket "(" indicates a bond to X, the right or bottom bracket and ")" indicates the bond between Y and Z, n is an integer from about 1 to 100, where present p is an integer from about 2 to 150, where present q is an integer from about 1 to 44, where present R⁸ is -CH₂- or -CH₂-CH₂-C(CH₃)(CN)-, and where present R⁹ is a direct bond or -CH₂-CH₂--NH-C(O)-, and Z comprises galactose, galactosamine, or N-acetylgalactosamine.

In several embodiments of such compositions, m is 1 to 3, Y is of a linker moiety having a formula of: 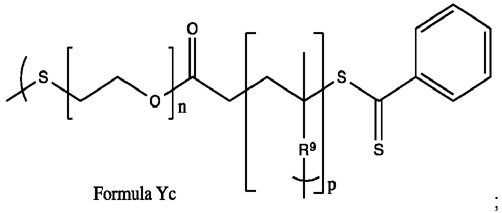 wherein CH₂-CH₂--NH-C(O)-; and Z comprises a liver-targeting moiety comprising one or more of galactose, galactosamine, or N-acetylgalactosamine. In several embodiments, Z is the β-anomer of the selected moiety.

As discussed above, in several embodiments, X is a self-antigen and the unwanted immune response is an autoimmune response.

A variety of self-antigens is disclosed herein, but in several particular embodiments, X is myelin oligodendrocyte glycoprotein or myelin proteolipid protein. In such embodiments, the unwanted immune response experienced by the subject is associated with multiple sclerosis. In additional embodiments, X is insulin, proinsulin, or preproinsulin and wherein the unwanted immune response is associated with diabetes mellitus. It shall be appreciated that being associated with multiple sclerosis, diabetes mellitus or other auto-immune disease need not necessarily require a formal diagnosis of such auto-immune condition, but rather can be associated with common symptoms or characteristics of a particular auto-immune disorder.

In additional embodiments, as discussed herein, an unwanted immune response can be raised against a therapeutic agent, such as a protein drug or drug derived from non-human and/or non-mammalian species. For example, in several embodiments, X is a therapeutic agent, such as Factor VIII, Factor IX, or other hemostasis-inducing agent. In such embodiments, the unwanted immune response is against the agent and the associated disease is hemophilia, which fails to improve (in the absence of the composition) because of the autoimmune response. However, upon administration of the composition, the hemophilia can improve because the composition aids in inducing tolerance to the agent, reducing the response to agent, and allowing reduced symptoms of hemophilia. In still additional embodiments, X is a therapeutic agent such as asparaginase and uricase. As discussed above, an unwanted immune response can result from administration of such agents, as they are derived from non-human sources. The ability of the compositions disclosed herein to induce tolerance to these agents allows these agents to continue to be used by a subject in need of therapy, while the side effects from an immune reaction are reduced, lessened, eliminated or otherwise ameliorated.

In several embodiments, X is a food antigen. Many food antigens are known to cause allergies upon ingestion, however, in several embodiments, X is selected from the group consisting of conarachin (Ara h 1), allergen II (Ara h 2), arachis agglutinin, conglutin (Ara h 6), a-lactalbumin (ALA), lactotransferrin, Pen a 1 allergen (Pen a 1), allergen Pen m 2 (Pen m 2), tropomyosin fast isoform, high molecular weight glutenin, low molecular weight glutenin, alpha- gliadin, gamma-gliadin, omega-gliadin, hordein, seclain, and avenin. In several embodiments, treatment with the compositions disclosed herein where X is a food antigen allows the subject to have a significantly reduced immune response to the antigen, e.g., many peanut allergies are so severe that exposure to peanut dust or oil can cause anaphylaxis. In some embodiments, treatment reduces and/or eliminates responses to such incidental exposure to the antigen. In additional embodiments, treatment allows the subject to ingest the food from which the antigen is derived with limited or no adverse immune response.

In several embodiments, administration of the composition to the subject results in a greater degree of proliferation of antigen-specific T cells as compared to proliferation of antigen-specific T cells resulting from administration of the antigen alone. In such embodiments, the proliferation of antigen-specific T cells indicates that delivery of the antigen (via the composition) to the molecular processing machinery that processes antigens as self/non-self is enhanced versus administration of the antigen alone. In other words, in such embodiments the targeted delivery is effective. In still additional embodiments, administration of the compositions disclosed herein results in a greater expression of exhaustion markers or markers of apoptosis on antigen-specific T cells as compared to expression of exhaustion markers or markers of apoptosis on antigen-specific T cells resulting from administration of the antigen alone. This result in indicative of specific reduction in activity of T cells directed against the antigen of interest and/or deletion of T cells directed against the antigen of interest. In several embodiments, these molecular hallmarks of induction of tolerance are the precursor of the reduction or amelioration of immune response symptoms that the subject would have previously experienced when exposed to the antigen.

In several embodiments, Z comprises a liver-targeting moiety that is a carbohydrate. In several embodiments, the carbohydrate is a short-chain carbohydrate. In several embodiments, Z is a sugar. In several embodiments, Z is galactose, galactosamine, N-acetylgalactosamine, glucose, glucosamine, or N-acetylglucosamine. In several embodiments, the induction of immune tolerance is greater when a glucose, glucosamine, or N-acetylglucosamine is used for Z. In still additional embodiments, enhancements in induction of immune tolerance can be achieved when the liver targeting moiety is a sugar and the sugar is in the β-anomer configuration. In several embodiments, Z is galactose, galactosamine, N-acetylgalactosamine, glucose, glucosamine, or N-acetylglucosamine and conjugated at its C1, C2 or C6 to Y.

Also provided herein are methods of inducing tolerance to antigens which, when administered alone (e.g., without the presently disclosed compositions) would result in an adverse immune response. Such methods, depending on the embodiments, involved the administration either before, or after, exposure to the antigen. In several embodiments, administration prior to exposure serves a prophylactic effect, which in several embodiments essentially avoids or significantly reduces in the immune response. Administration of the compositions can be via a variety of methods, including, but not limited to intravenous, intramuscular, oral, transdermal, or other infusion route. Administration can be daily, weekly, multiple times per day, or on an as needed basis (e.g., prior to an anticipated exposure).

Also provided for herein are uses of the compositions disclosed herein for the treatment of unwanted immune responses after exposure to an antigen. As discussed herein, such use can be for prophylactic effects and/or for reducing symptoms from prior exposure to antigens (or prior adverse immune effects, such as those in the auto-immune setting). For example, provided herein are uses of compositions according to Formula 1 for the treatment of unwanted side effects due to exposure to a therapeutic antigen, exposure to a food antigen, or an adverse effect from an immune response against a self-antigen. The compositions disclosed herein are suitable for administration to a subject in conjunction with such use, for example by oral, IV, IM, or other suitable route. Uses of the compositions disclosed herein, in several embodiments, unexpectedly result in the reduction, elimination or amelioration of adverse immune responses to antigens of interest.

Additional compositions and methods of using them are provided herein. For example, in several embodiments, there is provided a pharmaceutically acceptable composition for inducing tolerance to a therapeutic protein in a subject having an deficiency in production of a functional analogous native protein, comprising a compound of Formula 1 (X-⁅-Y-Z]ₘ), where m is an integer from about 1 to 10, X comprises an antigenic protein or protein fragment, Y is of a linker moiety having a formula selected from the group consisting of Formula Ya, Formula Yc, Formula Ym, Formula Yn, wherein, the left bracket "(" indicates a bond to X, the right or bottom bracket and ")" indicates the bond between Y and Z, n is an integer from about 1 to 100, where present p is an integer from about 2 to 150, where present q is an integer from about 1 to 44, where present R⁸ is -CH₂- or -CH₂-CH₂-C(CH₃)(CN)-, where present R⁹ is a direct bond or -CH₂-CH₂--NH-C(O)-, and Z comprises galactose, galactosamine, or N-acetylgalactosamine.

In several embodiments of the composition, m is 1 to 3, Y is of a linker moiety having a formula of: 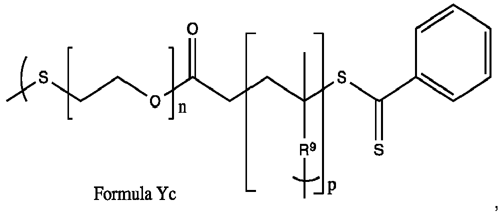 wherein CH₂-CH₂--NH-C(O)-, and Z comprises a liver-targeting moiety comprising one or more of glucose, glucosamine, N-acetylglucosamine, galactose, galactosamine, or N-acetylgalactosamine. In several embodiments, the galactose, galactosamine, or N-acetylgalactosamine are the β-anomers. In several embodiments, combinations of galactose, galactosamine, N-acetylgalactosamine, glucose, glucosamine, or N-acetylglucosamine are used.

Also provided for herein is a pharmaceutically acceptable composition for inducing tolerance to a therapeutic protein in a subject having an deficiency in production of a functional analogous native protein, comprising a compound of Formula 1 (X-⁅-Y-Z]ₘ), where m is an integer from about 1 to 10, X comprises a antigenic protein or protein fragment, Y is of a linker moiety having a formula selected from the group consisting of Formula Ya, Formula Yc, Formula Ym, or Formula Ym, wherein the left bracket "(" indicates a bond to X, where present the right ")" indicates a bond to Z, where present the bottom ")" indicates a bond to Z, where present n is an integer from about 1 to about 80, where present q is an integer from about 1 to about 4, where present p is an integer from about 1 to about 90, where present R⁸ is - CH₂- or -CH₂-CH₂-C(CH₃)(CN)-, and where present W represents a polymer of the Formula W¹ or W² group or W is a copolymer of Formula W¹ or W² where: 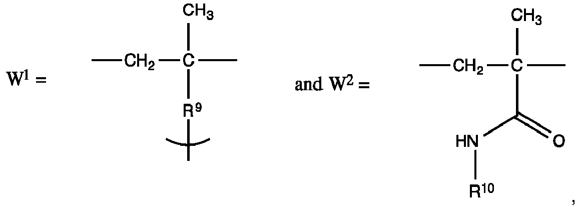 where R⁹ is a direct bond, -CH₂-CH₂--NH-C(O)- or -CH₂-CH₂-(O-CH₂-CH₂)ₜ-NH-C(O)- , t is an integer from 1 to 5, R¹⁰ is an aliphatic group, an alcohol or an aliphatic alcohol; and Z comprises glucose, glucosamine, N-acetylglucosamine, galactose, galactosamine, or N-acetylgalactosamine. In several embodiments, the galactose, galactosamine, or N-acetylgalactosamine are the β-anomers. In several embodiments, combinations of galactose, galactosamine, N-acetylgalactosamine, glucose, glucosamine, or N-acetylglucosamine are used. In several embodiments of the composition, m is 1 to 3, Y is represented by Formula Ym, wherein R⁸ is -CH₂-CH₂-C(CH₃)(CN)-, and W is represented by a block copolymer of W¹ and W² where R⁹ is -CH₂-CH₂-(O-CH₂-CH₂)ₜ-NH-C(O)-, t is 1, and R¹⁰ is 2-hydroxypropyl; and Z comprises a liver-targeting moiety comprising one or more of glucose, glucosamine, N-acetylglucosamine, galactose, galactosamine, or N-acetylgalactosamine. In several embodiments, the galactose, galactosamine, or N-acetylgalactosamine are the β-anomers. In several embodiments, combinations of galactose, galactosamine, N-acetylgalactosamine, glucose, glucosamine, or N-acetylglucosamine are used.

In several embodiments, X comprises an antigenic region of myelin basic protein, myelin oligodendrocyte glycoprotein, or myelin proteolipid protein. In additional embodiments, X comprises an antigenic region of Factor VIII, Factor IX, insulin, uricase, PAL, or asparaginase. In additional embodiments, X comprises a foreign antigen such as conarachin (Ara h 1), allergen II (Ara h 2), arachis agglutinin, conglutin (Ara h 6), a-lactalbumin (ALA), lactotransferrin, Pen a 1 allergen (Pen a 1), allergen Pen m 2 (Pen m 2), tropomyosin fast isoform, high molecular weight glutenin, low molecular weight glutenin, alpha- gliadin, gamma-gliadin, omega-gliadin, hordein, seclain, and avenin.

Additionally provided for herein are compositions comprising a compound of Formula 1 (X-[-Y---Z]m), where m is an integer from about 1 to 100, X comprises an antigen against which a patient develops an unwanted immune response, or a tolerogenic portion thereof, or X comprises an antibody, antibody fragment or ligand that specifically binds a circulating protein or peptide or antibody, which circulating protein or peptide or antibody is causatively involved in transplant rejection, immune response against a therapeutic agent, autoimmune disease, hypersensitivity and/or allergy, Y comprises a linker moiety, and Z comprises a liver-targeting moiety.

In several embodiments, Z galactose, galactosamine, N-acetylgalactosamine, glucose, glucosamine or N-acetylglucosamine. Combinations of galactose, galactosamine, N-acetylgalactosamine, glucose, glucosamine or N-acetylglucosamine may also be used, in several embodiments. Further, in several embodiments, the galactose, galactosamine, N-acetylgalactosamine, glucose, glucosamine or N-acetylglucosamine are optionally the β anomer. In several embodiments, Z is conjugated at its C1, C2 or C6 to Y.

In several embodiments, Y is selected from N-hydroxysuccinamidyl linkers, malaemide linkers, vinylsulfone linkers, pyridyl di-thiol-poly(ethylene glycol) linkers, pyridyl di-thiol linkers, n-nitrophenyl carbonate linkers, NHS-ester linkers, and nitrophenoxy polyethylene glycol)ester linkers. In several embodiments, Y comprises an antibody, antibody fragment, peptide or other ligand that specifically binds X, a disulfanyl ethyl ester, a structure represented by one of Formulae Ya to Yp, or Y has a portion represented by Formula Y'-CMP: 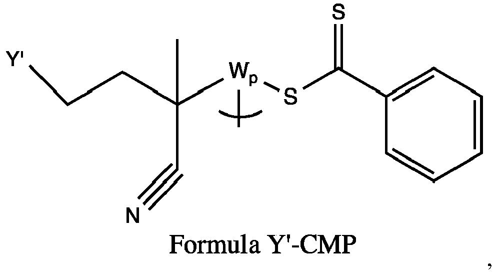 where the left bracket "(" indicates the bond between X and Y, the right or bottom bracket and ")" indicates the bond between Y and Z, n is an integer from about 1 to 100, q is an integer from about 1 to 44, R⁸ is -CH₂- or -CH₂-CH₂-C(CH₃)(CN)-, Y' represents the remaining portion of Y, and W represents a polymer of the same W¹ group, or W is a copolymer or a random copolymer of the same or different W¹ and W² groups, where: 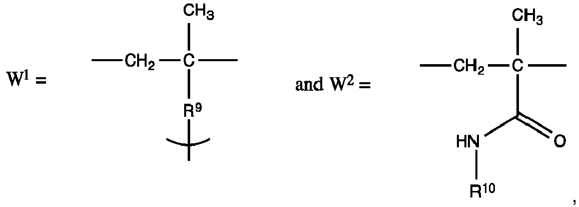 where p is an integer from 2 to about 150, R⁹ is a direct bond, -CH₂-CH₂--NH-C(O)- or -CH₂-CH₂-(O-CH₂-CH₂)ₜ-NH-C(O)-, t is an integer from 1 to 5; and R¹⁰ is an aliphatic group, an alcohol or an aliphatic alcohol.

In some such embodiments, n is about 40 to 80, p is about 10 to 100, q is about 3 to 20, R⁸ is -CH₂-CH₂-C(CH₃)(CN)-, when R9 is -CH2-CH2--NH-C(O)-, Z is glucose, galactose, N-acetylgalactosamine or N-acetylglucosamine conjugated at its C1, and when W is a copolymer, R10 is 2-hydroxypropyl. In some embodiments, Y comprises Formula Ya, Formula Yb, Formula Yc, Formula Yf, Formula Yg, Formula Yh, Formula Yi, Formula Yk, Formula Ym or Formula Yn. In some embodiments, Y comprises Formula Ya, Formula Yb, Formula Yc, Formula Ym or Formula Yn. In still additional embodiments, Y comprises Formula Ya, Formula Yb, Formula Yc, Formula Ym or Formula Yn.

In several embodiments, X comprises a foreign transplant antigen against which transplant recipients develop an unwanted immune response, a foreign food, animal, plant or environmental antigen against which patients develop an unwanted immune response, a foreign therapeutic agent against which patients develop an unwanted immune response, or a synthetic self-antigen against the endogenous version of which patients develop an unwanted immune response, or a tolerogenic portion thereof. Specific examples of various antigens are disclosed herein.

Also provided for herein is are methods of treatment for an unwanted immune response against an antigen by administering to a mammal in need of such treatment an effective amount of a composition comprising a compound of Formula 1 (X-[- Y---Z]m), where m is an integer from about 1 to 100, X comprises an antigen against which a patient develops an unwanted immune response, or a tolerogenic portion thereof or X comprises an antibody, antibody fragment or ligand that specifically binds a circulating protein or peptide or antibody, which circulating protein or peptide or antibody is causatively involved in transplant rejection, immune response against a therapeutic agent, autoimmune disease, hypersensitivity and/or allergy, Y comprises a linker moiety, and Z comprises a glucosylated liver-targeting moiety.

In several such embodiments, X comprises an antigen against which a patient develops an unwanted immune response, or a tolerogenic portion thereof, and Y comprises, an antibody, antibody fragment, peptide or other ligand that specifically binds X, a disulfanyl ethyl ester, a structure represented by one of Formulae Ya to Yp or Y has a portion represented by Formula Y'-CMP where, the left bracket "(" indicates the bond between X and Y, the right or bottom bracket and ")" indicates the bond between Y and Z, n is an integer from about 1 to 100, q is an integer from about 1 to 44, R⁸ is -CH₂- or -CH₂-CH₂-C(CH₃)(CN)-, Y' represents the remaining portion of Y, and W represents a polymer of the same W¹ group, or W is a copolymer or a random copolymer of the same or different W¹ and W² groups, where: 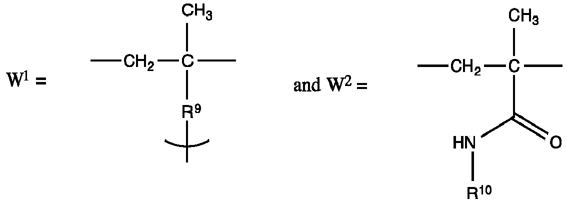 where p is an integer from 2 to about 150, R⁹ is a direct bond, -CH₂-CH₂--NH-C(O)- or -CH₂-CH₂-(O-CH₂-CH₂)ₜ-NH-C(O)-, t is an integer from 1 to 5, and R¹⁰ is an aliphatic group, an alcohol or an aliphatic alcohol. In several such treatment method embodiments, X comprises the antibody, antibody fragment or ligand, and the composition is administered for clearance of a circulating protein or peptide or antibody that specifically binds to X, which circulating protein or peptide or antibody is causatively involved in transplant rejection, immune response against a therapeutic agent, autoimmune disease, hypersensitivity and/or allergy.

In still additional embodiments, X comprises the antibody, antibody fragment or ligand, and the composition is administered in an amount effective to reduce a concentration of the antibodies that are causatively involved in transplant rejection, immune response against a therapeutic agent, autoimmune disease, hypersensitivity and/or allergy in blood of the patient by at least 50% w/w, as measured at a time between about 12 to about 48 hours after the administration.

In several such treatment embodiments, compositions are administered for tolerization of the patient with respect to antigen moiety X.

In several embodiments X comprises a foreign transplant antigen against which transplant recipients develop an unwanted immune response, a foreign food, animal, plant or environmental antigen against which patients develop an unwanted immune response, a foreign therapeutic agent against which patients develop an unwanted immune response, or a synthetic self-antigen against the endogenous version of which patients develop an unwanted immune response, or a tolerogenic portion thereof.

Several embodiments disclosed herein provide a composition comprising a compound of Formula 1: 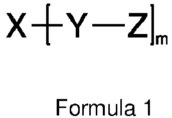 where:
m is an integer from about 1 to 100;
X comprises an antigen against which a patient develops an unwanted immune response, or a tolerogenic portion thereof; or
X comprises an antibody, antibody fragment or ligand that specifically binds a circulating protein or peptide or antibody, which circulating protein or peptide or antibody is causatively involved in transplant rejection, immune response against a therapeutic agent, autoimmune disease, hypersensitivity and/or allergy;
Y comprises a linker moiety; and
Z comprises a liver-targeting moiety.

Z can also comprise galactose, galactosamine, N-acetylgalactosamine, glucose, glucosamine or N-acetylglucosamine, for example, conjugated at its C1, C2 or C6 to Y. N-acetylglucosamine and glucose bind to different lectin receptors as do N-acetylgalactosamine and galactose. In the examples below the experimental data (and the full disclosure of this application) indicate that the selection of Z as N-acetylglucosamine leads to elevated levels of regulatory T cell responses compared to those achieved with N-acetylgalactosamine. In several embodiments, this results in unexpectedly enhanced induction of immune tolerance and/or clearance of antigens from the blood of a subject.

Y can be selected from N-hydroxysuccinamidyl linkers, malaemide linkers, vinylsulfone linkers, pyridyl di-thiol-poly(ethylene glycol) linkers, pyridyl di-thiol linkers, n-nitrophenyl carbonate linkers, NHS-ester linkers, and nitrophenoxy polyethylene glycol)ester linkers.

Y can also comprise: an antibody, antibody fragment, peptide or other ligand that specifically binds X; a disulfanyl ethyl ester; a structure represented by one of Formulae Ya to Yp: 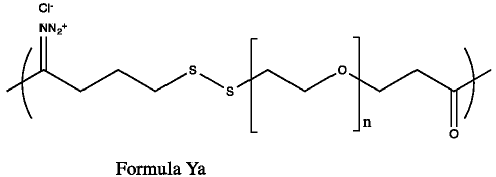 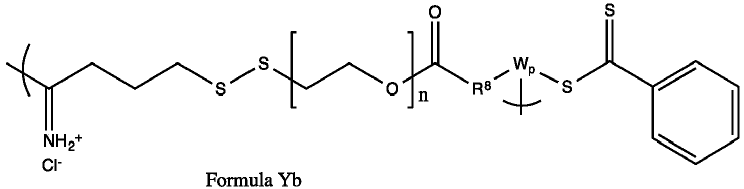 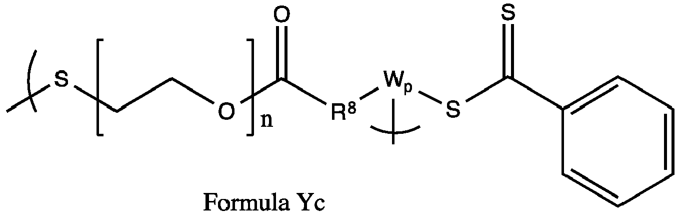 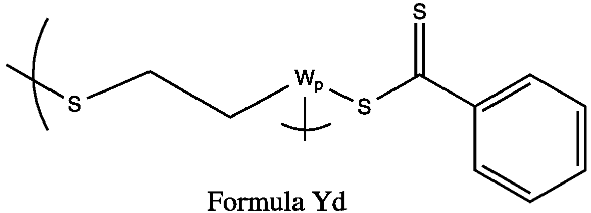 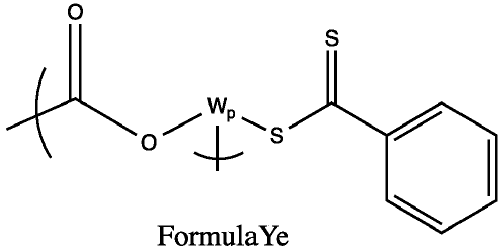 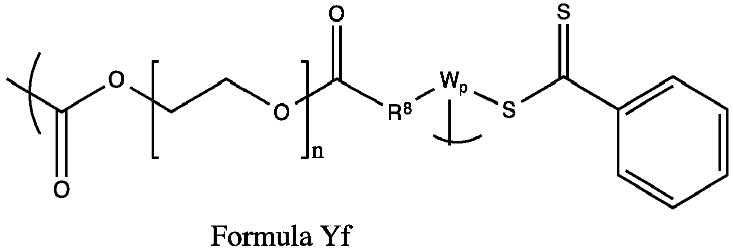 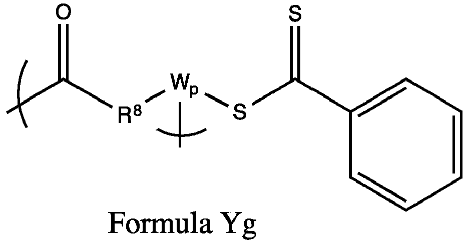 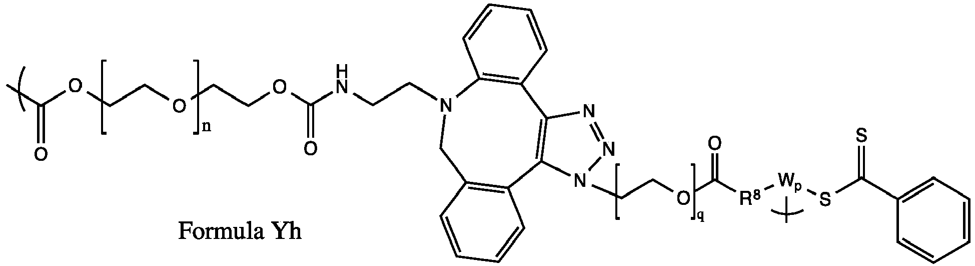 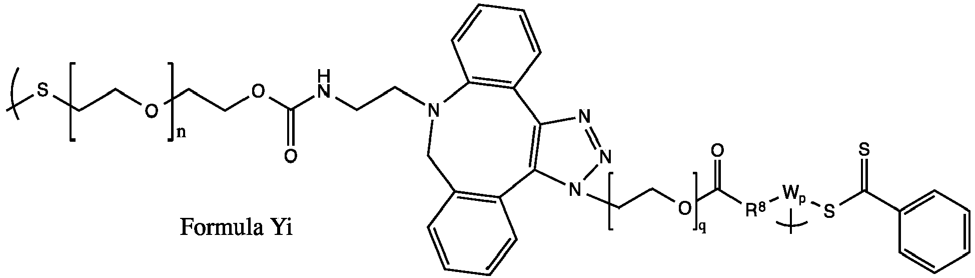 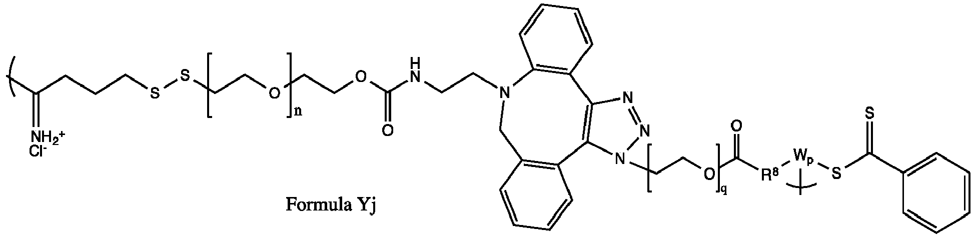 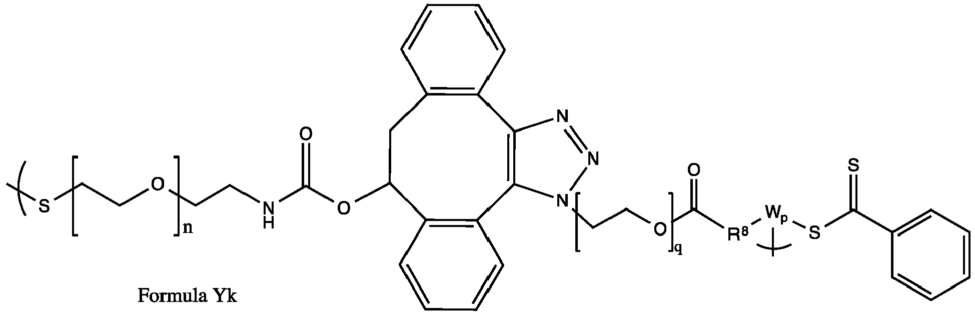 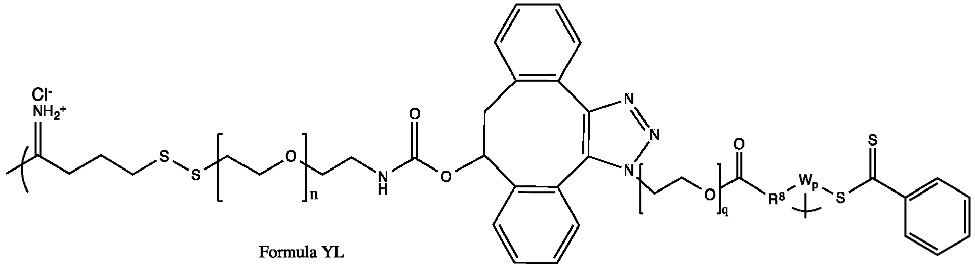 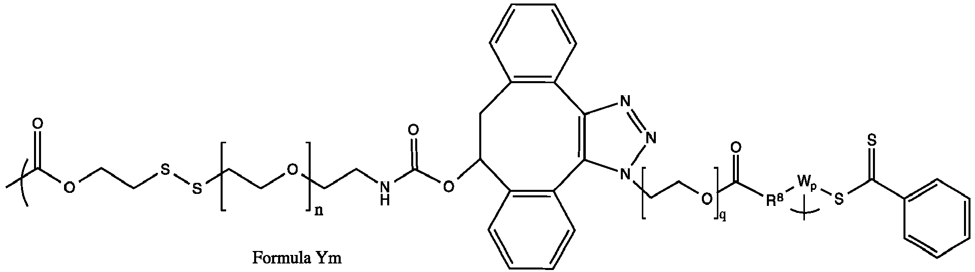 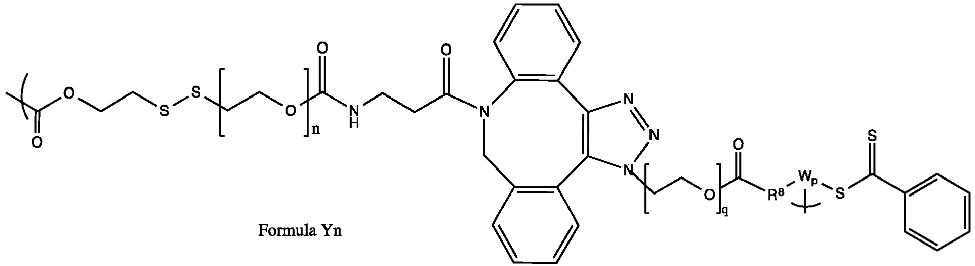 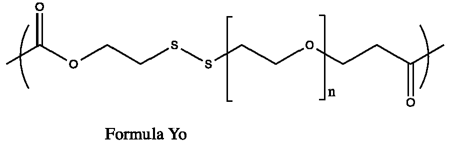 and 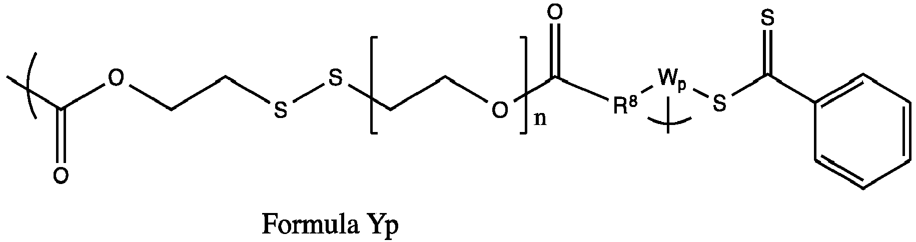 or Y has a portion represented by Formula Y'-CMP: 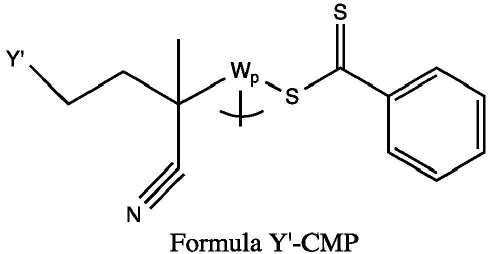 where:
the left bracket "(" indicates the bond between X and Y;
the right or bottom bracket and ")" indicates the bond between Y and Z;
n is an integer from about 1 to 100;
q is an integer from about 1 to 44;
R⁸ is -CH₂- or -CH₂-CH₂-C(CH₃)(CN)-;
Y' represents the remaining portion of Y (e.g., HS-PEG); and
W represents a polymer of the same W¹ group, or W is a copolymer (preferably a random copolymer) of the same or different W¹ and W² groups, where: 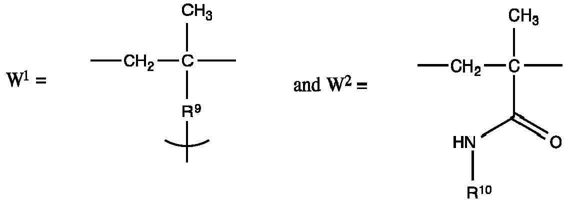 where:
   p is an integer from 2 to about 150;
   R⁹ is a direct bond, -CH₂-CH₂--NH-C(O)- (i.e., an ethylacetamido group or "EtAcN") or -CH₂-CH₂-(O-CH₂-CH₂)ₜ-NH-C(O)- (i.e., a pegylated ethylacetamido group or "Et-PEGₜ-AcN")
   t is an integer from 1 to 5, (particularly 1 to 3, and more particularly 1 or 2); and
   R¹⁰ is an aliphatic group, an alcohol or an aliphatic alcohol. In some embodiments, R¹⁰ is a Cfalkyl or C_{f}alkylOH_{g} where f is independently an integer between 0 and 10 and g is independently an integer between 0 and 10. In some embodiments, R¹⁰ is 2-hydroxypropyl.

In several embodiments, particular linkers are preferred. For example, in several embodiments, linkers according to Ym yield unexpectedly effective tolerance endpoints. In additional embodiments, linkers according to formula Yn yield unexpectedly effective tolerance endpoints. In still additional embodiments, formulations of F1m'-OVA-m₁₋₃-n₇₉-p₉₀-q₄-CMP-poly-(EtPEG₁AcN-1NAcGLU₃₀-ran-HPMA₆₀ achieve particularly effective tolerance-associated endpoints. In several embodiments, combinations of these linkers lead to synergistic results and still further unexpected increases in immune tolerance induction.

In another aspect of the above, n is about 40 to 80, p is about 10 to 100, q is about 3 to 20, R⁸ is -CH₂-CH₂-C(CH₃)(CN)-; and when R⁹ is -CH₂-CH₂--NH-C(O)-, Z is galactose or N-acetylgalactosamine conjugated at its C1.

In still another aspect of the above, Y comprises Formula Ya, Formula Yb, Formula Yh, Formula Yi, Formula Yk, Formula Ym or Formula Yn, particularly Formula Ya, Formula Yb, Formula Ym or Formula Yn.

X can further comprise: a foreign transplant antigen against which transplant recipients develop an unwanted immune response; a foreign food, animal, plant or environmental antigen against which patients develop an unwanted immune response; a foreign therapeutic agent against which patients develop an unwanted immune response; or a synthetic self-antigen against the endogenous version of which patients develop an unwanted immune response, or a tolerogenic portion thereof.

The disclosure also pertains to a method of treatment for an unwanted immune response against an antigen by administering to a mammal in need of such treatment an effective amount of a composition comprising a compound of Formula 1 as disclosed herein. In some such methods the composition can be administered for clearance of a circulating protein or peptide or antibody that specifically binds to antigen moiety X, which circulating protein or peptide or antibody is causatively involved in transplant rejection, immune response against a therapeutic agent, autoimmune disease, hypersensitivity and/or allergy. The composition can be administered in an amount effective to reduce a concentration of the antibodies that are causatively involved in transplant rejection, immune response against a therapeutic agent, autoimmune disease, hypersensitivity and/or allergy in blood of the patient by at least 50% w/w, as measured at a time between about 12 to about 48 hours after the administration. The composition can administered for tolerization of a patient with respect to antigen moiety X.

### BRIEF DESCRIPTION OF THE DRAWINGS

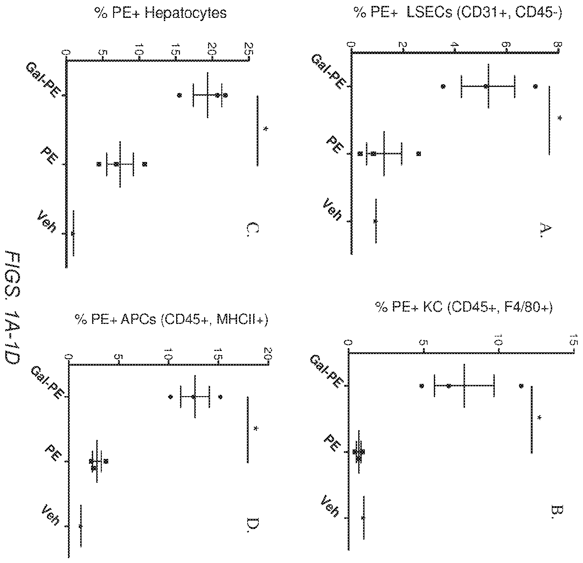

Figs. 1A-1D are a series of graphs showing differential cellular uptake of galactose conjugates. Figure 1A depicts that F1aA-PE-m₄-n₈₀ (Gal-PE) preferentially targets PE to sinusoidal endothelial cells (LSECs) of the liver. Figure 1B depicts that F1aA-PE-m₄-n₈₀ (Gal-PE) preferentially targets PE to Kupffer cells (KC) of the liver. Figure 1C depicts that F1 aA-PE-m₄-n₈₀ (Gal-PE) preferentially targets PE to hepatocytes. Figure 1D depicts that F1aA-PE-m₄-n₈₀ (Gal-PE) preferentially targets PE to other antigen presenting cells (APCs) of the liver. * = P < 0.05.

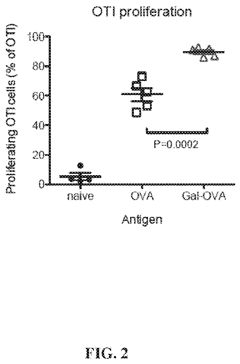

Fig. 2 is a graph showing proliferation of OT-I CD8+ T cells in mice treated with F1aA-OVA-m₄-n₈₀ (Gal-OVA), OVA or saline (i.e. naïve), with greatest proliferation seen in the Gal-OVA treated group.

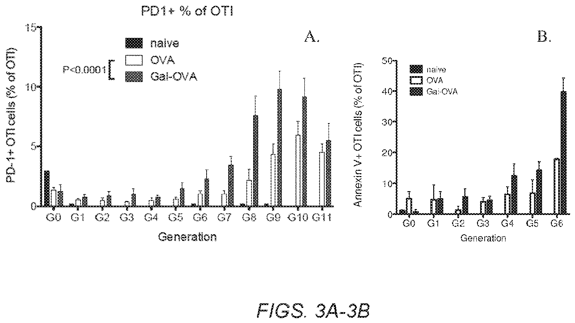

Figs. 3A-3B are a series of graphs depicting data related to marker expression on T cells. Figure 3A shows the percentage of OT-I CD8+ T cells expressing PD-1 ("PD1+") in generations of proliferating T cells treated with saline, OVA or F1aA-OVA-m₄-n₈₀ (GAL-OVA), with greatest level of PD-1 in the gal-OVA-treated group. Figure 3B shows the percentage of OT-I CD8+ T cells expressing phosphatidylserine (stained as "Annexin V+") in generations of proliferating T cells treated with saline, OVA or F1aA-OVA-m₄-n₈₀ (GAL-OVA), with greatest level of Annexin-V+ cells in the gal-OVA-treated group.

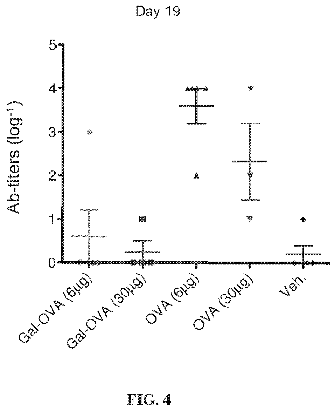

Fig. 4 is a graph showing that galactose conjugation [F1aA-OVA-m₄-n₈₀ (Gal-OVA)] decreases the immunogenicity of OVA as determined by OVA-specific antibody titers (shown in Ab titers log⁻¹).

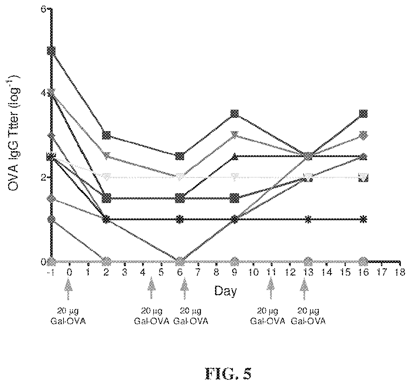

Fig. 5 shows that administration of F1aA-OVA-m₄-n₈₀ (Gal-OVA) in repeated doses over time is able to deplete OVA-specific antibodies from the serum of mice.

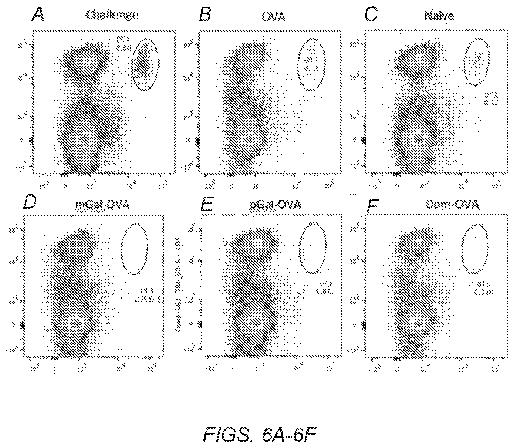

Figs. 6A-6F depict data related to the mitigation of the OVA-specific immune response. Figure 6A shows the immune response in mice challenged with OVA and LPS. Figure 6B shows the immune response in mice treated with OVA, while Figure 6C shows the immune response in naïve mice. Figures 6D and 6E (respectively) show that F1aA-OVA-m₄-n₈₀ (mGal-OVA; 6D) and F1b-OVA-m₁-n₄₄-p₃₄ (pGal-OVA; 6E) are able to mitigate the OVA-specific immune response in draining lymph nodes after intradermal challenge with OVA and the adjuvant LPS. Fig 6F is from a parent application and does not form a part of the present disclosure.

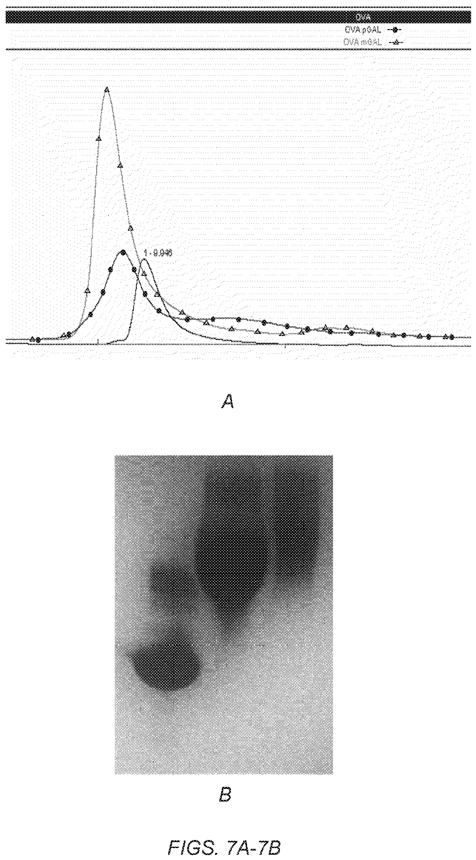

Figs. 7A-7B shows the characterization of F1aA-OVA-m₄-n₈₀ and F1b-OVA-m₁-n₄₄-p₃₄. Fig. 7A shows size-exclusion HPLC traces of F1aA-OVA-m₄-n₈₀ (open triangles), F1b-OVA-m₁-n₄₄-p₃₄ (filled circles) and unconjugated OVA (solid line). Shift to the left represents an increase in molecular weight. Fig. 7B shows polyacrylamide gel demonstrating increased molecular weight after OVA conjugation: (1.) Unconjugated OVA, (2.) F1aA-OVA-m₄-n₈₀ and (3.) F1b-OVA-m₁-n₄₄-p₃₄.

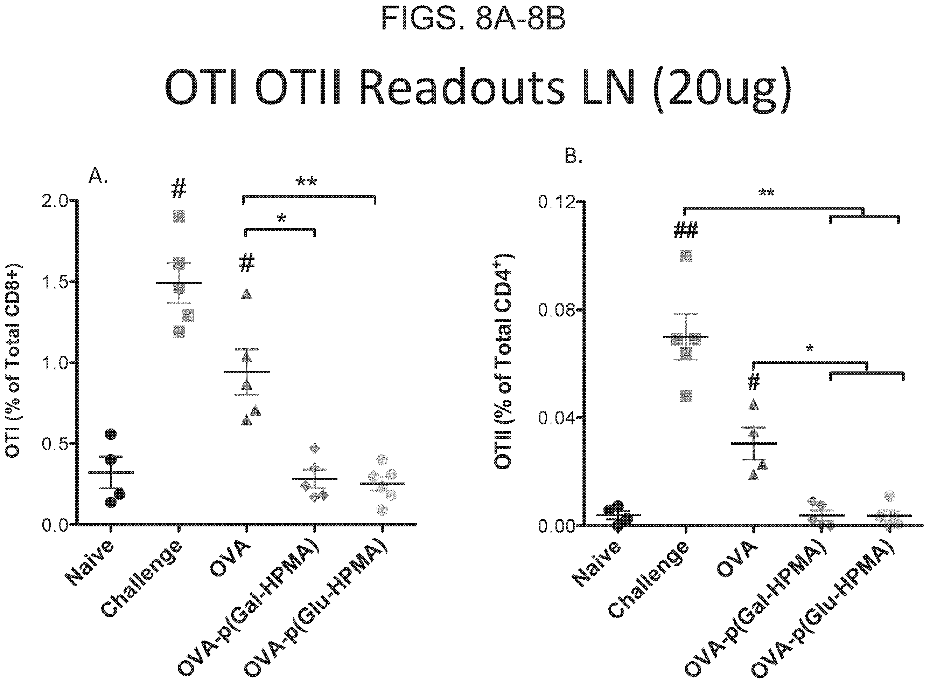

Figs. 8A-8B depict data related to the reduction in antigen-specific immune response after administration of F1m'-OVA-m₁₋₃-n₇₉-p₉₀-q₄-CMP-poly-(EtPEG₁AcN-1NAcGLU₃₀-ran-HPMA₆₀ [labeled OVA-p(Glu-HPMA) and shown as filled circles] or F1m'-OVA-m₁₋₃-n₇₉-p₉₀-q₄-CMP-poly-(EtPEG₁AcN-1NAcGAL₃₀-ran-HPMA₆₀ [labeled OVA-p(Gal-HPMA) and shown as filled diamonds]. Fig. 8A depicts flow cytometric detection of OTI CD8+ T-cell populations (CD3e⁺/CD8a⁺/CD45.2⁺) quantified from the draining lymph nodes (inguinal and popliteal) 4 days following antigen challenge in CD45.1⁺ mice. Significant reductions in OT-I CD8+ T-cells were detected following administration of OVA-p(Gal-HPMA) and OVA-p(Glu-HPMA). Fig. 8B depicts flow cytometric detection of OTII CD4+ T-cell populations (CD3e⁺/CD4⁺/CD45.2⁺) quantified from the draining lymph nodes (inguinal and popliteal) 4 d following antigen challenge in CD45.1⁺ mice. Significant reductions in OT-II CD4+ T-cells were detected following administration of OVA-p(Gal-HPMA) and OVA-p(Glu-HPMA) * = P < 0.05, ** = P < 0.01; # = P < 0.05, ## = P < 0.01 (#'s represent significance as compared to naïve animals).

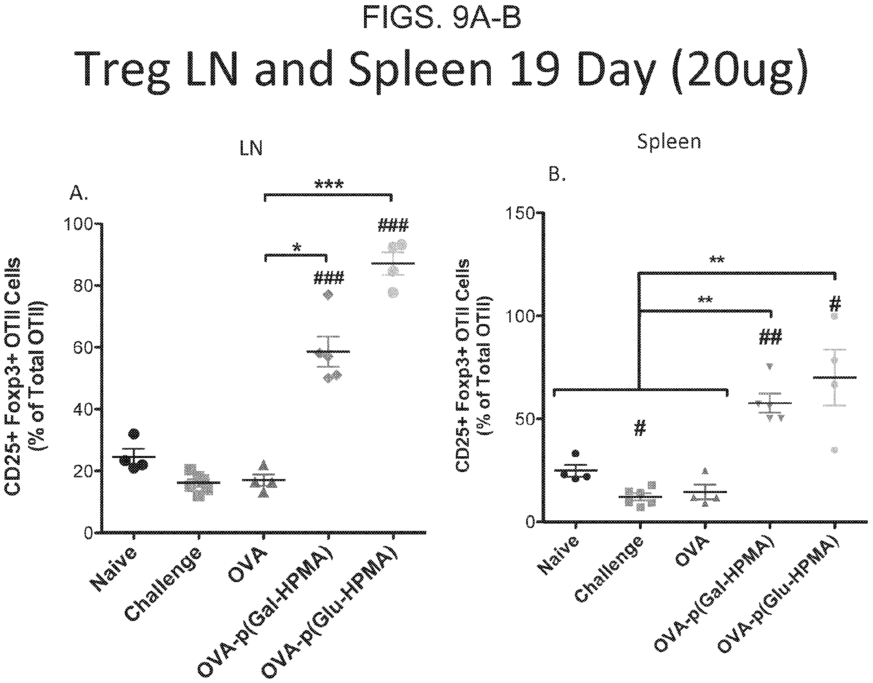

Figs. 9A-9B depict data related to the increase in antigen-specific regulatory T-cells in the lymph nodes and spleen of mice after antigen challenge. Fig. 9A depicts flow cytometric detection of an F1m'-OVA-m₁₋₃-n₇₉-p₉₀-q₄-CMP-poly-(EtPEG₁AcN-1NAcGLU₃₀-ran-HPMA₆₀ [labeled OVA-p(Glu-HPMA) and shown as filled circles] and F1m'-OVA-m₁₋₃-n₇₉-p₉₀-q₄-CMP-poly-(EtPEG₁AcN-1NAcGAL₃₀-ran-HPMA₆₀ [labeled OVA-p(Gal-HPMA) and shown as filled diamonds]-induced increase in OTII T-regulator cells (CD3e + CD4+ CD45.2+ CD25+ FoxP3+) collected from the lymph nodes 4 d following antigen challenge in CD45.1⁺ mice. Fig. 9B shows the corresponding analysis from the spleen of mice treated with OVA-p(Glu-HPMA) or OVA-p(Gal-HPMA) as compared to animals treated with OVA or saline (i.e. Challenge) * = P < 0.05, ** = P < 0.01; *** = P < 0.001; # = P < 0.01, ## = P < 0.01; ### = P < 0.001 (#'s represent significance as compared to naïve animals).

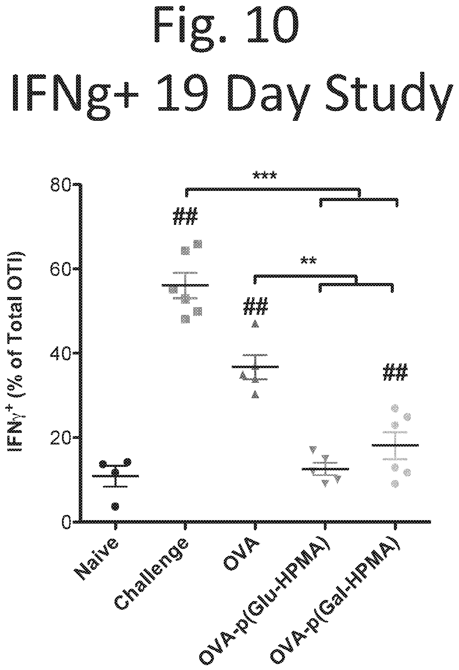

Fig. 10 depicts flow cytometry data related to a decrease in the percentage of antigen-specific effector cells (IFNγ+ OTI CD8+ T-cells (CD3e + CD8α+ CD45.2+ IFNγ+) 4 d following antigen challenge in CD45.1⁺ mice. Mice treated with F1m'-OVA-m₁₋₃-n₇₉-p₉₀-q₄-CMP-poly-(EtPEG₁AcN-1NAcGLU₃₀-ran-HPMA₆₀ [labeled OVA-p(Glu-HPMA) and shown as filled circles] or F1m'-OVA-m₁₋₃-n₇₉-p₉₀-q₄-CMP-poly-(EtPEG₁AcN-1NAcGAL₃₀-ran-HPMA₆₀ [labeled OVA-p(Gal-HPMA) and shown as filled diamonds] conjugates generated significantly fewer IFNγ+ OTI CD8+ T-cells after antigen challenge as compared to mice treated with OVA or saline (i.e. Challenge) * = P < 0.01, ** = P < 0.01; ## = P < 0.01 (#'s represent significance as compared to naïve animals).

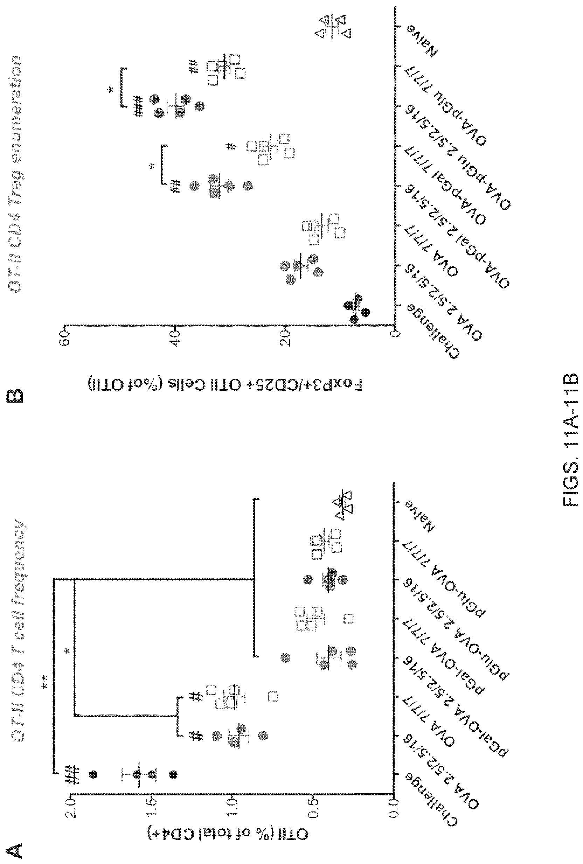

Figs. 11A-11B depict data related to T cell deletion and regulation in an OTII adoptive transfer model, in which OTII cells (CD4⁺ T cells from a CD45.2+ mouse) are adoptively transferred into a CD45.1⁺ recipient, which is treated with F1m'-OVA-m₁₋₃-n₇₉-p₉₀-q₄-CMP-poly-(EtPEG₁AcN-1NAcGAL₃₀-ran-HPMA₆₀ ["OVA-p(Gal-HPMA)"] or F1n'-OVA-m₁₋₃-n₇₉-p₉₀-q₄-CMP-poly-(EtPEG₁AcN-1NAcGLU₃₀-ran-HPMA₆₀ ["OVA-p(Glu-HPMA)"], or OVA not linked to a polymer ["OVA"] to induce T regulatory responses and prevent subsequent responses to vaccine-mediated antigen challenge. Both 3 × 10⁵ CFSE-labeled OTI and 3 × 10⁵ CFSE-labeled OTII cells were adoptively transferred to CD45.1⁺ mice (n = 8 mice per group) on day 0. On days 1, 4 and 7, tolerogenic doses or control doses were administered. In one regimen, OVA was provided at a dose of 2.5 µg at day 1, 2.5 µg at day 4, and 16 µg at day 7. In another, OVA was provided at a dose of 7 µg at day 1, 7 µg at day 4, and 7 µg at day 7, for the same total dose. Likewise, pGal-OVA and pGlu-OVA were each administered in other groups at the same dosings of 2.5 µg at day 1, 2.5 µg at day 4, and 16 µg at day 7 or 7 µg at day 1, 7 µg at day 4, and 7 µg at day 7, all doses being on an OVA equivalent dose basis. In a final group, saline was administered on the same days. On day 14, the recipient mice were then challenged with OVA (10 µg) adjuvanted with lipopolysaccharide (50 ng) by intradermal injection. Characterization of the draining lymph nodes was done on day 19, to allow determination as to whether or not deletion actually took place and whether regulatory T cells were induced from the adoptively transferred cells. Fig. 11A shows the number of OTII cells present after challenge, and Fig. 11B shows the frequency of FoxP3⁺CD25⁺ cells (markers of T regulatory cells). * and # indicate p<0.05, ** and ## indicate p<0.01, and ### indicates P < 0.001.

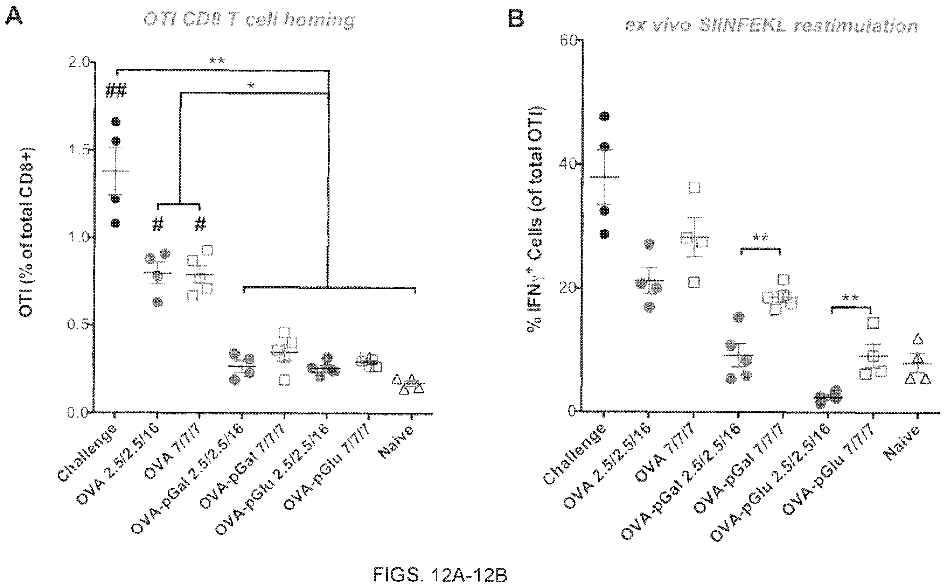

Figs. 12A-12B depicts data related to T cell deletion and regulation in an OTI adoptive transfer model, in which OTI cells (CD8⁺ T cells from a CD45.2+ mouse) are adoptively transferred into a CD45.1⁺ recipient, which is treated with F1m'-OVA-m₁₋₃-n₇₉-p₉₀-q₄-CMP-poly-(EtPEG₁AcN-1NAcGAL₃₀-ran-HPMA₆₀ ["OVA-p(Gal-HPMA)"] or F1m'-OVA-m₁₋₃-n₇₉-p₉₀-q₄-CMP-poly-(EtPEG₁AcN-1NAcGLU₃₀-ran-HPMA₆₀ ["OVA-p(Glu-HPMA)"], or OVA not linked to a polymer ["OVA"] to induce T regulatory responses and prevent subsequent responses to vaccine-mediated antigen challenge. Both 3 x 10⁵ CFSE-labeled OTI and 3 x 10⁵ CFSE-labeled OTII cells were adoptively transferred to CD45.1⁺ mice (n = 8 mice per group) on day 0. On days 1, 4 and 7, tolerogenic doses or control doses were administered. In one regimen, OVA was provided at a dose of 2.5 µg at day 1, 2.5 µg at day 4, and 16 µg at day 7. In another, OVA was provided at a dose of 7 µg at day 1, 7 µg at day 4, and 7 µg at day 7, for the same total dose. Likewise, pGal-OVA and pGlu-OVA were each administered in other groups at the same dosings of 2.5 µg at day 1, 2.5 µg at day 4, and 16 µg at day 7 or 7 µg at day 1, 7 µg at day 4, and 7 µg at day 7, all doses being on an OVA equivalent dose basis. In a final group, saline was administered on the same days. On day 14, the recipient mice were then challenged with OVA (10 µg) adjuvanted with lipopolysaccharide (50 ng) by intradermal injection. Characterization of the draining lymph nodes was done on day 19, to allow determination as to whether or not deletion actually took place and whether T cells were responsive to antigen re-exposure though their cytokine expression. Fig. 12A shows the number of OTI cells present after challenge, and Fig. 12B shows the frequency of IFNy-expressing cells (lack thereof indicating anergy). * and # indicate p<0.05, ** and ## indicate p<0.01).

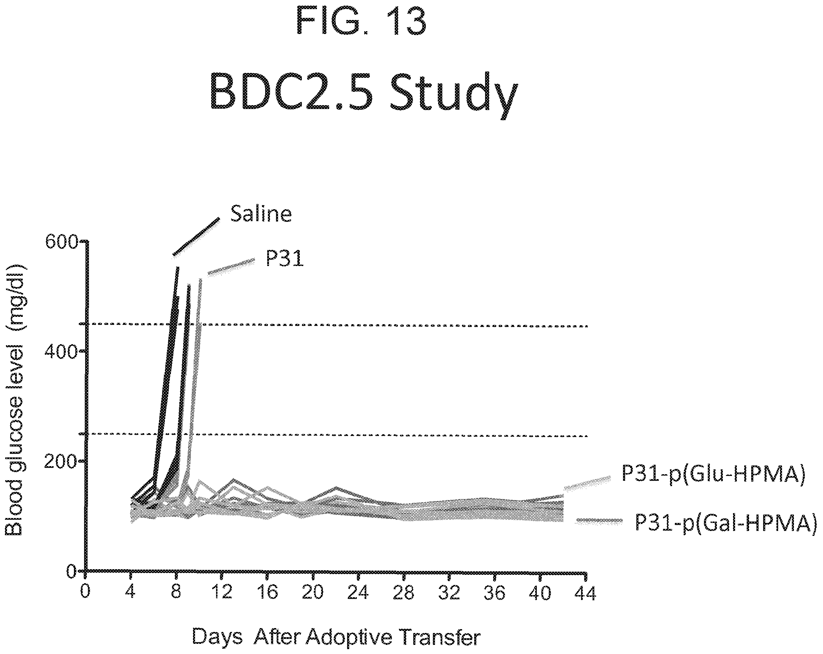

Fig. 13 depicts data related to blood glucose levels. Mice were treated with F1m'-P31-m₁₋₃-n₇₉-p₉₀-q₄-CMP-poly-(EtPEG₁AcN-1NAcGLU₃₀-ran-HPMA₆₀ [labeled P31-p(Glu-HPMA)], F1m'-P31-m₁₋₃-n₇₉-p₉₀-q₄-CMP-poly-(EtPEG₁AcN-1NAcGAL₃₀-ran-HPMA₆₀ [labeled P31-p(Gal-HPMA) conjugates (or saline). Animals receiving P31-p(Glu-HPMA) or P31-p(Gal-HPMA) maintained normal blood glucose levels for 42 days, whereas animals treated with P31 or Saline developed rapid hyperglycemia within 5-10 days, demonstrating that conjugates disclosed herein protect mice from T-cell induced autoimmune diabetes.

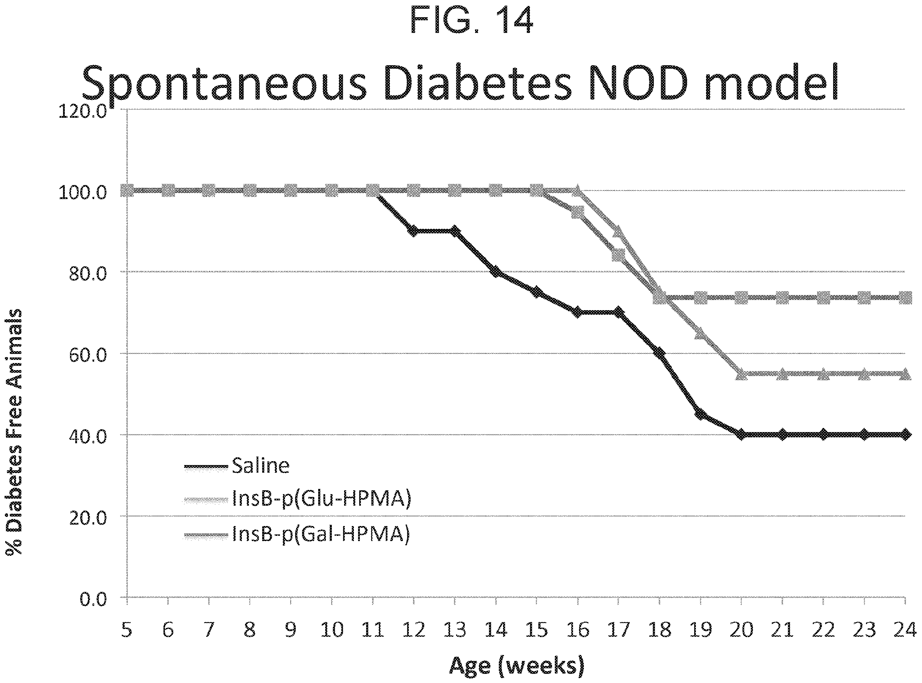

Fig. 14 depicts data related to the generation of spontaneous diabetes in non-obese diabetic (NOD) mice. Mice treated with F1c'-Insulin-B-m₁-n₄-p₉₀-CMP-poly-(EtPEG₁AcN-1NAcGLU₃₀-ran-HPMA₆₀ are shown as filled squares. Mice treated with F1c'-Insulin-B-m₁-n₄-p₉₀-CMP-poly-(EtPEG₁AcN-1NAcGAL₃₀-ran-HPMA₆₀ are shown as filled triangles. Mice treated with saline are shown as filled diamonds. Treating animals with the compounds of Formula 1 reduced the incidences of diabetes onset in NOD mice as compared to animals treated with saline.

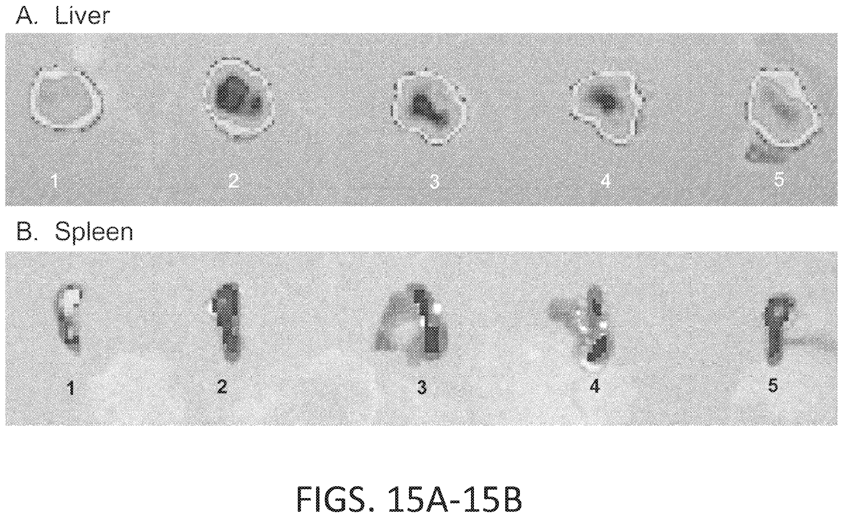

Figs. 15A-15B depicts data related to biodistribution of the model antigen OVA tethered to the synthetic glycopolymers, showing uptake in the liver while limiting uptake in the spleen. A. Fluorescent signal of perfused livers taken from animals treated with OVA (1) or OVA conjugated to various glycopolymers (2-5). B. Fluorescent images of spleens taken from animals treated with OVA (1) or OVA conjugated to various glycopolymers (2-5). Formulations are as follows: 1. OVA, 2. OVA-p(Galβ-HPMA), 3. OVA-p(Gal-HPMA), 4. OVA-p(Gluβ-HPMA), 5. OVA-p(Glu-HPMA).

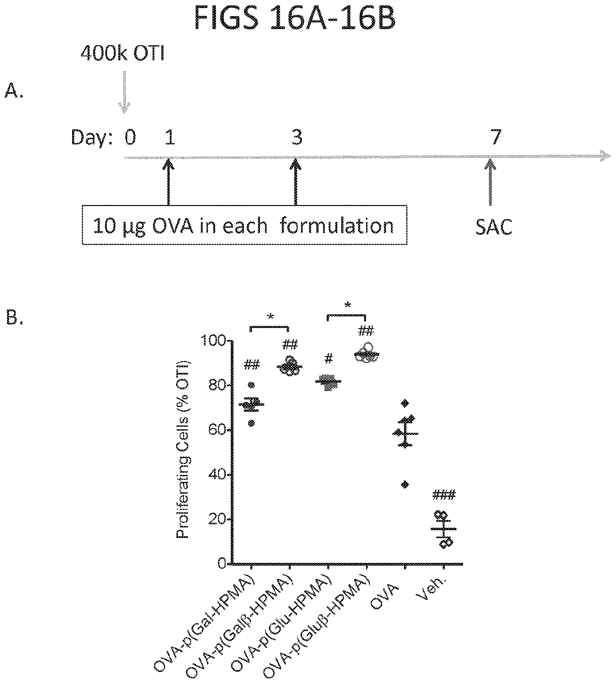

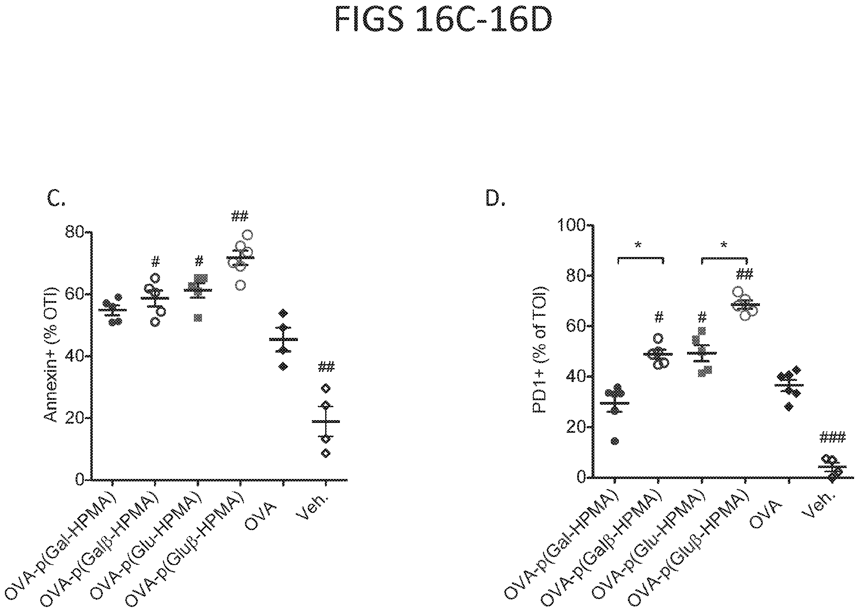

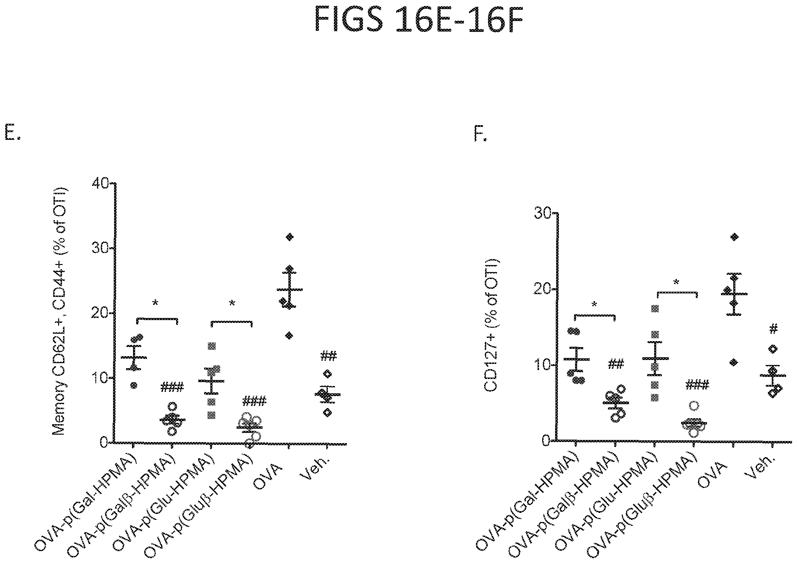

Figures 16A-16F depict data related to experiments comparing linker moieties. OVA-p(Gal-HPMA), OVA-p(Glu-HPMA), OVA-p(Galβ-HPMA), and OVA-p(Gluβ-HPMA) conjugates were synthesized and tested for their ability to induce antigen-specific T cell anergy and eliminate the T cell population responsible for long term memory. Fig. 16A shows a schematic of the treatment regimen for 7-day experiment. Fig. 16B depicts the percentage of proliferating OTI splenic T cells as assayed by CSFE dilution. Fig. 16C depicts the percentage of Annexin V+ OTI T cells in the spleens of animals treated with OVA-glycopolymer conjugates or free OVA. Fig. 16D depicts the percentage of PD-1+ splenic OTI cells. Fig. 16E depicts the percentage of T memory cells in the OTI population, where T memory cells was defined as CD62L+ and CD44+. Fig. 16 F depicts the percentage of OTI cells expressing CD127. Of particular note is the unexpectedly enhanced efficacy of compositions employing glucose or galactose in the β-conformation, as compared to the α-conformation. * = P < 0.05 ** = P < 0.01; # = P < 0.05, ## = P < 0.01 and ### = P< 0.001 (#'s represent significance as compared to animals treated with OVA alone).

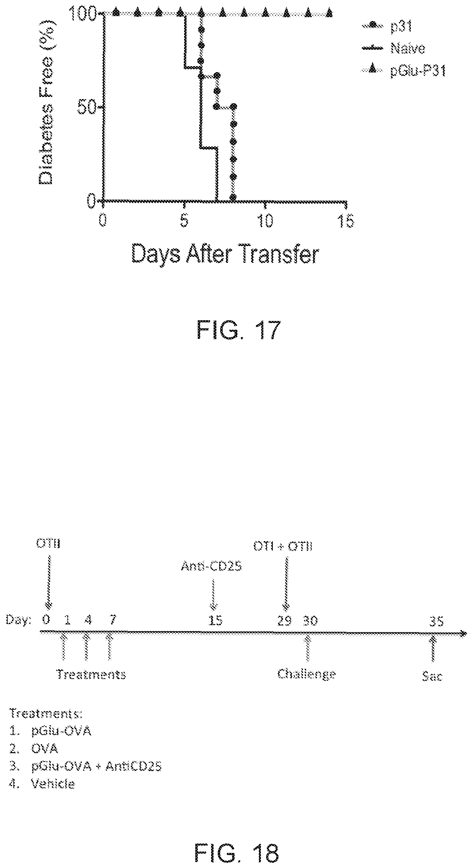

Fig. 17 depicts data related to development of symptoms of diabetes in naïve, control, and experimental groups.
Fig. 18 depicts the various treatment groups and the experimental timeline used in an experiment related to evaluation of induction of long-lasting tolerance in transferred T cells.

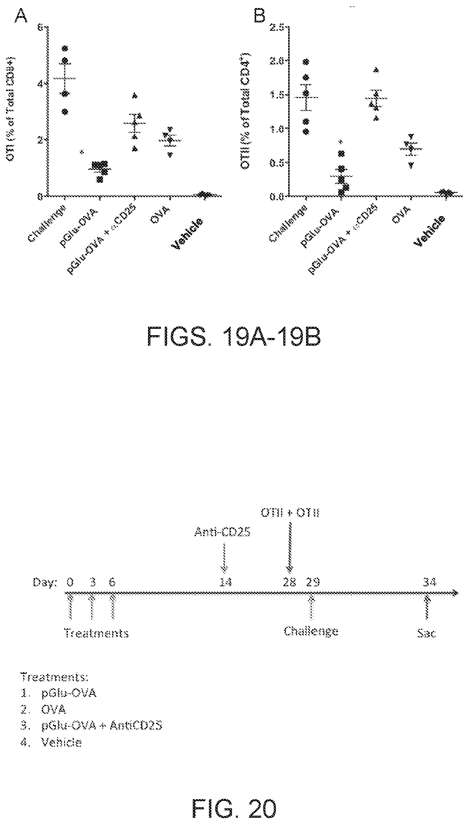

Figures 19A-19B depict data related to T cell composition in the lymph node. Figure 19A depicts the remaining OTI cells after antigen challenge (as a percentage of total CD8+ cells) in the various treatment groups. Figure 19B depicts the remaining OTII cells after antigen challenge (as a percentage of total CD4⁺ cells) in the various treatment groups.
Fig. 20 depicts the various treatment groups and the experimental timeline used in an experiment related to evaluation of induction of long-lasting tolerance in endogenous T cells.

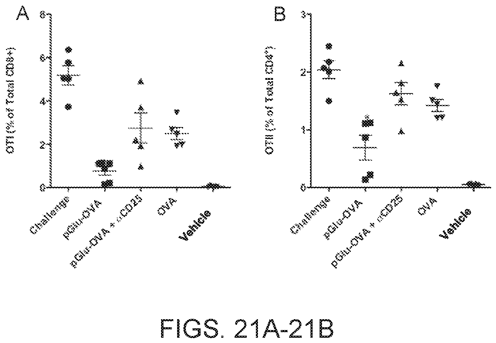

Figures 21A-21B depict data related to T cell composition in the lymph node. Figure 21A depicts the remaining OTI cells after antigen challenge (as a percentage of total CD8+ cells) in the various treatment groups. Figure 21B depicts the remaining OTII cells after antigen challenge (as a percentage of total CD4⁺ cells) in the various treatment groups.

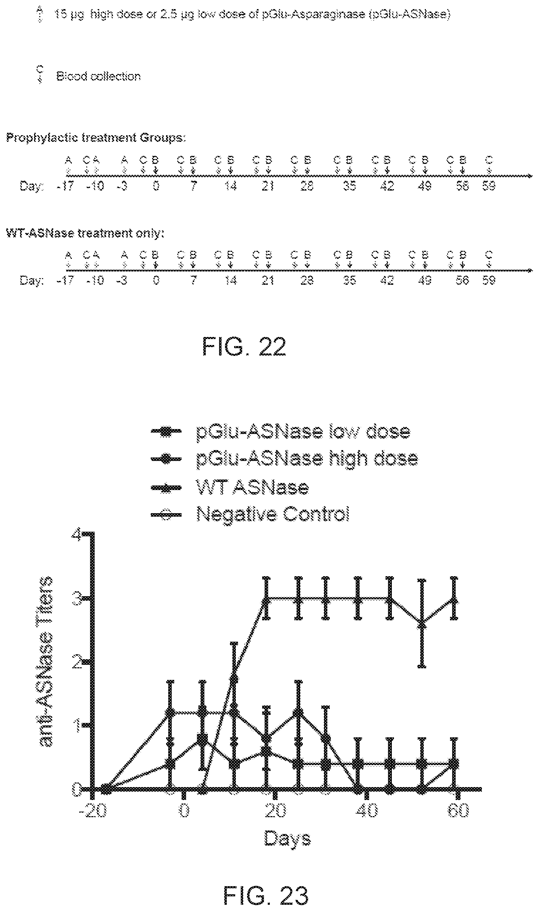

Fig. 22 depicts the experimental design used to evaluate the ability of compositions as disclosed herein to prophylactically reduce antibody response.
Fig. 23 depicts experimental data related to the amount of anti-asparaginase antibodies from mice in the various treatment groups.

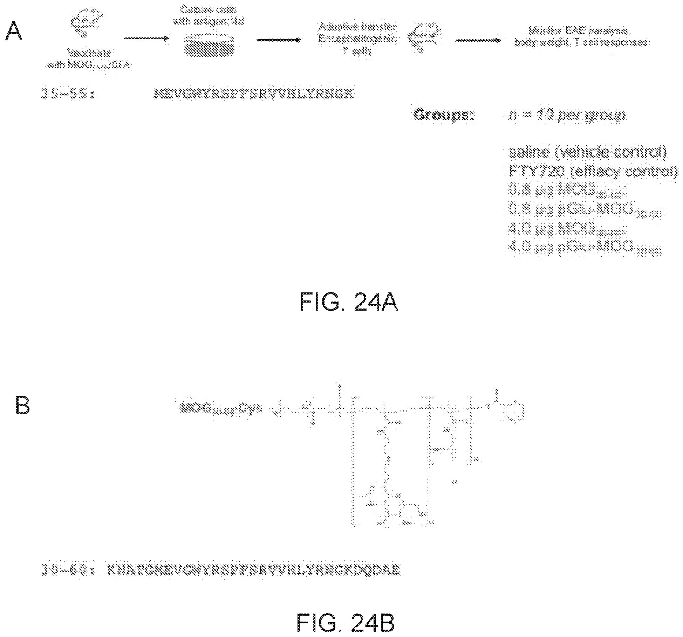

Figures 24A-24B depict the experimental design and composition used in evaluating tolerance to myelin oligodendrocyte glycoprotein (MOG). Fig. 24A shows the experimental protocol used in immunizing donor mice and treating recipient mice. Fig. 24B shows one example of a tolerogenic composition in accordance with several embodiments disclosed herein.

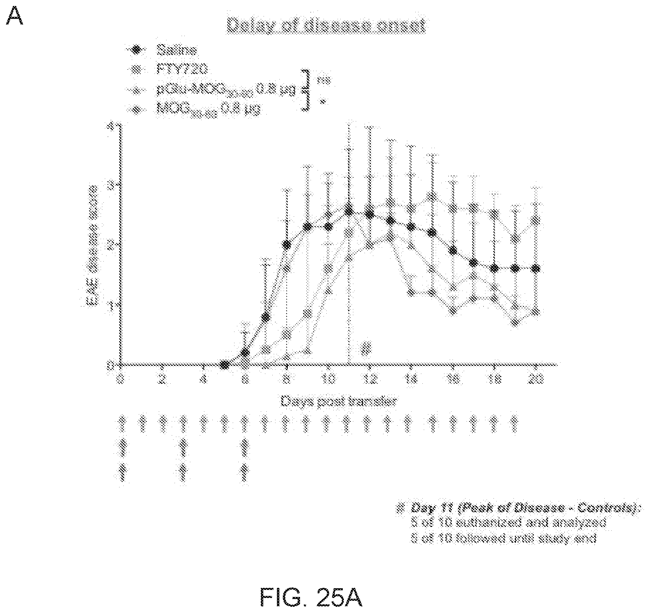

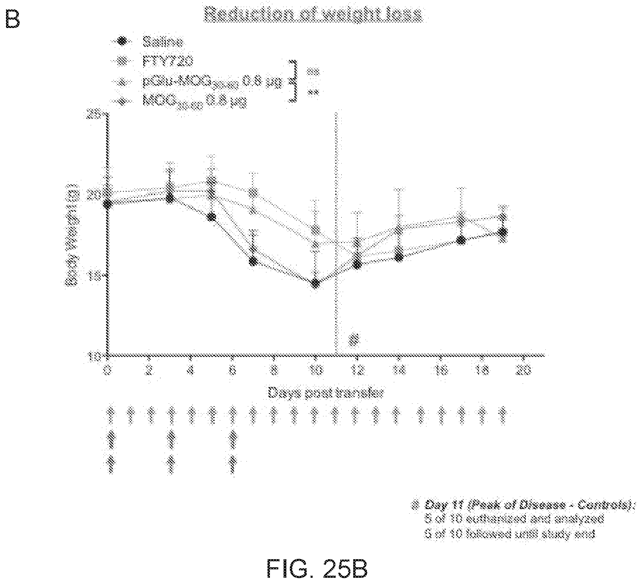

Figures 25A-25B depict experimental data related to induction of tolerance against MOG using a first concentration of the tolerogenic composition. Fig. 25A depicts data related to delay of disease onset in the various treatment groups. Fig. 25B depicts data related to reduction of weight loss in the various treatment groups.

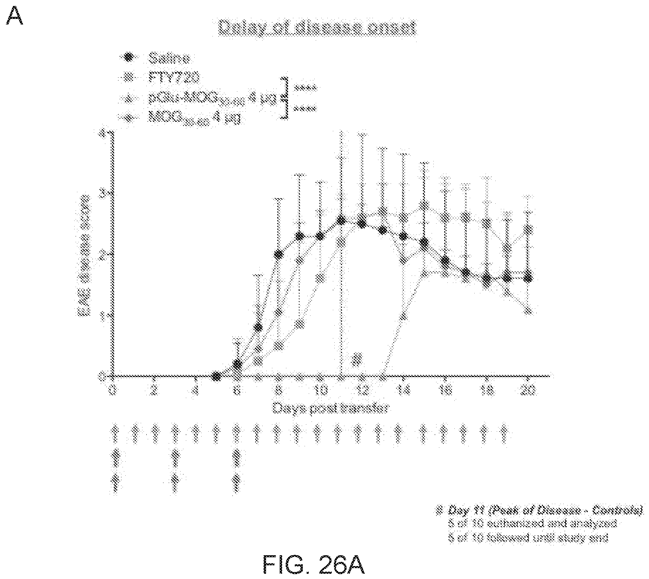

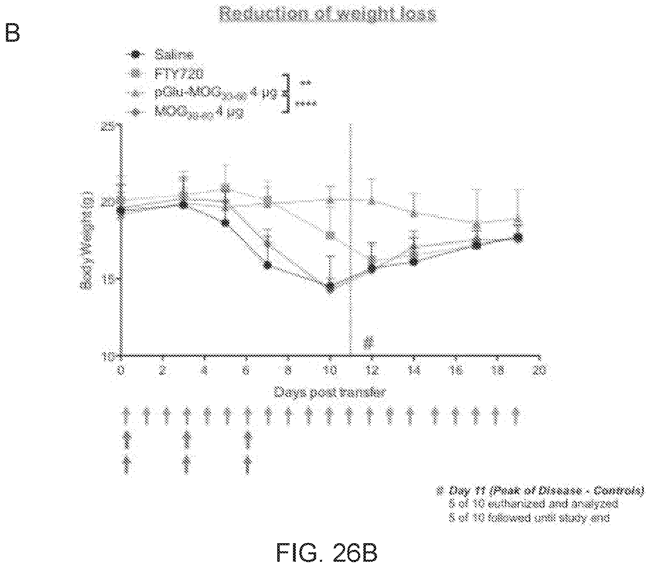

Figures 26A-26B depict experimental data related to induction of tolerance against MOG using an additional concentration of the tolerogenic composition. Fig. 26A depicts data related to delay of disease onset in the various treatment groups. Fig. 26B depicts data related to reduction of weight loss in the various treatment groups.

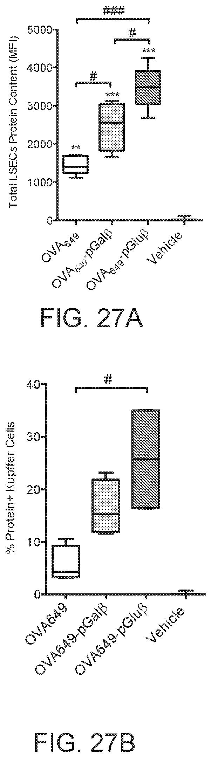

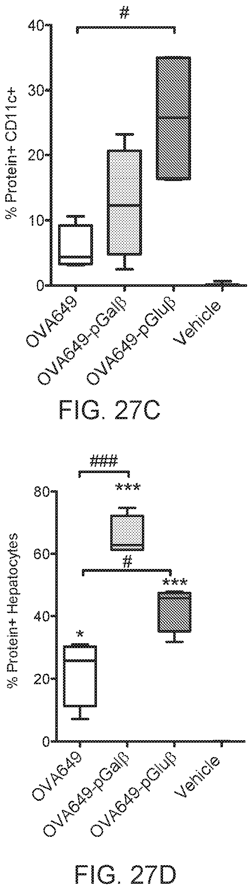

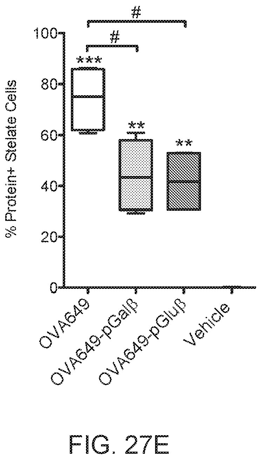

Figures 27A-27E depict experimental data related to the biodistribution of pGal and pGlu compositions in the beta conformation. Fig.27A depicts targeting of OVA via various conjugates to LSECs in the liver. Fig.27B depicts targeting of OVA via various conjugates to Kupffer cells in the liver. Fig.27C depicts targeting of OVA via various conjugates to CD11c+ cells in the liver. Fig.27D depicts targeting of OVA via various conjugates to hepatocytes in the liver. Fig.27E depicts targeting of OVA via various conjugates to stellate cells in the liver.

### DETAILED DESCRIPTION

Two known asialoglycoprotein receptors ("ASGPRs") are expressed on hepatocytes and liver sinusoidal endothelial cells (or "LSECs"). Other galactose/galactosamine/N-acetylgalactosamine receptors can be found in various forms on multiple cell types [e.g., dendritic cells, hepatocytes, LSECs, and Kupffer cells]. While the molecular and cellular targets of glucose, glucosamine and N-acetylglucosamine can be distinct from those of the corresponding galactose isomers, it has been found that the corresponding compounds of Formula 1 where Z is a glucosylating moiety are comparably effective in some instances, while in some embodiments disclosed herein, they are unexpectedly effective. Dendritic cells are considered "professional antigen presenting cells," because their primary function is to present antigens to the immune system for generating immune responses. Some cells within the liver are known to be able to present antigens, but the liver is more known to be involved in tolerogenesis. The liver is understood to be a tolerogenic organ. For example, lower incidences of rejection are reported in cases of multiple organ transplants when the liver is one of the organs transplanted. LSECs are much newer to the literature; consequently their role in tolerogenesis and/or moderation of inflammatory immune responses is not yet widely acknowledged or well understood. However, it is becoming clear that they also can play a significant role in the induction of antigen-specific tolerance.

One of the distinctive features of the erythrocyte surface is its glycosylation, i.e., the presence of significant numbers of glycosylated proteins. Indeed, the glycophorins (e.g., glycophorin A) have been employed as targets for erythrocyte binding. Glycophorins are proteins with many covalently attached sugar chains, the terminus of which is sialic acid. As an erythrocyte ages and becomes ripe for clearance, the terminal sialic acid of its glycophorins tends to be lost, leaving N-acetylgalactosamine at the free end. N-acetylgalactosamine is a ligand selectively received by the ASGPR associated with hepatic cells, leading to binding of N-acetylgalactosamine-containing substances by hepatic cells and their subsequent uptake and processing in the liver.

Heretofore, it has been understood by those skilled in the art that glycosylation of a therapeutic agent in a manner that results in hepatic targeting should be avoided due to first-pass clearance by the liver resulting in poor circulation half-life of the therapeutic agent. By the same token, some monoclonal antibodies need to be specifically glycosylated at ASN297 for optimal binding to their Fc receptors. It has now surprisingly been found, and is disclosed herein, that galactosylation and glucosylation can be used in a manner that induces tolerogenesis.

The present disclosure provides, in several embodiments, certain therapeutic compositions that are targeted for delivery to (and for uptake by) the liver, particularly hepatocytes, LSECs, Kupffer cells and/or stellate cells, more particularly hepatocytes and/or LSECs, and even more particularly to specifically bind ASGPR. Liver-targeting facilitates two mechanisms of treatment: tolerization and clearance. Tolerization takes advantage of the liver's role in clearing apoptotic cells and processing their proteins to be recognized by the immune system as "self," as well as the liver's role in sampling peripheral proteins for immune tolerance. Clearance takes advantage of the liver's role in blood purification by rapidly removing and breaking down toxins, polypeptides and the like. Targeting of these compositions to the liver is accomplished by a galactosylating moiety (e.g., galactose, galactosamine and N-acetylgalactosamine, particularly conjugated at C1, C2 or C6, though some embodiments involved conjugation at other or any carbon in the molecule), by a glucosylating moiety (e.g., glucose, glucosamine and N-acetylglucosamine, particularly conjugated at C1, C2 or C6, though some embodiments involved conjugation at other or any carbon in the molecule), or by de-sialylating a polypeptide for which such liver-targeting is desired. The galactosylating or glucosylating moiety can be chemically conjugated or recombinantly fused to an antigen, whereas desialylation exposes a galactose-like moiety on an antigen polypeptide. The antigen can be endogenous (a self-antigen) or exogenous (a foreign antigen), including but not limited to: a foreign transplant antigen against which transplant recipients develop an unwanted immune response (e.g., transplant rejection), a foreign food, animal, plant or environmental antigen to which patients develop an unwanted immune (e.g., allergic or hypersensitivity) response, a therapeutic agent to which patients develop an unwanted immune response (e.g., hypersensitivity and/or reduced therapeutic activity), a self-antigen to which patients develop an unwanted immune response (e.g., autoimmune disease), or a tolerogenic portion (e.g., a fragment or an epitope) thereof; these compositions are useful for inducing tolerization to the antigen. Alternatively, the galactosylating or other liver-targeting moiety can be conjugated to an antibody, antibody fragment or ligand that specifically binds a circulating protein or peptide or antibody, which circulating protein or peptide or antibody is causatively involved in transplant rejection, immune response against a therapeutic agent, autoimmune disease, and/or allergy (as discussed above); these compositions are useful for clearing the circulating protein, peptide or antibody. Accordingly, the compositions of the present disclosure can be used for treating an unwanted immune response, e.g., transplant rejection, an immune response against a therapeutic agent, an autoimmune disease, and/or an allergy, depending on the embodiment. Also provided are pharmaceutical compositions containing a therapeutically effective amount of a composition of the disclosure admixed with at least one pharmaceutically acceptable excipient. In another aspect, the disclosure provides methods for the treatment of an unwanted immune response, such as transplant rejection, response against a therapeutic agent, autoimmune disease or allergy.

### Definitions

As used in the present specification, the following words and phrases are generally intended to have the meanings as set forth below, except to the extent that the context in which they are used indicates otherwise.

The singular forms "a," "an," and "the" include plural referents, unless the context clearly indicates otherwise.

The term "about" when used in connection with a numerical value is meant to encompass numerical values within a range typically having a lower limit that is, e.g., 5-10% smaller than the indicated numerical value and having an upper limit that is, e.g., 5-10% larger than the indicated numerical value. Also included are any values within the disclosed range, including the listed endpoints.

As used herein, an "antigen" is any substance that serves as a target for the receptors of an adaptive immune response, such as the T cell receptor, major histocompatibility complex class I and II, B cell receptor or an antibody. In some embodiments, an antigen may originate from within the body (e.g., "self," "auto" or "endogenous"). In additional embodiments, an antigen may originate from outside the body ("non-self," "foreign" or "exogenous"), having entered, for example, by inhalation, ingestion, injection, or transplantation, transdermally, etc. In some embodiments, an exogenous antigen may be biochemically modified in the body. Foreign antigens include, but are not limited to, food antigens, animal antigens, plant antigens, environmental antigens, therapeutic agents, as well as antigens present in an allograft transplant.

An "antigen-binding molecule" as used herein relates to molecules, in particular to proteins such as immunoglobulin molecules, which contain antibody variable regions providing a binding (specific binding in some embodiments) to an epitope. The antibody variable region can be present in, for example, a complete antibody, an antibody fragment, and a recombinant derivative of an antibody or antibody fragment. The term "antigen-binding fragment" of an antibody (or "binding portion"), as used herein, refers to one or more fragments of an antibody that retain the ability to specifically bind a target sequence. Antigen-binding fragments containing antibody variable regions include (without limitation) "Fv", "Fab", and "F(ab')₂" regions, "single domain antibodies (sdAb)", "nanobodies", "single chain Fv (scFv)" fragments, "tandem scFvs" (V_{H}A-V_{L}A-V_{H}B-V_{L}B), "diabodies", "triabodies" or "tribodies", "single-chain diabodies (scDb)", and "bi-specific T-cell engagers (BiTEs)".

As used herein, a "chemical modification" refers to a change in the naturally occurring chemical structure of one or more amino acids of a polypeptide. Such modifications can be made to a side chain or a terminus, e.g., changing the amino-terminus or carboxyl terminus. In some embodiments, the modifications are useful for creating chemical groups that can conveniently be used to link the polypeptides to other materials, or to attach a therapeutic agent.

The term "comprising", which is synonymous with "including," "containing," or "characterized by," is inclusive or open-ended and does not exclude additional, unrecited elements or method steps. The phrase "consisting of" excludes any element, step, or ingredient not specified. The phrase "consisting essentially of" limits the scope of described subject matter to the specified materials or steps and those that do not materially affect its basic and novel characteristics. It is understood that wherever embodiments are described herein with the language "comprising", otherwise analogous embodiments described in terms of "consisting of" and/or "consisting essentially of" are also provided. When used in the claims as transitional phrases, each should be interpreted separately and in the appropriate legal and factual context (e.g., "comprising" is considered more of an open-ended phrase while "consisting of" is more exclusive and "consisting essentially of" achieves a middle ground).

"Conservative changes" can generally be made to an amino acid sequence without altering activity. These changes are termed "conservative substitutions" or mutations; that is, an amino acid belonging to a grouping of amino acids having a particular size or characteristic can be substituted for another amino acid. Substitutes for an amino acid sequence can be selected from other members of the class to which the amino acid belongs. For example, the nonpolar (hydrophobic) amino acids include alanine, leucine, isoleucine, valine, proline, phenylalanine, tryptophan, methionine, and tyrosine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Such substitutions are not expected to substantially affect apparent molecular weight as determined by polyacrylamide gel electrophoresis or isoelectric point. Conservative substitutions also include substituting optical isomers of the sequences for other optical isomers, specifically D amino acids for L amino acids for one or more residues of a sequence. Moreover, all of the amino acids in a sequence can undergo a D to L isomer substitution. Exemplary conservative substitutions include, but are not limited to, Lys for Arg and vice versa to maintain a positive charge; Glu for Asp and vice versa to maintain a negative charge; Ser for Thr so that a free -OH is maintained; and Gln for Asn to maintain a free -NH₂. Yet another type of conservative substitution constitutes the case where amino acids with desired chemical reactivities are introduced to impart reactive sites for chemical conjugation reactions, if the need for chemical derivatization arises. Such amino acids include but are not limited to Cys (to insert a sulfhydryl group), Lys (to insert a primary amine), Asp and Glu (to insert a carboxylic acid group), or specialized noncanonical amino acids containing ketone, azide, alkyne, alkene, and tetrazine side-chains. Conservative substitutions or additions of free -NH₂ or -SH bearing amino acids can be particularly advantageous for chemical conjugation with the linkers and galactosylating moieties of Formula 1. Moreover, point mutations, deletions, and insertions of the polypeptide sequences or corresponding nucleic acid sequences can in some cases be made without a loss of function of the polypeptide or nucleic acid fragment. Substitutions can include, e.g., 1, 2, 3, 4, 5, 6, 7, 8, 9, 10, 15, 20, 25, 30, 35, 40, 45, 50 or more residues (including any number of substitutions between those listed). A variant usable in the present invention may exhibit a total number of up to 200 (e.g., up to 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, or 200, including any number in between those listed) changes in the amino acid sequence (e.g., exchanges, insertions, deletions, N-terminal truncations, and/or C-terminal truncations). In several embodiments, the number of changes is greater than 200. Additionally, in several embodiments, the variants include polypeptide sequences or corresponding nucleic acid sequences that exhibit a degree of functional equivalence with a reference (e.g., unmodified or native sequence). In several embodiments, the variants exhibit about 80%, about 85%, about 90%, about 95%, about 97%, about 98%, about 99% functional equivalence to an unmodified or native reference sequence (and any degree of functional equivalence between those listed). The amino acid residues described herein employ either the single letter amino acid designator or the three-letter abbreviation in keeping with the standard polypeptide nomenclature, J. Biol. Chem., (1969), 243, 3552-3559. All amino acid residue sequences are represented herein by formulae with left and right orientation in the conventional direction of amino-terminus to carboxy-terminus.

The terms "effective amount" or "therapeutically effective amount" refer to that amount of a composition of the disclosure that is sufficient to effect treatment, as defined below, when administered to a mammal in need of such treatment. This amount will vary depending upon the subject and disease condition being treated, the weight and age of the subject, the severity of the disease condition, the particular composition of the disclosure chosen, the dosing regimen to be followed, timing of administration, manner of administration and the like, all of which can readily be determined by one of ordinary skill in the art.

An "epitope", also known as antigenic determinant, is the segment of a macromolecule, e.g. a protein, which is recognized by the adaptive immune system, such as by antibodies, B cells, major histocompatibility complex molecules, or T cells. An epitope is that part or segment of a macromolecule capable of binding to an antibody or antigen-binding fragment thereof. In this context, the term "binding" in particular relates to a specific binding. In the context of several embodiments of the present invention, it is preferred that the term "epitope" refers to the segment of protein or polyprotein that is recognized by the immune system.

The term galactose refers to a monosaccharide sugar that exists both in open-chain form and in cyclic form, having D- and L- isomers. In the cyclic form, there are two anomers, namely alpha and beta. In the alpha form, the C1 alcohol group is in the axial position, whereas in the beta form, the C1 alcohol group is in the equatorial position. In particular, "galactose" refers to the cyclic six-membered pyranose, more in particular the D-isomer and even more particularly the alpha-D-form (α-D-galactopyranose) the formal name for which is (2R,3R,4S,5R,6R)-6-(hydroxymethyl)tetrahydro-2H-pyran-2,3,4,5-tetraol. Glucose is an epimer of galactose; the formal name is (2R,3R,4S,5S,6R)-6-(hydroxymethyl)tetrahydro-2H-pyran-2,3,4,5-tetraol. The structure and numbering of galactose and glucose are shown giving two non-limiting examples of stereochemical illustration.

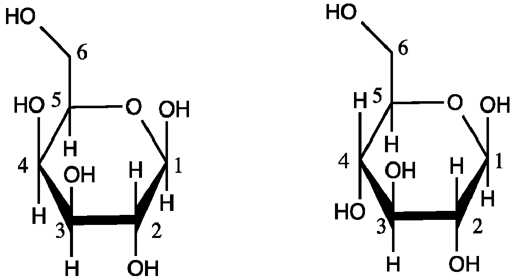

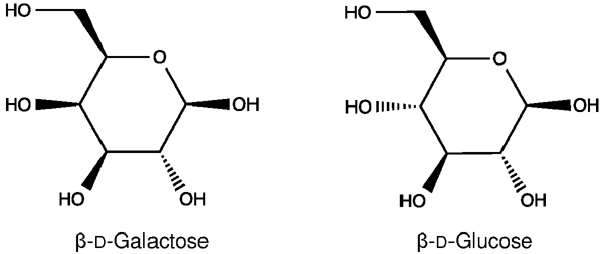

The term "galactosylating moiety" refers to a particular type of liver-targeting moiety. Galactosylating moieties include, but are not limited to a galactose, galactosamine and/or N-acetylgalactosamine residue. A "glucosylating moiety" refers to another particular type of liver-targeting moiety and includes, but is not limited to glucose, glucosamine and/or N-acetylglucosamine.

The term "liver-targeting moiety", refers to moieties having the ability to direct, e.g., a polypeptide, to the liver. The liver comprises different cell types, including but not limited to hepatocytes, sinusoidal epithelial cells, Kupffer cells, stellate cells, and/or dendritic cells. Typically, a liver-targeting moiety directs a polypeptide to one or more of these cells. On the surface of the respective liver cells, receptors are present which recognize and specifically bind the liver-targeting moiety. Liver-targeting can be achieved by chemical conjugation of an antigen or ligand to a galactosylating or glucosylating moiety, desialylation of an antigen or ligand to expose underlying galactosyl or glucosyl moieties, or specific binding of an endogenous antibody to an antigen or ligand, where the antigen or ligand is: desialylated to expose underlying galactosyl or glucosyl moieties, conjugated to a galactosylating or a glucosylating moiety. Naturally occurring desialylated proteins are not encompassed within the scope of certain embodiments of the present disclosure.

The "numerical values" and "ranges" provided for the various substituents are intended to encompass all integers within the recited range. For example, when defining n as an integer representing a mixture including from about 1 to 100, particularly about 8 to 90 and more particularly about 40 to 80 ethylene glycol groups, where the mixture typically encompasses the integer specified as n ± about 10% (or for smaller integers from 1 to about 25, ±3), it should be understood that n can be an integer from about 1 to 100 (e.g., 1, 2, 3, 4, 5, 6, 7, 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95, 99, 100, 105 or 110, or any between those listed, including the endpoints of the range) and that the disclosed mixture encompasses ranges such as 1-4, 2-4, 2-6, 3-8, 7-13, 6-14, 18-23, 26-30, 42-50, 46-57, 60-78, 85-90, 90-110 and 107-113 ethylene glycol groups. The combined terms "about" and "±10%" or "±3" should be understood to disclose and provide specific support for equivalent ranges wherever used.

The term "optional" or "optionally" means that the subsequently described event or circumstance may or may not occur, and that the description includes instances where said event or circumstance occurs and instances in which it does not.

A peptide that specifically binds a particular target is referred to as a "ligand" for that target.

A "polypeptide" is a term that refers to a chain of amino acid residues, regardless of post-translational modification (e.g., phosphorylation or glycosylation) and/or complexation with additional polypeptides, and/or synthesis into multisubunit complexes with nucleic acids and/or carbohydrates, or other molecules. Proteoglycans therefore also are referred to herein as polypeptides. A long polypeptide (having over about 50 amino acids) is referred to as a "protein." A short polypeptide (having fewer than about 50 amino acids) is referred to as a "peptide." Depending upon size, amino acid composition and three dimensional structure, certain polypeptides can be referred to as an "antigen-binding molecule," "antibody," an "antibody fragment" or a "ligand." Polypeptides can be produced by a number of methods, many of which are well known in the art. For example, polypeptides can be obtained by extraction (e.g., from isolated cells), by expression of a recombinant nucleic acid encoding the polypeptide, or by chemical synthesis. Polypeptides can be produced by, for example, recombinant technology, and expression vectors encoding the polypeptide introduced into host cells (e.g., by transformation or transfection) for expression of the encoded polypeptide

As used herein, "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

The term "purified" as used herein with reference to a polypeptide refers to a polypeptide that has been chemically synthesized and is thus substantially uncontaminated by other polypeptides, or has been separated or isolated from most other cellular components by which it is naturally accompanied (e.g., other cellular proteins, polynucleotides, or cellular components). An example of a purified polypeptide is one that is at least 70%, by dry weight, free from the proteins and naturally occurring organic molecules with which it naturally associates. A preparation of a purified polypeptide therefore can be, for example, at least 80%, at least 90%, or at least 99%, by dry weight, the polypeptide. Polypeptides also can be engineered to contain a tag sequence (e.g., a polyhistidine tag, a myc tag, a FLAG^{®} tag, or other affinity tag) that facilitates purification or marking (e.g., capture onto an affinity matrix, visualization under a microscope). Thus, a purified composition that comprises a polypeptide refers to a purified polypeptide unless otherwise indicated. The term "isolated" indicates that the polypeptides or nucleic acids of the disclosure are not in their natural environment. Isolated products of the disclosure can thus be contained in a culture supernatant, partially enriched, produced from heterologous sources, cloned in a vector or formulated with a vehicle, etc.

The term "random copolymer" refers to the product of simultaneous polymerization of two or more monomers in admixture, where the probability of finding a given monomeric unit at any given site in a polymer chain is independent of the nature of the neighboring units at that position (Bernoullian distribution). Thus, when the variable group identified as Wₚ represents a random copolymer, the chain can comprise any sequence from 2 up to about 150 W¹ and W² groups, such as: -W¹-W²-W¹-W²-; - W²-W¹-W²-W¹-; -W¹-W¹-W¹-W²-; -W¹-W¹-W²-W²-; -W¹-W²-W²-W¹-; -W¹-W²-W¹-W²-W²-W¹-W²-W¹-; - W¹-W¹-W²-W²-W¹-W²-W²-W¹-; and W²-W²-W¹-W²-W¹-W¹-W¹-W²-W²-W¹-W²-W²-W¹; *ad infinitum,* where Z attached to the various W¹ groups and the W¹ and W² groups themselves can be the same or different.

The term "sequence identity" is used with regard to polypeptide (or nucleic acid) sequence comparisons. This expression in particular refers to a percentage of sequence identity, for example at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% to the respective reference polypeptide or to the respective reference polynucleotide. Particularly, the polypeptide in question and the reference polypeptide exhibit the indicated sequence identity over a continuous stretch of 20, 30, 40, 45, 50, 60, 70, 80, 90, 100 or more amino acids or over the entire length of the reference polypeptide.

"Specific binding," as that term is commonly used in the biological arts, refers to a molecule that binds to a target with a relatively high affinity as compared to non-target tissues, and generally involves a plurality of non-covalent interactions, such as electrostatic interactions, van der Waals interactions, hydrogen bonding, and the like. Specific binding interactions characterize antibody-antigen binding, enzyme-substrate binding, and certain protein-receptor interactions; while such molecules might bind tissues besides their specific targets from time to time, to the extent that such non-target binding is inconsequential, the high-affinity binding pair can still fall within the definition of specific binding.

The term "treatment" or "treating" means any treatment of a disease or disorder in a mammal, including:
preventing or protecting against the disease or disorder, that is, causing the clinical symptoms not to develop;
inhibiting the disease or disorder, that is, arresting or suppressing the development of clinical symptoms; and/or
relieving the disease or disorder, that is, causing the regression of clinical symptoms.

The term "unwanted immune response" refers to a reaction by the immune system of a subject, which in the given situation is not desirable. The reaction of the immune system is unwanted if such reaction does not lead to the prevention, reduction, or healing of a disease or disorder but instead causes, enhances or worsens, or is otherwise associated with induction or worsening of a disorder or disease. Typically, a reaction of the immune system causes, enhances or worsens a disease if it is directed against an inappropriate target. Exemplified, an unwanted immune response includes but is not limited to transplant rejection, immune response against a therapeutic agent, autoimmune disease, and allergy or hypersensitivity.

The term "variant" is to be understood as a protein (or nucleic acid) which differs in comparison to the protein from which it is derived by one or more changes in its length, sequence, or structure. The polypeptide from which a protein variant is derived is also known as the parent polypeptide or polynucleotide. The term "variant" comprises "fragments" or "derivatives" of the parent molecule. Typically, "fragments" are smaller in length or size than the parent molecule, whilst "derivatives" exhibit one or more differences in their sequence or structure in comparison to the parent molecule. Also encompassed are modified molecules such as but not limited to post-translationally modified proteins (e.g. glycosylated, phosphorylated, ubiquitinated, palmitoylated, or proteolytically cleaved proteins) and modified nucleic acids such as methylated DNA. Also mixtures of different molecules such as but not limited to RNA-DNA hybrids, are encompassed by the term "variant". Naturally occurring and artificially constructed variants are to be understood to be encompassed by the term "variant" as used herein. Further, the variants usable in the present invention may also be derived from homologs, orthologs, or paralogs of the parent molecule or from artificially constructed variant, provided that the variant exhibits at least one biological activity of the parent molecule, e.g., is functionally active. A variant can be characterized by a certain degree of sequence identity to the parent polypeptide from which it is derived. More precisely, a protein variant in the context of the present disclosure may exhibit at least 80% sequence identity to its parent polypeptide. Preferably, the sequence identity of protein variants is over a continuous stretch of 20, 30, 40, 45, 50, 60, 70, 80, 90, 100 or more amino acids. As discussed above, in several embodiments variants exhibit about 80%, about 85%, about 90%, about 95%, about 97%, about 98%, about 99% functional equivalence to an unmodified or native reference sequence (and any degree of functional equivalence between those listed).

### Compositions

One aspect of the present disclosure relates to compositions, pharmaceutical formulations, and methods of treatment employing such compositions, as represented by Formula 1: 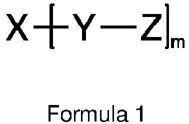 where:
m is an integer from about 1 to 100, particularly from about 1 to 20, and most particularly 1 to about 10;
X is an antigen moiety, particularly a foreign antigen or self-antigen against which a patient develops an unwanted immune response, or a tolerogenic portion (e.g., a fragment or an epitope) of such an antigen moiety;
Y is a linker moiety or a direct bond, or an antibody, antibody fragment, peptide or other ligand that specifically binds X; and
Z is a liver-targeting moiety, in particular galactosylating or a glucosylating moiety.
The value for m in Formula 1 will depend upon the nature of X, in that each antigen, antibody, antibody fragment or ligand will have an individual number and density of sites (predominantly the N-terminal amine, lysine residues and cysteine residues) to which a linker, a galactosylating moiety or a glucosylating moiety can be bound. Antigens having a limited number of such sites can be derivatized, for example, at the N or C terminus, by adding lysine or cysteine residues (optionally via a cleavable linker, particularly a linker having an immunoproteosome cleavage site). Generally, it is preferred to provide an adequate degree of galactosylation/glucosylation in compositions of Formula 1 so as to facilitate uptake by liver cells. Pharmaceutical formulations and methods of the disclosure can employ a cocktail of compositions of Formula 1, respectively bearing different X moieties (e.g., several epitopes associated with a particular unwanted immune response).

The compositions of Formula 1 include the sub-genuses where X is a foreign transplant antigen against which transplant recipients develop an unwanted immune response (e.g., transplant rejection), a foreign food, animal, plant or environmental antigen against which patients develop an unwanted immune (e.g., allergic or hypersensitivity) response, a foreign therapeutic agent against which patients develop an unwanted immune response (e.g., hypersensitivity and/or reduced therapeutic activity), or a self-antigen against which patients develop an unwanted immune response (e.g., autoimmune disease); where Y is a linker of Formulae Ya through Yp; and/or where Z is galactose, galactosamine, N-acetylgalactosamine, glucose, glucosamine or N-acetylglucosamine as illustrated by Formulae 1a through 1p as described below with reference to the Reaction Schemes.

In additional embodiments, in the compositions of Formula 1, X can be an antibody, antibody fragment or ligand that specifically binds a circulating protein or peptide or antibody, which circulating protein or peptide or antibody is causatively involved in transplant rejection, immune response against a therapeutic agent, autoimmune disease, hypersensitivity and/or allergy.

### Antigens

The antigen employed as X in the compositions of Formula 1 can be a protein or a peptide, e.g. the antigen may be a complete or partial therapeutic agent, a full-length transplant protein or peptide thereof, a full-length autoantigen or peptide thereof, a full-length allergen or peptide thereof, and/or a nucleic acid, or a mimetic of an aforementioned antigen. A listing of any particular antigen in a category or association with any particular disease or reaction does not preclude that antigen from being considered part of another category or associated with another disease or reaction.

Antigens employed in the practice of the present disclosure can be one or more of the following:
- Therapeutic agents that are proteins, peptides, antibodies and antibody-like molecules, including antibody fragments and fusion proteins with antibodies and antibody fragments. These include human, non-human (such as mouse) and non-natural (i.e., engineered) proteins, antibodies, chimeric antibodies, humanized antibodies, and non-antibody binding scaffolds, such as fibronectins, DARPins, knottins, and the like.
- Human allograft transplantation antigens against which transplant recipients develop an unwanted immune response.
- Self-antigens that cause an unwanted, autoimmune response. Those skilled in the art will appreciate that while self-antigens are of an endogenous origin in an autoimmune disease patient, the polypeptides employed in the disclosed compositions are typically synthesized exogenously (as opposed to being purified and concentrated from a source of origin).
- Foreign antigens, such as food, animal, plant and environmental antigens, against which a patient experiences an unwanted immune response. Those skilled in the art will appreciate that while a therapeutic protein can also be considered a foreign antigen due to its exogenous origin, for purposes of clarity in the description of the present disclosure such therapeutics are described as a separate group. Similarly, a plant or an animal antigen can be eaten and considered a food antigen, and an environmental antigen may originate from a plant. They are, however, all foreign antigens. In the interest of simplicity no attempt will be made to describe distinguish and define all of such potentially overlapping groups, as those skilled in the art can appreciate the antigens that can be employed in the compositions of the disclosure, particularly in light of the detailed description and examples.
The antigen can be a complete protein, a portion of a complete protein, a peptide, or the like, and can be derivatized (as discussed above) for attachment to a linker and/or galactosylating moiety, can be a variant and/or can contain conservative substitutions, particularly maintaining sequence identity, and/or can be desialylated.

In the embodiments where the antigen is a therapeutic protein, peptide, antibody or antibody-like molecule, specific antigens can be selected from: Abatacept, Abciximab, Adalimumab, Adenosine deaminase, Ado-trastuzumab emtansine, Agalsidase alfa, Agalsidase beta, Aldeslukin, Alglucerase, Alglucosidase alfa, α-1-proteinase inhibitor, Anakinra, Anistreplase (anisoylated plasminogen streptokinase activator complex), Antithrombin III, Antithymocyte globulin, Ateplase, Bevacizumab, Bivalirudin, Botulinum toxin type A, Botulinum toxin type B, C1-esterase inhibitor, Canakinumab, Carboxypeptidase G2 (Glucarpidase and Voraxaze), Certolizumab pegol, Cetuximab, Collagenase, Crotalidae immune Fab, Darbepoetin-α, Denosumab, Digoxin immune Fab, Dornase alfa, Eculizumab, Etanercept, Factor Vlla, Factor VIII, Factor IX, Factor XI, Factor XIII, Fibrinogen, Filgrastim, Galsulfase, Golimumab, Histrelin acetate, Hyaluronidase, Idursulphase, Imiglucerase, Infliximab, Insulin [including recombinant human insulin ("rHu insulin") and bovine insulin], Interferon-α2a, Interferon-α2b, Interferon-β1a, Interferon-β1b, Interferon-γ1b, Ipilimumab, L-arginase, L-asparaginase, L-methionase, Lactase, Laronidase, Lepirudin / hirudin, Mecasermin, Mecasermin rinfabate, Methoxy Natalizumab, Octreotide, Ofatumumab, Oprelvekin, Pancreatic amylase, Pancreatic lipase, Papain, Peg-asparaginase, Peg-doxorubicin HCl, PEG-epoetin-β, Pegfilgrastim, Peg-Interferon-α2a, Peg-Interferon-α2b, Pegloticase, Pegvisomant, Phenylalanine ammonia-lyase (PAL), Protein C, Rasburicase (uricase), Sacrosidase, Salmon calcitonin, Sargramostim, Streptokinase, Tenecteplase, Teriparatide, Tocilizumab (atlizumab), Trastuzumab, Type 1 alpha-interferon, Ustekinumab, vW factor. The therapeutic protein can be obtained from natural sources (e.g., concentrated and purified) or synthesized, e.g., recombinantly, and includes antibody therapeutics that are typically IgG monoclonal or fragments or fusions.

Particular therapeutic protein, peptide, antibody or antibody-like molecules include Abciximab, Adalimumab, Agalsidase alfa, Agalsidase beta, Aldeslukin, Alglucosidase alfa, Factor VIII, Factor IX, Infliximab, Insulin (including rHu Insulin), L-asparaginase, Laronidase, Natalizumab, Octreotide, Phenylalanine ammonia-lyase (PAL), or Rasburicase (uricase) and generally IgG monoclonal antibodies in their varying formats.

Another particular group includes the hemostatic agents (Factor VIII and IX), Insulin (including rHu Insulin), and the non-human therapeutics uricase, PAL and asparaginase.

Unwanted immune response in hematology and transplant includes autoimmune aplastic anemia, transplant rejection (generally), and Graft vs. Host Disease (bone marrow transplant rejection). In the embodiments where the antigen is a human allograft transplantation antigen, specific sequences can be selected from: subunits of the various MHC class I and MHC class II haplotype proteins (for example, donor/recipient differences identified in tissue cross-matching), and single-amino-acid polymorphisms on minor blood group antigens including RhCE, Kell, Kidd, Duffy and Ss. Such compositions can be prepared individually for a given donor/recipient pair.

In the embodiments where the antigen is a self-antigen, specific antigens (and the autoimmune disease with which they are associated) can be selected from:
- In type 1 diabetes mellitus, several main antigens have been identified: insulin, proinsulin, preproinsulin, glutamic acid decarboxylase-65 (GAD-65 or glutamate decarboxylase 2), GAD-67, glucose-6 phosphatase 2 (IGRP or islet-specific glucose 6 phosphatase catalytic subunit related protein), insulinoma-associated protein 2 (IA-2), and insulinoma-associated protein 2β (IA-2β); other antigens include ICA69, ICA12 (SOX-13), carboxypeptidase H, Imogen 38, GLIMA 38, chromogranin-A, HSP-60, carboxypeptidase E, peripherin, glucose transporter 2, hepatocarcinoma-intestine-pancreas/pancreatic associated protein, S100β, glial fibrillary acidic protein, regenerating gene II, pancreatic duodenal homeobox 1, dystrophia myotonica kinase, islet-specific glucose-6-phosphatase catalytic subunit-related protein, and SST G-protein coupled receptors 1-5. It should be noted that insulin is an example of an antigen that can be characterized both as a self-antigen and a therapeutic protein antigen. For example, rHu Insulin and bovine insulin are therapeutic protein antigens (that are the subject of unwanted immune attack), whereas endogenous human insulin is a self-antigen (that is the subject of an unwanted immune attack). Because endogenous human insulin is not available to be employed in a pharmaceutical composition, a recombinant form is employed in certain embodiments of the compositions of the disclosure.
   ∘ Human insulin, including an exogenously obtained form useful in the compositions of the disclosure, has the following sequence (UNIPROT P01308): 


 (SEQ ID NO:1).
   ∘ GAD-65, including an exogenously obtained form useful in the compositions of the disclosure, has the following sequence (UNIPROT Q05329): 
MASPGSGFWS FGSEDGSGDS ENPGTARAWC QVAQKFTGGI GNKLCALLYG
DAEKPAESGG SQPPRAAARK AACACDQKPC SCSKVDVNYA FLHATDLLPA
CDGERPTLAF LQDVMNILLQ YVVKSFDRST KVIDFHYPNE LLQEYNWELA
DQPQNLEEIL MHCQTTLKYA IKTGHPRYFN QLSTGLDMVG LAADWLTSTA
NTNMFTYEIA PVFVLLEYVT LKKMREIIGW PGGSGDGIFS PGGAISNMYA
MMIARFKMFP EVKEKGMAAL PRLIAFTSEH SHFSLKKGAA ALGIGTDSVI
LIKCDERGKM IPSDLERRIL EAKQKGFVPF LVSATAGTTV YGAFDPLLAV
ADICKKYKIW MHVDAAWGGG LLMSRKHKWK LSGVERANSV TWNPHKMMGV
PLQCSALLVR EEGLMQNCNQ MHASYLFQQD KHYDLSYDTG DKALQCGRHV
DVFKLWLMWR AKGTTGFEAH VDKCLELAEY LYNIIKNREG YEMVFDGKPQ
HTNVCFWYIP PSLRTLEDNE ERMSRLSKVA PVIKARMMEY GTTMVSYQPL
GDKVNFFRMV ISNPAATHQD IDFLIEEIER LGQDL (SEQ ID NO:2).
   ∘ IGRP, including an exogenously obtained form useful in the compositions of the disclosure, has the following sequence (UNIPROT QN9QR9): 
MDFLHRNGVLIIQHLQKDYRAYYTFLNFMSNVGDPRNIFFIYFPLCFQFNQTVGTKMIW
VAVIGDWLNLIFKWILFGHRPYWWVQETQIYPNHSSPCLEQFPTTCETGPGSPSGHAM
GASCVWYVMVTAALSHTVCGMDKFSITLHRLTWSFLWSVFWLIQISVCISRVFIATHFP
HQVILGVIGGMLVAEAFEHTPGIQTASLGTYLKTNLFLFLFAVGFYLLLRVLNIDLLWSVP
IAKKWCANPDWIHIDTTPFAGLVRNLGVLFGLGFAINSEMFLLSCRGGNNYTLSFRLLC
ALTSLTILQLYHFLQIPTHEEHLFYVLSFCKSASIPLTVVAFIPYSVHMLMKQSGKKSQ
(SEQ ID NO:3).
- In autoimmune diseases of the thyroid, including Hashimoto's thyroiditis and Graves' disease, main antigens include thyroglobulin (TG), thyroid peroxidase (TPO) and thyrotropin receptor (TSHR); other antigens include sodium iodine symporter (NIS) and megalin. In thyroid-associated ophthalmopathy and dermopathy, in addition to thyroid autoantigens including TSHR, an antigen is insulin-like growth factor 1 receptor. In hypoparathyroidism, a main antigen is calcium sensitive receptor.
- In Addison's Disease, main antigens include 21-hydroxylase, 17α-hydroxylase, and P450 side chain cleavage enzyme (P450scc); other antigens include ACTH receptor, P450c21 and P450c17.
- In premature ovarian failure, main antigens include FSH receptor and α-enolase.
- In autoimmune hypophysitis, or pituitary autoimmune disease, main antigens include pituitary gland-specific protein factor (PGSF) 1a and 2; another antigen is type 2 iodothyronine deiodinase.
- In multiple sclerosis, main antigens include myelin basic protein ("MBP"), myelin oligodendrocyte glycoprotein ("MOG") and myelin proteolipid protein ("PLP").
   ∘ MBP, including an exogenously obtained form useful in the compositions of the disclosure, has the following sequence (UNIPROT P02686): 
MGNHAGKRELNAEKASTNSETNRGESEKKRNLGELSRTTSEDNEVFGEADANQNNG
TSSQDTAVTDSKRTADPKNAWQDAHPADPGSRPHLIRLFSRDAPGREDNTFKDRPSE
SDELQTIQEDSAATSESLDVMASQKRPSQRHGSKYLATASTMDHARHGFLPRHRDTGI
LDSIGRFFGGDRGAPKRGSGKDSHHPARTAHYGSLPQKSHGRTQDENPVVHFFKNIV
TPRTPPPSQGKGRGLSLSRFSWGAEGQRPGFGYGGRASDYKSAHKGFKGVDAQGT
LSKIFKLGGRDSRSGSPMARR (SEQ ID NO:4).
   ∘ MOG, including an exogenously obtained form useful in the compositions of the disclosure, has the following sequence (UNIPROT Q16653): 
MASLSRPSLPSCLCSFLLLLLLQVSSSYAGQFRVIGPRHPIRALVGDEVELPCRISPGKN
ATGMEVGWYRPPFSRVVHLYRNGKDQDGDQAPEYRGRTELLKDAIGEGKVTLRIRNV
RFSDEGGFTCFFRDHSYQEEAAMELKVEDPFYWVSPGVLVLLAVLPVLLLQITVGLIFL
CLQYRLRGKLRAEIENLHRTFDPHFLRVPCWKITLFVIVPVLGPLVALIICYNWLHRRLA
GQFLEELRNPF (SEQ ID NO:5).
   ∘ PLP, including an exogenously obtained form useful in the compositions of the disclosure, has the following sequence (UNIPROT P60201): 




 (SEQ ID NO:6).
   ∘ Peptides/epitopes useful in the compositions of the disclosure for treating multiple sclerosis include some or all of the following sequences, individually in a composition of Formula 1 or together in a cocktail of compositions of Formula 1:
      ▪ MBP13-32: KYLATASTMDHARHGFLPRH (SEQ ID NO:7);
      ▪ MBP83-99: ENPWHFFKNIVTPRTP (SEQ ID NO:8);
      ▪ MBP111-129: LSRFSWGAEGQRPGFGYGG (SEQ ID NO:9);
      ▪ MBP146-170: AQGTLSKIFKLGGRDSRSGSPMARR (SEQ ID NO:10);
      ▪ MOG1-20: GQFRVIGPRHPIRALVGDEV (SEQ ID NO:11);
      ▪ MOG35-55: MEVGWYRPPFSRWHLYRNGK (SEQ ID NO:12); and
      ▪ PLP139-154: HCLGKWLGHPDKFVGI (SEQ ID NO:13).
- In rheumatoid arthritis, main antigens include collagen II, immunoglobulin binding protein, the fragment crystallizable region of immunoglobulin G, double-stranded DNA, and the natural and cirtullinated forms of proteins implicated in rheumatoid arthritis pathology, including fibrin/fibrinogen, vimentin, collagen I and II, and alpha-enolase.
- In autoimmune gastritis, a main antigen is H+,K+-ATPase.
- In pernicious angemis, a main antigen is intrinsic factor.
- In celiac disease, main antigens are tissue transglutaminase and the natural and deamidated forms of gluten or gluten-like proteins, such as alpha-, gamma-, and omega-gliadin, glutenin, hordein, secalin, and avenin. Those skilled in the art will appreciate, for example, that while the main antigen of celiac disease is alpha gliadin, alpha gliadin turns more immunogenic in the body through deamidation by tissue glutaminase converting alpha gliadin's glutamines to glutamic acid. Thus, while alpha gliadin is originally a foreign food antigen, once it has been modified in the body to become more immunogenic it can be characterized as a self-antigen.
- In vitiligo, a main antigen is tyrosinase, and tyrosinase related protein 1 and 2.
   ∘ MART1, Melanoma antigen recognized by T cells 1, Melan-A, including an exogenously obtained form useful in the compositions of the disclosure, has the following sequence (UNIPROT Q16655): 
MPREDAHFIYGYPKKGHGHSYTTAEEAAGIGILTVILGVLLLIGCWYCRRRNGYRALMD
KSLHVGTQCALTRRCPQEGFDHRDSKVSLQEKNCEPVVPNAPPAYEKLSAEQSPPPY
SP (SEQ ID NO:14).
   ∘ Tyrosinase, including an exogenously obtained form useful in the compositions of the disclosure, has the following sequence (UNIPROT P14679): 









  (SEQ ID NO:15).
   ∘ Melanocyte protein PMEL, gp100, including an exogenously obtained form useful in the compositions of the disclosure, has the following sequence (UNIPROT P40967): 
MDLVLKRCLLHLAVIGALLAVGATKVPRNQDWLGVSRQLRTKAWNRQLYPEWTEAQR
LDCWRGGQVSLKVSNDGPTLIGANASFSIALNFPGSQKVLPDGQVIWVNNTIINGSQV
WGGQPVYPQETDDACIFPDGGPCPSGSWSQKRSFVYVWKTWGQYWQVLGGPVSG
LSIGTGRAMLGTHTMEVTVYHRRGSRSYVPLAHSSSAFTITDQVPFSVSVSQLRALDG
GNKHFLRNQPLTFALQLHDPSGYLAEADLSYTWDFGDSSGTLISRALVVTHTYLEPGP
VTAQVVLQAAIPLTSCGSSPVPGTTDGHRPTAEAPNTTAGQVPTTEVVGTTPGQAPTA
EPSGTTSVQVPTTEVISTAPVQMPTAESTGMTPEKVPVSEVMGTTLAEMSTPEATGMT
PAEVSIVVLSGTTAAQVTTTEWVETTARELPIPEPEGPDASSIMSTESITGSLGPLLDGT
ATLRLVKRQVPLDCVLYRYGSFSVTLDIVQGIESAEILQAVPSGEGDAFELTVSCQGGL
PKEACMEISSPGCQPPAQRLCQPVLPSPACQLVLHQILKGGSGTYCLNVSLADTNSLA
VVSTQLIMPGQEAGLGQVPLIVGILLVLMAVVLASLIYRRRLMKQDFSVPQLPHSSSHW
LRLPRIFCSCPIGENSPLLSGQQV (SEQ ID NO:16).
- In myasthenia gravis, a main antigen is acetylcholine receptor.
- In pemphigus vulgaris and variants, main antigens are desmoglein 3, 1 and 4; other antigens include pemphaxin, desmocollins, plakoglobin, perplakin, desmoplakins, and acetylcholine receptor.
- In bullous pemphigoid, main antigens include BP180 and BP230; other antigens include plectin and laminin 5.
- In dermatitis herpetiformis Duhring, main antigens include endomysium and tissue transglutaminase.
- In epidermolysis bullosa acquisita, a main antigen is collagen VII.
- In systemic sclerosis, main antigens include matrix metalloproteinase 1 and 3, the collagen-specific molecular chaperone heat-shock protein 47, fibrillin-1, and PDGF receptor; other antigens include Scl-70, U1 RNP, Th/To, Ku, Jo1, NAG-2, centromere proteins, topoisomerase I, nucleolar proteins, RNA polymerase I, II and III, PM-Sic, fibrillarin, and B23.
- In mixed connective tissue disease, a main antigen is U1snRNP.
- In Sjogren's syndrome, the main antigens are nuclear antigens SS-A and SS-B; other antigens include fodrin, poly(ADP-ribose) polymerase and topoisomerase, muscarinic receptors, and the Fc-gamma receptor IIIb.
- In systemic lupus erythematosus, main antigens include nuclear proteins including the "Smith antigen," SS-A, high mobility group box 1 (HMGB1), nucleosomes, histone proteins and double-stranded DNA (against which auto-antibodies are made in the disease process).
- In Goodpasture's syndrome, main antigens include glomerular basement membrane proteins including collagen IV.
- In rheumatic heart disease, a main antigen is cardiac myosin.
- In autoimmune polyendocrine syndrome type 1 antigens include aromatic L-amino acid decarboxylase, histidine decarboxylase, cysteine sulfinic acid decarboxylase, tryptophan hydroxylase, tyrosine hydroxylase, phenylalanine hydroxylase, hepatic P450 cytochromes P4501A2 and 2A6, SOX-9, SOX-10, calcium-sensing receptor protein, and the type 1 interferons interferon alpha, beta and omega.
- In neuromyelitis optica, a main antigen is AQP4.
   ∘ Aquaporin-4, including an exogenously obtained form useful in the compositions of the disclosure, has the following sequence (UNIPROT P55087): 
MSDRPTARRWGKCGPLCTRENIMVAFKGVWTQAFWKAVTAEFLAMLIFVLLSLGSTIN
WGGTEKPLPVDMVLISLCFGLSIATMVQCFGHISGGHINPAVTVAMVCTRKISIAKSVFY
IAAQCLGAIIGAGILYLVTPPSVVGGLGVTMVHGNLTAGHGLLVELIITFQLVFTIFASCDS
KRTDVTGSIALAIGFSVAIGHLFAINYTGASMNPARSFGPAVIMGNWENHWIYWVGPIIG
AVLAGGLYEYVFCPDVEFKRRFKEAFSKAAQQTKGSYMEVEDNRSQVETDDLILKPGV
VHVIDVDRGEEKKGKDQSGEVLSSV (SEQ ID NO:17).
- In uveitis, main antigens include Retinal S-antigen or "S-arrestin" and interphotoreceptor retinoid binding protein (IRBP) or retinol-binding protein 3.
   ∘ S-arrestin, including an exogenously obtained form useful in the compositions of the disclosure, has the following sequence (UNIPROT P10523): 







 (SEQ ID
NO:18).
   ∘ IRBP, including an exogenously obtained form useful in the compositions of the disclosure, has the following sequence (UNIPROT P10745):























 (SEQ ID NO:19).

In the embodiments where the antigen is a foreign antigen against which an unwanted immune response can be developed, such as food antigens, specific antigens can be:
- from peanut: conarachin (Ara h 1), allergen II (Ara h 2), arachis agglutinin, conglutin (Ara h 6);
   ∘ conarachin, for example has the sequence identified as UNIPROT Q6PSU6
- from apple: 31 kda major allergen/disease resistance protein homolog (Mal d 2), lipid transfer protein precursor (Mal d 3), major allergen Mal d 1.03D (Mal d 1);
- from milk: a-lactalbumin (ALA), lactotransferrin; from kiwi: actinidin (Act c 1, Act d 1), phytocystatin, thaumatin-like protein (Act d 2), kiwellin (Act d 5);
- from egg whites: ovomucoid, ovalbumin, ovotransferrin, and lysozyme;
- from egg yolks: livetin, apovitillin, and vosvetin;
- from mustard: 2S albumin (Sin a 1), 11S globulin (Sin a 2), lipid transfer protein (Sin a 3), profilin (Sin a 4);
- from celery: profilin (Api g 4), high molecular weight glycoprotein (Api g 5);
- from shrimp: Pen a 1 allergen (Pen a 1), allergen Pen m 2 (Pen m 2), tropomyosin fast isoform;
- from wheat and/or other cereals: high molecular weight glutenin, low molecular weight glutenin, alpha-, gamma- and omega-gliadin, hordein, secalin and/or avenin;
   ∘ peptides/epitopes useful in the compositions of the disclosure for treating Celiac Disease include some or all of the following sequences, individually in a composition of Formula 1 or together in a cocktail of compositions of Formula 1:
      ▪ DQ-2 relevant, Alpha-gliadin "33-mer" native: LQLQPFPQPQLPYPQPQLPYPQPQLPYPQPQPF (SEQ ID NO:20)
      ▪ DQ-2 relevant, Alpha-gliadin "33-mer" deamidated: LQLQPFPQPELPYPQPELPYPQPELPYPQPQPF (SEQ ID NO:21)
      ▪ DQ-8 relevant, Alpha-gliadin: QQYPSGQGSFQPSQQNPQ (SEQ ID NO:22)
      ▪ DQ-8 relevant, Omega-gliadin (wheat, U5UA46): QPFPQPEQPFPW (SEQ ID NO:23)
- from strawberry: major strawberry allergy Fra a 1-E (Fra a 1); and
- from banana: profilin (Mus xp 1).

In the embodiments where the antigen is a foreign antigen against which an unwanted immune response is developed, such as to animal, plant and environmental antigens, specific antigens can, for example, be: cat, mouse, dog, horse, bee, dust, tree and goldenrod, including the following proteins or peptides derived from:
- weeds, (including ragweed allergens amb a 1, 2, 3, 5, and 6, and Amb t 5; pigweed Che a 2 and 5; and other weed allergens Par j 1, 2, and 3, and Par o 1);
- grass (including major allergens Cyn d 1, 7, and 12; Dac g 1, 2, and 5; Hol I 1.01203; Lol p 1, 2, 3, 5, and 11; Mer a 1; Pha a 1; Poa p 1 and 5);
- pollen from ragweed and other weeds (including curly dock, lambs quarters, pigweed, plantain, sheep sorrel, and sagebrush), grass (including Bermuda, Johnson, Kentucky, Orchard, Sweet vernal, and Timothy grass), and trees (including catalpa, elm, hickory, olive, pecan, sycamore, and walnut);
- dust (including major allergens from species *Dermatophagoides pteronyssinus,* such as Der p 1, 2, 3, 4, 5, 6, 7, 8, 9, 10, 11, 14, 15, 18, 20, 21, and 23; from species *Dermatophagoides farina,* such as Der f 1, 2, 3, 6, 7, 10, 11, 13, 14, 15, 16, 18, 22, and 24; from species *Blomia tropicalis* such as Blo 11, 2, 3, 4, 5, 6, 10, 11, 12, 13, 19, and 21; also allergens Eur m 2 from *Euroglyphus maynei,* Tyr p 13 from *Tyrophagus putrescentiae,* and allergens Bla g 1, 2, and 4; Per a 1, 3, and 7 from cockroach);
- pets (including cats, dogs, rodents, and farm animals; major cat allergens include Fel d 1 through 8, cat IgA, BLa g 2, and cat albumin; major dog allergens include Can f 1 through 6, and dog albumin);
- bee stings, including major allergens Api m 1 through 12; and
- fungus, including allergens derived from, species of *Aspergillus* and *Penicillium,* as well as the species *Alternaria alternata, Davidiella tassiana,* and *Trichophyton rubrum.*

As will be appreciated by those skilled in the art, a patient can be tested to identify an antigen against which an unwanted immune response has developed, and a protein, peptide or the like can be developed based on that antigen and incorporated as X in a composition of the present disclosure.

### Sialated Antigens, Antibodies, Antibody Fragments

Following are non-limiting examples of antigens, antibodies, antibody fragments having sialylation that can be removed to leave glycosylation specifically targeting the ASGPR: follicle stimulating hormone (FSH), human chorionic gonadotropin (HCG), luteinizing hormone (LH), osteopontin, thyroid stimulating hormone (TSH), agalsidase alfa, agalsidase beta (FABRAZYME^{®}; Genzyme), epoetin alfa and epoetin beta, follitropin alfa (GONAL-F^{®}; Merck/Serono) and follitropin beta (FOLLISTIM^{®}; Schering-Plough), insulin growth factor binding protein 6 (IGFBP-6), lutropin alfa (LUVERISO; Merck/Serono), transforming growth factor β1, antithrombin (ATryn^{®}/TROMBATE-III^{®}; Genzyme/Talecris Biotherapeutics), thyrotropin alfa (THYROGEN^{®}; Genzyme), lenograstim, sargramostim (LEUKINE^{®}; Genzyme), interleukin-3, prourokinase, lymphotoxin, C1-esterase inhibitor (Berinert^{®}; CSL), IgG-like antibodies, interferon beta, coagulation factor Vlla (NOVOSEVEN^{®}; Novo Nordisk), coagulation factor VIII (moroctocog alfa), coagulation factor IX (nonacog alfa) (BENEFIX^{®}; Wyeth), and the p55 tumor necrosis receptor fusion protein. (See: Byrne et al., Drug Discovery Today, Vol 12, No. 7/8, pages 319-326, Apr. 2007 and Sola et al., BioDrugs. 2010; 24(1): 9-21). Pharmaceutically relevant proteins that have previously been hyperglycosylated and can be desialylated for hepatocyte-ASGPR targeting include: interferon alfa and gamma, luteinizing hormone, Fv antibody fragments, asparaginase, cholinesterase, darbepoetin alfa (AraNESP^{®}; Amgen), thrombopoietin, leptin, FSH, IFN-α2, serum albumin, and corifollitropin alfa.

Proteins with glycans that do not normally terminate in sialic acids, including proteins produced in bacteria or yeast (such as arginase, some insulins, and uricase) would not be amenable to desialylation.

Those skilled in the art will appreciate that publicly available references, such as UNIPROT, disclose the presence and location of sites for desialylation on most if not all antigens, antibodies, antibody fragments and ligands of interest.

### Antibodies and Peptide Ligands

In the embodiments employing an antibody, antibody fragment or ligand, such moieties are chosen to specifically bind a targeted circulating protein or peptide or antibody, and result in hepatic uptake of the circulating targeted moiety, possibly as an adduct with the targeting moiety, ultimately resulting in the clearance and inactivation of the circulating targeted moiety. For example, liver-targeted Factor VIII will bind and clear circulating anti-Factor VIII antibodies. Procedures for the identification of such moieties will be familiar to those skilled in the art.

### Linkers

The linkers employed in the compositions of the present disclosure ("Y" in Formula 1) can include N-hydroxysuccinamidyl linkers, malaemide linkers, vinylsulfone linkers, pyridyl di-thiol-poly(ethylene glycol) linkers, pyridyl di-thiol linkers, n-nitrophenyl carbonate linkers, NHS-ester linkers, nitrophenoxy polyethylene glycol)ester linkers and the like.

One particular group of linkers comprises linkers of Formula Y'-CMP below (where Y' indicates the remaining portion of the linker and R⁹ and Z are as defined). More particularly, in the group of linkers including Formula Y'-CMP, in several embodiments the R⁹ substituent is an ethylacetamido group, and even more particularly the ethylacetamido is conjugated with C1 of N-acetylgalactosamine or N-acetylglucosamine.

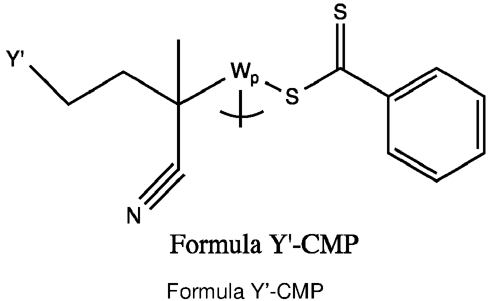

Di-thiol-containing linkers, particularly disulfanylethyl carbamate-containing linkers (named including a free amine of X, otherwise named a "disulfanyl ethyl ester" without including the free amine of X) are particularly advantageous in the present compositions as having the ability to cleave and release an antigen in its original form once inside a cell, for example as illustrated below (where Y' indicates the remaining portion of the linker and X' and Z are as defined).

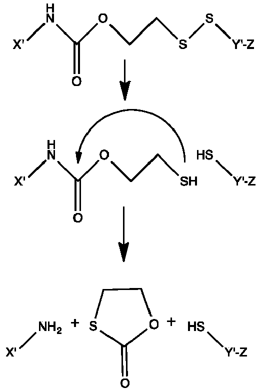

Particularly with regard to the linkers illustrated below in Formula Ya through Formula Yp:
the left bracket "(" indicates the bond between X and Y;
the right or bottom bracket ")" indicates the bond between Y and Z;
n is an integer representing a mixture including from about 1 to 100, particularly about 8 to 90 (e.g., 7, 8, 9, 10, 11, 12, 13, 14, 15, 16, 17, 18, 19, 20, 25, 30, 35, 40, 45, 50, 55, 60, 65 70, 75, 80, 85, 90 or 95), more particularly about 40 to 80 (e.g., 39, 40, 43, 45, 46, 48, 50, 52, 53, 55, 57, 60, 62, 65, 66, 68, 70, 73, 75, 78, 80 or 81) ethylene glycol groups, where the mixture typically encompasses the integer specified as n ±10%;
p is an integer representing a mixture including from about 2 to 150, particularly about 20 to 100 (e.g., 18, 19, 20, 25, 30, 35, 40, 45, 50, 55, 60, 65 70, 75, 80, 85, 90, 95, 100 or 105) and more particularly about 30 to 40 (e.g., 28, 29, 30, 31, 32, 33, 34, 35, 36, 37, 38, 39, 40, 41, 42, 43 or 44), where the mixture typically encompasses the integer specified as p ±10%;
q is an integer representing a mixture including from about 1 to 44, particularly about 3 to 20 (e.g., 3, 4, 5, 6, 7, 8, 9, 10, 11, 12, 13, 14, 15, 16, 17, 18, 19, 20, 21 or 22) and more particularly about 4 to 12 (e.g., 4, 5, 6, 7, 8, 9, 10, 11, 12 or 13), where the mixture typically encompasses the integer specified as q ±10%; and
R⁸ is -CH₂- ("methyl") or -CH₂-CH₂-C(CH₃)(CN)- ("1-cyano-1-methyl-propyl" or "CMP").
Y' represents the remaining portion of Y (e.g., HS-PEG); and
W represents a polymer of the same W¹ group, or W is a copolymer (preferably a random copolymer) of the same or different W¹ and W² groups, where: 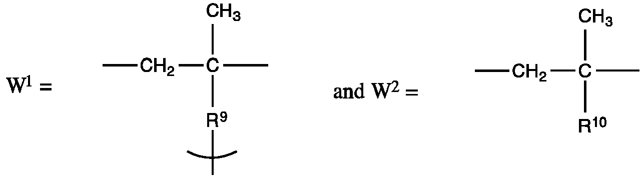 where:
   p is an integer from 2 to about 150;
   R⁹ is a direct bond, -CH₂-CH₂--NH-C(O)- (i.e., an ethylacetamido group or "EtAcN") or -CH₂-CH₂-(O-CH₂-CH₂)ₜ-NH-C(O)- (i.e., a pegylated ethylacetamido group or "Et-PEGₜ-AcN")
   t is an integer from 1 to 5, (particularly 1 to 3, and more particularly 1 or 2); and
   R¹⁰ is an aliphatic group, an alcohol or an aliphatic alcohol, particularly N-(2-hydroxypropyl)methylacrylamide; and
   Z (not shown) is galactose, glucose, galactosamine, glucosamine, N-acetylgalactosamine or N-acetylglucosamine.

### Formulae Ya through Yp

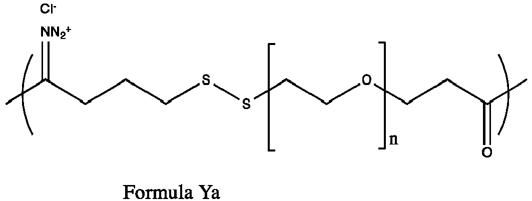

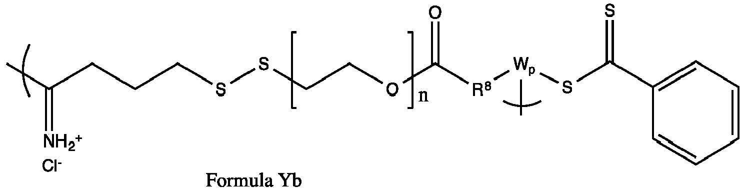

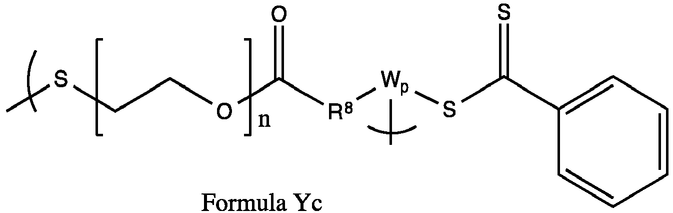

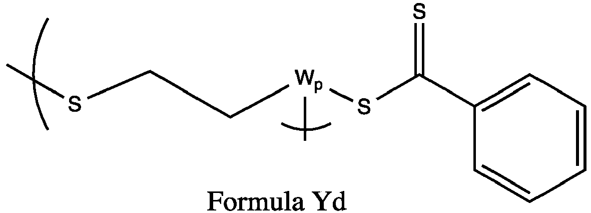

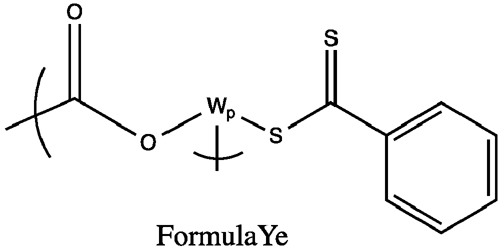

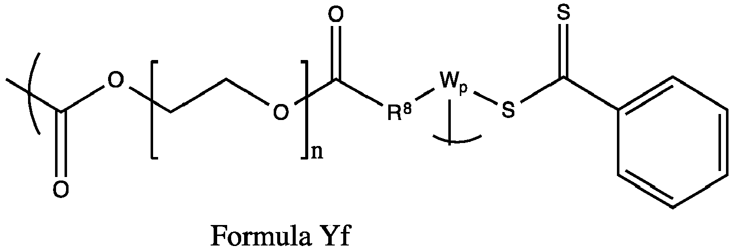

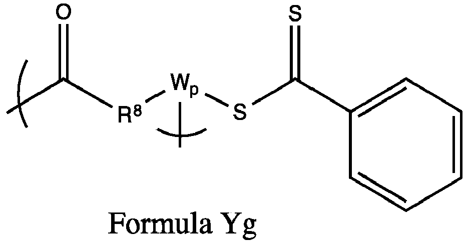

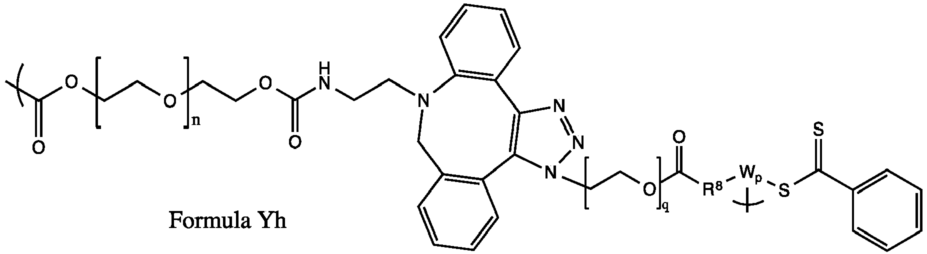

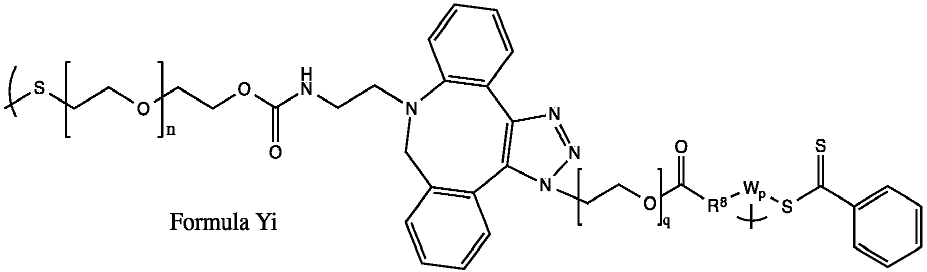

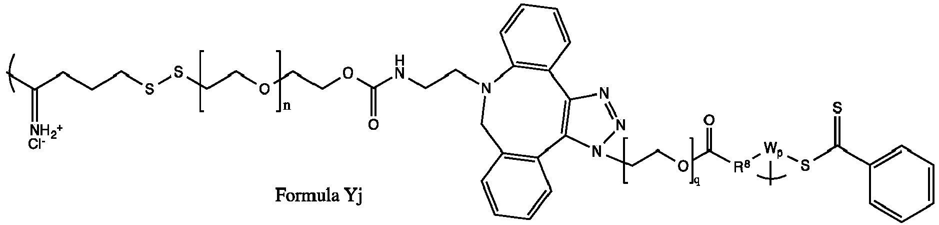

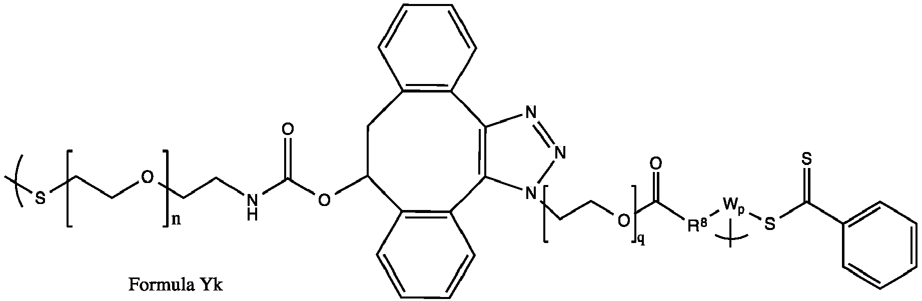

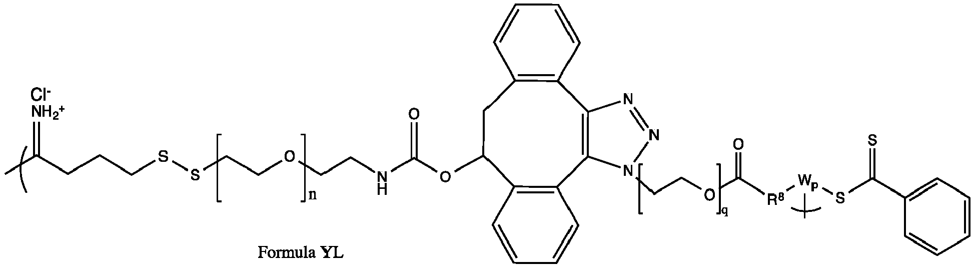

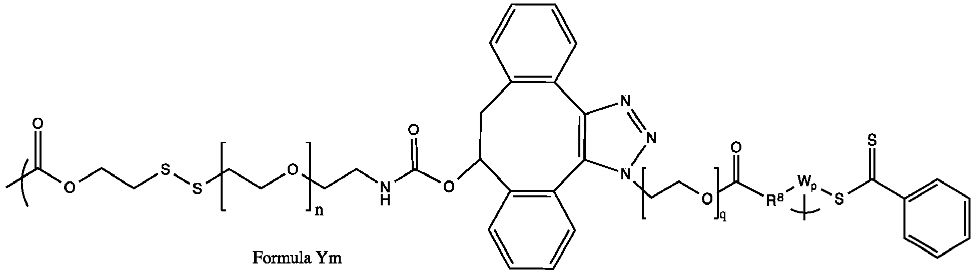

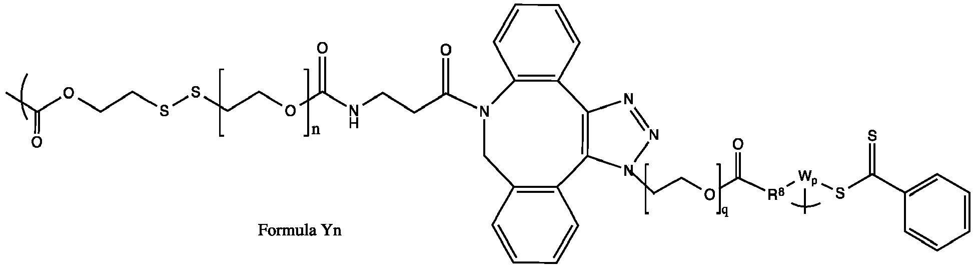

(Linkers of Formula Yn can be synthesized via certain precursors that render Yn particularly suitable for conjugation to hydrophobic antigens.) 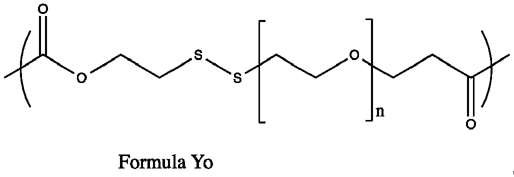 and

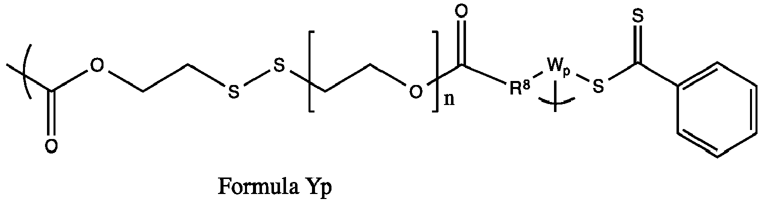

The linkers shown above as Formulae Yh through Yn are synthesized as isomers that are employed without separation. For example, the linkers of Formulae Yh, Yi, Yj and Yn will be a mixture of the 8,9-dihydro-**1*****H**-*dibenzo[*b,f*][1,2,3]triazolo[4,5-*d*]azocin-8yl and 8,9-dihydro-3***H-***dibenzo[*b,f*][1,2,3]triazolo[4,5-*d*]azocin-8yl structures illustrated below: 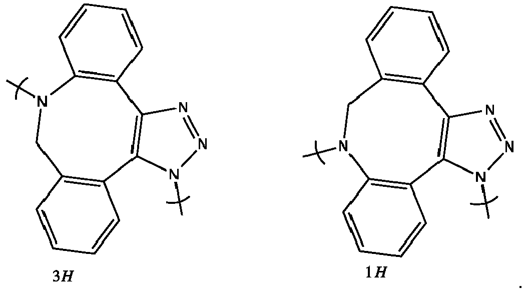 The linkers of Formulae Yk, YL and Ym will be a mixture of the 8,9-dihydro-1*H-*dibenzo[3,4:7,8]cycloocta[1,2-*d*][1,2,3]triazol-**8-yl** and 8,9-dihydro-1*H*-dibenzo[3,4:7,8]cycloocta[1,2-*d*][1,2,3]triazol-**9**-**yl** structures illustrated below: 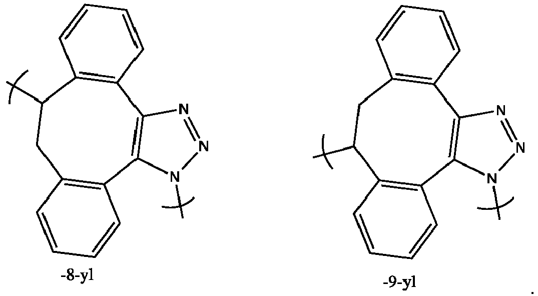 The presence of such isomeric mixtures does not impair the functionality of the compositions employing such linkers.

### Liver-targeting Moieties

The galactosylating moieties employed in the compositions of the present disclosure serve to target the compositions to liver cells (for example, specifically binding hepatocytes) and can be selected from: galactose, galactosamine or N-acetylgalactosamine. The glucosylating moieties employed in the compositions of the present disclosure serve to target the compositions to liver cells (for example, specifically binding hepatocytes or LSECs) and can be selected from: glucose, glucosamine or N-acetylglucosamine. It has been reported that ASGPR affinity can be retained while modifying either side of galactose's C3/C4 -diol anchor (Mamidyala, Sreeman K., et al., J. Am. Chem. Soc. 2012, 134, 1978-1981), therefore the points of conjugation used in several embodiments are particularly at C1, C2 and C6.

Particular liver-targeting moieties include galactose or glucose conjugated at C1 or C6, galactosamine or glucosamine conjugated at C2, and N-acetylgalactosamine or N-acetylglucosamine conjugated at C6. Other particular liver-targeting moieties include N-acetylgalactosamine or N-acetylglucosamine conjugated at C2, more particularly conjugated to a linker bearing an R⁹ substituent that is CH₂. Still other particular liver-targeting moieties include galactose, galactosamine, N-acetylgalactosamine, glucose, glucosamine or N-acetylglucosamine conjugated at C1, more particularly conjugated to a linker bearing an R⁹ substituent that is an ethylacetamido group.

### Nomenclature

The compositions of Formula 1 can be named using a combination of IUPAC and trivial names. For example, a compound corresponding to Formula 1 where X is a cyclobutyl moiety (shown instead of an antigen for illustrative purposes), Y is Formula Ya, m is 1, n is 4 and Z is N-acetylgalactosamin-2-yl or N-acetylglucosamin-2-yl: 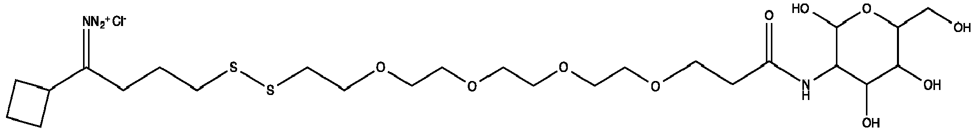 can be named (*Z*)-(21-cyclobutyl-1-oxo-1-((2,4,5-trihydroxy-6-(hydroxymethyl)tetrahydro-2*H*-pyran-3-yl)amino)-4,7,10,13-tetraoxa-16,17-dithiahenicosan-21-ylidene)triaz-1-yn-2-ium chloride, so the corresponding composition of the disclosure where X is tissue transglutaminase can be named (*Z*)-(21-(tissue transglutaminase)-1-oxo-1-((2,4,5-trihydroxy-6-(hydroxymethyl)tetrahydro-2*H*-pyran-3-yl)amino)-4,7,10,13-tetraoxa-16,17-dithiahenicosan-21-ylidene)triaz-1-yn-2-ium chloride. The corresponding composition of the disclosure where X' is tissue transglutaminase, m is 2, n is 4 and Z' is N-acetylgalactosamin-2-yl or N-acetylglucosamin-2-yl can be named (3Z)-((tissue transgultaminase)-1,3-diylbis(1-oxo-1-((2,4,5-trihydroxy-6-(hydroxymethyl)tetrahydro-2*H*-pyran-3-yl)amino)-4,7,10,13-tetraoxa-16,17-dithiahenicosan-21-yl-21-ylidene))bis(triaz-1-yn-2-ium) chloride.

In the interest of simplification, the compositions of Formula 1 can be named using an alternative naming system by reference to X and correspondence to one of Formulae 1a to 1p (as illustrated in the reaction schemes) followed by recitation of the integers for variables m, n, p and/or q, R⁸, R⁹ and identification of the galactosylating moiety and the position at which it is conjugated. In some embodiments, the compounds where W is a copolymer are designated by the letter of the "Y group" followed by a "prime" (e.g., F1c') and include the number and an identification of the comonomers. Under this system, the composition of Formula 1a where X is ovalbumin, m is 2, n is 4 and Z is N-acetylgalactosamin-2-yl can be named "F1a-OVA-m₂-n₄-2NAcGAL." The corresponding composition of Formula 1a where X is ovalbumin, m is 2, n is 4 and Z is N-acetylglucosamin-2-yl can be named "F1a-OVA-m₂-n₄-2NAcGLU."

Similarly, the following composition of Formula 1 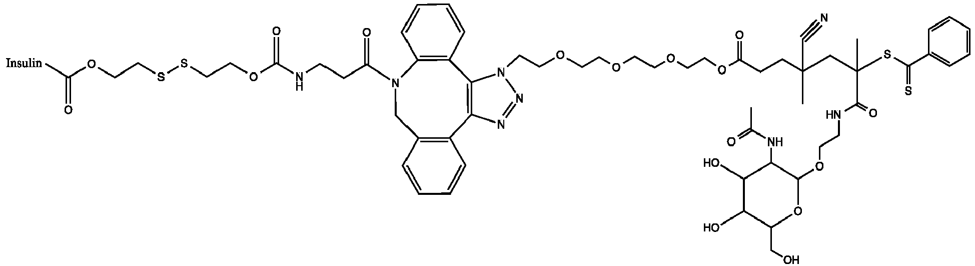 can be named "2-((2-(((3-(**3**-(22-((3-acetamido-4,5-dihydroxy-6-(hydroxymethyl)tetrahydro-2*H*-pyran-2-yl)oxy)-16-cyano-16,18-dimethyl-13,19-dioxo-18-((phenylcarbonothioyl)thio)-3,6,9,12-tetraoxa-20-azadocosyl)-**3,9-dihydro**-8*H-*dibenzo[*b*,*f*][1,2,3]triazolo[4,5-*d*]azocin-8-yl)-3-oxopropyl)carbamoyl)oxy)ethyl)disulfanyl)ethyl insulin carboxylate." The isomer: 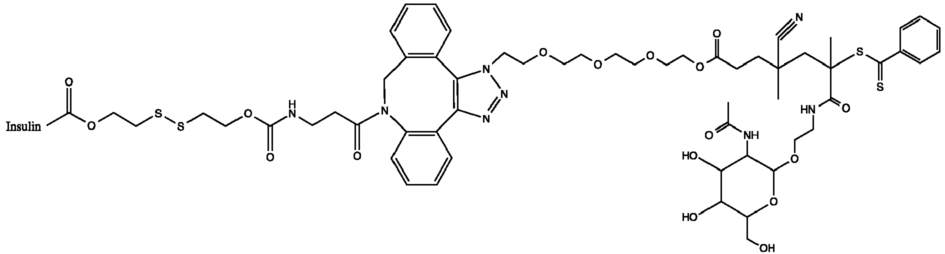 can be named "2-((2-(((3-(**1**-(22-((3-acetamido-4,5-dihydroxy-6-(hydroxymethyl)tetrahydro-2*H-*pyran-2-yl)oxy)-16-cyano-16,18-dimethyl-13,19-dioxo-18-((phenylcarbonothioyl)thio)-3,6,9,12-tetraoxa-20-azadocosyl)-**1,9-dihydro**-8*H-*dibenzo[*b*,*f*][1,2,3]triazolo[4,5-*d*]azocin-8-yl)-3-oxopropyl)carbamoyl)-oxy)ethyl)disulfanyl)ethyl insulin carboxylate" (bold lettering highlights added for convenience in identifying the difference between the formal names). Employing the naming system adopted for the present disclosure, both isomers can be named "F1n-insulin-m₁-n₁-p₁-q₄-CMP-EtAcN-1NAcGAL" (or ""F1 n-insulin-m₁-n₁-p₁-q₄-CMP-EtAcN-1NAcGLU" because no stereochemistry is shown for the sugar ring) where CMP indicates that R⁸ is 1-cyano-1-methyl-propyl, EtAcN indicates that R⁹ is ethylacetamido and 1NAcGAL indicates Z" is N-acetylgalactosamine conjugated at C1. Absence of the abbreviation EtAcN before the designation for Z would indicate that R⁹ is a direct bond.

The following composition of Formula 1 exemplifies compounds where W is a copolymer: 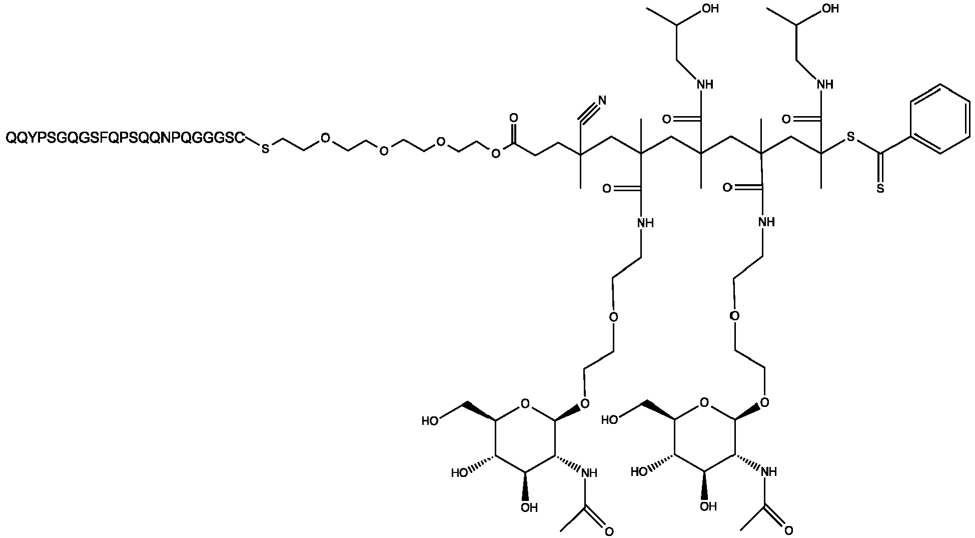 and can be named 2-(2-(2-(2-(QQYPSGQGSFQPSQQNPQGGGSC-sulfanyl)ethoxy)ethoxy)ethoxy)ethyl 6,10-bis((2-(2-(((2*R*,3*R*,4*R*,5*S*,6*R*)-3-acetamido-4,5-dihydroxy-6-(hydroxymethyl)tetrahydro-2*H*-pyran-2-yl)oxy)ethoxy)ethyl)carbamoyl)-4-cyano-13-((2-hydroxypropyl)amino)-8-((2-hydroxypropyl)carbamoyl)-4,6,8,10,12-pentamethyl-13-oxo-12-((phenylcarbonothioyl)thio)tridecanoate. Employing the naming system adopted for the present disclosure the compound can be named "F1c'-DQ8-relevant Alpha Gliadin-m₁-n₄-p₄-CMP-poly-(EtPEG₁AcN-1NAcGLU₂-HPMA₂)".

### Preparation of the Compositions of The Disclosure

The compositions of Formula 1 can be prepared, for example, by adjusting the procedures described in Zhu, L., et al., Bioconjugate Chem. 2010, 21, 2119-2127. Syntheses of certain compositions of Formula 1 are also described below with reference to Reaction Schemes 1 to 14. Other synthetic approaches will be apparent to those skilled in the art.

Formula 101 (below) is an alternative representation of X 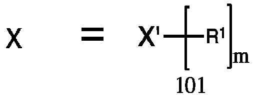 where R¹ is a free surface amino (-NH₂) or thiol (-SH) moiety positioned on X's three-dimensional structure so as to be accessible for conjugation to a linker, and X' represents the remainder of X excluding the identified free amino group(s) [(X" is used in the reaction schemes to represent the remainder of X excluding free thiol group(s)]. Depending upon the identity of X, there will be at least one (the N-terminal amine) and can be multiple R¹ groups (predominantly from lysine residues or cysteine residues that are not involved in disulfide bonding), as represented by m, which is an integer from about 1 to 100, more typically 1 or from about 4 to 20, and most typically 1 to about 10.

Variables employed in the reaction schemes are as defined above, and additionally include the following, which should be understood to have these meanings absent any specific indication otherwise with respect to a particular reaction scheme or step.
- R² is OH or a protecting group;
- R³ is OH, NH₂, NHAc, a protecting group or NH-protecting group;
- R⁴ is OH or a protecting group;
- R⁵ is OH or a protecting group;
- R⁶ is OH or a protecting group;
- Z' is galactose or glucose conjugated at C1 or C6, galactosamine or glucosamine conjugated at C2, or N-acetylgalactosamine conjugated or N-acetylglucosamine at C6;
- R⁸ is -CH₂- or -CH₂-CH₂-C(CH₃)(CN)-; and
- R⁹ is a direct bond and Z" is N-acetylgalactosamine conjugated at C2; or
- R⁹ is an ethylacetamido or a pegylated ethylacetamido group and Z" is galactose, glucose, galactosamine, glucosamine, N-acetylgalactosamine or N-acetylglucosamine conjugated at C1.

### Synthetic Reaction Parameters

The terms "solvent", "inert organic solvent" or "inert solvent" mean a solvent inert under the conditions of the reaction being described in conjunction therewith [including, for example, benzene, toluene, acetonitrile, tetrahydrofuran ("THF"), dimethylformamide ("DMF"), chloroform, methylene chloride (or dichloromethane), diethyl ether, methanol, pyridine and the like]. Unless specified to the contrary, the solvents used in the reactions of the present disclosure are inert organic solvents.

The term "q.s." means adding a quantity sufficient to achieve a stated function, e.g., to bring a solution to the desired volume (i.e., 100%).

Isolation and purification of the compounds and intermediates described herein can be effected, if desired, by any suitable separation or purification procedure such as, for example, filtration, extraction, crystallization, column chromatography, thin-layer chromatography or thick-layer chromatography, centrifugal size exclusion chromatography, high-performance liquid chromatography, recrystallization, sublimation, fast protein liquid chromatography, gel electrophoresis, dialysis, or a combination of these procedures. Specific illustrations of suitable separation and isolation procedures can be had by reference to the examples hereinbelow. However, other equivalent separation or isolation procedures can, of course, also be used.

Unless otherwise specified (including in the examples), all reactions are conducted at standard atmospheric pressure (about 1 atmosphere) and ambient (or room) temperature (about 20°C), at about pH 7.0-8.0.

Characterization of reaction products can be made by customary means, e.g., proton and carbon NMR, mass spectrometry, size exclusion chromatography, infrared spectroscopy, gel electrophoresis.

Reaction Scheme 1 illustrates the preparation of compositions of Formula 1 where Z can be galactose, glucose, galactosamine, glucosamine, N-acetylgalactosamine or N-acetylglucosamine. In that regard and as defined above, Z' as employed in Reaction Scheme 1 encompasses galactose or glucose conjugated at C1 and C6 and corresponding to the following structures according to Formula 1: 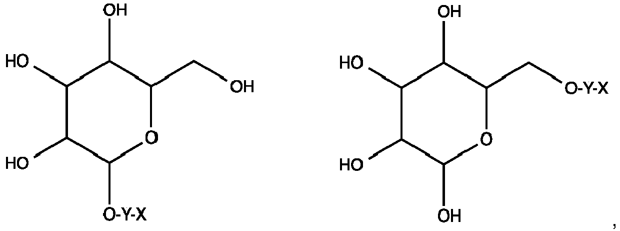 galactosamine or glucosamine conjugated at C2 and corresponding to the following structure according to Formula 1: 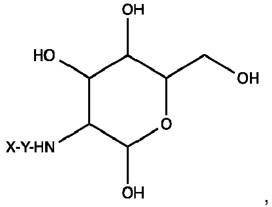 and N-acetylgalactosamine or N-acetylglucosamine conjugated at C6 and corresponding to the following structure according to Formula 1:

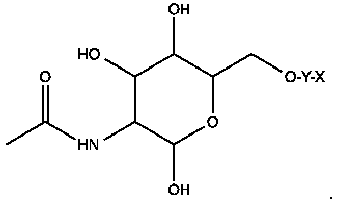

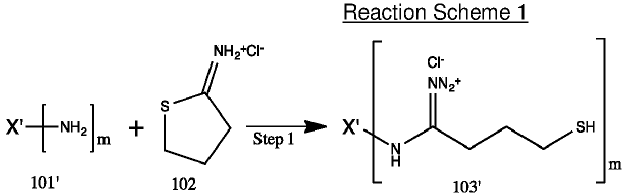

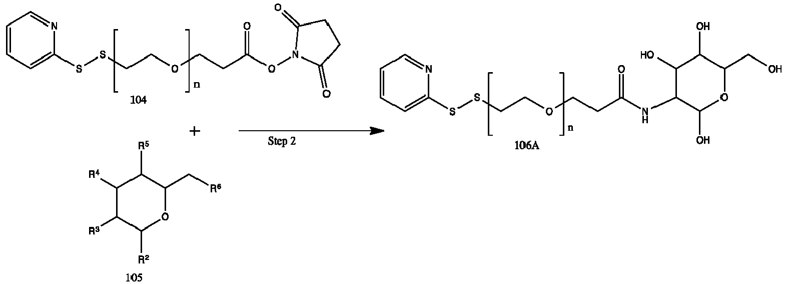

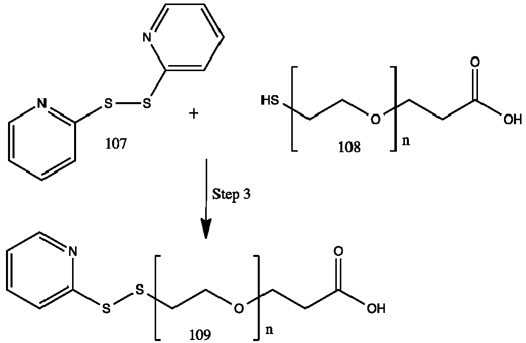

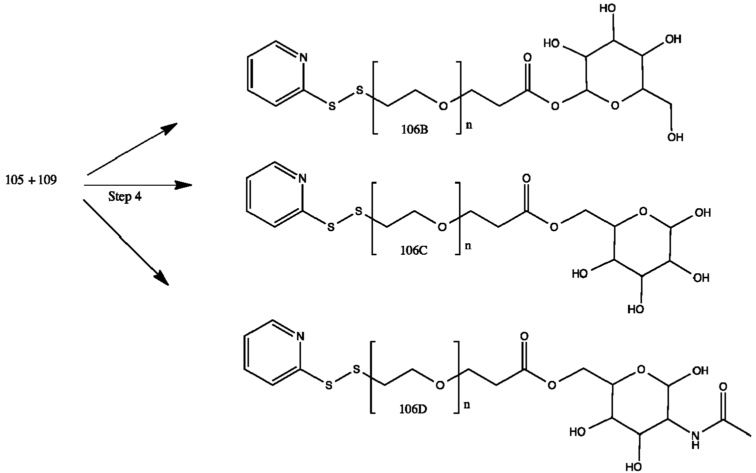

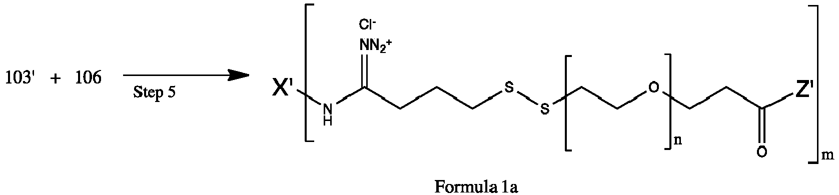

As illustrated above in Reaction Scheme 1, Step 1, surface thiol group(s) can be generated on an antigen, antibody, antibody fragment or ligand having free surface amino group(s) (Formula 101') by contact with a Traut reagent (Formula 102) at a pH of about 8.0 for about 1 hour to give the Formula 103', from which unreacted Traut's reagent is removed, e.g., via centrifugal size exclusion chromatography. The two structures shown below, illustrate the product of Reaction Scheme 1, Step 1, respectively showing the free surface amino group(s) originally found on X (i.e., Formula 103' where X' represents the remainder of X excluding the identified free surface amino groups) and omitting the free surface amino group(s) (i.e., Formula 103). This parallels the distinction illustrated as between X and Formula 101. The convention has been followed in the subsequent reaction schemes.

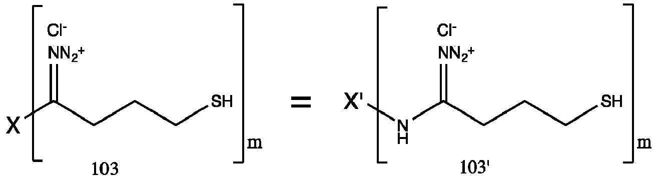

In Reaction Scheme 1, Step 2, a pyridyl di-thiol-poly(ethylene glycol)-NHS ester (Formula 104) is contacted with galactosamine or glucosamine (Formula 105 where R³ is NH₂ and R², R⁴, R⁵ and R⁶ are OH) with stirring at about pH 8 for about 1 hour to give the corresponding pyridyl di-thiol-poly(ethylene glycol)-sugar of Formula 106A, which can be used without further purification.

In Reaction Scheme 1, Step 3, 4,4'-dithiodipyridine (Formula 107) is contacted with a thiol-poly(ethylene glycol)propanoic acid (Formula 108) to give the corresponding pyridyl di-thiol-poly(ethylene glycol)propanoic acid (Formula 109).

In Reaction Scheme 1, Step 4, the acid of Formula 109 is contacted with a protected galactose or N-acetylgalactosamine of Formula 105 where R² is OH and R³, R⁴, R⁵ and R⁶ are protecting groups ("PG"), where R⁶ is OH and R², R³, R⁴ and R⁵ are PG, or where R⁶ is N-acetyl and R², R³, R⁴ and R⁵ are PG to give the corresponding pyridyl di-thiol-poly(ethylene glycol)-sugars of Formulae 106B, 106C and 106D, which can be used following de-protection.

In Reaction Scheme 1, Step 5, to a stirred solution of the product of Step 1 (Formula 103') is added an excess (corresponding to the value of m) of the product of Step 2 or Step 4 (Formula 106, i.e., 106A, 106B, 106C or 106D) for about 1 hour, followed by centrifugal sized exclusion chromatography to remove any free remaining reactants to yield the corresponding product according to Formula 1a, respectively, Formula 1aA, Formula 1aB, Formula 1aC and Formula 1aD.

The compositions corresponding to Formula 1a can be named, respectively, e.g., as follows:
"F1aA-X'-mₘ-nₙ" or "F1 a-X'-mₘ-nₙ-2NGAL"
"F1aB-X'-mₘ-nₙ" or "F1a-X'-mₘ-nₙ-1GAL"
"F1aC-X'-mₘ-nₙ" or "F1a-X'-mₘ-nₙ-6GAL"
"F1aD-X'-mₘ-nₙ" or "F1a-X'-mₘ-nₙ-6NAcGAL"
"F1aA-X'-mₘ-nₙ" or "F1a-X'-mₘ-nₙ-2NGLU"
"F1aB-X'-mₘ-nₙ" or "F1a-X'-mₘ-nₙ-1GLU"
"F1aC-X'-mₘ-nₙ" or "F1a-X'-mₘ-nₙ-6GLU"
"F1aD-X'-mₘ-nₙ" or "F1a-X'-mₘ-nₙ-6NAcGLU"
respectively, for products made employing an intermediate according to Formulae 106A-D.

Reaction Schemes 2-14 illustrate preparation of the compounds where W is a polymer of the same W¹ group. For the purposes of the nomenclature employed therewith, except as expressly stated otherwise, Z" refers to N-acetylgalactosamine or N-acetylglucosamine conjugated at C2: 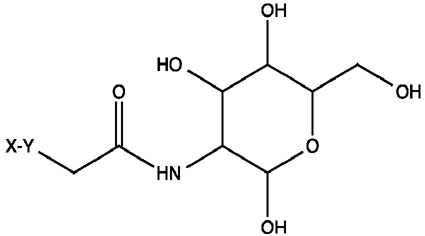 or to galactose, glucose, galactosamine, glucosamine, N-acetylgalactosamine or N-acetylglucosamine conjugated at C1. It should be noted that, according to several embodiments, in order to improve yields, the C1 conjugated compositions can be protected during synthesis, for example by cyclizing the amine with the C3 hydroxyl and de-protecting following incorporation of the protected galactosamine into the adjacent portion of the linker.

The poly(galactose methacrylate) and poly(glucose methacrylate) reactants of Formulae 201, 401, 501, 601, 701, 803 and 1401 can be prepared by methacrylating galactose or glucose, e.g., contacting galactosamine or glucosamine and methacrylate anhydride, followed by reversible addition-fragmentation chain transfer (RAFT) polymerization with a corresponding RAFT agent in the presence of azobisisobutyronitrile (AIBN) in a suitable solvent, starting with freeze-thaw cycles followed by heating at about 60-80°C, preferably 70°C for about 5-8, preferably about 6 hours. The polymer can be precipitated in a lower alkanol, preferably methanol.

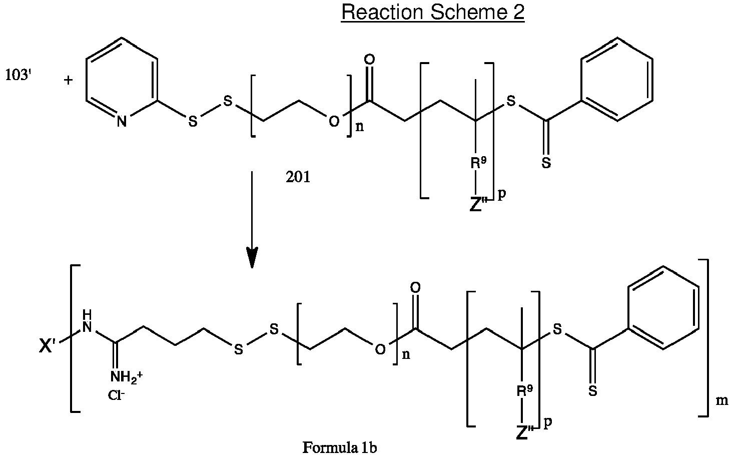

As illustrated in Reaction Scheme 2, an antigen, antibody, antibody fragment or ligand having free surface thiol group(s) prepared, e.g., as described with reference to Reaction Scheme 1, Step 1 (Formula 103') is contacted with an excess (corresponding to the value of m) of a pyridyl di-thiol-poly(ethylene glycol) of Formula 201 for about 1 hour to yield the corresponding product according to Formula 1b.

The compositions of Formula 1b can be named as follows:
"F1b-X'-mₘ-nₙ-pₚ-2NAcGAL" "F1b-X'-mₘ-nₙ-pₚ-2NAcGLU" or "F1b-X'-mₘ-nₙ-pₚ-EtAcN-Z".
For example, the composition of Formula 1b where X' is uricase, m is 1, n is 4, p is 4 and Z" is N-acetylgalactosamine conjugated at C2 can be named "F1b-uricase-m₁-n₄-p₄-2NAcGAL" or "30-(uricase)-3,5,7,9-tetramethyl-12-oxo-1-phenyl-1-thioxo-3,5,7,9-tetrakis((2,4,5-trihydroxy-6-(hydroxymethyl)tetrahydro-2H pyran-3-yl)carbamoyl)-13,16,19,22-tetraoxa-2,25,26-trithiatriacontan-30-iminium".

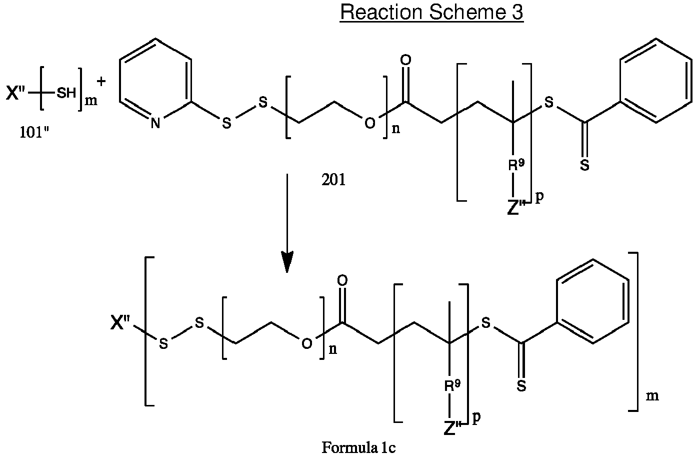

As illustrated in Reaction Scheme 3, an antigen, antibody, antibody fragment or ligand having native free surface thiol group(s) (cysteines) [Formula 101" corresponding to Formula 101 and illustrating where X", as the term will be subsequently employed, represents X excluding the identified free surface thiol group(s)] is contacted with an excess (corresponding to the value of m) of a pyridyl di-thiol-poly(ethylene glycol) of Formula 201 to yield the corresponding product according to Formula 1c.

The compositions corresponding to Formula 1c can be named as follows:
"F1c-X'-mₘ-nₙ-pₚ-2NAcGAL" "F1c-X'-mₘ-nₙ-pₚ-2NAcGLU" or "F1c-X'-mₘ-nₙ-pₚ-EtAcN-Z".

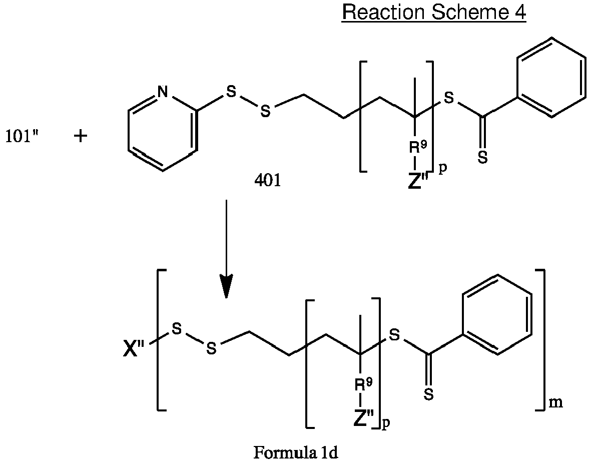

As illustrated in Reaction Scheme 4, an antigen, antibody, antibody fragment or ligand having native free surface thiol group(s) of Formula 101" is contacted with an excess (corresponding to the value of m) of a pyridyl di-thiol of Formula 401 to yield the corresponding product according to Formula 1d.

The compositions corresponding to Formula 1d can be named as follows:
"F1d-X'-mₘ-p_{P}-2NAcGAL" "F1d-X'-mₘ-p_{P}-2NAcGLU" or "F1d-X'-mₘ-pₚ-EtAcN-Z".

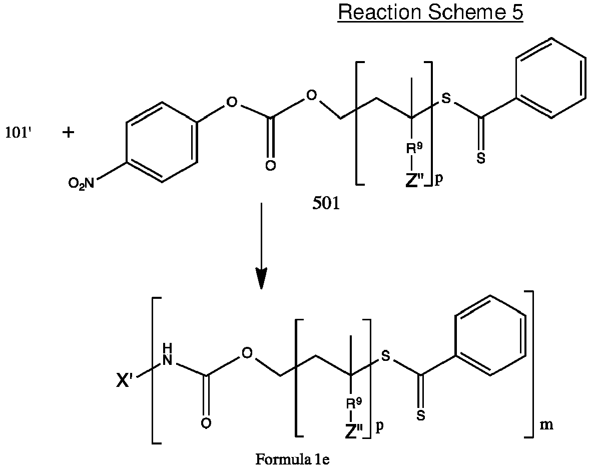

As illustrated in Reaction Scheme 5, an antigen, antibody, antibody fragment or ligand having native free surface amino group(s) of Formula 101' is contacted with an excess (corresponding to the value of m) of a n-nitrophenyl carbonate of Formula 501 to yield the corresponding product according to Formula 1e.

The compositions corresponding to Formula 1e can be named as follows:
"F1e-X'-mₘ-pₚ-2NAcGAL" "F1e-X'-mₘ-pₚ-2NAcGLU" or "F1e-X'-mₘ-pₚ-EtAcN-Z".

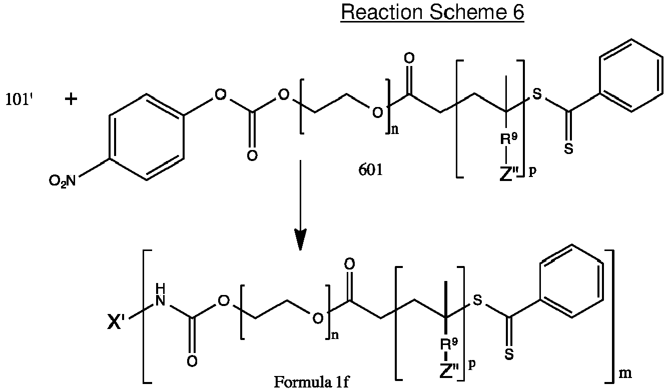

As illustrated in Reaction Scheme 6, an antigen, antibody, antibody fragment or ligand having native free surface amino group(s) of Formula 101' is contacted with an excess (corresponding to the value of m) of a n-nitrophenyl carbonate polyethylene glycol)ester of Formula 601 to yield the corresponding product according to Formula 1f.

The compositions corresponding to Formula 1f can be named as follows:
"F1f-X'-mₘ-nₙ-pₚ-2NAcGAL" "F1f-X'-mₘ-nₙ-pₚ-2NAcGLU" or "F1f-X'-mₘ-nₙ-pₚ-EtAcN-Z".

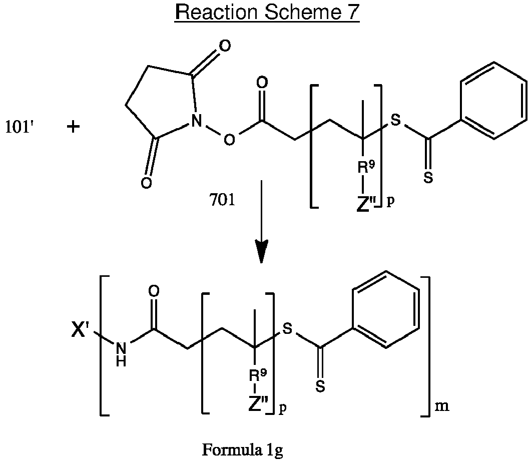

As illustrated in Reaction Scheme 7, an antigen, antibody, antibody fragment or ligand having native free surface amino group(s) of Formula 101' is contacted with an excess (corresponding to the value of m) of a NHS-ester polyethylene glycol)ester of Formula 701 to yield the corresponding product according to Formula 1g.

The compositions corresponding to Formula 1g can be named as follows:
"F1g-X'-mₘ-pₚ-2NAcGAL" "F1g-X'-mₘ-pₚ-2NAcGLU" or "F1g-X'-mₘ-pₚ-EtAcN-Z"

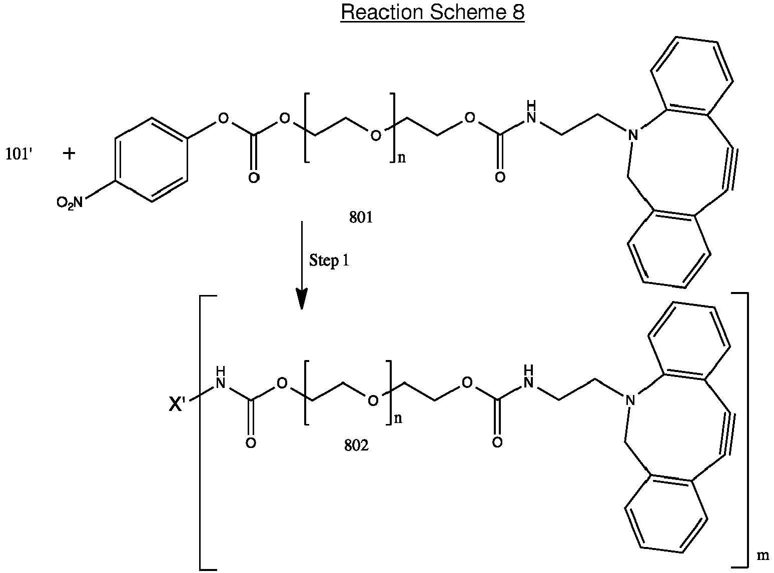

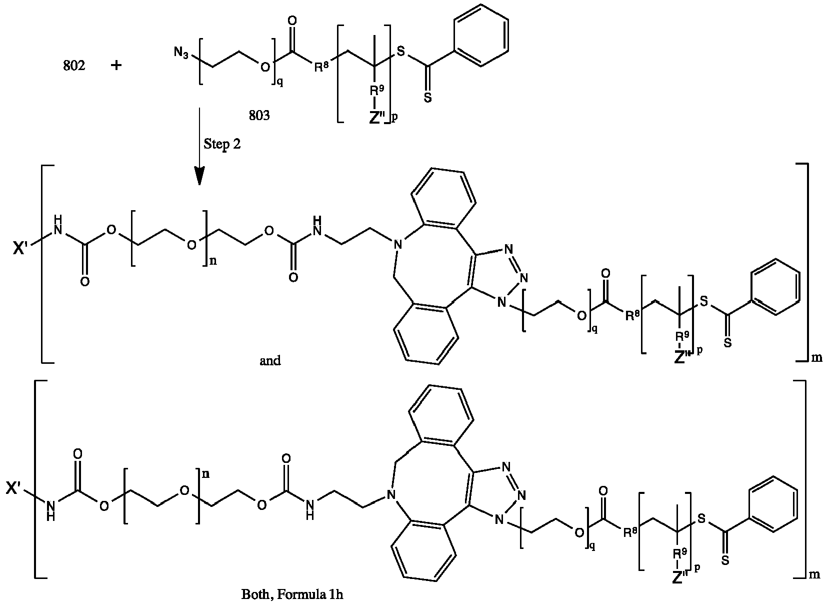

As illustrated in Reaction Scheme 8, Step 1, an antigen, antibody, antibody fragment or ligand having native free surface amino group(s) of Formula 101' is contacted with an excess (corresponding to the value of m) of an amine-reactive linker for Click chemistry of Formula 801 to yield the corresponding product according to Formula 802.

In Reaction Scheme 8, Step 2, the product of Formula 802 is then contacted with an equivalent amount (again corresponding to the value of m) of a galactos(amine) polymer of Formula 803 to yield the corresponding isomeric product according to Formula 1h. The two isomers, illustrated above, result from non-specific cyclization of the azide of Formula 803 with the triple bond of Formula 802. Such non-specific cyclization occurs in the synthesis of other compositions where Y is selected from Formulae Yh through Yn, but will not be illustrated in each instance.

The compositions corresponding to Formula 1h can be named as follows:
"F1h-X'-mₘ-nₙ-pₚ-q_{q}-2NAcGAL" "F1h-X'-mₘ-nₙ-pₚ-q_{q}-2NAcGLU" or "F1h-X'-mₘ-nₙ-pₚ-q_{q}-EtAcN-Z".

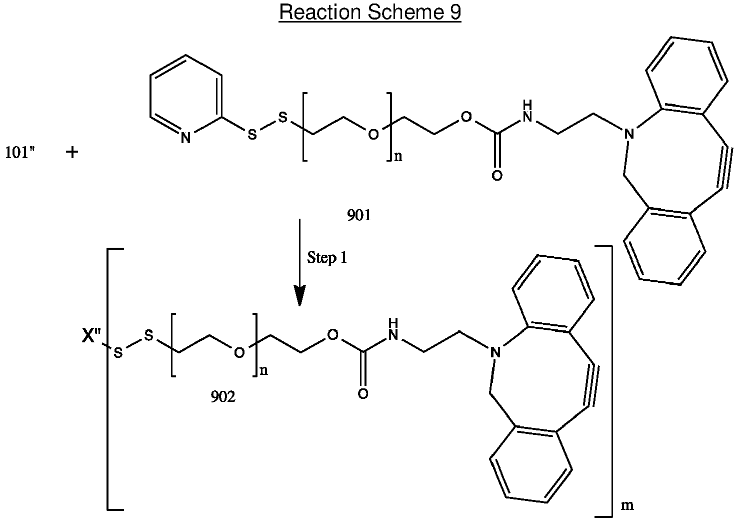

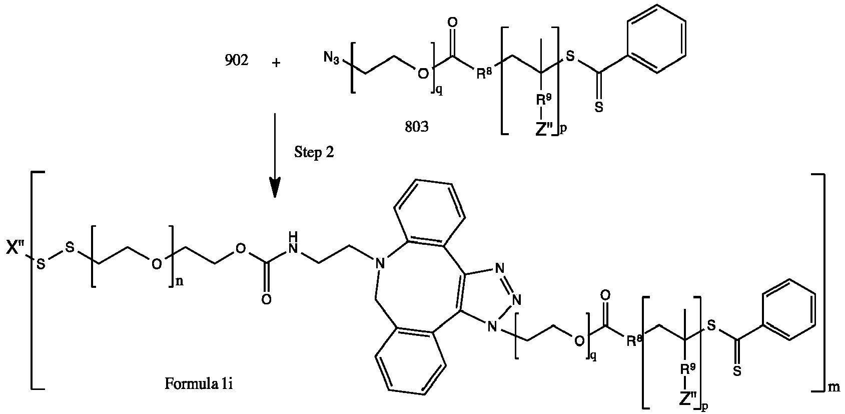

As illustrated in Reaction Scheme 9, Step 1, an antigen, antibody, antibody fragment or ligand having native free surface thiol group(s) of Formula 101" is contacted with an excess (corresponding to the value of m) of a thiol-reactive linker for Click chemistry of Formula 901 to yield the corresponding product according to Formula 902".

In Reaction Scheme 9, Step 2, the product of Formula 902" is then contacted with an equivalent amount (again corresponding to the value of m) of a galactos(amine) polymer of Formula 803 to yield the corresponding isomeric product according to Formula 1i.

The compositions corresponding to Formula 1i can be named as follows:
"F1i-X'-mₘ-nₙ-pₚ-q_{q}-2NAcGAL" "F1i-X'-mₘ-nₙ-pₚ-q_{q}-2NAcGLU" or "F1i-X'-mₘ-nₙ-pₚ-q_{q}-EtAcN-Z".

By following the procedures described with regard to Reaction Scheme 9, but substituting starting material 101" with a compound of Formula 103' (derivatized with the Traut reagent) there is obtained the corresponding isomeric product of Formula 1j as shown below.

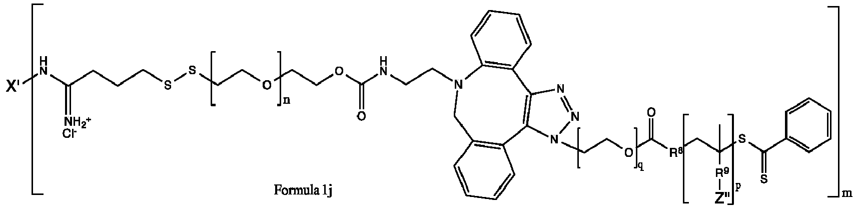

The compositions corresponding to Formula 1j can be named as follows:
"F1j-X'-mₘ-nₙ-pₚ-q_{q}-2NAcGAL" "F1j-X'-mₘ-nₙ-pₚ-q_{q}-2NAcGLU" or "F1j-X'-mₘ-nₙ-pₚ-q_{q}-EtAcN-Z".

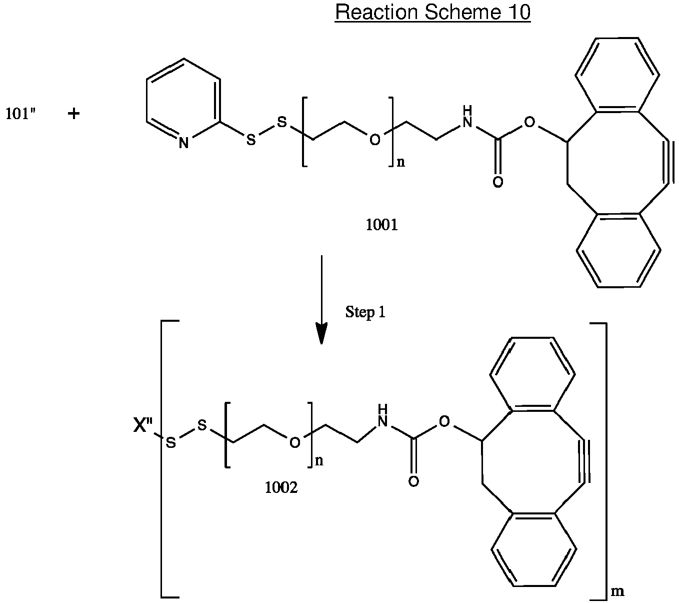

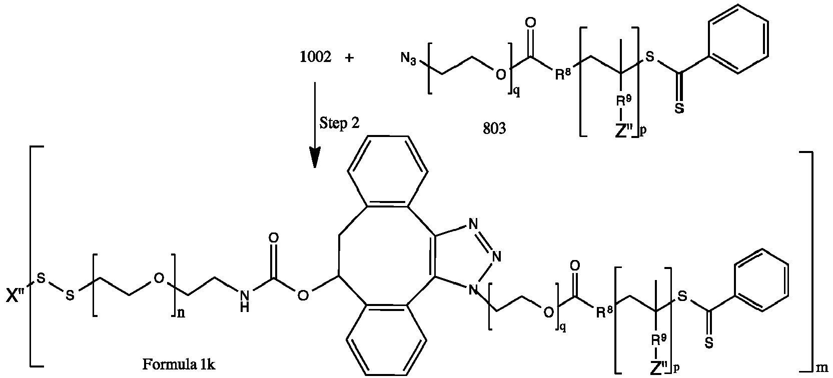

As illustrated in Reaction Scheme 10, Step 1, an antigen, antibody, antibody fragment or ligand having native free surface thiol group(s) of Formula 101" is contacted with an excess (corresponding to the value of m) of a thiol-reactive linker for Click chemistry of Formula 1001 to yield the corresponding product according to Formula 1002.

In Reaction Scheme 10, Step 2, the product of Formula 1002 is then contacted with an equivalent amount (again corresponding to the value of m) of a galactos(amine) polymer of Formula 803 to yield the corresponding isomeric product according to Formula 1k.

The compositions corresponding to Formula 1k can be named as follows:
"F1k-X'-mₘ-nₙ-pₚ-q_{q}-2NAcGAL" "F1k-X'-mₘ-nₙ-pₚ-q_{q}-2NAcGLU" or "F1k-X'-mₘ-nₙ-pₚ-q_{q}-EtAcN-Z".

By following the procedures described with regard to Reaction Scheme 10, but substituting starting material 101" with a compound of Formula 103' (derivatized with the Traut reagent) there is obtained the corresponding isomeric product of Formula 1L as shown below.

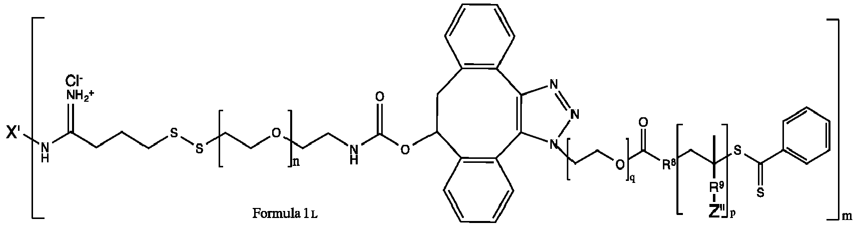

The compositions corresponding to Formula 1L can be named as follows:
"F1L-X'-mₘ-nₙ-pₚ-q_{q}-2NAcGAL" "F1L-X'-mₘ-nₙ-pₚ-q_{q}-2NAcGLU" or "F1L-X'-mₘ-nₙ-pₚ-q_{q}-EtAcN-Z".

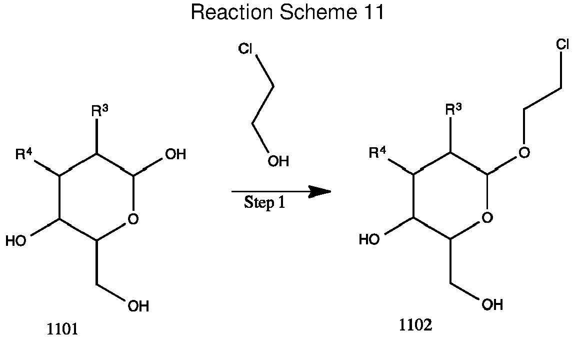

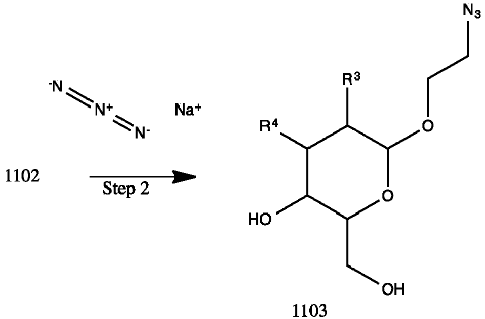

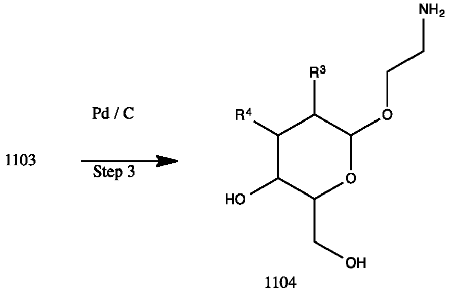

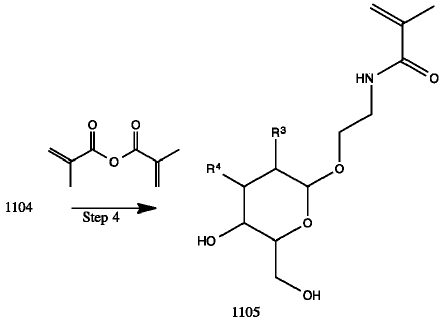

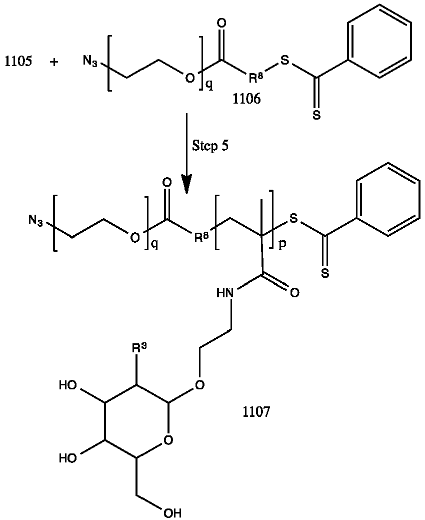

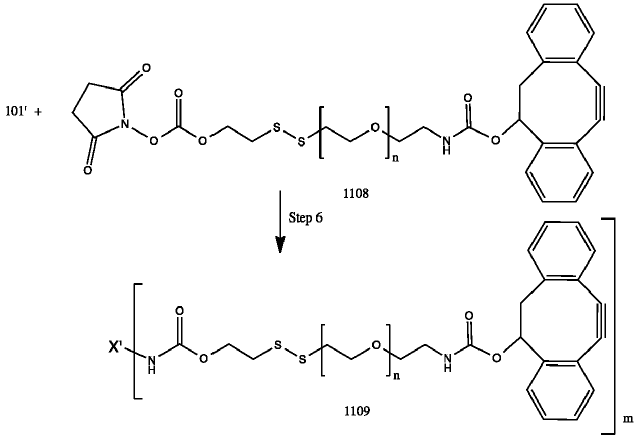

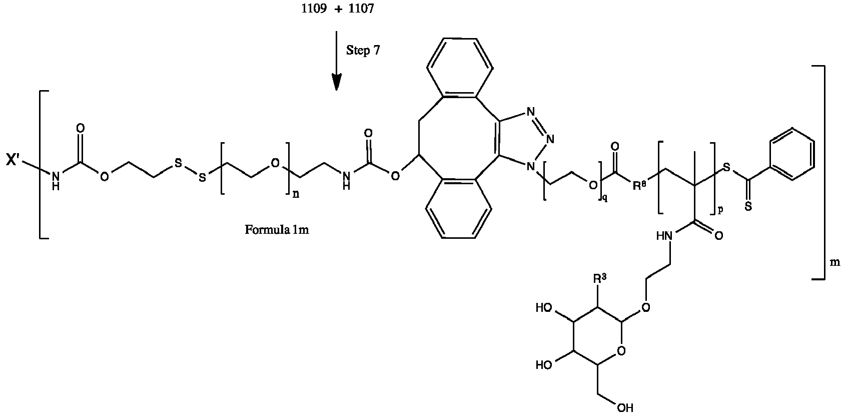

As illustrated in Reaction Scheme 11, Step 1, galactose, protected galactosamine or N-Acetyl-D-galactosamine (Formula1101 where R³ and R⁴ are OH, R³ is NH-protecting group (e.g., cyclized with R⁴) or R³ is NHAc and R⁴ is OH, respectively) is contacted with 2-chloroethan-1-ol followed by cooling and the dropwise addition of acetylchloride. The solution is warmed to room temperature and then heated to 70°C for several hours. Ethanol is added to the crude product and the resulting solution is stirred in the presence of carbon and then filtered followed by solvent removal to yield the corresponding product of Formula 1102.

As illustrated in Reaction Scheme 11, Step 2, the product of Formula 1102 is added to an excess of sodium azide and heated to 90°C for several hours, then filtered followed by solvent removal to yield the corresponding product of Formula 1103.

As illustrated in Reaction Scheme 11, Step 3, the product of Formula 1103 is added to a solution of palladium on carbon and ethanol, and stirred under hydrogen gas (3 atm) for several hours, then filtered followed by solvent removal to yield the corresponding product of Formula 1104.

As illustrated in Reaction Scheme 11, Step 4, the product of Formula 1104 is added to a solution of methacrylate anhydride. Triethylamine is added and the reaction stirred for 2 hours followed by solvent removal and isolation to yield the corresponding product of Formula 1105.

As illustrated in Reaction Scheme 11, Step 5, an azide-modified uRAFT agent (Formula 1106) is added to a solution of the product of Formula 1105 with azobisisobutyronitrile, subjected to 4 free-pump-thaw cycles and then stirred at 70°C. After several hours the corresponding polymer product of Formula 1107 is precipitated by addition of a lower alkanol followed by solvent removal. Where R³ is NH-protecting group (e.g., cyclized with R⁴) the protecting group(s) is(are) removed at this point.

As illustrated in Reaction Scheme 11, Step 6, an antigen, antibody, antibody fragment or ligand having native free surface amino group(s) of Formula 101' is added to a pH 8.0 buffer and contacted with an excess (corresponding to the value of m) of a dioxopyrrolidine of Formula 1108 with stirring. After 1 hour, unreacted Formula 1108 is removed and the resulting product of Formula 1109 is used without further purification.

As illustrated in Reaction Scheme 11, Step 7, the product of Formula 1107 is added to a pH 8.0 buffer, to which is added the product of Formula 1109. After stirring for 2 hours, the excess Formula 1107 is removed to yield the corresponding isomeric product of Formula 1m.

By substituting *N*-(2,4,5-trihydroxy-6-(hydroxymethyl)tetrahydro-2*H*-pyran-3-yl)methacrylamide for the product of Formula 1105 in Step 5 and continuing with Steps 6 and 7, the corresponding isomeric product of Formula 1m where Z" is N-acetylgalactosamine conjugated at C2 are obtained.

The compositions corresponding to Formula 1m can be named as follows:
"F1m-X'-mₘ-nₙ-pₚ-q_{q-}EtAcN-Z" where Z" is 1GAL, 1NGAL, 1NAcGAL, "F1m-X'-mₘ-nₙ-pₚ-q_{q-}2NAcGAL" or "F1m-X'-mₘ-nₙ-pₚ-q_{q-}2NAcGLU" (or the corresponding 1GAL, 1GLU, 1NGAL, 1NGLU, 1NAcGAL or
1NAcGLU compounds).

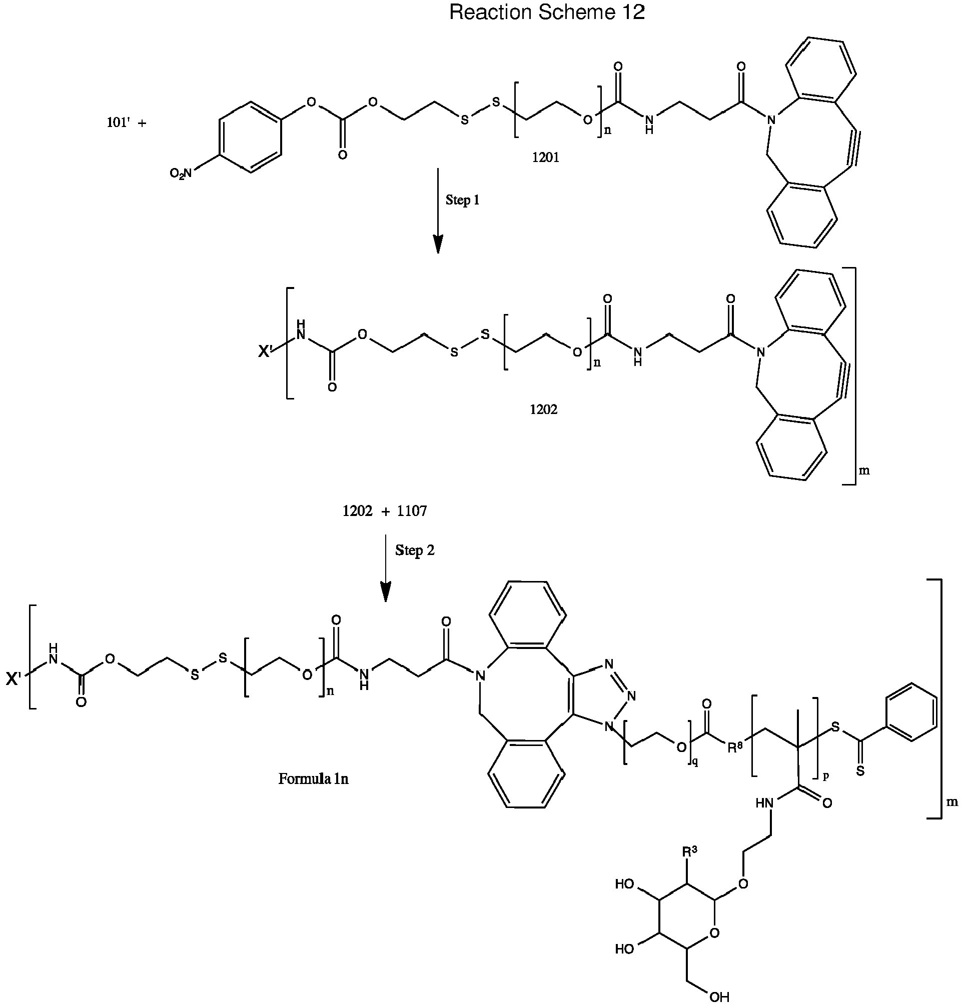

The synthetic approach of Reaction Scheme 12 is particularly suitable for hydrophobic antigens, antibodies, antibody fragments and ligands (e.g., Insulin) due to the use of organic solvents.

As illustrated in Reaction Scheme 12, Step 1, an antigen, antibody, antibody fragment or ligand having native free surface amino group(s) of Formula 101' is dissolved in an organic solvent (e.g., DMF) containing triethylamine. To this is added an amount (corresponding to the value of m) of a compound of Formula 1201 followed by stirring and the addition of t-butyl methyl ether. The corresponding product of Formula 1202 is recovered as a precipitate.

The product of Formula 1202 is resuspended in the organic solvent and an amount (corresponding to the value of m) of Formula 1107 (obtained, e.g., as described with reference to Reaction Scheme 11) is added followed by stirring. The reaction product is precipitated via the addition of dichloromethane, followed by filtration and solvent removal. Purification (e.g., resuspension in PBS followed by centrifugal size exclusion chromatography yields the corresponding isomeric product of Formula 1n.

The compositions corresponding to Formula 1n can be named as follows:
"F1n-X'-mₘ-nₙ-pₚ-q_{q-}EtAcN-Z" where Z" is 1GAL, 1NGAL, 1NAcGAL, 1GLU, 1NGLU, 1NAcGLU, or
"F1m-X'-nₘ-nₙ-pₚ-q_{q-}2NAcGAL" or "F1m-X'-nₘ-nₙ-pₚ-q_{q-}2NAcGLU".

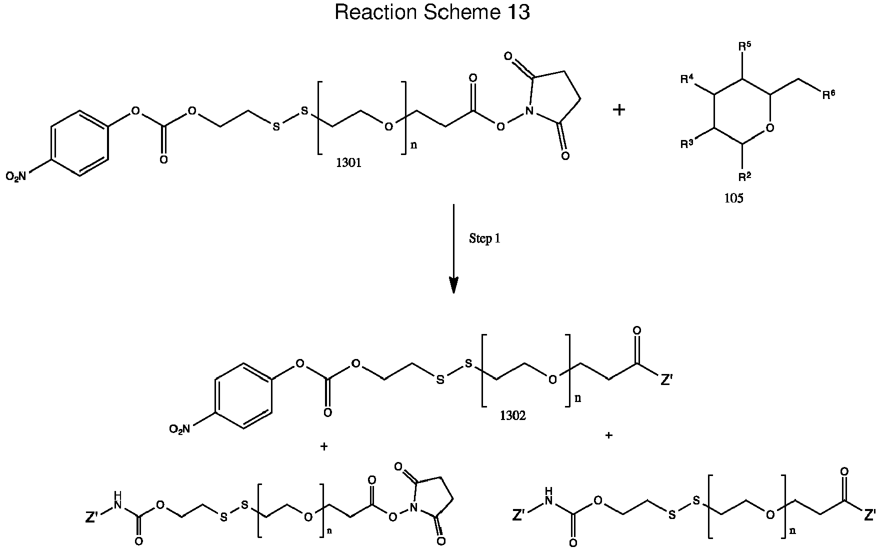

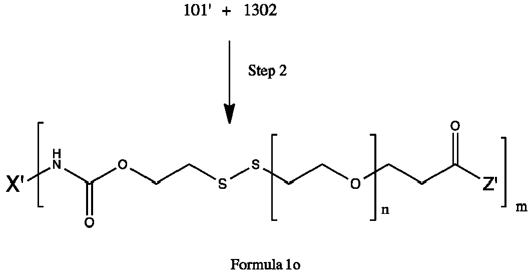

In Reaction Scheme 13, Step 1, a nitrophenoxycarbonyl-oxyalkyl di-thiol-poly(ethylene glycol)-NHS ester (Formula 1301) is contacted with galactose, galactosamine or N-acetylgalactosamine (Formula 105) to give the corresponding product of Formula 1302, along with the other two illustrated products, from which the desired nitrophenoxycarbonyl di-thiol-poly(ethylene glycol)-carboxyethyl galactose, galactosamine or N-acetylgalactosamine of Formula 1302 is isolated before proceeding to the next step.

As illustrated in Reaction Scheme 13, Step 2, an antigen, antibody, antibody fragment or ligand having native free surface amino group(s) of Formula 101' is contacted with an excess (corresponding to the value of m) of the product of Formula 1302 to yield the corresponding product according to Formula 1o.

The compositions corresponding to Formula 1o can be named as follows:
"F1o-X'-mₘ-nₙ-Z'."

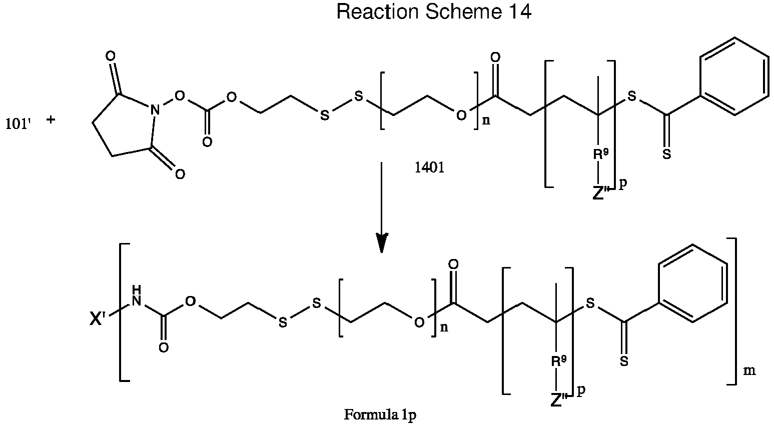

As illustrated in Reaction Scheme 14, an antigen, antibody, antibody fragment or ligand having native free surface amino group(s) (Formula 101') is contacted with an excess (corresponding to the value of m) of a pyridyl di-thiol-poly(ethylene glycol)-NHS ester of Formula 1401 to yield the corresponding product according to Formula 1p.

The compositions corresponding to Formula 1p can be named as follows:
"F1p-X'-mₘ-nₙ-pₚ-2NAcGAL" "F1p-X'-mₘ-nₙ-pₚ-2NAcGLU" or "F1p-X'-mₘ-nₙ-pₚ-EtAcN-Z".

Reaction Schemes 15-18 illustrate preparation of the compounds where W is a copolymer of the same or different W¹ and W² groups.

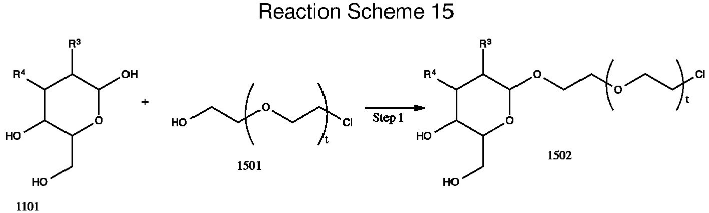

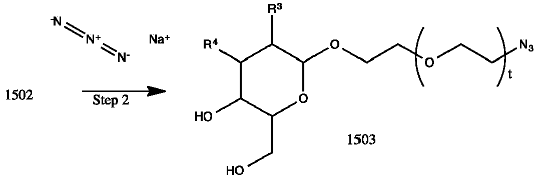

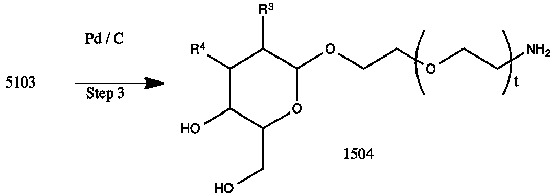

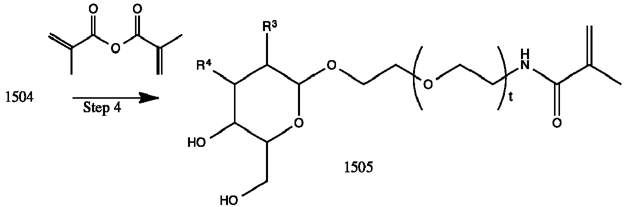

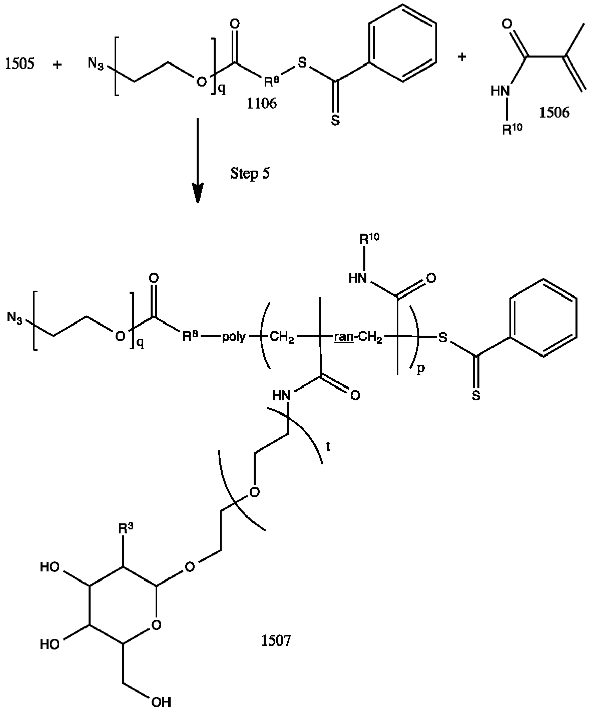

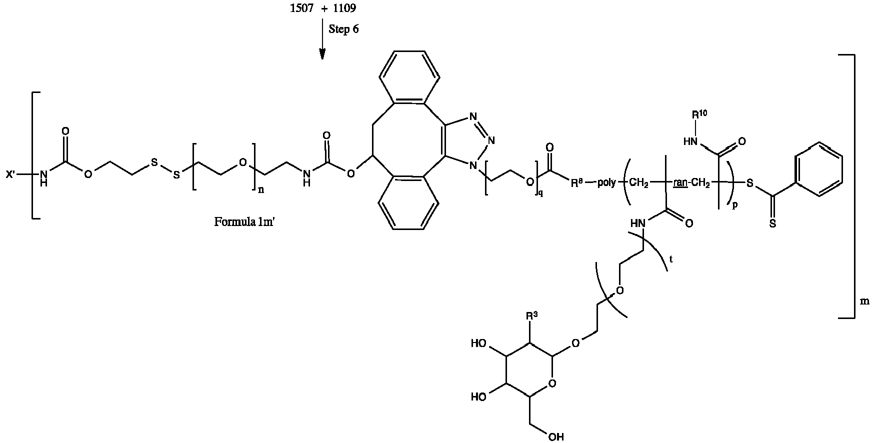

As illustrated in Reaction Scheme 15, Step 1, galactose or glucose (Formula1101 where R³ and R⁴ are OH), protected galactosamine or protected glucosamine (Formula 1101 where R³ is NH-protecting group, e.g., cyclized with R⁴) or N-acetyl-D-galactosamine or N-acetyl-D-glucosamine (Formula1101 where R³ is NHAc and R⁴ is OH) is contacted with a 2-(poly-(2-chloroethoxy)ethoxy)ethan-1-ol of Formula 1501 (where t is 1 to 5) followed by cooling and the dropwise addition of acetylchloride. The solution is warmed to room temperature and then heated to 70°C for several hours. Ethanol is added to the crude product and the resulting solution is stirred in the presence of carbon and then filtered followed by solvent removal to yield the corresponding product of Formula 1502.

As illustrated in Reaction Scheme 15, Step 2, the product of Formula 1502 is added to an excess of sodium azide and heated to 90°C for several hours, then filtered followed by solvent removal to yield the corresponding product of Formula 1503.

As illustrated in Reaction Scheme 15, Step 3, the product of Formula 1503 is added to a solution of palladium on carbon and ethanol, and stirred under hydrogen gas (3 atm) for several hours, then filtered followed by solvent removal to yield the corresponding product of Formula 1504.

As illustrated in Reaction Scheme 15, Step 4, the product of Formula 1504 is added to a solution of methacrylate anhydride. Triethylamine is added and the reaction stirred for 2 hours followed by solvent removal and isolation to yield the corresponding product of Formula 1505. Alternatively, pentafluorophenyl methacrylate (or another acrylating agent) can be used to prepare the corresponding product of Formula 1505. In some embodiments, the product of formula 1504 is added to DMF. Triethyl amine (e.g., an organic base) is added and the mixture is cooled (e.g., to 4°C using an ice bath). Subsequently, pentafluorophenyl methacrylate (or another acrylating agent) is added (e.g., drop-wise with constant stirring). After a period of time (e.g., 30 minutes), the cooling (e.g., ice-bath) is removed and the reaction is allowed to stir at room temperature for a period of time (e.g., 4 hours). In some embodiments, the solvent is then removed. In some embodiments, the product is purified using flash chromatography.

As illustrated in Reaction Scheme 15, Step 5, an azide-modified uRAFT agent of Formula 1106 and a methacrylamide of Formula 1506 are added to a solution of the product of Formula 1505 with azobisisobutyronitrile, subjected to 4 free-pump-thaw cycles and then stirred at 70°C. After several hours the corresponding random copolymer product of Formula 1507 is precipitated by addition of a lower alkanol or acetone followed by solvent removal. Where R³ is NH-protecting group (e.g., cyclized with R⁴) the protecting group(s) is(are) removed at this point.

As illustrated in Reaction Scheme 15, Step 6, the product of Formula 1507 is added to a pH 8.0 buffer, to which is added the product of Formula 1109 (prepared, for example, as described with reference to Reaction Scheme 11). After stirring for 2 hours, the excess Formula 1109 is removed to yield the corresponding isomeric random copolymer product of Formula 1m'.

By adding more than one methacrylamide of Formula 1505 in Step 5 (for example, glucose and galactose methacrylamides, or two or more methacrylamides having different values for t) and/or two or more methacrylamides of Formula 1506, and continuing with Step 6, the corresponding product of Formula 1m' having a mixture of R³ and/or PEG ("t") and/or R¹⁰ groups, i.e., compounds of Formula 1 where W is a random copolymer of different W¹ and W² groups are obtained.

The compositions corresponding to Formula 1m' can be named as follows:
"F1m'-X'-mₘ-nₙ-pₚ-q_{q}-R⁸-poly-(W¹ₜZ"_{W1p}-ran-W²_{W2p})".

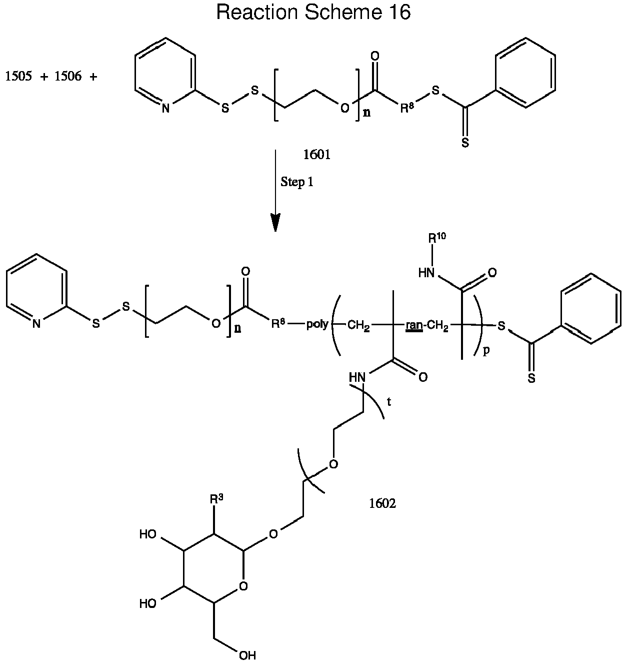

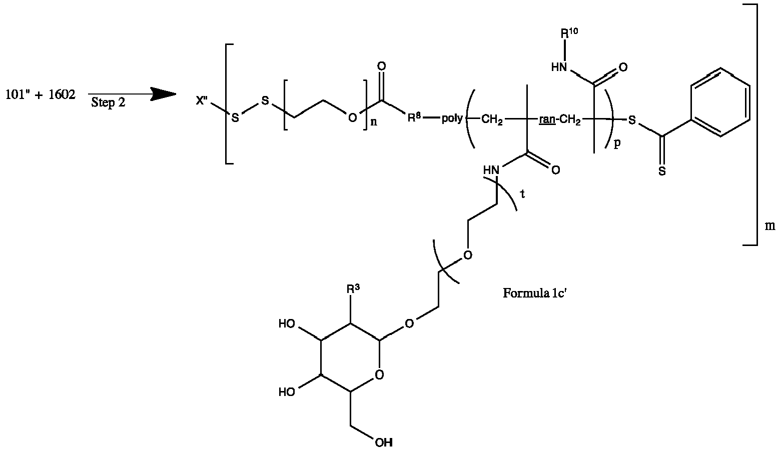

As illustrated in Reaction Scheme 16, Step 1, a compound of Formula 1601 is contacted with compounds of Formulae 1505 and 1506 under conditions analogous to those of Reaction Scheme 15, Step 5, to afford the corresponding compound of Formula 1602.

In some embodiments, the following synthesis is performed to form compound 1601a (an embodiment of 1601):

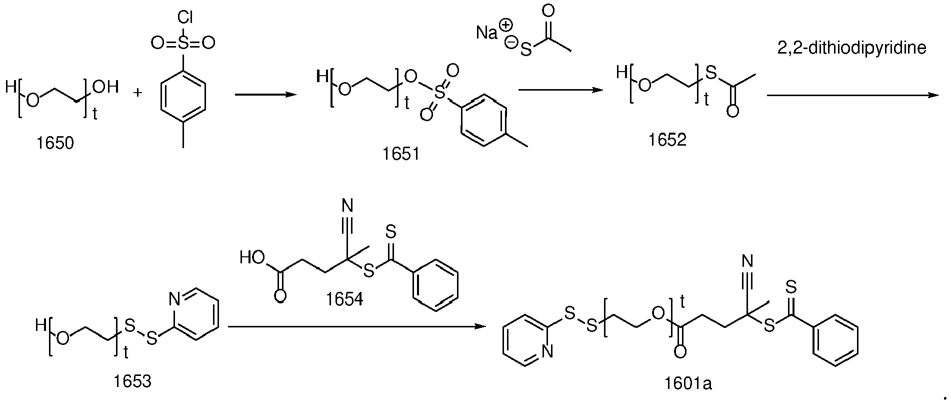

In some embodiments, t is an integer from about 1 to about 10 or about 1 to about 5. In some embodiments, an oligoethylene glycol (1650) is reacted with p-toluenesulfonyl chloride (or some other agent capable of functionalizing 1650 with a leaving group) to form oligoethylene glycol mono p-toluenesulfonate (1651)(or some other oligoethylene glycol functionalized with a leaving group). In some embodiments, compound 1651 can be reacted with potassium thioacetate to form compound 1652. In some embodiments, compound 1652 is reacted with 2,2-dithiodipyridine to form compound 1653. In some embodiments, compound 1653 is coupled to compound 1654 to form compound 1601a.

As illustrated in Reaction Scheme 16, Step 2, the compound of Formula 1602 is contacted with a compound of Formula 101" under conditions analogous to those of Reaction Scheme 15, Step 6, to afford the corresponding compound of Formula 1c'.

The compositions corresponding to Formula 1c' can be named as follows:
"F1c'-X'-mₘ-nₙ-pₚ-R⁸-poly-(W¹ₜZ"-ran-W²)".

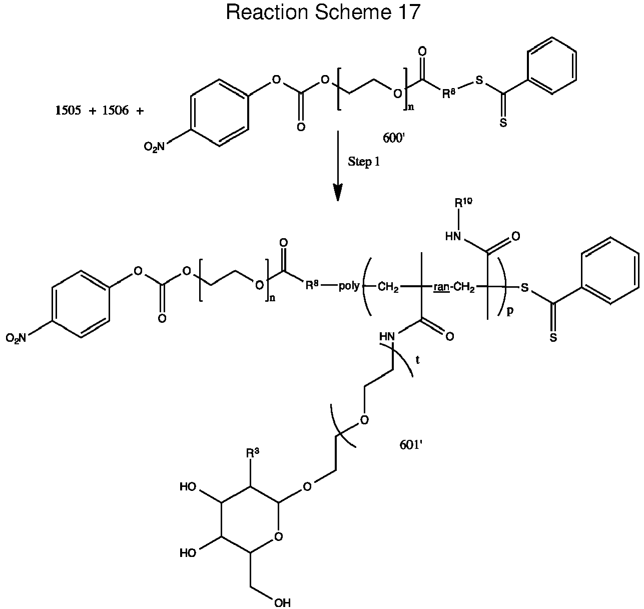

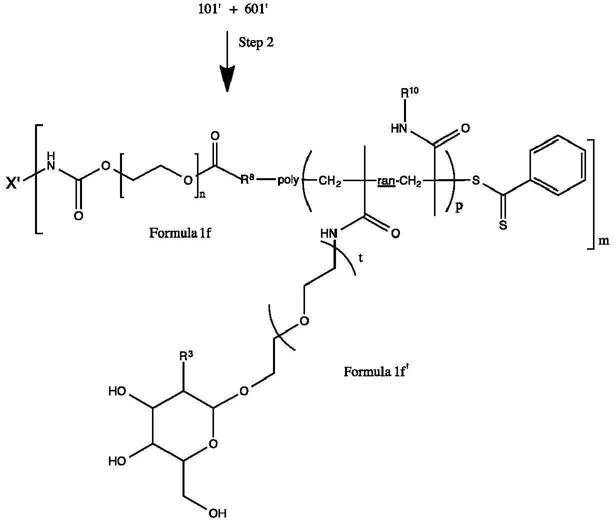

As illustrated in Reaction Scheme 17, Step 1, a compound of Formula 600' is contacted with compounds of Formulae 1505 and 1506 under conditions analogous to those of Reaction Scheme 15, Step 5, to afford the corresponding compound of Formula 601'.

As illustrated in Reaction Scheme 17, Step 2, the compound of Formula 601' is contacted with a compound of Formula 101' under conditions analogous to those of Reaction Scheme 15, Step 6, to afford the corresponding compound of Formula 1f'.

The compositions corresponding to Formula 1f' can be named as follows:
"F1f'-X'-mₘ-nₙ-pₚ-R⁸-poly-(W¹ₜZ"-ran-W²)".

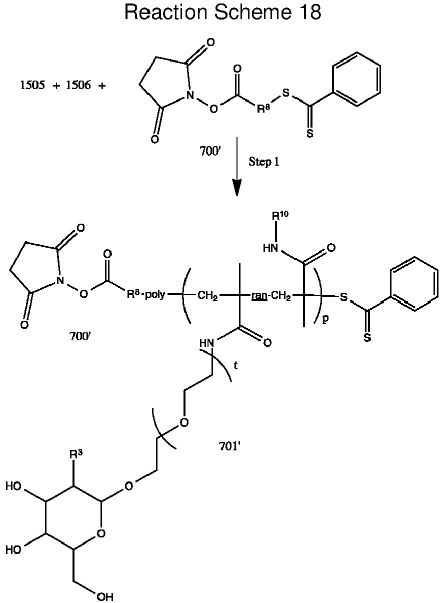

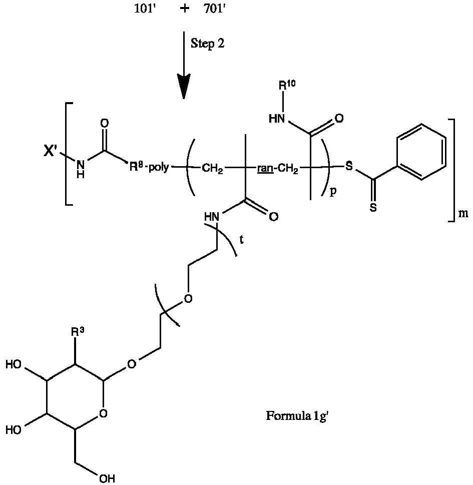

As illustrated in Reaction Scheme 18, Step 1, a compound of Formula 700' is contacted with compounds of Formulae 1505 and 1506 under conditions analogous to those of Reaction Scheme 15, Step 5, to afford the corresponding compound of Formula 701'.

As illustrated in Reaction Scheme 18, Step 2, the compound of Formula 701' is contacted with a compound of Formula 101' under conditions analogous to those of Reaction Scheme 15, Step 6, to afford the corresponding compound of Formula 1g'.

The compositions corresponding to Formula 1g' can be named as follows:
"F1g'-X'-mₘ-pₚ-R⁸-poly-(W¹ₜZ"-ran-W²)".

### Particular Processes and Last Steps

A compound of Formula 103' is contacted with an excess (corresponding to the value of m) of a compound of Formula 106 to give the corresponding product of Formula 1a.

A compound of Formula 103' is contacted with an excess (corresponding to the value of m) of a compound of Formula 201 to give the corresponding product of Formula 1b.

A compound of Formula 802, 902 or 1002 is contacted with an excess (corresponding to the value of m) of a compound of Formula 803 to give the corresponding product of Formula 1h, Formula 1i or Formula 1k, respectively.

A compound of Formula 1109 is contacted with an excess (corresponding to the value of m) of a compound of Formula 1107 to give the corresponding product of Formula 1m, particularly where n is about 80, p is about 30, q is about 4, and m being a function of the antigen is about 2 to 10.

A compound of Formula 1202 is contacted with an excess (corresponding to the value of m) of a compound of Formula 1107 to give the corresponding product of Formula 1n, particularly where n is about 1, p is about 30, q is about 4, and m being a function of the antigen is about 2 to 10.

A compound of Formula 1507 is contacted with a compound of Formula 1109 to give the corresponding product of Formula 1m', particularly where n is about 4, p is about 90, q is about 4, t is about 1 or 2, R³ is NHAc, R⁴ is OH, R⁸ is CMP, R¹⁰ is 2-hydroxypropyl and m being a function of the antigen is about 1 to 10.

A compound of Formula 101" is contacted with a compound of Formula 1602 to give the corresponding product of Formula 1c', particularly where n is about 4, p is about 90, t is about 1 or 2, R³ is NHAc, R⁴ is OH, R⁸ is CMP, R¹⁰ is 2-hydroxypropyl and m being a function of the antigen is about 1 to 10.

A compound of Formula 101' is contacted with a compound of Formula 601' to give the corresponding product of Formula 1f', particularly where n is about 4, p is about 90, t is about 1 or 2, R³ is NHAc, R⁴ is OH, R⁸ is CMP, R¹⁰ is 2-hydroxypropyl and m being a function of the antigen is about 1 to 10.

A compound of Formula 101' is contacted with a compound of Formula 701' to give the corresponding product of Formula 1g', particularly where n is about 4, p is about 90, t is about 1 or 2, R³ is NHAc, R⁴ is OH, R⁸ is CMP, R¹⁰ is 2-hydroxypropyl and m being a function of the antigen is about 1 to 10.

### Particular Compositions

By way of non-limiting example, a particular group preferred for the compositions, pharmaceutical formulations, methods of manufacture and use of the present disclosure are the following combinations and permutations of substituent groups of Formula 1 (sub-grouped, respectively, in increasing order of preference):
- X is a foreign transplant antigen against which transplant recipients develop an unwanted immune response, a foreign antigen to which patients develop an unwanted immune response, a therapeutic protein to which patients develop an unwanted immune response, a self-antigen to which patients develop an unwanted immune response, or a tolerogenic portion thereof.
- X is a therapeutic protein to which patients develop an unwanted immune response selected from: Abatacept, Abciximab, Adalimumab, Adenosine deaminase, Ado-trastuzumab emtansine, Agalsidase alfa, Agalsidase beta, Aldeslukin, Alglucerase, Alglucosidase alfa, α-1-proteinase inhibitor, Anakinra, Anistreplase (anisoylated plasminogen streptokinase activator complex), Antithrombin III, Antithymocyte globulin, Ateplase, Bevacizumab, Bivalirudin, Botulinum toxin type A, Botulinum toxin type B, C1-esterase inhibitor, Canakinumab, Carboxypeptidase G2 (Glucarpidase and Voraxaze), Certolizumab pegol, Cetuximab, Collagenase, Crotalidae immune Fab, Darbepoetin-α, Denosumab, Digoxin immune Fab, Dornase alfa, Eculizumab, Etanercept, Factor VIIa, Factor VIII, Factor IX, Factor XI, Factor XIII, Fibrinogen, Filgrastim, Galsulfase, Golimumab, Histrelin acetate, Hyaluronidase, Idursulphase, Imiglucerase, Infliximab, Insulin (including rHu insulin and bovine insulin), Interferon-α2a, Interferon-α2b, Interferon-β1a, Interferon-β1b, Interferon-γ1b, Ipilimumab, L-arginase, L-asparaginase, L-methionase, Lactase, Laronidase, Lepirudin / hirudin, Mecasermin, Mecasermin rinfabate, Methoxy Ofatumumab, Natalizumab, Octreotide, Oprelvekin, Pancreatic amylase, Pancreatic lipase, Papain, Peg-asparaginase, Peg-doxorubicin HCl, PEG-epoetin-β, Pegfilgrastim, Peg-Interferon-α2a, Peg-Interferon-α2b, Pegloticase, Pegvisomant, Phenylalanine ammonia-lyase (PAL), Protein C, Rasburicase (uricase), Sacrosidase, Salmon calcitonin, Sargramostim, Streptokinase, Tenecteplase, Teriparatide, Tocilizumab (atlizumab), Trastuzumab, Type 1 alpha-interferon, Ustekinumab, and vW factor.
   ∘ Especially where X is Abciximab, Adalimumab, Agalsidase alfa, Agalsidase beta, Aldeslukin, Alglucosidase alfa, Factor VIII, Factor IX, Infliximab, L-asparaginase, Laronidase, Natalizumab, Octreotide, Phenylalanine ammonia-lyase (PAL), or Rasburicase (uricase).
      ▪ Particularly where X is Factor VIII, Factor IX, uricase, PAL or asparaginase.
- X is a self-antigen polypeptide selected for treating type 1 diabetes mellitus, pediatric multiple sclerosis, juvenile rheumatoid arthritis, celiac disease, or alopecia universalis.
   ∘ Especially where X is a self-antigen polypeptide selected for treating new onset type 1 diabetes mellitus, pediatric multiple sclerosis or celiac disease.
- X is a foreign antigen to which patients develop an unwanted immune response
   ∘ From peanut, including conarachin (Ara h 1)
   ∘ From wheat, including Alpha-gliadin "33-mer" native (SEQ ID NO:20), Alpha-gliadin "33-mer" deamidated (SEQ ID NO:21), Alpha-gliadin (SEQ ID NO:22) and Omega-gliadin (SEQ ID NO:23).
   ∘ From cat, including Fel d 1A (UNIPROT P30438) and Cat albumin (UNIPROT P49064).
   ∘ From dog, including Can f 1 (UNIPROT 018873) and Dog albumin (UNIPROT P49822).
- X is a foreign transplant antigen against which transplant recipients develop an unwanted immune response, e.g. a human leukocyte antigen protein.
- X is an antibody, antibody fragment or ligand that specifically binds a circulating protein or peptide or antibody, which circulating protein or peptide or antibody gives rise to transplant rejection, immune response against a therapeutic agent, autoimmune disease, and/or allergy.
   ∘ Especially where X binds an endogenous circulating protein or peptide or antibody.
- Y is a linker selected from: Formula Ya, Formula Yb, Formula Yh, Formula Yi, Formula Yk, Formula Ym, Formula Yn, Formula Yo and Formula Yp.
   ∘ Especially where n is 8 to 90 ±10%, p is 20 to 100 ±10%, and q is 3 to 20 ±3.
      ▪ Particularly where n is 40 to 80 ±10%, p is 30 to 40 ±10%, and q is 4 to 12 ±3.
   ∘ Especially where Y is Formula Ya, Formula Yb, Formula Ym or Formula Yn.
      ▪ Particularly where n is 8 to 90 ±10%, p is 20 to 100 ±10% and q is 3 to 20 ±3.
         - More particularly where n is 40 to 80 ±10%, p is 30 to 40 ±10%, and q is 4 to 12 ±3.
      ▪ Particularly where Z is conjugated to Y via an ethylacetamido group.
         - More particularly where Z is conjugated to Y at its C1.
            ∘ More particularly where R⁸ is CMP.
         - More particularly where R⁸ is CMP.
      ▪ Particularly where R⁸ is CMP.
- Y is a linker selected from: Formula Yc, Formula Yf, Formula Yg and Formula Ym.
   ∘ Especially where Wₚ is a random copolymer in which R⁹ is Et-PEGₜ-AcN and R¹⁰ is 2-hydroxypropyl.
      ▪ Particularly where t is 1 or 2
         - More particularly where t is 1.
      ▪ Particularly where p is about 90 and includes about 30 W¹ and 60 W² comonomers.
- Z is galactose, galactosamine, N-acetylgalactosamine, glucose, glucosamine or N-acetylglucosamine.
   ∘ Especially where Z is galactose or N-acetylgalactosamine conjugated at C1, C2 or C6.
      ▪ Particularly where Z is galactose or N-acetylgalactosamine conjugated at C1 or C2.
         - More particularly where Z is N-acetylgalactosamine conjugated at C1.
   o Especially where Z is glucose or N-acetylglucosamine conjugated at C1, C2 or C6.
      ▪ Particularly where Z is glucose or N-acetylglucosamine conjugated at C1 or C2.
         - More particularly where Z is N-acetylglucosamine conjugated at C1.

Each of the above-described groups and sub-groups are individually preferred and can be combined to describe further preferred aspects of the disclosure, for example but not by way of limitation, as follows:
- X is a self-antigen polypeptide selected for treating type 1 diabetes mellitus, pediatric multiple sclerosis, juvenile rheumatoid arthritis, celiac disease, or alopecia universalis.
   ∘ Especially where X is a self-antigen polypeptide selected for treating new onset type 1 diabetes mellitus, pediatric multiple sclerosis or celiac disease.
      ▪ Particularly where Y is a linker selected from: Formula Ya, Formula Yb, Formula Yc, Formula Yf, Formula Yg, Formula Yh, Formula Yi, Formula Yk, Formula Ym, Formula Yn, Formula Yo and Formula Yp.
         - Especially where Wₚ is a W¹ polymer in which R⁹ is Et-PEGₜ-AcN or a random copolymer in which R⁹ is Et-PEGₜ-AcN and R¹⁰ is 2-hydroxypropyl.
            ∘ Particularly where t is 1 or 2
               ▪ More particularly where t is 2.
               ▪ More particularly where t is 1.
            ∘ Particularly where p is about 90
               ▪ More particularly where Wₚ is a random copolymer and includes about 30 W¹ and 60 W² comonomers.
         - Especially where n is 8 to 90 ±10%, p is 20 to 100 ±10%, and q is 3 to 20 ±3.
            ∘ Particularly where n is 40 to 80 ±10%, p is 30 to 40 ±10%, and q is 4 to 12 ±3.
         - Especially where Y is Formula Ya, Formula Yb, Formula Ym or Formula Yn.
            ∘ Particularly where n is 8 to 90 ±10%, p is 20 to 100 ±10% and q is 3 to 20 ±3.
               ▪ More particularly where n is 40 to 80 ±10%, p is 30 to 40 ±10%, and q is 4 to 12 ±3.
                  - Even more particularly where Z is conjugated to Y via an ethylacetamido group.
               ▪ More particularly where Z is conjugated to Y via an ethylacetamido group.
            ∘ Particularly where Z is conjugated to Y via an ethylacetamido group.
         - Especially where Z is galactose, galactosamine or N-acetylgalactosamine.
            ∘ Particularly where Z is galactose or N-acetylgalactosamine conjugated at C1, C2 or C6.
               ▪ More particularly where Z is galactose or N-acetylgalactosamine conjugated at C1 or C2.
                  - Even more particularly where Z is N-acetylgalactosamine conjugated at C1.
         - Especially where Z is glucose, glucosamine or N-acetylglucosamine.
            ∘ Particularly where Z is glucose or N-acetylglucosamine conjugated at C1, C2 or C6.
               ▪ More particularly where Z is glucose or N-acetylglucosamine conjugated at C1 or C2.
                  - Even more particularly where Z is N-acetylglucosamine conjugated at C1.
      ▪ Particularly where Y is a linker selected from: Formula Yc, Formula Yf, Formula Yg and Formula Ym.
         • Especially where Wₚ is a random copolymer in which R⁹ is Et-PEGₜ-AcN and R¹⁰ is 2-hydroxypropyl.
            ∘ Particularly where t is 1 or 2
               ▪ More particularly where t is 1.
            o Particularly where p is about 90 and includes about 30 W¹ and 60 W² comonomers.
      ▪ Particularly where Y is a linker selected from: Formula Yc and Formula Ym.
         - Especially where Wₚ is a random copolymer in which R⁹ is Et-PEGₜ-AcN and R¹⁰ is 2-hydroxypropyl.
            ∘ Particularly where t is 1 or 2
               ▪ More particularly where t is 1.
            o Particularly where p is about 90 and includes about 30 W¹ and 60 W² comonomers.
      ▪ Particularly where Z is galactose, galactosamine or N-acetylgalactosamine.
         - Especially where Z is galactose or N-acetylgalactosamine conjugated at C1, C2 or C6.
            ∘ Particularly where Z is galactose or N-acetylgalactosamine conjugated at C1 or C2.
               ▪ More particularly where Z is N-acetylgalactosamine conjugated at C1.
      ▪ Particularly where Z is glucose, glucosamine or N-acetylglucosamine.
         - Especially where Z is glucose or N-acetylglucosamine conjugated at C1, C2 or C6.
            ∘ More particularly where Z is glucose or N-acetylglucosamine conjugated at C1 or C2.
               ▪ Even more particularly where Z is N-acetylglucosamine conjugated at C1.
   ∘ Especially where Y is a linker selected from: Formula Ya, Formula Yb, Formula Yh, Formula Yi, Formula Yk, Formula Ym, Formula Yn, Formula Yo and Formula Yp.
      ▪ Particularly where Y is a linker selected from: Formula Yc, Formula Yf, Formula Yg and Formula Ym.
         - Especially where Wₚ is a random copolymer in which R⁹ is Et-PEGₜ-AcN and R¹⁰ is 2-hydroxypropyl.
            ∘ Particularly where t is 1 or 2
               ▪ More particularly where t is 1.
            o Particularly where p is about 90 and includes about 30 W¹ and 60 W² comonomers.
      ▪ Particularly where Y is a linker selected from: Formula Yc and Formula Ym.
         - Especially where Wₚ is a random copolymer in which R⁹ is Et-PEGₜ-AcN and R¹⁰ is 2-hydroxypropyl.
            ∘ Particularly where t is 1 or 2
               ▪ More particularly where t is 1.
            o Particularly where p is about 90 and includes about 30 W¹ and 60 W² comonomers.
      ▪ Particularly where n is 8 to 90 ±10%, p is 20 to 100 ±10%, and q is 3 to 20 ±3.
         - More particularly where n is 40 to 80 ±10%, p is 30 to 40 ±10%, and q is 4 to 12 ±3.
      ▪ Particularly where Y is Formula Ya, Formula Yb, Formula Ym or Formula Yn.
         - More particularly where n is 8 to 90 ±10%, p is 20 to 100 ±10% and q is 3 to 20 ±3.
            ∘ More preferably where n is 40 to 80 ±10%, p is 30 to 40 ±10%, and q is 4 to 12 ±3.
         - More particularly where Z is conjugated to Y via an ethylacetamido group.
   ∘ Especially where Z is galactose, galactosamine or N-acetylgalactosamine.
      ▪ Particularly where Z is galactose or N-acetylgalactosamine conjugated at C1, C2 or C6.
         - More particularly where Z is galactose or N-acetylgalactosamine conjugated at C1 or C2.
            ∘ More preferably where Z is N-acetylgalactosamine conjugated at C1.
         - More particularly where Y is a linker selected from: Formula Yc, Formula Yf, Formula Yg and Formula Ym.
            ∘ Especially where Wₚ is a random copolymer in which R⁹ is Et-PEGₜ-AcN and R¹⁰ is 2-hydroxypropyl.
               ▪ Particularly where t is 1 or 2
                  - More particularly where t is 1.
               ▪ Particularly where p is about 90 and includes about 30 W¹ and 60 W² comonomers.
   ∘ Especially where Z is glucose, glucosamine or N-acetylglucosamine.
      ▪ Particularly where Z is glucose or N-acetylglucosamine conjugated at C1, C2 or C6.
         - More particularly where Z is glucose or N-acetylglucosamine conjugated at C1 or C2.
            ∘ More preferably where Z is N-acetylglucosamine conjugated at C1.
         - More particularly where Y is a linker selected from: Formula Yc, Formula Yf, Formula Yg and Formula Ym.
            ∘ Especially where Wₚ is a random copolymer in which R⁹ is Et-PEGₜ-AcN and R¹⁰ is 2-hydroxypropyl.
               ▪ Particularly where t is 1 or 2
                  - More particularly where t is 1.
               ▪ Particularly where p is about 90 and includes about 30 W¹ and 60 W² comonomers.
         - More particularly where Y is a linker selected from: Formula Yc and Formula Ym.
            ∘ Especially where Wₚ is a random copolymer in which R⁹ is Et-PEGₜ-AcN and R¹⁰ is 2-hydroxypropyl.
               ▪ Particularly where t is 1 or 2
                  - More particularly where t is 1.
               ▪ Particularly where p is about 90 and includes about 30 W¹ and 60 W² comonomers.
- m is an integer from about 1 to 100.
   o m is 1, 2, 3, 4, 5, 6, 7, 8, 9, 10, 11, 12, 13, 14, 15, 16, 17, 18, 19, 20, 25, 30, 35, 40, 45, 50, 55, 60, 65 70, 75, 80, 85, 90, 95, 100 or 110.
   o Particularly m is from about 1 to 20.
      ▪ m is 1, 2, 3, 4, 5, 6, 7, 8, 9, 10, 11, 12, 13, 14, 15, 16, 17, 18, 19, 20, 21 or 22.
      ▪ More particularly m is about 10.
         - m is 9, 10 or 11.
- n is an integer representing a mixture including from about 1 to 100
   ∘ n is 1, 2, 3, 4, 5, 6, 7, 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95, 99, 100, 105 or 110.
      ▪ Particularly n is about 8 to 90.
      ▪ Particularly n is 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95 or 99.
         - More particularly n is about 40 to 80.
         - More particularly n is 37, 40, 41, 45, 50, 54, 55, 59, 60, 65, 70, 75, 80, 82, 83 or 88.
   ∘ n represents a mixture encompassing the ranges 1-4, 2-4, 2-6, 3-8, 7-13, 6-14, 15-25, 26-30, 42-50, 46-57, 60-82, 85-90, 90-110 and 107-113.
      ▪ Particularly n represents a mixture encompassing the ranges 7-13, 6-14, 15-25, 26-30, 42-50, 46-57, 60-82, 85-90 and 82-99.
         - More particularly n represents a mixture encompassing the ranges 36-44, 42-50, 46-57, 60-82 and 75-85.
   ∘ p is an integer representing a mixture including from about 2 to 150.
      ▪ p is 2, 3, 4, 5, 6, 7, 8, 9, 10, 11, 12, 13, 14, 15, 16, 17, 18, 19, 20, 25, 30, 35, 40, 45, 50, 55, 60, 65 70, 75, 80, 85, 90, 95, 100, 110, 120, 130, 140, 150, 160 or 165.
      ▪ Particularly where n is an integer representing a mixture including from about 1 to 100.
         - Particularly n is 1, 2, 3, 4, 5, 6, 7, 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95, 99, 100, 105 or 110.
            ∘ More particularly where n is about 8 to 90.
            ∘ More particularly n is 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95 or 99.
               ▪ Even more particularly where n is about 40 to 80.
               ▪ Even more particularly n is 37, 40, 41, 45, 50, 54, 55, 59, 60, 65, 70, 75, 80, 82, 83 or 88.
         - More particularly p is 18, 19, 20, 25, 30, 35, 40, 45, 50, 55, 60, 65 70, 75, 80, 85, 90, 95, 100 or 110.
            ∘ Particularly where n is an integer representing a mixture including from about 1 to 100.
               ▪ Particularly n is 1, 2, 3, 4, 5, 6, 7, 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95, 99, 100, 105 or 110.
                  - More particularly where n is about 8 to 90.
                  - More particularly n is 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95 or 99.
                     ∘ Even more particularly where n is about 40 to 80.
                     ∘ Even more particularly n is 37, 40, 41, 45, 50, 54, 55, 59, 60, 65, 70, 75, 80, 82, 83 or 88.
            ∘ More particularly p is 27, 28, 29, 30, 31, 32, 33, 34, 35, 36, 37, 38, 39, 40, 41, 42, 43, or 44.
               ▪ Particularly where n is 1, 2, 3, 4, 5, 6, 7, 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95, 99, 100, 105 or 110.
                  - More particularly where n is about 8 to 90.
                  - More particularly n is 8, 9, 10, 11, 12, 13, 14, 15, 16, 17, 18, 19, 20, 22, 25, 30, 34, 35, 37, 40, 41, 45, 50, 54, 55, 59, 60, 65, 70, 75, 80, 82, 83, 85, 88, 90, 95 or 99.
                     ∘ Even more particularly where n is about 40 to 80.
                     ∘ Even more particularly n is 37, 40, 41, 45, 50, 54, 55, 59, 60, 65, 70, 75, 80, 82, 83 or 88.
   ∘ q is an integer representing a mixture including from about 1 to 44.
      ▪ q is 1, 2, 3, 4, 5, 6, 7, 8, 9, 10, 11, 12, 13, 14, 15, 16, 17, 18, 19, 20, 25, 30, 35, 40, 44 or 48.

### Utility, Testing and Administration

### General Utility

The compositions of the disclosure find use in a variety of applications including, as will be appreciated by those in the art, treatment of transplant rejection, immune response against a therapeutic agent, autoimmune disease, and food allergy, among other uses.

In a preferred embodiment, the compositions of the disclosure are used to modulate, particularly down-regulate, antigen-specific undesirable immune response.

The compositions of the disclosure are useful, in additional embodiments, to bind and clear from the circulation specific undesired proteins, including antibodies endogenously generated in a patient (i.e., not exogenous antibodies administered to a patient), peptides and the like, which cause autoimmunity and associated pathologies, allergy, inflammatory immune responses, and anaphylaxis.

In several embodiments according to the present disclosure, antigens are targeted to the liver for presentation via antigen-presenting cells to specifically down-regulate the immune system or for clearance of unwanted circulating proteins. This is distinct from previous uses of liver targeting, for example as described in US 2013/0078216, where the purpose of liver-targeting molecules such as DOM26h-196-61 was the delivery of therapeutic agents to treat liver diseases such as fibrosis, hepatitis, Cirrhosis and liver cancer.

According to several embodiments, the present disclosure provides compositions and methods to treat unwanted immune response to self-antigens and foreign antigens, including but not limited to: a foreign transplant antigen against which transplant recipients develop an unwanted immune response (e.g., transplant rejection), a foreign antigen to which patients develop an unwanted immune (e.g., allergic or hypersensitivity) response, a therapeutic agent to which patients develop an unwanted immune response (e.g., hypersensitivity and/or reduced therapeutic activity), a self-antigen to which patients develop an unwanted immune response (e.g., autoimmune disease)

Autoimmune disease states that can be treated using the methods and compositions provided herein include, but are not limited to: Acute Disseminated Encephalomyelitis (ADEM); Acute interstitial allergic nephritis (drug allergies); Acute necrotizing hemorrhagic leukoencephalitis; Addison's Disease; Alopecia areata; Alopecia universalis; Ankylosing Spondylitis; Arthritis, juvenile; Arthritis, psoriatic; Arthritis, rheumatoid; Atopic Dermatitis; Autoimmune aplastic anemia; Autoimmune gastritis; Autoimmune hepatitis; Autoimmune hypophysitis; Autoimmune oophoritis; Autoimmune orchitis; Autoimmune polyendocrine syndrome type 1; Autoimmune polyendocrine syndrome type 2; Autoimmune thyroiditis; Behcet's disease; Bronchiolitis obliterans; Bullous pemphigoid; Celiac disease; Churg-Strauss syndrome; Chronic inflammatory demyelinating polyneuropathy; Cicatricial pemphigoid; Crohn's disease; Coxsackie myocarditis; Dermatitis herpetiformis Duhring; Diabetes mellitus (Type 1); Erythema nodosum; Epidermolysis bullosa acquisita, Giant cell arteritis (temporal arteritis); Giant cell myocarditis; Goodpasture's syndrome; Graves' disease; Guillain-Barre syndrome; Hashimoto's encephalitis; Hashimoto's thyroiditis; IgG4-related sclerosing disease; Lambert-Eaton syndrome; Mixed connective tissue disease; Mucha-Habermann disease; Multiple sclerosis; Myasthenia gravis; Optic neuritis; Neuromyelitis optica; Pemphigus vulgaris and variants; Pernicious angemis; Pituitary autoimmune disease; Polymyositis; Postpericardiotomy syndrome; Premature ovarian failure; Primary Biliary Cirrhosis; Primary sclerosing cholangitis; Psoriasis; Rheumatic heart disease; Sjogren's syndrome; Systemic lupus erythematosus; Systemic sclerosis; Ulcerative colitis; Undifferentiated connective tissue disease (UCTD); Uveitis; Vitiligo; and Wegener's granulomatosis.

A particular group of autoimmune disease states that can be treated using the methods and compositions provided herein include, but are not limited to: Acute necrotizing hemorrhagic leukoencephalitis; Addison's Disease; Arthritis, psoriatic; Arthritis, rheumatoid; Autoimmune aplastic anemia; Autoimmune hypophysitis; Autoimmune gastritis; Autoimmune polyendocrine syndrome type 1; Bullous pemphigoid; Celiac disease; Coxsackie myocarditis; Dermatitis herpetiformis Duhring; Diabetes mellitus (Type 1); Epidermolysis bullosa acquisita; Giant cell myocarditis; Goodpasture's syndrome; Graves' disease; Hashimoto's thyroiditis; Mixed connective tissue disease; Multiple sclerosis; Myasthenia gravis; Neuromyelitis optica; Pernicious angemis; Pemphigus vulgaris and variants; Pituitary autoimmune disease; Premature ovarian failure; Rheumatic heart disease; Systemic sclerosis; Sjogren's syndrome; Systemic lupus erythematosus; and Vitiligo.

In the embodiments employing an antigen against which an unwanted immune response is developed, such as food antigens, treatment can be provided for reactions against, for example: peanut, apple, milk, egg whites, egg yolks, mustard, celery, shrimp, wheat (and other cereals), strawberry and banana.

As will be appreciated by those skilled in the art, a patient can be tested to identify a foreign antigen against which an unwanted immune response has developed, and a composition of the disclosure can be developed based on that antigen.

### Testing

In establishing the utility of the compositions and methods of the disclosure, specificity in binding to antigen-presenting cells in the liver (particularly binding to hepatocytes and specifically ASGPR) should initially be determined. This can be accomplished, for example, by employing a marker (such as the fluorescent marker phycoerythrin ("PE")) in a composition of the disclosure. The composition is administered to suitable experimental subjects. Controls, e.g., unconjugated PE or vehicle (saline) are administered to other group(s) of subjects. The composition and controls are allowed to circulate for a period of 1 to 5 hours, after which the spleens and livers of the subjects are harvested and measured for fluorescence. The specific cells in which fluorescence is found can be subsequently identified. Compositions of the disclosure, when tested in this manner, show higher levels of concentration in the antigen-presenting cells of the liver as compared with unconjugated PE or vehicle.

Effectiveness in immune modulation can be tested by measuring the proliferation of OT-I CD8⁺ cells (transplanted into host mice) in response to the administration of a composition of the disclosure incorporating a known antigen, such as ovalbumin ("OVA"), as compared with administration of the antigen alone or just vehicle. Compositions of the disclosure, when tested in this manner, show an increase of OT-I cell proliferation as compared with antigen alone or vehicle, demonstrating increased CD8⁺ T-cell cross-priming. To distinguish T cells being expanded into a functional effector phenotype from those being expanded and deleted, the proliferating OT-I CD8⁺ T cells can be phenotypically analyzed for molecular signatures of exhaustion [such as programmed death-1 (PD-1), FasL, and others], as well as Annexin-V binding as a hallmark of apoptosis and thus deletion. The OT-I CD8⁺ T cells can also be assessed for their responsiveness to an antigen challenge with adjuvant in order to demonstrate functional non-responsiveness, and thus immune tolerance, towards the antigen. To do so, the cells are analyzed for inflammatory signatures after administration of compositions of the disclosure into host mice followed by an antigen challenge. Compositions of the disclosure when tested in this manner demonstrate very low (e.g., background) levels of inflammatory OT-I CD8⁺ T cell responses towards OVA in comparison to control groups, thus demonstrating immune tolerance.

Humoral immune response can be tested by administering a composition of the disclosure incorporating a known antigen, such as OVA, as compared with the administration of the antigen alone or just vehicle, and measuring the levels of resulting antibodies. Compositions of the disclosure when tested in this manner show very low (e.g., background) levels of antibody formation responsive to their administration and the administration of vehicle, with significantly higher levels of antibody formation responsive to administration of the antigen.

Effectiveness in tolerization against an antigen can be tested as above with reference to humoral immune response, where several weeks following treatment(s) with a composition of the disclosure a group of subjects is challenged by administration of the antigen alone, followed by measuring the levels of antibodies to the antigen. Compositions of the disclosure when tested in this manner show low levels of antibody formation responsive to challenge with the antigen in groups pretreated with such compositions as compared to groups that are not pretreated.

Disease-focused experimental models are well known to those skilled in the art and include the NOD (or non-obese diabetic) mouse model of autoimmunity and tolerance and the EAE (experimental autoimmune encephalomyelitis) model for the human inflammatory demyelinating disease, multiple sclerosis. In particular, the NOD mouse develops spontaneous autoimmune diabetes (similar to type 1a diabetes in humans). Groups of NOD mice are treated with test compound or a negative control, followed by measurement of blood glucose. Successful treatment corresponds to likelihood of treating diabetes in humans or proof of mechanism for approaches to the treatment of other autoimmune diseases. (See, e.g., Anderson and Bluestone, Annu. Rev. Immunol. 2005;23:447-85.)

### Administration

The compositions of the disclosure are administered at a therapeutically effective dosage, e.g., a dosage sufficient to provide treatment for the disease states previously described. Administration of the compounds of the disclosure or the pharmaceutically acceptable salts thereof can be via any of the accepted modes of administration for agents that serve similar utilities.

While human dosage levels have yet to be optimized for the compounds of the disclosure, these can initially be extrapolated from the about 10 µg to 100 µg doses administered for mice. Generally, an individual human dose is from about 0.01 to 2.0 mg/kg of body weight, preferably about 0.1 to 1.5 mg/kg of body weight, and most preferably about 0.3 to 1.0 mg/kg of body weight. Treatment can be administered for a single day or a period of days, and can be repeated at intervals of several days, one or several weeks, or one or several months. Administration can be as a single dose (e.g., as a bolus) or as an initial bolus followed by continuous infusion of the remaining portion of a complete dose over time, e.g., 1 to 7 days. The amount of active compound administered will, of course, be dependent on any or all of the following: the subject and disease state being treated, the severity of the affliction, the manner and schedule of administration and the judgment of the prescribing physician. It will also be appreciated that amounts administered will depend upon the molecular weight of the antigen, antibody, antibody fragment or ligand as well as the size of the linker.

The compositions of the disclosure can be administered either alone or in combination with other pharmaceutically acceptable excipients. While all typical routes of administration are contemplated (e.g. oral, topical, transdermal, injection (intramuscular, intravenous, or intra-arterial)), it is presently preferred to provide liquid dosage forms suitable for injection. The formulations will typically include a conventional pharmaceutical carrier or excipient and a composition of the disclosure or a pharmaceutically acceptable salt thereof. In addition, these compositions can include other medicinal agents, pharmaceutical agents, carriers, and the like, including, but not limited to the therapeutic protein, peptide, antibody or antibody-like molecule corresponding to the antigen (X) employed in the composition of the disclosure, and other active agents that can act as immune-modulating agents and more specifically can have inhibitory effects on B-cells, including anti-folates, immune suppressants, cyostatics, mitotic inhibitors, and anti-metabolites, or combinations thereof.

Generally, depending on the intended mode of administration, the pharmaceutically acceptable composition will contain about 0.1% to 95%, preferably about 0.5% to 50%, by weight of a composition of the disclosure, the remainder being suitable pharmaceutical excipients, carriers, etc. Dosage forms or compositions containing active ingredient in the range of 0.005% to 95% with the balance made up from non-toxic carrier can be prepared.

Liquid pharmaceutically administrable compositions can, for example, be prepared by dissolving, dispersing, etc. an active composition of the disclosure (e.g., a lyophilized powder) and optional pharmaceutical adjuvants in a carrier, such as, for example, water (water for injection), saline, aqueous dextrose, glycerol, glycols, ethanol or the like (excluding galactoses), to thereby form a solution or suspension. If desired, the pharmaceutical composition to be administered can also contain minor amounts of nontoxic auxiliary substances such as wetting agents, emulsifying agents, stabilizing agents, solubilizing agents, pH buffering agents and the like, for example, sodium acetate, sodium citrate, cyclodextrine derivatives, sorbitan monolaurate, triethanolamine acetate and triethanolamine oleate, etc., osmolytes, amino acids, sugars and carbohydrates, proteins and polymers, salts, surfactants, chelators and antioxidants, preservatives, and specific ligands. Actual methods of preparing such dosage forms are known, or will be apparent, to those skilled in this art; for example, see Remington: The Science and Practice of Pharmacy, Pharmaceutical Press, 22nd Edition, 2012. The composition or formulation to be administered will, in any event, contain a quantity of the active compound in an amount effective to treat the symptoms of the subject being treated.

### EXAMPLES

The following examples serve to more fully describe the manner of using the above-described disclosure, as well as to set forth the best modes contemplated for carrying out various aspects of the disclosure. It is understood that these examples in no way serve to limit the true scope of this disclosure, but rather are presented for illustrative purposes. All references cited herein are incorporated by reference in their entirety.

### Example 1

### F1aA-OVA-m₄-n₈₀ (or F1a-OVA-m₄-n₈₀-2NGAL)

### 1A. Formula 103' where X' is OVA and m is 4

In an endotoxin-free tube, OVA (5.0 mg, 0.00012 mmol) was added to 100 µl of pH 8.0 PBS containing 5 mM EDTA and stirred. Separately, 1 mg of Traut's Reagent was dissolved in 100 µl of pH 7.0 PBS, and 16 µl (0.00119 mmol) of the Traut's Reagent solution so obtained was added to the stirred solution of OVA with continued stirring. After 1 hour, excess Traut's Reagent was removed using a centrifugal size exclusion column to afford the corresponding product of Formula 103'.

### 1B. Formula 106A where n is 80

In an endotoxin-free tube, galactosamine (10.0 mg, 0.04638 mmol) was dissolved with stirring in 100 µl of pH 8.0 PBS containing 5 mM EDTA. Pyridyl dithiol-poly(ethylene glycol)-NHS ester (Formula 104 where n is 80) (16.23 mg, 0.00464 mmol) dissolved in 100 µl of pH 7.0 PBS was added to the stirring solution of galactosamine. After 1 hour, the resulting pyridyl dithiol-poly(ethylene glycol)-N-acetylgalactosamine (Formula 106A) was ready to be used without further purification.

### 1C. Formula 1aA where X' is OVA, m is 4, n is 80 (and Z' is C2 galactosamine)

The purified OVA-Traut conjugate of Formula 103' prepared in Example 1A was added directly to the stirring product of Formula 106A prepared in Example 1B. After 1hour, the resulting product of Formula 1a was purified by passing the reaction mixture through a centrifugal size exclusion column. Characterization (UHPLC SEC, gel electrophoresis) confirmed product identity. (See Fig. 5.)

### 1D. Other Compounds of Formula 103'

By following the procedure described in Example 1A and substituting OVA with the following:
- Abciximab,
- Adalimumab,
- Agalsidase alfa,
- Agalsidase beta,
- Aldeslukin,
- Alglucosidase alfa,
- Factor VIII,
- Factor IX,
- L-asparaginase,
- Laronidase,
- Octreotide,
- Phenylalanine ammonia-lyase,
- Rasburicase,
- Insulin (SEQ ID NO:1),
- GAD-65 (SEQ ID NO:2),
- IGRP (SEQ ID NO:3)
- MBP (SEQ ID NO:4),
- MOG (SEQ ID NO:5),
- PLP (SEQ ID NO:6),
- MBP13-32 (SEQ ID NO:7),
- MBP83-99 (SEQ ID NO:8),
- MBP111-129 (SEQ ID NO:9),
- MBP146-170 (SEQ ID NO:10),
- MOG1-20 (SEQ ID NO:11),
- MOG35-55 (SEQ ID NO:12),
- PLP139-154 (SEQ ID NO:13),
- MART1 (SEQ ID NO:14),
- Tyrosinase (SEQ ID NO:15),
- PMEL (SEQ ID NO:16),
- Aquaporin-4 (SEQ ID NO:17),
- S-arrestin (SEQ ID NO:18),
- IRBP (SEQ ID NO:19),
- Conarachin (UNIPROT Q6PSU6),
- Alpha-gliadin "33-mer" native (SEQ ID NO:20),
- Alpha-gliadin "33-mer" deamidated (SEQ ID NO:21),
- Alpha-gliadin (SEQ ID NO:22),
- Omega-gliadin (SEQ ID NO:23),
- Fel d 1A (UNIPROT P30438),
- Cat albumin (UNIPROT P49064),
- Can f 1 (UNIPROT 018873),
- Dog albumin (UNIPROT P49822), and
- RhCE (UNIPROT P18577),
there are obtained the following corresponding compounds of Formula 103' where:
- X is Abciximab and m is 10,
- X is Adalimumab and m is 11,
- X is Agalsidase alfa and m is 14,
- X is Agalsidase beta and m is 14,
- X is Aldeslukin and m is 6,
- X is Alglucosidase alfa and m is 13,
- X is Factor VIII and m is 100,
- X is Factor IX and m is 18,
- X is L-asparaginase and m is 5,
- X is Laronidase and m is 7,
- X is Octreotide and m is 1,
- X is Phenylalanine ammonia-lyase and m is 12,
- X is Rasburicase and m is 12,
- X is Insulin (SEQ ID NO:1) and m is 2,
- X is GAD-65 (SEQ ID NO:2) and m is 8,
- X is IGRP (SEQ ID NO:3) and m is 7,
- X is MBP (SEQ ID NO:4) and m is 6,
- X is MOG (SEQ ID NO:5) and m is 5,
- X is PLP (SEQ ID NO:6) and m is 8,
- X is MBP13-32 (SEQ ID NO:7) and m is 1,
- X is MBP83-99 (SEQ ID NO:8) and m is 1,
- X is MBP111-129 (SEQ ID NO:9) and m is 1,
- X is MBP146-170 (SEQ ID NO:10) and m is 2,
- X is MOG1-20 (SEQ ID NO:11) and m is 1,
- X is MOG35-55 (SEQ ID NO:12) and m is 2,
- X is PLP139-154 (SEQ ID NO:13) and m is 3,
- X is MART1 (SEQ ID NO:14) and m is 4,
- X is Tyrosinase (SEQ ID NO:15) and m is 8,
- X is PMEL (SEQ ID NO:16) and m is 5,
- X is Aquaporin-4 (SEQ ID NO:17) and m is 4,
- X is S-arrestin (SEQ ID NO:18) and m is 12,
- X is IRBP (SEQ ID NO:19) and m is 21,
- X is Conarachin and m is 21,
- X is Alpha-gliadin "33-mer" native (SEQ ID NO:20) and m is 1,
- X is Alpha-gliadin "33-mer" deamidated (SEQ ID NO:21) and m is 1,
- X is Alpha-gliadin (SEQ ID NO:22) and m is 1,
- X is Omega-gliadin (SEQ ID NO:23) and m is 1,
- X is Fel d 1 and m is 4,
- X is Cat albumin and m is 16,
- X is Can f 1 and m is 6,
- X is Dog albumin and m is 23, and
- X is RhCE and m is 10.

### 1E. Other Compounds of Formula 1aA

By following the procedure described in Example 1C and substituting the compounds of Formula 103', for example as obtained in Example 1D, there are obtained the following corresponding compounds of Formula 1aA:
- F1aA-Abciximab-m₁₀-n₈₀,
- F1aA-Adalimumab-m₁₁-n₈₀,
- F1aA-Agalsidase alfa-m₁₄-n₈₀,
- F1aA-Agalsidase beta-m₁₄-n₈₀,
- F1aA-Aldeslukin-m₆-n₈₀,
- F1aA-Alglucosidase alfa-m₁₃-n₈₀,
- F1aA-Factor VIII-m₁₀₀-n₈₀,
- F1aA-Factor IX-m₁₈-n₈₀,
- F1aA-L-asparaginase-m₅-n₈₀,
- F1aA-Laronidase-m₇-n₈₀,
- F1aA-Octreotide-m₁-n₈₀,
- F1aA-Phenylalanine ammonia-lyase-m₁₂-n₈₀,
- F1aA-Rasburicase-m₁₂-n₈₀,
- F1aA-Insulin-m₂-n₈₀,
- F1aA-GAD-65-m₈-n₈₀,
- F1aA-IGRP-m₇-n₈₀,
- F1aA-MBP-m₆-n₈₀,
- F1aA-MOG-m₅-n₈₀,
- F1aA-PLP-m₈-n₈₀,
- F1aA-MBP13-32-m₁-n₈₀,
- F1aA-MBP83-99-m₁-n₈₀,
- F1aA-MBP111-129-m₁-n₈₀,
- F1aA-MBP146-170-m₂-n₈₀,
- F1aA-MOG1-20-m₁-n₈₀,
- F1aA-MOG35-55-m₂-n₈₀,
- F1aA-PLP139-154-m₃-n₈₀,
- F1aA-MART1-m₄-n₈₀,
- F1aA-Tyrosinase-m₈-n₈₀,
- F1aA-PMEL-m₅-n₈₀,
- F1aA-Aquaporin-4-m₄-n₈₀,
- F1aA-S-arrestin-m₁₂-n₈₀,
- F1aA-IRBP-m₂₁-n₈₀,
- F1aA-Conarachin-m₂₁-n₈₀,
- F1aA-Alpha-gliadin "33-mer" native-m₁-n₈₀,
- F1aA-Alpha-gliadin "33-mer" deamidated-m₁-n₈₀,
- F1aA-Alpha-gliadin-m₁-n₈₀,
- F1aA-Omega-gliadin-m₁-n₈₀,
- F1aA-Fel d 1-m₄-n₈₀,
- F1aA-Cat albumin-m₁₆-n₈₀,
- F1aA-Can f 1-m₆-n₈₀,
- F1aA-Dog albumin-m₂₃-n₈₀, and
- F1aA-RhCE-m₁₀-n₈₀.

### 1F. Other Compounds of Formula 106A

By following the procedure described in Example 1B and substituting the pyridyl dithiol-poly(ethylene glycol)-NHS ester (Formula 104 where n is 80) with the following:
- Formula 104 where n is 12,
- Formula 104 where n is 33,
- Formula 104 where n is 40,
- Formula 104 where n is 43,
- Formula 104 where n is 50,
- Formula 104 where n is 60,
- Formula 104 where n is 75, and
- Formula 104 where n is 80,
there are obtained the following corresponding compounds of Formula 106A where:
- n is 12,
- n is 33,
- n is 40,
- n is 43,
- n is 50,
- n is 60,
- n is 75, and
- n is 84,

### 1G. Other Compounds of Formula 1aA

By following the procedure described in Example 1E and substituting the compound of Formula 106A with the compounds obtained in Example 1F, there are obtained the corresponding compounds of Formula 1aA where n is 12, 33, 40, 43, 50, 60, 75 and 84, such as:
- F1aA-Insulin-m₂-n₁₂,
- F1aA-Insulin-m₂-n₃₃,
- F1aA-Insulin-m₂-n₄₀,
- F1aA-Insulin-m₂-n₄₃,
- F1aA-Insulin-m₂-n₅₀,
- F1aA-Insulin-m₂-n₆₀,
- F1aA-Insulin-m₂-n₇₅, and
- F1aA-Insulin-m₂-n₈₄.

### 1H. Other Compounds of Formula 1aA

Similarly, by following the procedures described in Example 1A-G and substituting the compound glucosamine for galactosamine, there are obtained the corresponding compounds of Formula 1aA where Z' is C2 glucosamine.

### Example 2

### F1b-OVA-m₁-n₄-p₃₄-2NAcGAL

### 2A. Formula 103' where X' is Ovalbumin and m is 1

In an endotoxin-free tube, OVA (6.5 mg, 0.000155 mmol) was added to 200 µl of pH 8.0 PBS containing 5 mM EDTA and stirred. Separately, 1 mg of Taut's Reagent was dissolved in 100 µl of pH 7.0 PBS, and 43 µl (0.00310 mmol) of the Traut's Reagent solution so obtained was added to the stirred solution of OVA with continued stirring. After 1 hour, non-reacted Traut's Reagent was removed using a centrifugal size exclusion column to afford the product of Formula 103'.

### 2B. Formula 1b where X' is Ovalbumin, m is 1, n is 4, p is 34, R⁹ is a direct bond and Z" is 2NAcGAL

In a micro centrifuge tube, poly(Galactosamine Methacrylate)-(pyridyl disulfide) (Formula 201) (20.0 mg, 0.0020 mmol) was solubilized in 50 µl of pH 8.0 PBS containing 5 mM EDTA. To this was added the purified OVA-Traut product from Example 2A followed by stirring for 1 hour. The resulting product of Formula 1b was purified by passing the reaction mixture through a centrifugal size exclusion column. Characterization (UHPLC SEC, gel electrophoresis) confirmed the identity of the product. (See Fig. 5.)

### 2C. Other Compounds of Formula 1b

By following the procedure described in Example 2B and substituting the compounds of Formula 103', for example as obtained in Example 1D, there are obtained the following corresponding compounds of Formula 1b:
- F1b-Abciximab-m₁₀-n₄-p₃₄-2NAcGAL,
- F1b-Adalimumab-m₁₁-n₄-p₃₄-2NAcGAL,
- F1b-Agalsidase alfa-m₁₄-n₄-p₃₄-2NAcGAL,
- F1b-Agalsidase beta-m₁₄-n₄-p₃₄-2NAcGAL,
- F1b-Aldeslukin-m₆-n₄-p₃₄-2NAcGAL,
- F1b-Alglucosidase alfa-m₁₃-n₄-p₃₄-2NAcGAL,
- F1b-Factor VIII-m₁₀₀-n₄-p₃₄-2NAcGAL,
- F1b-Factor IX-m₁₈-n₄-p₃₄-2NAcGAL,
- F1b-L-asparaginase-m₅-n₄-p₃₄-2NAcGAL,
- F1b-Laronidase-m₇-n₄-p₃₄-2NAcGAL,
- F1b-Octreotide-m₁-n₄-p₃₄-2NAcGAL,
- F1b-Phenylalanine ammonia-lyase-m₁₂-n₄-p₃₄-2NAcGAL,
- F1b-Rasburicase-m₁₂-n₄-p₃₄-2NAcGAL,
- F1b-Insulin-m₂-n₄-p₃₄-2NAcGAL,
- F1b-GAD-65-m₈-n₄-p₃₄-2NA_{C}GAL,
- F1b-IGRP-m₇-n₄-p₃₄-2NAcGAL,
- F1b-MBP-m₆-n₄-p₃₄-2NAcGAL,
- F1b-MOG-m₅-n₄-p₃₄-2NAcGAL,
- F1b-PLP-m₈-n₄-p₃₄-2NAcGAL,
- F1b-MBP13-32-m₁-n₄-p₃₄-2NAcGAL,
- F1b-MBP83-99-m₁-n₄-p₃₄-2NAcGAL,
- F1b-MBP111-129-m₁-n₄-p₃₄-2NAcGAL,
- F1b-MBP146-170-m₂-n₄-p₃₄-2NAcGAL,
- F1b-MOG1-20-m₁-n₄-p₃₄-2NAcGAL,
- F1b-MOG35-55-m₂-n₄-p₃₄-2NAcGAL,
- F1b-PLP139-154-m₃-n₄-p₃₄-2NAcGAL,
- F1b-MART1-m₄-n₄-p₃₄-2NAcGAL,
- F1b-Tyrosinase-m₈-n₄-p₃₄-2NAcGAL,
- F1b-PMEL-m₅-n₄-p₃₄-2NAcGAL,
- F1b-Aquaporin-4-m₄-n₄-p₃₄-2NAcGAL,
- F1b-S-arrestin-m₁₂-n₄-p₃₄-2NAcGAL,
- F1b-IRBP-m₂₁-n₄-p₃₄-2NAcGAL,
- F1b-Conarachin-m₂₁-n₄-p₃₄-2NAcGAL,
- F1b-Alpha-gliadin "33-mer" native-m₁-n₄-p₃₄-2NAcGAL,
- F1b-Alpha-gliadin "33-mer" deamidated-m₁-n₄-p₃₄-2NAcGAL,
- F1b-Alpha-gliadin-m₁-n₄-p₃₄-2NAcGAL,
- F1b-Omega-gliadin-m₁-n₄-p₃₄-2NAcGAL,
- F1b-Fel d 1-m₄-n₄-p₃₄-2NAcGAL,
- F1b-Cat albumin-m₁₆-n₄-p₃₄-2NAcGAL,
- F1b-Can f 1-m₆-n₄-p₃₄-2NAcGAL,
- F1b-Dog albumin-m₂₃-n₄-p₃₄-2NAcGAL, and
- F1b-RhCE-m₁₀-n₄-p₃₄-2NAcGAL.

### 1D. Other Compounds of Formula 1b

Similarly, by following the procedures described in Example 2B-C and substituting the compound poly(Glucosamine Methacrylate)-(pyridyl disulfide) or poly(Galactosamine Methacrylate)-(pyridyl disulfide), there are obtained the corresponding compounds of Formula 1b where Z" is 2-NAcGLU.

### Example 3

### F1f-OVA-m₁-n₄-p₃₃-2NAcGAL

### 3A. Formula 1f where X' is Ovalbumin and m is 1, n is 4, p is 33, R⁹ is a direct bond and Z" is 2NAcGAL

In an endotoxin-free tube, OVA (4.0 mg, 0.0000952381 mmol) was added to 0.1 ml of pH 7.4 PBS and stirred. Separately, poly-(n-Acetylgalactosamine)-p-nitrophenyol carbonate of Formula 601 where n is 4 and p is 33 (33.0 mg, 0.002380952 mmol) was added to 100 µl of pH 7.5 PBS and vortexed until dissolved. The two solutions were combined and the mixture was stirred vigorously for 1 hour. The mixture was then collected and dialyzed for 3 days against pH 7.4 PBS (30 kDa molecular weight cut off) to afford the product of Formula 1f.

### 3B. Formula 1f where X' is Ovalbumin and m is 1, n is 4, p is 33, R⁹ is a direct bond and Z" is 2NAcGLU

Similarly, by following the procedure of Example 3A and substituting poly-(n-Acetylglucosamine)-p-nitrophenyol carbonate for poly-(n-Acetylgalactosamine)-p-nitrophenyol carbonate, there is obtained the corresponding compound of Formula 1f where Z" is 2NAcGLU.

### Example 4

### F1g-PVA-m₁-p₉₀-2NAcGAL

### 4A. Formula 1g where X' is Ovalbumin and m is 1, p is 90, R⁹ is a direct bond and Z" is 2NAcGAL

In an endotoxin-free tube, OVA (5.0 mg, 0.000119048 mmol) was added to 0.2 ml of pH 7.4 PBS and stirred. To the stirring solution was added 75 mg (0.00297619 mmol) of Poly(Galactosamine Methacrylate)-NHS (Formula 701) dissolved in 0.4 ml of pH 7.4 PBS. The mixture was allowed to stir for 2 hours. The mixture was then collected and dialyzed for 3 days against pH 7.4 PBS (30 kDa molecular weight cut off) to afford the product of Formula 1g.

### 4B. Formula 1g where X' is Ovalbumin and m is 1, p is 90, R⁹ is a direct bond and Z" is 2NAcGLU

Similarly, by following the procedure of Example 4A and substituting Poly(Glucosamine Methacrylate)-NHS for Poly(Galactosamine Methacrylate)-NHS, there is obtained the corresponding compound of Formula 1g where Z" is 2NAcGLU.

### Example 5

### F1h-OVA-m₂-n₄₅-p₅₅-q₄-2NAcGAL

### 5A. Formula 802' where X' is Ovalbumin, m is 2 and n is 45

In an endotoxin-free tube, OVA (3.0 mg, 0.0000714286 mmol) was added to 150 µl of pH 8.0 PBS containing 5 mM EDTA and stirred. Dibenzocyclooctyne-PEG-(p-nitrophenyl carbonate) (Formula 801) (5.265 mg, 0.002142857 mmol) dissolved in DMF was added to the OVA solution and stirred for 1 hour. The excess dibenzocyclooctyne-PEG-(p-nitrophenyl carbonate) was removed using a centrifugal size exclusion column to afford the product of Formula 802'.

### 5B. Formula 1h where X' is Ovalbumin, m is 2, n is 45, p is 55, q is 4, R⁸ is CH₂, R⁹ is a direct bond and Z" is 2NAcGAL

Poly(Galactosamine Methacrylate)-N3 (Formula 803 where p is 55, q is 4 and Z" is N-acetylgalactosamine) (33 mg, 0.002142857 mmol) was dissolved in 100 µl of pH 7.4 PBS and added to the product of Example 5A with stirring. After 1 hour, the resulting product of Formula 1h was purified by centrifugal size exclusion chromatography.

### 5C. Formula 1h where X' is Ovalbumin, m is 2, n is 45, p is 55, q is 4, R⁸ is CH₂, R⁹ is a direct bond and Z" is 2NAcGLU

Similarly, by following the procedure of Example 5B and substituting Poly(Glucosamine Methacrylate)-NHS for Poly(Galactosamine Methacrylate)-NHS, there is obtained the corresponding compound of Formula 1h where Z" is 2NAcGLU.

### Example 6

### F1j-OVA-m₁₀-n₄₅-p₅₅-q₄-2NAcGAL

### 6A. Formula 103' where X' is Ovalbumin and m is 10

In an endotoxin-free tube, OVA (5.0 mg, 0.00019 mmol) was added to 150 µl of pH 8.0 PBS containing 5 mM EDTA and stirred. Separately, 1 mg of Taut's Reagent was dissolved in 100 µl of pH 7.0 PBS, and 16 µl (0.0019 mmol) of the Traut's Reagent solution so obtained was added to the stirred solution of OVA with continued stirring. After 1 hour, non-reacted Traut's Reagent was removed using a centrifugal size exclusion column to afford the product of Formula 103'.

### 6B. Formula 902" where X' is Ovalbumin, m is 10 and n is 45

Dibenzocyclooctyne-PEG-(pyridyl disulfide) (Formula 901 where n is 45) (6.0 mg, 0.00238 mmol) was dissolved in DMF and the resulting solution was added to the OVA solution obtained in Example 6A and stirred for 1 hour. The excess dibenzocyclooctyne-PEG-(pyridyl disulfide) was removed using centrifugal size exclusion chromatography to afford the product of Formula 902".

### 6C. Formula 1j where X' is Ovalbumin, m is 10, n is 45, p is 55, q is 4, R⁸ is CH₂, R⁹ is a direct bond and Z" is 2NAcGAL

Poly(Galactosamine Methacrylate)-N3 (Formula 803 where p is 55, q is 4 and Z" is N-acetylgalactosamine) (36 mg, 0.00238 mmol) was dissolved in 150 µl of pH 7.4 PBS and added to the product of Example 6B with stirring. After 1 hour, the resulting product of Formula 1j was purified (excess p(GMA)-N3 removed) by centrifugal size exclusion chromatography. Characterization (UHPLC SEC, gel electrophoresis) confirmed the identity of the product.

### 6D. Formula 1j where X' is Ovalbumin, m is 10, n is 45, p is 55, q is 4, R⁸ is CH₂, R⁹ is a direct bond and Z" is 2NAcGLU

Similarly, by following the procedure of Example 6C and substituting Poly(Glucosamine Methacrylate)-NHS for Poly(Galactosamine Methacrylate)-NHS, there is obtained the corresponding compound of Formula 1j where Z" is 2NAcGLU.

### Example 7

### F1L-OVA-m₂-n₈₀-p₅₅-q₄-2NAcGAL

### 7A. Formula 1002 where X' is Ovalbumin, m is 2 and n is 80

Dibenzocyclooctyne-PEG-(pyridyl disulfide) (Formula 1001 where n is 80) (9.0 mg, 0.00238 mmol) was dissolved in DMF and the resulting solution was added to a purified OVA solution of Formula 103' (where X' is Ovalbumin and m is 2), for example prepared as described in Example 6A and stirred for 1 hour. The excess dibenzocyclooctyne-PEG-(pyridyl disulfide) was removed using centrifugal size exclusion chromatography to afford the product of Formula 1002.

### 7B. Formula 1L where X' is Ovalbumin, m is 2, n is 80, p is 55, q is 4, R⁸ is CH₂, R⁹ is a direct bond and Z" is 2NAcGAL

Poly(Galactosamine Methacrylate)-N3 (Formula 803 where p is 55, q is 4 and Z" is N-Acetylgalactosamine) (36 mg, 0.00238 mmol) was dissolved in 150 µl of pH 7.4 PBS and added to the product of Example 7A with stirring. After 1 hour, the resulting product of Formula 1L was purified (excess poly(Galactosamine Methacrylate)-N3 removed) by centrifugal size exclusion chromatography. Characterization (UHPLC SEC, gel electrophoresis) confirmed the identity of the product.

### 7C. Formula 1L where X' is Ovalbumin, m is 2, n is 80, p is 55, q is 4, R⁸ is CH₂, R⁹ is a direct bond and Z" is 2NAcGLU

Similarly, by following the procedure of Example 7B and substituting Poly(Glucosamine Methacrylate)-NHS for Poly(Galactosamine Methacrylate)-NHS, there is obtained the corresponding compound of Formula 1jLwhere Z" is 2NAcGLU.

### Example 8

### Preparation of poly(Galactosamine methacrylate) Polymers

### 8A. Galactosamine Methacrylate

To stirred galactosamine hydrochloride (2.15 g, 10.0 mmol) was added 0.5 M sodium methoxide (22 ml, 11.0 mmol). After 30 minutes, methacrylate anhydride (14.694 g, 11.0 mmol) was added and stirring continued for 4 hours. The resulting galactosamine methacrylate was loaded onto silica gel via rotovap and purified via column chromatography using DCM:MeOH (85:15).

### 8B. Formula 201 where n is 4 and p is 30

Galactose methacrylate (600 mg, 2.43 mmol), 2-(2-(2-(2-(pyridin-2-yldisulfanyl)ethoxy)ethoxy)ethoxy)ethyl 2-((phenylcarbonothioyl)thio)acetate (44.8 mg, 0.081 mmol) and AIBN (3.174089069 mg, 0.016 mmol) were added to 1.5 ml of DMF in a Schlenk Flask. The reaction mixture was subjected to 4 freeze-thaw cycles and then stirred at 70°C for 6 hours. The desired polymer product of Formula 201 was precipitated in 12 ml of methanol, and excess solvent was removed under reduced pressure.

### 8C. Formula 201 where n is 4 and p is 30

Similarly, by following the procedure of Example 8B and substituting Glucose methacrylate for galactose methacrylate there are obtained the corresponding poly(Glucosamine methacrylate) polymers.

### Example 9

### Preparation of F1aA-PE-m₃-n₈₀

### 9A. Formula 103' where X' is Phycoerythrin

In an endotoxin-free tube, phycoerythrin ("PE") (purchased from Pierce) (200 µl, 0.000004 mmol) was added to 50 µl of pH 8.0 PBS containing 5 mM EDTA and stirred. Separately, 1 mg of Taut's Reagent was dissolved in 100 µl of pH 7.0 PBS, and 2 µl (0.00013 mmol) of the Traut's Reagent solution so obtained was added to the stirred solution of PE with continued stirring. After 1 hour, excess Traut's Reagent was removed using a centrifugal size exclusion column to afford the product of Formula 103'.

### 9B. Formula 106A where n is 80

In an endotoxin-free tube, galactosamine (7.0 mg, 0.03246 mmol) was dissolved with stirring in 100 µl of pH 8.0 PBS containing 5 mM EDTA. Pyridyl dithiol-poly(ethylene glycol)-NHS ester (Formula 104 where n is 80) (16.23 mg, 0.00464 mmol) dissolved in 50 µl of pH 7.0 PBS was added to the stirring solution of galactosamine. After 1 hour, the resulting product of Formula 106A was ready to be used without further purification.

### 9C. Formula 1a where X' is Phycoerythrin, m is 3, n is 80 and Z' is galactosamine

The purified PE-Traut conjugates prepared in Example 9A were added directly to the stirring product of Formula 106A prepared in Example 9B. After 1hour, the resulting product of Formula 1a was purified by passing the reaction mixture through a centrifugal size exclusion column. Characterization (UHPLC SEC, gel electrophoresis) confirmed the identity of the product.

### 9D. Formula 1a where X' is Phycoerythrin, m is 3, n is 80 and Z' is glucosamine

Similarly, by following the procedure of Example 9B and C and substituting glucosamine for galactosamine there is obtained the corresponding compound of Formula 1a where Z" is glucosamine.

### Example 10

### Hepatic Distribution

10A. F1aA-PE-m₃-n₈₀ was prepared, for example, as described in Example 9. A 30µg/100µl solution in sterile saline was prepared for injection.

The F1aA-PE-m₃-n₈₀ solution (30µg) was administered to one of three groups of C57 black 6 mice 3 per group) via tail vein injection. The two other groups of mice received an equivalent volume of phycoerythrin in 100 µl of saline or saline vehicle. Three hours after administration, the livers and spleens of these animals were harvested and the level of cellular fluorescents in these organs was determined by flow cytometry as an indication of cellular PE content.

As shown in Figs. 1A-1D, sinusoidal endothelial cells (LSECs) (1A), hepatocytes (1C), Kupffer cells (KC) (1B), and other antigen-presenting cells (APCs) (1D) from the livers of mice treated with F1aA-PE-m₃-n₈₀ exhibited at least a three-fold increase in fluorescence as compared with animals that received PE solution. No detectible difference in fluorescence was found in spleen cells harvested from the three groups. These results confirm that F1aA-PE-m₃-n₈₀ has sufficient specificity for binding to antigen-presenting cells in the liver.

10B. By following the procedure described in Example 10A and substituting F1aA-PE-m₃-n₈₀ with the compounds F1b-PE-m₃-n₄-p₃₄-2NAcGAL, F1f-PE-m₃-n₄-p₃₃-2NAcGAL, F1g-PE-m₃-p₉₀-2NAcGAL, F1h-PE-m₃-n₄₅-p₅₅-q₄-2NAcGAL, F1j-PE-m₃-n₄₅-p₅₅-q₄-2NAcGAL, F1L-PE-m₃-n₈₀-p₅₅-q₄-2NAcGAL, F1m-PE-m₃-n₈₀-p₃₀-q₄-CMP-2NHAc, F1m-PE-m₃-n₆₂-p₃₀-q₈-CMP-2OH, F1n-PE-m₃-n₁-p₃₀-q₄-CMP-2NHAc and F1n-PE-m₃-n₃₃-p₃₀-q₈-CMP-2OH, prepared, for example, as described with reference to Example 9 by substitution for X in Examples 2B, 3, 4, 5B, 6B, 7B, 15G, 15L, 16B and 16F, respectively it is confirmed that the compounds F1aA-PE-m₃-n₈₀ with the compounds F1b-PE-m₃-n₄-p₃₄-2NAcGAL, F1f-PE-m₃-n₄-p₃₃-2NAcGAL, F1g-PE-m₃-p₉₀-2NAcGAL, F1h-PE-m₃-n₄₅-p₅₅-q₄-2NAcGAL, F1j-PE-m₃-n₄₅-p₅₅-q₄-2NAcGAL, F1L-PE-m₃-n₈₀-p₅₅-q₄-2NAcGAL, F1m-PE-m₃-n₈₀-p₃₀-q₄-CMP-2NHAc, F1m-PE-m₃-n₆₂-p₃₀-q₈-CMP-2OH, F1n-PE-m₃-n₁-p₃₀-q₄-CMP-2NHAc and F1n-PE-m₃-n₃₃-p₃₀-q₈-CMP-2OH have sufficient specificity for binding to antigen-presenting cells in the liver.

10C. By following the procedure described in Example 10A and 10B and substituting the corresponding glucosylated compounds for the galactosylated compounds, it is confirmed that the glucolsylated compounds have sufficient specificity for binding to antigen-presenting cells in the liver.

### Example 11

### Proliferation of Antigen-specific OT1 CD8+ T cells

11A. F1aA-OVA-m₄-n₈₀ synthesized, for example, as described in Example 1, was prepared as a 10µg/100µl saline solution for injection. On day 0, 106 OT-I T cells were fluorescently labeled and adoptively transferred into 3 groups of CD 45.2 mice (5 per group) via tail vein injection. The next day (i.e. Day 1), to each of the 3 groups of mice were administered, respectively, 10 µg of F1aA-OVA-m4-n80, OVA or saline via tail vein injection. On day 6, the animals were sacrificed and the % of splenic proliferating OT-I cells was determined via florescence activated cell sorting.

The results from this study (see Fig. 2) show that the percentage of proliferating OTI T cells in mice treated with F1aA-OVA-m₄-n₈₀ ("Gal-OVA" in Fig. 2) was significantly greater than the percentage of proliferating OTI cells in the spleens of mice treated with OVA or saline ("naïve" in Fig. 2). The increase in OTI cell-proliferation demonstrates the increased CD8+ T-cell cross-priming in animals treated with F1aA-OVA-m₄-n₈₀ versus the other therapies. In concert with the results from Example 12, these results indicate that the ability of F1aA-OVA-m₄-n₈₀ to target antigens to the liver increases OVA presentation by antigen presenting cells in the liver to OVA-specific OTI T cells.

11B. To distinguish T cells being expanded into a functional effector phenotype from those being expanded and deleted, the proliferating OTI CD8⁺ T cells were analyzed for phosphatidylserine exposure by way of Annexin-V binding, as a hallmark of apoptosis and thus deletion, as well as the exhaustion marker programmed death-1 (PD-1). As shown in Figs. 3A-3B, F1aA-OVA-m₄-n₈₀ ("Gal-OVA" in Figs. 3A-3B) induced much higher numbers of Annexin-V⁺ and PD-1⁺ proliferating OTI CD8⁺ T cells than soluble OVA. These data demonstrate that, in accordance with several embodiments disclosed herein, coupling an antigen to which tolerance is to be induced with linkers and liver targeting moieties as disclosed herein result in unexpectedly enhanced generation of T cells having the capacity to be immunologically functional.

11C. By following the procedure described in Examples 11A and 11B, and substituting F1aA-OVA-m₄-n₈ with the compounds of Formula 1 obtained, for example, as described in Examples 3A, 4A, 5B, 6C, 7B and 19G, it is shown the compounds from Examples 3A, 4A, 5B, 6C, 7B and 19G induce much higher numbers of Annexin-V⁺ and PD-1⁺ proliferating OTI CD8⁺ T cells than soluble OVA.

11D. By following the procedure described in Examples 11A and 11B and substituting F1aA-OVA-m₄-n₈ with the compounds of Formulae 1 and 2 obtained, for example, as described in Examples 1E, 1G, 2C, 15I, 15L, 16B, 16D and 16F, and substituting OVA with the antigens corresponding to X (or X' or X"), respectively, it is shown that the compounds from Examples 1E, 1G, 2C, 15I, 15L, 16B, 16D and 16F induce much higher numbers of Annexin-V⁺ and PD-1⁺ proliferating OTI CD8⁺ T cells than soluble antigen X.

11E. By following the procedure described in Example 11A-D and substituting the corresponding glucosylated compounds for the galactosylated compounds, it is confirmed that the glucolsylated compounds induce much higher numbers of Annexin-V⁺ and PD-1⁺ proliferating OTI CD8⁺ T cells than soluble antigen X.

### Example 12

### F1aA-OVA-m₄-n₈ does not induce an OVA-specific antibody response

12A. In order to assess the humoral immune response to F1aA-OVA-m₄-n₈ we treated mice with a weekly i.v. injection of either F1aA-OVA-m₄-n₈ or OVA, then measured the levels of OVA-specific antibodies in the blood. On day 0, 7, and 14 of the experiment, mice were administered an i.v. injection of 100 µl of saline containing one of the following: 1.) 6 µg of OVA; 2.) 6 µg of F1aA-OVA-m₄-n₈; 3.) 30 µg of OVA; 4.) 30 µg of F1aA-OVA-m₄-n₈, or 5.) saline alone. Each group contained 5 mice. On day 19, the mice were bled via cheek puncture, and the titer of OVA-specific antibodies in each mouse's blood was determined via ELISA. The results for this study show that although mice treated with 6 and 30 µg of OVA had increased OVA-specific antibody titers, mice treated with both 6 and 30 µg of F1aA-OVA-m₄-n₈ ("Gal-OVA" in Fig. 4) had blood titers similar to mice treated with saline (i.e. vehicle treated animals) (Fig. 4). For example mice treated with 6 and 30 µg of OVA had an average antibody titer of 3.5 and 2.5, respectively; whereas, mice treated with 6 and 30 µg of OVA had an average antibody titer of 0.75 and 0.25, respectively. Thus, these data demonstrate that coupling an antigen to which immune tolerance is desired to a linker and liver targeting moiety according to several embodiments disclosed herein results in significantly less antigen specific antibody generation. As such, these data demonstrate that the immune response to the antigen delivered to the liver by the compositions disclosed herein is reduced.

12B. By following the procedure described in Example 12A and substituting F1aA-OVA-m₄-n₈ with the compounds of Formula 1 obtained, for example, as described in Examples 3A, 4A, 5B, 6C, 7B and 15G, it is shown that mice treated with the compounds from Examples 3A, 4A, 5B, 6C, 7B and 15G have OVA-specific antibody titers similar to mice treated with saline.

12C. By following the procedure described in Example 12B and substituting F1aA-OVA-m₄-n₈ with the compounds of Formula 1 obtained, for example, as described in Examples 1E, 1G, 2C, 15!, 15L, 16B, 16D and 16F, and substituting OVA with the antigens corresponding to X (or X' or X"), respectively, it is shown that mice treated with the compounds from Examples 1E, 1G, 2C, 15I, 15L, 16B, 16D and 16F have antigen X-specific antibody titers similar to mice treated with saline.

12D. By following the procedure described in Example 12A-C and substituting the corresponding glucosylated compounds for the galactosylated compounds, it is confirmed that the glucolsylated compounds have antigen X-specific antibody titers similar to mice treated with saline.

### Example 13

### F1aA-OVA-m₄-n₈ depletes OVA-specific antibodies

13A. Mice that had different OVA-antibody blood titers (each mouse had a titer from 0 to 4.5) were treated with an i.v. injection of 20 µg of F1aA-OVA-m₄-n₈ solubilized in 100 µl saline. Mice were given i.v. injections of F1aA-OVA-m₄-n₈ on days 0, 5, 7, 12, and 14 (Injections of F1aA-OVA-m₄-n₈ are labeled as "Gal-OVA" and shown as green arrows on the x-axis of Fig. 5). In order to determine the ability of F1aA-OVA-m₄-n₈ to deplete serum OVA-specific antibodies, the mice were bled on day -1 to establish an initial antibody titer and then subsequent bleeds were carried out after each injection of F1aA-OVA-m₄-n₈ on days 2, 6, 9. 13, and 16. The antibody titer for each mouse was determined via ELISA. The results from this study show that F1aA-OVA-m₄-n₈ is able to deplete serum antibody levels in mice. For example, one day after the first F1aA-OVA-m₄-n₈ injection (i.e. day 2), mice with positive OVA-antibody titers experience a 5 to 100-fold decrease in serum antibody levels (Fig. 5). These results show that although over the course of the 19 day experiment, antibody titers did increase for certain mice, the titer levels never reached the initial antibody titer measured on Day -1 and subsequent doses of F1aA-OVA-m₄-n₈ were effective in reducing these transient increases in antibody titers. These results demonstrate that F1aA-OVA-m₄-n₈ has the specificity to bind serum OVA-specific antibodies and the kinetics required to deplete OVA-specific serum antibodies.

13B. By following the procedure described in Example 13A and substituting F1aA-OVA-m₄-n₈ with the compounds of Formula 1 obtained, for example, as described in Examples 3A, 4A, 5B, 6C, 7B and 15G, it is shown that the compounds from Examples 3A, 4A, 5B, 6C, 7B and 15G have the specificity to bind serum OVA-specific antibodies and the kinetics required to deplete OVA-specific serum antibodies.

13C. By following the procedure described in Example 13A and substituting F1aA-OVA-m₄-n₈ with the compounds of Formula 1 obtained, for example, as described in Examples 1E, 1G, 2C, 10D, 151, 15L, 16B, 16D and 16F, and substituting OVA with the antigens corresponding to X (or X' or X"), respectively, it is shown that the compounds from Examples 1E, 1G, 2C, 151, 15L, 16B, 16D and 16F have the specificity to bind serum antigen X-specific antibodies and the kinetics required to deplete antigen X-specific serum antibodies.

13D. By following the procedure described in Example 13A-C and substituting the corresponding glucosylated compounds for the galactosylated compounds, it is confirmed that the glucolsylated compounds have the specificity to bind serum antigen X-specific antibodies and the kinetics required to deplete antigen X-specific serum antibodies.

### Example 14

### OT-1 challenge-to-tolerance model

14A. Using an established OTI challenge-to-tolerance model (Liu, Iyoda, et al., 2002), the ability of F1aA-OVA-m₄-n₈ (mGal-OVA) and F1b-OVA-m₁-n₄-p₃₄ (pGal-OVA) to prevent subsequent immune responses to vaccine-mediated antigen challenge were demonstrated - even with a challenge involving a very strong bacterially-derived adjuvant (i.e. lipopolysaccharide). To tolerize, 233 nmol of either F1aA-OVA-m₄-n₈, F1b-OVA-m₁-n₄-p₃₄, or soluble OVA were intravenously administered in 100 µl saline at 1 and 6 days following adoptive transfer of OTI CD8⁺ (CD45.2⁺) T cells to CD45.1⁺ mice (n = 5 mice per group). After 9 additional days to allow potential deletion of the transferred T cells, the recipient mice were then challenged with OVA (10 µg) adjuvanted with lipopolysaccharide (LPS) (50 ng) by intradermal injection. Characterization of the draining lymph nodes 4 d after challenge allowed a determination as to whether or not deletion actually took place.

14B. Intravenous administration of F1aA-OVA-m₄-n₈ and F1b-OVA-m₁-n₄-p₃₄ resulted in profound reductions in OTI CD8⁺ T cell populations in the draining lymph nodes as compared to mice treated with unmodified OVA prior to antigen challenge with LPS, demonstrating deletional tolerance. For example, Figs. 6A-6F show that the draining lymph nodes from mice treated with either F1aA-OVA-m₄-n₈ (mGal-OVA) and F1b-OVA-m₁-n₄-p₃₄ (pGal-OVA) contained over 9-fold fewer OTI CD8⁺ T cells as compared to OVA-treated mice, and more than 43-fold fewer than the challenge control mice that did not receive intravenous injections of antigen; responses in spleen cells were similar. These results demonstrate that F1aA-OVA-m₄-n₈ and F1b-OVA-m₁-n₄-p₃₄ mitigated an OVA-specific immune response after adjuvented OVA challenge, thus establishing that the compositions disclosed herein are suitable for induction of immune tolerance. As to characterization, Fig. 7 shows characterization of F1aA-OVA-m₄-n₈₀ and F1b-OVA-m₁-n₄₄-p₃₄.

14C. By following the procedure described in Examples 14A and B, and substituting F1aA-OVA-m₄-n₈ and F1b-OVA-m₁-n₄-p₃₄ with the compounds of Formula 1 obtained, for example, as described in Examples 3A, 4A, 5B, 6C, 7B and 15G, it is shown that the compounds from Examples 3A, 4A, 5B, 6C, 7B and 15G mitigate an OVA-specific immune response after adjuvented OVA challenge.

14D. By following the procedure described in Examples 14A and B, and substituting F1aA-OVA-m₄-n₈ and F1b-OVA-m₁-n₄-p₃₄ with the compounds of Formula 1 obtained, for example, as described in Examples 1E, 1G, 2C, 15!, 15L, 16B, 16D and 16F, and substituting OVA with the antigens corresponding to X (or X' or X"), respectively, it is shown that the compounds from Examples 1E, 1G, 2C, 15!, 15L, 16B, 16D and 16F mitigate an antigen X-specific immune response after adjuvented antigen X challenge.

14E. By following the procedure described in Example 14A-D and substituting the corresponding glucosylated compounds for the galactosylated compounds, it is confirmed that the glucolsylated compounds mitigate an antigen X-specific immune response after adjuvanted antigen X challenge.

### Example 15

### F1m-OVA-m₂-n₈₀-p₃₀-q₄-CMP-2NHAc

### 15A. Formula 1102 where R³ is NHAc and R⁴ is OH

*N*-Acetyl-D-galactosamine (Formula 1101 where R³ is NHAc and R⁴ is OH) (5g, 22.6 mmol) was added to a stirred solution of chloroethanol (200 ml) at room temperature. The solution was cooled to 4°C and acetylchloride was added drop-wise to the solution. The solution was brought to room temperature and then heated to 70°C. After 4 hours, the unreacted choroethanol was removed under reduced pressure. 100 ml of ethanol was added to the crude product and the resulting solution was stirred in the presence of carbon for 2 hours. The solution was filtered, and the solvent was removed under reduced pressure. The corresponding product of Formula 1102, *N*-(2-(2-chloroethoxy)-4,5-dihydroxy-6-(hydroxymethyl)tetrahydro-2H-pyran-3-yl)acetamide, was used without further purification.

### 15B. Formula 1103 where R³ is NHAc and R⁴ is OH

The *N*-(2-(2-chloroethoxy)-4,5-dihydroxy-6-(hydroxymethyl)tetrahydro-2H-pyran-3-yl)acetamide prepared in Example 15A (2g, 7.4 mmol) was added to a stirred solution of DMF (100 ml) and sodium azide (4g, 61.5 mmol). The solution was headed at 90°C for 12 hours and then filtered. The residual solvent was removed under reduced pressure and the crude product was purified via flash chromatography (10% MeOH in dichloromethane) to give the corresponding product of Formula 1103, *N-*(2-(2-azidoethoxy)-4,5-dihydroxy-6-(hydroxymethyl)tetrahydro-2H-pyran-3-yl)acetamide.

### 15C. Formula 1104 where R³ is NHAc and R⁴ is OH

The *N-*(2-(2-azidoethoxy)-4,5-dihydroxy-6-(hydroxymethyl)tetrahydro-2*H-*pyran-3-yl)acetamide prepared in Example 15B (2 g, 6.9 mmol) was added to a solution of palladium on carbon and ethanol (50 ml). The solution was stirred under hydrogen gas (3 atm) for 4 hours. The resulting solution was filtered and the residual solvent was removed under reduced pressure to afford the corresponding product of Formula 1104, *N*-(2-(2-aminoethoxy)-4,5-dihydroxy-6-(hydroxymethyl)tetrahydro-2*H*-pyran-3-yl)acetamide, which was used without further purification.

### 15D. Formula 1105 where R³ is NHAc and R⁴ is OH

The *N-*(2-(2-aminoethoxy)-4,5-dihydroxy-6-(hydroxymethyl)tetrahydro-2*H*-pyran-3-yl)acetamide prepared in Example 15C (1.0 g, 3.78 mmol) was added to a solution of methacrylate anhydride (0.583 g, 3.78 mmol) in DMF (50 ml). Triethylamine was then added to the solution and the reaction was stirred for 2 hours at room temperature. After 2 hours, the excess solvent was removed under reduced pressure, and the corresponding product of Formula 1105, *N*-(2-((3-acetamido-4,5-dihydroxy-6-(hydroxymethyl)tetrahydro-2*H*-pyran-2-yl)oxy)ethyl)methacrylamide, was isolated via flash chromatography.

### 15E. Formula 1107 where p is 30, q is 4, R³ is NHAc, R⁴ is OH and R⁸ is CMP

An azide-modified uRAFT agent of Formula 1106 where q is 4 (28 mg) was added to a solution of *N-*(2-((3-acetamido-4,5-dihydroxy-6-(hydroxymethyl)tetrahydro-2*H-*pyran-2-yl)oxy)ethyl)methacrylamide prepared in Example 15D (579 mg, 1.74 mmol) and azobisisobutyronitrile (2.2 mg, 0.0116 mmol) in DMF. The reaction mixture was subjected to 4 free-pump-thaw cycles, and then stirred at 70°C. After 12 hours, the polymer product of Formula 1107, where p is 30 and q is 4 was precipitated from the reaction mixture via the addition of methanol. The solvent was decanted from the solid and the solid was collected and residual solvent was removed via reduced pressure.

### 15F. Formula 1109 where X' is OVA, m is 2 and n is 80

Ovalbumin (5 mg, 0.00012 mmol) was added to 100 µl of sodium phosphate buffer (pH 8.0) and stirred. To this solution was added 5 mg of the compound of Formula 1108 where n is 80. After 1 hour, the unreacted compound of Formula 1108 was removed from the solution via centrifugal size-exclusion chromatography. The resulting buffered solution containing the corresponding product of Formula 1109 was used in the next reaction without further purification.

### 15G. Formula 1m where X' is OVA, m is 2, n is 80, p is 30, q is 4, R³ is NHAc and R⁸ is CMP

The solution prepared in Example 15F was added to 100 µl of sodium phosphate buffer (pH 8.0) which contained 10 mg of the product of Formula 1107 prepared in Example 15E. The reaction was allowed to stir for 2 hours and then the excess Formula 1107 was removed via centrifugal size exclusion chromatography to afford the corresponding isomeric product of Formula 1m in solution, which was used in biological studies without further purification. The R³ substituent is shown in the name of the title compound as 2NHAc.

### 15H. Other Compounds of Formula 1109

By following the procedure described in Example 15F and substituting OVA with the following:
- Abciximab,
- Adalimumab,
- Agalsidase alfa,
- Agalsidase beta,
- Aldeslukin,
- Alglucosidase alfa,
- Factor VIII,
- Factor IX,
- L-asparaginase,
- Laronidase,
- Octreotide,
- Phenylalanine ammonia-lyase,
- Rasburicase,
- Insulin (SEQ ID NO:1),
- GAD-65 (SEQ ID NO:2),
- IGRP (SEQ ID NO:3)
- MBP (SEQ ID NO:4),
- MOG (SEQ ID NO:5),
- PLP (SEQ ID NO:6),
- MBP13-32 (SEQ ID NO:7),
- MBP83-99 (SEQ ID NO:8),
- MBP111-129 (SEQ ID NO:9),
- MBP146-170 (SEQ ID NO:10),
- MOG1-20 (SEQ ID NO:11),
- MOG35-55 (SEQ ID NO:12),
- PLP139-154 (SEQ ID NO:13),
- MART1 (SEQ ID NO:14),
- Tyrosinase (SEQ ID NO:15),
- PMEL (SEQ ID NO:16),
- Aquaporin-4 (SEQ ID NO:17),
- S-arrestin (SEQ ID NO:18),
- IRBP (SEQ ID NO:19),
- Conarachin (UNIPROT Q6PSU6),
- Alpha-gliadin "33-mer" native (SEQ ID NO:20),
- Alpha-gliadin "33-mer" deamidated (SEQ ID NO:21),
- Alpha-gliadin (SEQ ID NO:22),
- Omega-gliadin (SEQ ID NO:23),
- Fel d 1A (UNIPROT P30438),
- Cat albumin (UNIPROT P49064),
- Can f 1 (UNIPROT 018873),
- Dog albumin (UNIPROT P49822), and
- RhCE (UNIPROT P18577),
there are obtained the following corresponding compounds of Formula 1109 where n is 80:
- X is Abciximab and m is 10,
- X is Adalimumab and m is 11,
- X is Agalsidase alfa and m is 14,
- X is Agalsidase beta and m is 14,
- X is Aldeslukin and m is 6,
- X is Alglucosidase alfa and m is 13,
- X is Factor VIII and m is 100,
- X is Factor IX and m is 18,
- X is L-asparaginase and m is 5,
- X is Laronidase and m is 7,
- X is Octreotide and m is 1,
- X is Phenylalanine ammonia-lyase and m is 12,
- X is Rasburicase and m is 12,
- X is Insulin (SEQ ID NO:1) and m is 2,
- X is GAD-65 (SEQ ID NO:2) and m is 8,
- X is IGRP (SEQ ID NO:3) and m is 7,
- X is MBP (SEQ ID NO:4) and m is 6,
- X is MOG (SEQ ID NO:5) and m is 5,
- X is PLP (SEQ ID NO:6) and m is 8,
- X is MBP13-32 (SEQ ID NO:7) and m is 1,
- X is MBP83-99 (SEQ ID NO:8) and m is 1,
- X is MBP111-129 (SEQ ID NO:9) and m is 1,
- X is MBP146-170 (SEQ ID NO:10) and m is 2,
- X is MOG1-20 (SEQ ID NO:11) and m is 1,
- X is MOG35-55 (SEQ ID NO:12) and m is 2,
- X is PLP139-154 (SEQ ID NO:13) and m is 3,
- X is MART1 (SEQ ID NO:14) and m is 4,
- X is Tyrosinase (SEQ ID NO:15) and m is 8,
- X is PMEL (SEQ ID NO:16) and m is 5,
- X is Aquaporin-4 (SEQ ID NO:17) and m is 4,
- X is S-arrestin (SEQ ID NO:18) and m is 12,
- X is IRBP (SEQ ID NO:19) and m is 21,
- X is Conarachin and m is 21,
- X is Alpha-gliadin "33-mer" native (SEQ ID NO:20) and m is 1,
- X is Alpha-gliadin "33-mer" deamidated (SEQ ID NO:21) and m is 1,
- X is Alpha-gliadin (SEQ ID NO:22) and m is 1,
- X is Omega-gliadin (SEQ ID NO:23) and m is 1,
- X is Fel d 1 and m is 4,
- X is Cat albumin and m is 16,
- X is Can f 1 and m is 6,
- X is Dog albumin and m is 23, and
- X is RhCE and m is 10.

### 151. Other Compounds of Formula 1m

By following the procedure described in Example 15G and substituting the compounds of Formula 1109, for example as obtained in Example 15H, there are obtained the following corresponding compounds of Formula 1m:
- F1m-Abciximab-m₁₀-n₈₀-p₃₀-q₄-CMP-2NHAc,
- F1m-Adalimumab-m₁₁-n₈₀-p₃₀-q₄-CMP-2NHAc,
- F1m-Agalsidase alfa-m₁₄-n₈₀-p₃₀-q₄-CMP-2NHAc,
- F1m-Agalsidase beta-m₁₄-n₈₀-p₃₀-q₄-CMP-2NHAc,
- F1m-Aldeslukin-m₆-n₈₀-p₃₀-q₄-CMP-2NHAc,
- F1m-Alglucosidase alfa-m₁₃-n₈₀-p₃₀-q₄-CMP-2NHAc,
- F1m-Factor VIII-m₁₀₀-n₈₀-p₃₀-q₄-CMP-2NHAc,
- F1m-Factor IX-m₁₈-n₈₀-p₃₀-q₄-CMP-2NHAc,
- F1m-L-asparaginase-m₅-n₈₀-p₃₀-q₄-CMP-2NHAc,
- F1m-Laronidase-m₇-n₈₀-p₃₀-q₄-CMP-2NHAc,
- F1m-Octreotide-m₁-n₈₀-p₃₀-q₄-CMP-2NHAc,
- F1m-Phenylalanine ammonia-lyase-m₁₂-n₈₀-p₃₀-q₄-CMP-2NHAc,
- F1m-Rasburicase-m₁₂-n₈₀-p₃₀-q₄-CMP-2NHAc,
- F1m-Insulin-m₂-n₈₀-p₃₀-q₄-CMP-2NHAc,
- F1m-GAD-65-m₈-n₈₀-p₃₀-q₄-CMP-2NHAc,
- F1m-IGRP-m₇-n₈₀-p₃₀-q₄-CMP-2NHAc,
- F1m-MBP-m₆-n₈₀-p₃₀-q₄-CMP-2NHAc,
- F1m-MOG-m₅-n₈₀-p₃₀-q₄-CMP-2NHAc,
- F1m-PLP-m₈-n₈₀-p₃₀-q₄-CMP-2NHAc,
- F1m-MBP13-32-m₁-n₈₀-p₃₀-q₄-CMP-2NHAc,
- F1m-MBP83-99-m₁-n₈₀-p₃₀-q₄-CMP-2NHAc,
- F1m-MBP111-129-m₁-n₈₀-p₃₀-q₄-CMP-2NHAc,
- F1m-MBP146-170-m₂-n₈₀-p₃₀-q₄-CMP-2NHAc,
- F1m-MOG1-20-m₁-n₈₀-p₃₀-q₄-CMP-2NHAC,
- F1m-MOG35-55-m₂-n₈₀-p₃₀-q₄-CMP-2NHAc,
- F1m-PLP139-154-m₃-n₈₀-p₃₀-q₄-CMP-2NHAc,
- F1m-MART1-m₄-n₈₀-p₃₀-q₄-CMP-2NHAc,
- F1m-Tyrosinase-m₈-n₈₀-p₃₀-q₄-CMP-2NHAc,
- F1m-PMEL-m₅-n₈₀-p₃₀-q₄-CMP-2NHAc,
- F1m-Aquaporin-4-m₄-n₈₀-p₃₀-q₄-CMP-2NHAc,
- F1m-S-arrestin-m₁₂-n₈₀-p₃₀-q₄-CMP-2NHAc,
- F1m-IRBP-m₂₁-n₈₀-p₃₀-q₄-CMP-2NHAc,
- F1m-Conarachin-m₂₁-n₈₀-p₃₀-q₄-CMP-2NHAc,
- F1m-Alpha-gliadin "33-mer" native-m₁-n₈₀-p₃₀-q₄-CMP-2NHAc,
- F1m-Alpha-gliadin "33-mer" deamidated-m₁-n₈₀-p₃₀-q₄-CMP-2NHAc,
- F1m-Alpha-gliadin-m₁-n₈₀-p₃₀-q₄-CMP-2NHAc,
- F1m-Omega-gliadin-m₁-n₈₀-p₃₀-q₄-CMP-2NHAc,
- F1m-Fel d 1-m₄-n₈₀-p₃₀-q₄-CMP-2NHAc,
- F1m-Cat albumin-m₁₆-n₈₀-p₃₀-q₄-CMP-2NHAc,
- F1m-Can f 1-m₆-n₈₀-p₃₀-q₄-CMP-2NHAc,
- F1m-Dog albumin-m₂₃-n₈₀-p₃₀-q₄-CMP-2NHAc, and
- F1m-RhCE-m₁₀-n₈₀-p₃₀-q₄-CMP-2NHAc.

### 15J. Formula 1107 where p is 30, q is 8, R³ is OH, R⁴ is OH and R⁸ is CMP

By following the procedure described in Example 15A and substituting the N-acetyl-D-galactosamine with galactose, and following through to the procedure described in Example 15E except using an azide-modified uRAFT agent of Formula 1106 where q is 8, there is obtained the compound of Formula 1107 where p is 30, q is 8, R³ is OH, R⁴ is OH and R⁸ is CMP.

### 15K. Formula 1109 where n is 62 and where X' and m are as in Example 19H

By following the procedure described in Example 15F, substituting the OVA with the compounds as described in Example 15H and employing the compound of Formula 1108 where n is 62, there are obtained the corresponding compounds of Formula 1109 where n is 62.

### 15L. Other Compounds of Formula 1m

By following the procedure described in Example 15G and substituting the compound of Formula 1107 with the compounds obtained in Example 15J, and substituting the compound of Formula 1109 with the compounds obtained in Example 15K, there are obtained the following corresponding compounds of Formula 1m:
- F1m-Abciximab-m₁₀-n₆₂-p₃₀-q₈-CMP-2OH,
- F1m-Adalimumab-m₁₁-n₆₂-p₃₀-q₈-CMP-2OH,
- F1m-Agalsidase alfa-m₁₄-n₆₂-p₃₀-q₈-CMP-2OH,
- F1m-Agalsidase beta-m₁₄-n₆₂-p₃₀-q₈-CMP-2OH,
- F1m-Aldeslukin-m₆-n₆₂-p₃₀-q₈-CMP-2OH,
- F1m-Alglucosidase alfa-m₁₃-n₆₂-p₃₀-q₈-CMP-2OH,
- F1m-Factor VIII-m₁₀₀-n₆₂-p₃₀-q₈-CMP-2OH,
- F1m-Factor IX-m₁₈-n₆₂-p₃₀-q₈-CMP-2OH,
- F1m-L-asparaginase-m₅-n₆₂-p₃₀-q₈-CMP-2OH,
- F1m-Laronidase-m₇-n₆₂-p₃₀-q₈-CMP-2OH,
- F1m-Octreotide-m₁-n₆₂-p₃₀-q₈-CMP-2OH,
- F1m-Phenylalanine ammonia-lyase-m₁₂-n₆₂-p₃₀-q₈-CMP-2OH,
- F1m-Rasburicase-m₁₂-n₆₂-p₃₀-q₈-CMP-2OH,
- F1m-Insulin-m₂-n₆₂-p₃₀-q₈-CMP-2OH,
- F1m-GAD-65-m₈-n₆₂-p₃₀-q₈-CMP-2OH,
- F1m-IGRP-m₇-n₆₂-p₃₀-q₈-CMP-2OH,
- F1m-MBP-m₆-n₆₂-p₃₀-q₈-CMP-2OH,
- F1m-MOG-m₅-n₆₂-p₃₀-q₈-CMP-2OH,
- F1m-PLP-m₈-n₆₂-p₃₀-q₈-CMP-2OH,
- F1m-MBP13-32-m₁-n₆₂-p₃₀-q₈-CMP-2OH,
- F1m-MBP83-99-m₁-n₆₂-p₃₀-q₈-CMP-2OH,
- F1m-MBP111-129-m₁-n₆₂-p₃₀-q₈-CMP-2OH,
- F1m-MBP146-170-m₂-n₆₂-p₃₀-q₈-CMP-2OH,
- F1m-MOG1-20-m₁-n₆₂-p₃₀-q₈-CMP-2OH,
- F1m-MOG35-55-m₂-n₆₂-p₃₀-q₈-CMP-2OH,
- F1m-PLP139-154-m₃-n₆₂-p₃₀-q₈-CMP-2OH,
- F1m-MART1-m₄-n₆₂-p₃₀-q₈-CMP-2OH,
- F1m-Tyrosinase-m₈-n₆₂-p₃₀-q₈-CMP-2OH,
- F1m-PMEL-m₅-n₆₂-p₃₀-q₈-CMP-2OH,
- F1m-Aquaporin-4-m₄-n₆₂-p₃₀-q₈-CMP-2OH,
- F1m-S-arrestin-m₁₂-n₆₂-p₃₀-q₈-CMP-2OH,
- F1m-IRBP-m₂₁-n₆₂-p₃₀-q₈-CMP-2OH,
- F1m-Conarachin-m₂₁-n₆₂-p₃₀-q₈-CMP-2OH,
- F1m-Alpha-gliadin "33-mer" native-m₁-n₆₂-p₃₀-q₈-CMP-2OH,
- F1m-Alpha-gliadin "33-mer" deamidated-m₁-n₆₂-p₃₀-q₈-CMP-2OH,
- F1m-Alpha-gliadin-m₁-n₆₂-p₃₀-q₈-CMP-2OH,
- F1m-Omega-gliadin-m₁-n₆₂-p₃₀-q₈-CMP-2OH,
- F1m-Fel d 1-m₄-n₆₂-p₃₀-q₈-CMP-2OH,
- F1m-Cat albumin-m₁₆-n₆₂-p₃₀-q₈-CMP-2OH,
- F1m-Can f 1-m₆-n₆₂-p₃₀-q₈-CMP-2OH,
- F1m-Dog albumin-m₂₃-n₆₂-p₃₀-q₈-CMP-2OH, and
- F1m-RhCE-m₁₀-n₆₂-p₃₀-q₈-CMP-2OH.

### 15M. Other Compounds of Formula 1m

By following the procedure described in Examples 15A-L and substituting the galactosamine or galactose with glucosamine or glucose, respectively, there are obtained the corresponding glucosylated compounds of Formula 1m.

### Example 16

### F1n-insulin-m₂-n₁-p₃₀-q₄-CMP-2NHAc

### 16A. Formula 1202 where X' is Insulin, m is 2 and n is 1

Recombinant human insulin (5 mg) was added to 100 µl of DMF containing 10 µl of triethylamine and stirred until the insulin became soluble. To this solution was added 10 mg (0.0161 mmol) of a linker precursor of Formula 1201 where n is 1 and the reaction was allowed to stir. After 1 hour, 1.3 ml of tert-butyl methyl ether was added to isolate the corresponding product of Formula 1202, which was recovered as the precipitate. Residual DMF and tert-butyl methyl ether were removed under reduced pressure. Characterization via liquid chromatography, mass spectroscopy and polyacrylamide gel electrophoresis confirmed the identity of the product. The modified insulin product of Formula 1202 was used without further purification.

### 16B. Formula 1n where X' is Insulin, m is 2, n is 1, p is 30, a is 4 and R⁸ is CMP

The product of Formula 1202 obtained in Example 16A was resuspended in 100 µl of DMF. The polymer product of Formula 1107 obtained in Example 15E (10 mg) was added and the reaction was allowed to stir for 1 hour. After 1 hour, the reaction products were precipitated via the addition of dichloromethane (1.3 ml). The product was filtered and the residual solvent was removed under reduced pressure. The crude product was then resuspended in 500 µl of PBS, and the low molecular weight components were removed via centrifugal size exclusion chromatography to afford the corresponding isomeric product of Formula 1n. Characterization via liquid chromatography, mass spectroscopy and polyacrylamide gel electrophoresis confirmed the identity of the product. The modified insulin product of Formula 1202 was used without further purification.

### 16C. Other Compounds of Formula 1202

By following the procedure described in Example 16A and substituting insulin with the following:
- Abciximab,
- Adalimumab,
- Agalsidase alfa,
- Agalsidase beta,
- Aldeslukin,
- Alglucosidase alfa,
- Factor VIII,
- Factor IX,
- L-asparaginase,
- Laronidase,
- Octreotide,
- Phenylalanine ammonia-lyase,
- Rasburicase,
- GAD-65 (SEQ ID NO:2),
- IGRP (SEQ ID NO:3)
- MBP (SEQ ID NO:4),
- MOG (SEQ ID NO:5),
- PLP (SEQ ID NO:6),
- MBP13-32 (SEQ ID NO:7),
- MBP83-99 (SEQ ID NO:8),
- MBP111-129 (SEQ ID NO:9),
- MBP146-170 (SEQ ID NO:10),
- MOG1-20 (SEQ ID NO:11),
- MOG35-55 (SEQ ID NO:12),
- PLP139-154 (SEQ ID NO:13),
- MART1 (SEQ ID NO:14),
- Tyrosinase (SEQ ID NO:15),
- PMEL (SEQ ID NO:16),
- Aquaporin-4 (SEQ ID NO:17),
- S-arrestin (SEQ ID NO:18),
- IRBP (SEQ ID NO:19),
- Conarachin (UNIPROT Q6PSU6),
- Alpha-gliadin "33-mer" native (SEQ ID NO:20),
- Alpha-gliadin "33-mer" deamidated (SEQ ID NO:21),
- Alpha-gliadin (SEQ ID NO:22),
- Omega-gliadin (SEQ ID NO:23),
- Fel d 1A (UNIPROT P30438),
- Cat albumin (UNIPROT P49064),
- Can f 1 (UNIPROT 018873),
- Dog albumin (UNIPROT P49822), and
- RhCE (UNIPROT P18577),
there are obtained the following corresponding compounds of Formula 1202 where n is 1:
- X is Abciximab and m is 10,
- X is Adalimumab and m is 11,
- X is Agalsidase alfa and m is 14,
- X is Agalsidase beta and m is 14,
- X is Aldeslukin and m is 6,
- X is Alglucosidase alfa and m is 13,
- X is Factor VIII and m is 100,
- X is Factor IX and m is 18,
- X is L-asparaginase and m is 5,
- X is Laronidase and m is 7,
- X is Octreotide and m is 1,
- X is Phenylalanine ammonia-lyase and m is 12,
- X is Rasburicase and m is 12,
- X is GAD-65 (SEQ ID NO:2) and m is 8,
- X is IGRP (SEQ ID NO:3) and m is 7,
- X is MBP (SEQ ID NO:4) and m is 6,
- X is MOG (SEQ ID NO:5) and m is 5,
- X is PLP (SEQ ID NO:6) and m is 8,
- X is MBP13-32 (SEQ ID NO:7) and m is 1,
- X is MBP83-99 (SEQ ID NO:8) and m is 1,
- X is MBP111-129 (SEQ ID NO:9) and m is 1,
- X is MBP146-170 (SEQ ID NO:10) and m is 2,
- X is MOG1-20 (SEQ ID NO:11) and m is 1,
- X is MOG35-55 (SEQ ID NO:12) and m is 2,
- X is PLP139-154 (SEQ ID NO:13) and m is 3,
- X is MART1 (SEQ ID NO:14) and m is 4,
- X is Tyrosinase (SEQ ID NO:15) and m is 8,
- X is PMEL (SEQ ID NO:20) and m is 5,
- X is Aquaporin-4 (SEQ ID NO:21) and m is 4,
- X is S-arrestin (SEQ ID NO:22) and m is 12,
- X is IRBP (SEQ ID NO:19) and m is 21,
- X is Conarachin and m is 21,
- X is Alpha-gliadin "33-mer" native (SEQ ID NO:20) and m is 1,
- X is Alpha-gliadin "33-mer" deamidated (SEQ ID NO:21) and m is 1,
- X is Alpha-gliadin (SEQ ID NO:22) and m is 1,
- X is Omega-gliadin (SEQ ID NO:27) and m is 1,
- X is Fel d 1 and m is 4,
- X is Cat albumin and m is 16,
- X is Can f 1 and m is 6,
- X is Dog albumin and m is 23, and
- X is RhCE and m is 10.

### 16D. Other Compounds of Formula 1n

By following the procedure described in Example 16B and substituting the compounds of Formula 1202, for example as obtained in Example 16C, there are obtained the following corresponding compounds of Formula 1m:
- F1n-Abciximab-m₁₀-n₁-p₃₀-q₄-CMP-2NHAc,
- F1n-Adalimumab-m₁₁-n₁-p₃₀-q₄-CMP-2NHAc,
- F1n-Agalsidase alfa-m₁₄-n₁-p₃₀-q₄-CMP-2NHAc,
- F1n-Agalsidase beta-m₁₄-n₁-p₃₀-q₄-CMP-2NHAc,
- F1n-Aldeslukin-m₆-n₁-p₃₀-q₄-CMP-2NHAc,
- F1n-Alglucosidase alfa-m₁₃-n₁-p₃₀-q₄-CMP-2NHAc,
- F1n-Factor VIII-m_{1O0}-n₁-p₃₀-q₄-CMP-2NHAc,
- F1n-Factor IX-m₁₈-n₁-p₃₀-q₄-CMP-2NHAc,
- F1n-L-asparaginase-m₅-n₁-p₃₀-q₄-CMP-2NHAc,
- F1n-Laronidase-m₇-n₁-p₃₀-q₄-CMP-2NHAc,
- F1n-Octreotide-m₁-n₁-p₃₀-q₄-CMP-2NHAc,
- F1n-Phenylalanine ammonia-lyase-m₁₂-n₁-p₃₀-q₄-CMP-2NHAc,
- F1n-Rasburicase-m₁₂-n₁-p₃₀-q₄-CMP-2NHAc,
- F1n-GAD-65-m₈-n₁-p₃₀-q₄-CMP-2NHAc,
- F1n-IGRP-m₇-n₁-p₃₀-q₄-CMP-2NHAc,
- F1n-MBP-m₆-n₁-p₃₀-q₄-CMP-2NHAc,
- F1n-MOG-m₅-n₁-p₃₀-q₄-CMP-2NHAc,
- F1n-PLP-m₈-n₁-p₃₀-q₄-CMP-2NHAc,
- F1n-MBP13-32-m₁-n₁-p₃₀-q₄-CMP-2NHAc,
- F1n-MBP83-99-m₁-n₁-p₃₀-q₄-CMP-2NHAc,
- F1n-MBP111-129-m₁-n₁-p₃₀-q₄-CMP-2NHAc,
- F1n-MBP146-170-m₂-n₁-p₃₀-q₄-CMP-2NHAc,
- F1n-MOG1-20-m₁-n₁-p₃₀-q₄-CMP-2NHAc,
- F1n-MOG35-55-m₂-n₁-p₃₀-q₄-CMP-2NHAc,
- F1n-PLP139-154-m₃-n₁-p₃₀-q₄-CMP-2NHAc,
- F1n-MART1-m₄-n₁-p₃₀-q₄-CMP-2NHAc,
- F1n-Tyrosinase-m₈-n₁-p₃₀-q₄-CMP-2NHAc,
- F1n-PMEL-m₅-n₁-p₃₀-q₄-CMP-2NHAc,
- F1n-Aquaporin-4-m₄-n₁-p₃₀-q₄-CMP-2NHAc,
- F1n-S-arrestin-m₁₂-n₁-p₃₀-q₄-CMP-2NHAc,
- F1n-IRBP-m₂₁-n₁-p₃₀-q₄-CMP-2NHAc,
- F1n-Conarachin-m₂₁-n₁-p₃₀-q₄-CMP-2NHAc,
- F1n-Alpha-gliadin "33-mer" native-m₁-n₁-p₃₀-q₄-CMP-2NHAc,
- F1n-Alpha-gliadin "33-mer" deamidated-m₁-n₁-p₃₀-q₄-CMP-2NHAc,
- F1n-Alpha-gliadin-m₁-n₁-p₃₀-q₄-CMP-2NHAc,
- F1n-Omega-gliadin-m₁-n₁-p₃₀-q₄-CMP-2NHAc,
- F1n-Fel d 1-m₄-n₁-p₃₀-q₄-CMP-2NHAc,
- F1n-Cat albumin-m₁₆-n₁-p₃₀-q₄-CMP-2NHAc,
- F1n-Can f 1-m₆-n₁-p₃₀-q₄-CMP-2NHAc,
- F1n-Dog albumin-m₂₃-n₁-p₃₀-q₄-CMP-2NHAc, and
- F1n-RhCE-m₁₀-n₁-p₃₀-q₄-CMP-2NHAc.

### 16E. Formula 1202 where n is 33 and where X' and m are as in Example 20C

By following the procedure described in Example 16F, substituting the insulin with the compounds as described in Example 16C and employing the compound of Formula 1201 where n is 33, there are obtained the corresponding compounds of Formula 1202 where n is 33.

### 16F. Other Compounds of Formula 1n

By following the procedure described in Example 16B and substituting the compound of Formula 1107 with the compounds obtained in Example 15J, and substituting the compound of Formula 1202 with the compounds obtained in Example 16E, there are obtained the following corresponding compounds of Formula 1n:
- F1n-Abciximab-m₁₀-n₃₃-p₃₀-q₈-CMP-2OH,
- F1n-Adalimumab-m₁₁-n₃₃-p₃₀-q₈-CMP-2OH,
- F1n-Agalsidase alfa-m₁₄-n₃₃-p₃₀-q₈-CMP-2OH,
- F1n-Agalsidase beta-m ₄-n₃₃-p₃₀-q₈-CMP-2OH,
- F1n-Aldeslukin-m₆-n₃₃-p₃₀-q₈-CMP-2OH,
- F1n-Alglucosidase alfa-m₁₃-n₃₃-p₃₀-q₈-CMP-2OH,
- F1n-Factor VIII-m₁₀₀-n₃₃-p₃₀-q₈-CMP-2OH,
- F1n-Factor IX-m₁₈-n₃₃-p₃₀-q₈-CMP-2OH,
- F1n-L-asparaginase-m₅-n₃₃-p₃₀-q₈-CMP-2OH,
- F1n-Laronidase-m₇-n₃₃-p₃₀-q₈-CMP-2OH,
- F1n-Octreotide-m₁-n₃₃-p₃₀-q₈-CMP-2OH,
- F1n-Phenylalanine ammonia-lyase-m₁₂-n₃₃-p₃₀-q₈-CMP-2OH,
- F1n-Rasburicase-m₁₂-n₃₃-p₃₀-q₈-CMP-2OH,
- F1n-GAD-65-m₈-n₃₃-p₃₀-q₈-CMP-2OH,
- F1n-IGRP-m₇-n₃₃-p₃₀-q₈-CMP-2OH,
- F1n-MBP-m₆-n₃₃-p₃₀-q₈-CMP-2OH,
- F1n-MOG-m₅-n₃₃-p₃₀-q₈-CMP-2OH,
- F1n-PLP-m₈-n₃₃-p₃₀-q₈-CMP-2OH,
- F1n-MBP13-32-m₁-n₃₃-p₃₀-q₈-CMP-2OH,
- F1n-MBP83-99-m₁-n₃₃-p₃₀-q₈-CMP-2OH,
- F1n-MBP111-129-m₁-n₃₃-p₃₀-q₈-CMP-2OH,
- F1n-MBP146-170-m₂-n₃₃-p₃₀-q₈-CMP-2OH,
- F1n-MOG1-20-m₁-n₃₃-p₃₀-q₈-CMP-2OH,
- F1n-MOG35-55-m₂-n₃₃-p₃₀-q₈-CMP-2OH,
- F1n-PLP139-154-m₃-n₃₃-p₃₀-q₈-CMP-2OH,
- F1n-MART1-m₄-n₃₃-p₃₀-q₈-CMP-2OH,
- F1n-Tyrosinase-m₈-n₃₃-p₃₀-q₈-CMP-2OH,
- F1n-PMEL-m₅-n₃₃-p₃₀-q₈-CMP-2OH,
- F1n-Aquaporin-4-m₄-n₃₃-p₃₀-q₈-CMP-2OH,
- F1n-S-arrestin-m₁₂-n₃₃-p₃₀-q₈-CMP-2OH,
- F1n-IRBP-m₂₁-n₃₃-p₃₀-q₈-CMP-2OH,
- F1n-Conarachin-m₂₁-n₃₃-p₃₀-q₈-CMP-2OH,
- F1n-Alpha-gliadin "33-mer" native-m₁-n₃₃-p₃₀-q₈-CMP-2OH,
- F1n-Alpha-gliadin "33-mer" deamidated-m₁-n₃₃-p₃₀-q₈-CMP-2OH,
- F1n-Alpha-gliadin-m₁-n₃₃-p₃₀-q₈-CMP-2OH,
- F1n-Omega-gliadin-m₁-n₃₃-p₃₀-q₈-CMP-2OH,
- F1n-Fel d 1-m₄-n₃₃-p₃₀-q₈-CMP-2OH,
- F1n-Cat albumin-m₁₆-n₃₃-p₃₀-q₈-CMP-2OH,
- F1n-Can f 1-m₆-n₃₃-p₃₀-q₈-CMP-2OH,
- F1n-Dog albumin-m₂₃-n₃₃-p₃₀-q₈-CMP-2OH, and
- F1n-RhCE-m₁₀-n₃₃-p₃₀-q₈-CMP-2OH.

### 16G. Other Compounds of Formula 1n

By following the procedure described in Examples 16A-F and substituting the galactosylating moieties with glucosylating moieties, there are obtained the corresponding glucosylated compounds of Formula 1n.

### Example 17

### Formula 1507 where p is 90, t is 1, R³ is NHAc and R⁴ is OH

### 17A. Formula 1502 where t is 1, R³ is NHAc and R⁴ is OH

*N-*Acetyl-D-glucosamine (Formula 1101 where R³ is NHAc and R⁴ is OH) (5.0 g, 22.6 mmol) was added to a stirred solution of 2-(2-chloroethoxy)ethan-1-ol (50 ml) at room temperature. The solution was cooled to 4°C and acetylchloride was added drop-wise to the solution. The solution was brought to room temperature and then heated to 70°C. After 4 hours, the reaction mixture was added to 200 ml of ethyl acetate. The precipitate that formed was collected, added to 100 ml of ethanol and stirred in the presence of carbon for 2 hours. The solution was filtered, and the solvent was removed under reduced pressure. The corresponding product of Formula 1502, *N-*acetyl-D-glucosamine-2-(chloroethoxy)ethanol, was used without further purification.

### 17B. Formula 1503 where t is 1, R³ is NHAc and R⁴ is OH

*N-*Acetyl-D-glucosamine-2-(chloroethoxy)ethanol (2.0 g, 6.11 mmol) was added to a stirred solution of DMF (100 ml) and sodium azide (4.0 g, 61.5 mmol). The solution was headed at 90°C for 12 hours and then filtered. The residual solvent was removed under reduced pressure and the crude product was purified via flash chromatography (10% MeOH in dichloromethane) to give the corresponding product of Formula 1503, *N-*acetyl-D-glucosamine-2-(azideoethoxy)ethanol.

### 17C. Formula 1504 where t is 1, R³ is NHAc and R⁴ is OH

*N-*Acetyl-D-glucosamine-2-(azideoethoxy)ethanol (2.0 g, 5.9 mmol) was added to a solution of palladium on carbon and ethanol (50 ml). The solution was stirred under hydrogen gas (3 atm) for 4 hours. The resulting solution was filtered and the residual solvent was removed under reduced pressure to afford the corresponding product of Formula 1504, *N-*acetyl-D-glucosamine-2-(amineoethoxy)ethanol.

### 17D. Formula 1505 where t is 1, R³ is NHAc and R⁴ is OH

*N-*Acetyl-D-glucosamine-2-(amineoethoxy)ethanol (1.0 g, 3.25 mmol) was added to a solution of methacrylate anhydride (0.583 g, 3.78 mmol) in DMF (50 ml). Triethylamine was then added to the solution and the reaction was stirred for 2 hours at room temperature. After 2 hours, the excess solvent was removed under reduced pressure, and the corresponding product of Formula 1505, ((2*S*,3*S*,4*S*,5*R*,6*S*)-4,5-dihydroxy-6-(hydroxymethyl)-2-(2-(2-methacrylamidoethoxy)ethoxy)tetrahydro-2*H*-pyran-3-yl)carbamic acid, was isolated via flash chromatography.

### 17E. Formula 1507 where p is 90, q is 4, t is 1, R³ is NHAc, R⁴ is OH, R⁸ is CMP, and R¹⁰ is 2-hydroxypropyl

A 25 ml Schlenk flask was charged with a compound of Formula 1505, the product of Example 17D (272 mg, 0.72 mmol), N-(2-hydroxypropyl)methacrylamide ("HPMA", used as received from the manufacturer) (211 mg, 1.47 mmol), an azide-modified uRAFT agent of Formula 1106 where q is 4 and R⁸ is CMP (10.41 mg, 0.0217 mmol), azobis(isobutyronitril) (0.98 mg, 0.005 mmol), and 1.2 ml dimethylformamide. The reaction mixture was subjected to four freeze-pump-thaw degassing cycles and then stirred at 70°C for 20 hours. The corresponding random polymeric product of Formula 1507 was recovered by precipitating the reaction mixture in acetone. Excess acetone was removed at reduced pressure to provide the random polymeric product, which was used without further purification.

### 17F. Formula 1507 where p is 90, q is 4, t is 1, R³ is NHAc, R⁴ is OH, R⁸ is CMP, and R¹⁰ is 2-hydroxypropyl, using N-acetyl-D-galactosamine

By following the procedures of Examples 17A through 17E and substituting N-acetyl-D-galactosamine for N-acetyl-D-glucosamine in the procedure of Example 17A, there was obtained the corresponding galactosyl compound of Formula 1507.

### 17G. Compounds of Formula 1507 where t is other than 1

By following the procedures of Examples 17A through 17E and substituting 2-(2-chloroethoxy)ethan-1-ol with:
- 2-(2-(2-chloroethoxy)ethoxy)ethan-1-ol will afford the corresponding compound of Formula 1507 where t is 2,
- 2-(2-(2-(2-chloroethoxy)ethoxy)ethoxy)ethan-1-ol will afford the corresponding compound of Formula 1507 where t is 3,
- 2-(2-(2-(2-(2-chloroethoxy)ethoxy)ethoxy)ethoxy)ethan-1-ol will afford the corresponding compound of Formula 1507 where t is 4,
- 2-(2-(2-(2-(2-(2-chloroethoxy)ethoxy)ethoxy)ethoxy)ethoxy)ethan-1-ol will afford the corresponding compound of Formula 1507 where t is 5, and
- 2-(2-(2-(2-(2-(2-(2-chloroethoxy)ethoxy)ethoxy)ethoxy)ethoxy)ethoxy)ethan-1-ol will afford the corresponding compound of Formula 1507 where t is 6.

### 17H. Compounds of Formula 1507 having a plurality of W¹ groups where t varies

By following the procedure of Example 17E and substituting the compound of Formula 1505 where t is 1 with 0.36 mmol each of Formula 1505 where t is 2 and 4, (prepared, for example, as described in Example 17F by following the procedures of Examples 17A through 17D) there is obtained the corresponding random copolymer of Formula 1507 having about 15 W¹ groups where t is 2, 15W¹ groups where t is 4 and 60 W² groups.

### 171. Compounds of Formula 1507 having a mixture of glucosyl and galactosyl moieties

By following the procedure of Example 17E and substituting the compound of Formula 1505 with 0.36 mmol each of glucosyl and galactosyl Formula 1505 (prepared, for example, as described in Example 17D and in Example 17F by following the procedures of Examples 17A through 17D) there is obtained the corresponding random copolymer of Formula 1507 having about 15 glucosyl W¹ groups, 15 galactosyl W¹ groups and 60 W² groups.

### Example 17.1

### Formula 1507 where t is 1, R³ is NHAc and R⁴ is OH

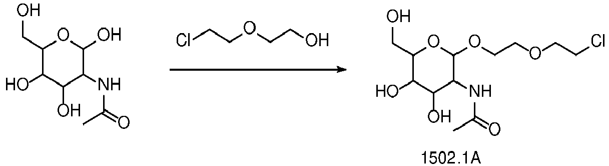

### 17.1A. Formula 1502 where t is 1, R³ is NHAc and R⁴ is OH: 2-(2-(2-chloroethoxy)ethoxy)-α-NAc-Galactosamine (1502.1A).

Acetyl chloride (4.35 mL, 61.05 mmol) was added dropwise to the ice-cold solution of NHAc protected D-Galactosamine (10.0 g) in 2-(2'-Chloroethoxy)ethanol (40 mL). The mixture was stirred for 15 minutes at 4°C and then was transferred to the oil bath at 70°C. The reaction was left mixing under cooling condenser for 4 hours. After that time, a dark brown solution was cooled down and poured into 400 mL solution of ethyl acetate and dichloromethane (3:1, v/v) in order to get rid of an excess of unreacted chloroethanol. The mixture was placed in a freezer for 30 minutes and then decanted from dark brown, sticky precipitate. The precipitate was dissolved in anhydrous ethanol and activated charcoal was added. The suspension was mixed for 1.5 hours and then filtered off through Celite and washed with ethanol. In the last step, ethanol was evaporated in vacuum to provide 12.8 g of product (1502.1A) (95.24% yield).

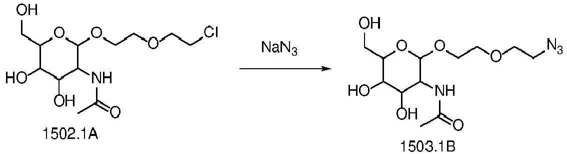

### 17.1B. Formula 1503 where t is 1, R³ is NHAc and R⁴ is OH; 2-(2-(2-Azidoethoxy)ethoxy)-α-NAc-Galactosamine (1503.1B).

A compound (1502.1A) (5.0 g) was dissolved in 20 mL of N,N-dimethylformamide. To that solution, sodium azide (26628-22-8) was added (5.0 g). The suspension was placed in an oil bath and stirred over night at 80°C. After the night, the reaction mixture was filtered off through Celite. The solvent was then evaporated under high pressure to provide an oily, brown substance. Final product was purified via flash chromatography (82.2% yield).

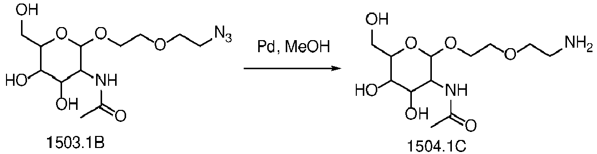

### 17.1C. Formula 1504 where t is 1, R³ is NHAc and R⁴ is OH; 2-(2-(2-aminoethoxy)ethoxy)-α-NAc-Galactosamine (1504.1C).

A suspension of (1503.1B) (5.5 g) and 10% palladium on carbon (ca. 500 mg) in 20 mL of ethanol was hydrogenated in a Shlenk flask with an initial pressure of 2 bars of hydrogen gas. The reduction process was controlled by TLC. After 3 hours reaction was completed and the suspension was filtered through Celite (78% yield).

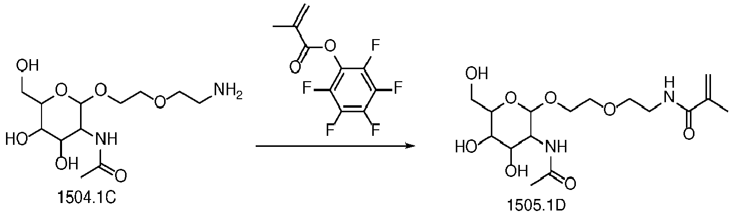

### 17.1D. Formula 1505 where t is 1, R³ is NHAc and R⁴ is OH; α-NAc-Glactosamine-amine-methacrylate (1505.1D)

A compound (1504.1C) (4.5 g) was dissolved in 10 mL of N,N-dimethylformamide. To that solution, triethylamine (3 mL) was added and the mixture was cooled down to 4°C. Subsequently, pentafluorophenyl methacrylate (13642-97-2) (4.38 mL) was added drop-wise with constant stirring. After 30 minutes, ice-bath was removed and the reaction was allowed to stir at room temperature for the next 4 hours. Next, the solvent was evaporated and the residual was adsorbed on silica gel. The purification of crude material using flash chromatography (dichloromethane : methanol 95:5, v/v) provided 3.8g of NAc-Galactosamine monomer (α-NAc-Glactosamine-amine-methacrylate (1505.1D)) (64.73% yield).

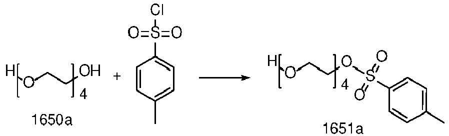

### Tetraethylene glycol mono p-toluenesulfonate (1651a).

Tetraethylene glycol (1650a) (112-60-7) (2.5 q) and pyridine (1.0 g) were added to 50 mL of dichloromethane and stirred for 20 minutes at 0°C. To that solution, p-toluenesulfonyl chloride (98-59-9)(2.37) in 15 mL of dichloromethane was added slowly. The reaction mixture was then stirred for 2h at 0°C followed by 4h at room temperature. After that time, the solvent was evaporated and crude product was purified via flash chromatography (ethyl acetate: hexane 6:4, v/v) to afford 1651a (44% yield).

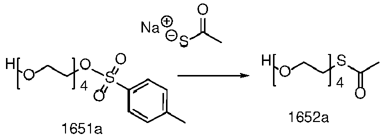

### S-[2-[2-[2-(2-hydroxyethoxy)ethoxy]ethoxy]ethyl] ester (1652a)

To a suspension of potassium thioacetate (10387-40-3) (10.1 g, 88 mmol) in 650 mL of DMF was added a solution of (1651a) (15.4 g) in 100 mL of DMF. The mixture was stirred at room temperature for 1 h and then at 90C for 4 h. After filtration, the solvent was evaporated under reduced pressure. The residue was dissolved in ethyl acetate (150 mL) and washed with water (2 × 50 mL) and brine (2 × 50 mL). The aqueous wash solutions were reextracted with ethyl acetate (2 × 50 mL), and the combined organic layers were dried over magnesium sulfate and evaporated under reduced pressure to give a yellow oil product 1652a (45% yield)

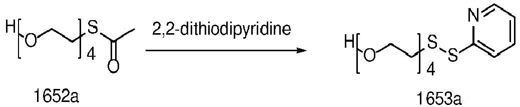

### 2-(2-(2-(2-(pyridin-2-yldisulfanyl)ethoxy)ethoxy)ethoxy)ethan-1-ol (1653a)

Sodium methoxide (1.40 ml of 0.5M in methanol) was added dropwise to a stirred solution of (1652a) (70.9 mg) and 2,2-dithiodipyridine (2127-03-9) (77.4 mg, 0.351 mmol) in anhydrous methanol (3 mL) under an argon atmosphere. After 2 h the reaction was concentrated with silica to a powder, and the crude product was purified by flash chromatography over silica (1:1 hexanes:EtOAc) to afford 1653a as a clear, pale yellow liquid (26.3 mg, 44% yield).

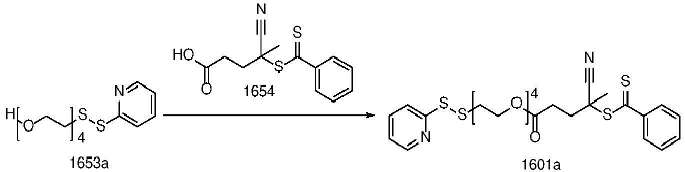

### uRAFT Agent (1601a)

Compound 1653a (1g) was added dropwise to a stirred solution of 4-Cyano-4-(thiobenzoylthio)pentanoic acid (1.1g) (201611-92-9), *N,N*'-Dicyclohexylcarbodiimide (538-75-0) (0.5 g) and 4-Dimethylaminopyridine (DMAP) (1122-58-3) (0.1 g) in DCM (15ml). The reaction was stirred at 0C for 2 h then allowed to warm to room temperature. After 3 h, the reaction was filtered through celite and the solvent was removed via reduced pressure. The final product (1601a) was recovered from flash chromatography (67% yield).

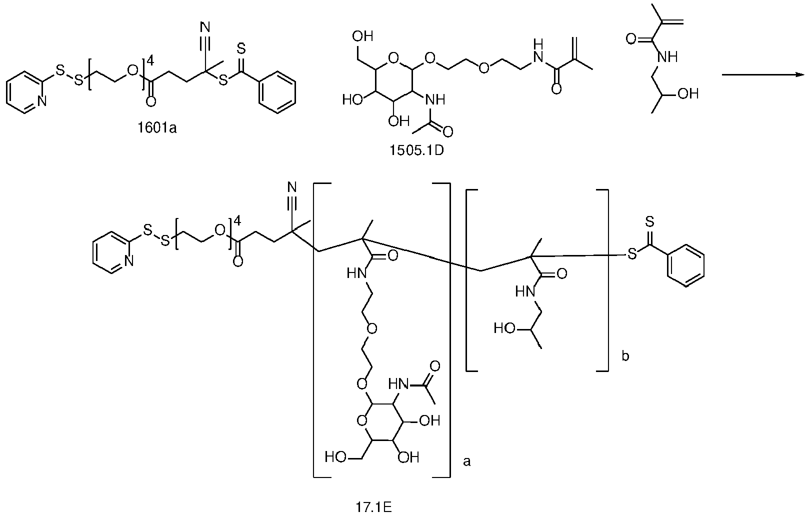

### pGal (17.1E)

The following conditions were performed using the α-NAc-Glactosamine-amine-methacrylate (e.g., 1505.1D) monomer to afford 17.1E. In some embodiments, the α-NAc-Glucosamine-amine-methacrylate monomer (e.g., 1505.2D) can be used instead to afford a glucosamine-based polymer. In some embodiments, a is an integer between about 0 to about 150, about 1 to about 100, about 1 to about 50, about 1 to about 10, or about 1 to about 5. In some embodiments, b is an integer between about 0 to about 150, about 1 to about 100, about 1 to about 50, about 1 to about 10, or about 1 to about 5.

Compound 1601a, 1505.1D, Azobisisobutyronitrile (78-67-1), and N-(2-hydroxypropyl)methacrylamide (21442-01-3) were added to DMF (1ml). The reaction mixture was subjected to 4 freeze-pump-thaw degassing cycles before being stirred for 20 h at 70C. The polymeric product was recovered via precipitation from acetone. The excess solvent was removed under reduced pressure (55% yield).

### Example 17.2

### Formula 1507 where t is 1, R³ is NHAc and R⁴ is OH

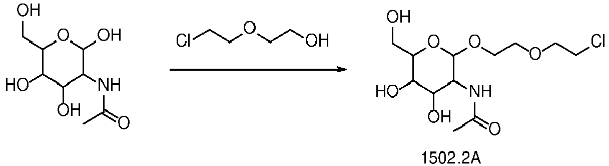

### 17.2A. Formula 1502 where t is 1, R³ is NHAc and R⁴ is OH; 2-(2-(2-chloroethoxy)ethoxy)-α-NAc-glucosamine (1502.2A).

Acetyl chloride (75-36-5) (4.35 mL, 61.05 mmol) was added dropwise to the ice-cold solution of D-Glucosamine (7512-17-6) (10.0 g) in 2-(2'-Chloroethoxy)ethanol (628-89-7) (40 mL). The mixture was stirred for 15 minutes at 4°C and then was transferred to the oil bath at 70°C. The reaction was left mixing under cooling condenser for 4 hours. After that time, a dark brown solution was cooled down and poured into 400 mL solution of ethyl acetate and dichloromethane (3:1, v/v) in order to get rid of an excess of unreacted chloroethanol. The mixture was placed in a freezer for 30 minutes and then decanted from dark brown, sticky precipitate. The precipitate was dissolved in anhydrous ethanol and activated charcoal was added. The suspension was mixed for 1.5 hours and then filtered off through Celite and washed with ethanol. In the last step, ethanol was evaporated in vacuum to afford 1502.2A (76% yield).

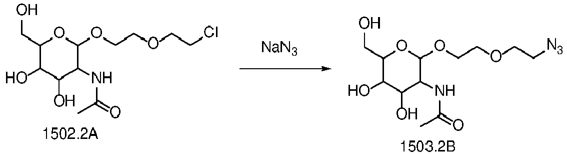

### 17.2B. Formula 1503 where t is 1, R³ is NHAc and R⁴ is OH; 2-(2-(2-Azidoethoxy)ethoxy)-α-NAc-Glucosamine (1503.2B).

A compound (1502.2A) (5.0 g) was dissolved in 20 mL of N,N-dimethylformamide. To that solution, sodium azide (26628-22-8) was added (5.0 g). The suspension was placed in an oil bath and stirred over night at 80°C. After the night, the reaction mixture was filtered off through Celite. The solvent was then evaporated under high pressure to provide an oily, brown substance. The final product 1503.2B was purified via flash chromatography (75.4% yield).

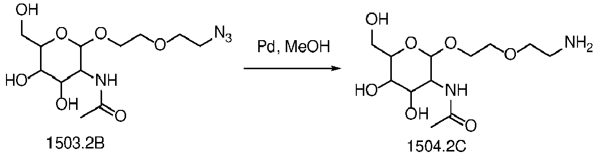

### 17.2C. Formula 1504 where t is 1, R³ is NHAc and R⁴ is OH; 2-(2-(2-aminoethoxy)ethoxy)-α-NAc-Glucosamine (1504.2C).

A suspension of (1503.2B) (5.5 g) and 10% palladium on carbon (ca. 500 mg) in 20 mL of ethanol was hydrogenated in a Shlenk flask with an initial pressure of 2 bars of hydrogen gas. The reduction process was controlled by TLC. After 3 hours reaction was completed and the suspension was filtered through Celite to afford 1504.2C (65% yield).

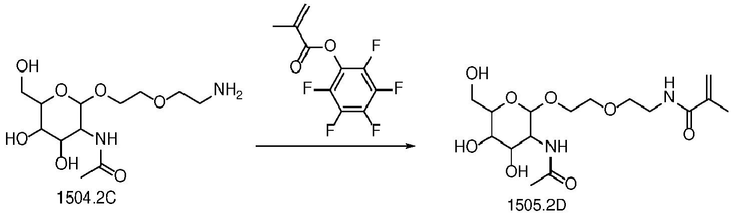

### 17.2D. Formula 1505 where t is 1, R³ is NHAc and R⁴ is OH; α-NAc-Glucosamine-amine-methacrylate (1505.2D).

Compound 1504.2C (4.5 g) was dissolved in 10 mL of N,N-dimethylformamide. To that solution, triethylamine (3 mL) was added and the mixture was cooled down to 4°C. Subsequently, pentafluorophenyl methacrylate (13642-97-2) (4.38 mL) was added drop-wise with constant stirring. After 30 minutes, ice-bath was removed and the reaction was allowed to stir at room temperature for the next 4 hours. Next, the solvent was evaporated and the residual was adsorbed on silica gel. The purification of crude material using flash chromatography (dichloromethane : methanol 95:5, v/v) provided 3.8g of NAc-Glucosamine monomer 1505.2D (74% yield).

### Example 18

### Formula 1m' where X' is OVA, m is 1-3, n is 79, p is 90 (30 W¹ + 60 W²), q is 4, t is 1, R³ is NHAc, R⁴ is OH, R⁸ is CMP, and R¹⁰ is 2-hydroxypropyl

### 18A. Formula 1109 where X' is OVA, m is 1-3 and n is 79

A solution of Formula 101' where X' is OVA (10 mg of endotoxin-free ovalbumin) in pH 7.6 PBS was added to Formula 1108 where n Is 79 (10 mg) in an endotoxin-free tube. The reaction mixture was allowed to stir at room temperature. After 1 hour, any unconjugated Formula 1108 was removed via centrifugal size exclusion chromatography to afford the corresponding product of Formula 1109, which was used without further purification.

### 18B. Formula 1m' where X' is OVA, m is 1-3, n is 79, p is 90, a is 4, t is 1, R³ is NHAc, R⁴ is OH, R⁸ is CMP, and R¹⁰ is 2-hydroxypropyl

The Formula 1109 solution obtained in Example 18A was then added to Formula 1507 as obtained in Example 17E (20 mg) in an endotoxin-free tube and stirred at room temperature to afford the corresponding product of Formula 1m' ("F1m'-OVA-m₁₋₃-n₇₉-p₉₀-q₄-CMP-poly-(EtPEG₁AcN-1NAcGLU₃₀-ran-HPMA₆₀"), which was purified from the reaction mixture via fast protein liquid chromatography (FPLC) using a Superdex 200 prep grade column and used without further purification.

### 18C. Formula 1m' where X' is OVA, m is 1-3, n is 79, p is 90, a is 4, t is 1, R³ is NHAc, R⁴ is OH, R⁸ is CMP, and R¹⁰ is 2-hydroxypropyl, using N-acetyl-D-galactosamine

By following the procedure of Example 18B and substituting the galactosyl compound of Formula 1507 as obtained in Example 17F there was obtained the corresponding galactosyl compound of Formula 1m' ("F1m'-OV A-m₁₋₃-n₇₉-p₉₀- q₄-CMP-poly-(EtPEG₁AcN-1NAcGAL₃₀-ran-HPMA₆₀").

### 18D. Other Compounds of Formula 1m' where X' is OVA, m is 1-3, n is 79, p is 90, q is 4, t is 1, R³ is NHAc, R⁴ is OH, R⁸ is CMP, and R¹⁰ is 2-hydroxypropyl

By following the procedures described in Example 18A, 18B and 18C and substituting OVA with the following:
- Abciximab,
- Adalimumab,
- Agalsidase alfa,
- Agalsidase beta,
- Aldeslukin,
- Alglucosidase alfa,
- Factor VIII,
- Factor IX,
- L-asparaginase,
- Laronidase,
- Octreotide,
- Phenylalanine ammonia-lyase,
- Rasburicase,
- GAD-65 (SEQ ID NO:2),
- IGRP (SEQ ID NO:3)
- MBP (SEQ ID NO:4),
- MOG (SEQ ID NO:5),
- PLP (SEQ ID NO:6),
- MBP13-32 (SEQ ID NO:7),
- MBP83-99 (SEQ ID NO:8),
- MBP111-129 (SEQ ID NO:9),
- MBP146-170 (SEQ ID NO:10),
- MOG1-20 (SEQ ID NO:11),
- MOG35-55 (SEQ ID NO:12),
- PLP139-154 (SEQ ID NO:13),
- MART1 (SEQ ID NO:14),
- Tyrosinase (SEQ ID NO:15),
- PMEL (SEQ ID NO:16),
- Aquaporin-4 (SEQ ID NO:17),
- S-arrestin (SEQ ID NO:18),
- IRBP (SEQ ID NO:19),
- Conarachin (UNIPROT Q6PSU6),
- Alpha-gliadin "33-mer" native (SEQ ID NO:20),
- Alpha-gliadin "33-mer" deamidated (SEQ ID NO:21),
- Alpha-gliadin (SEQ ID NO:22),
- Omega-gliadin (SEQ ID NO:23),
- Fel d 1A (UNIPROT P30438),
- Cat albumin (UNIPROT P49064),
- Can f 1 (UNIPROT 018873),
- Dog albumin (UNIPROT P49822), and
- RhCE (UNIPROT P18577),
there are obtained the following corresponding glucosyl and galactosyl compounds of Formula 1m':
- X' is Abciximab and m is 10,
- X' is Adalimumab and m is 11,
- X' is Agalsidase alfa and m is 14,
- X' is Agalsidase beta and m is 14,
- X' is Aldeslukin and m is 6,
- X' is Alglucosidase alfa and m is 13,
- X' is Factor VIII and m is 100,
- X' is Factor IX and m is 18,
- X' is L-asparaginase and m is 5,
- X' is Laronidase and m is 7,
- X' is Octreotide and m is 1,
- X' is Phenylalanine ammonia-lyase and m is 12,
- X' is Rasburicase and m is 12,
- X' is GAD-65 (SEQ ID NO:2) and m is 8,
- X' is IGRP (SEQ ID NO:3) and m is 7,
- X' is MBP (SEQ ID NO:4) and m is 6,
- X' is MOG (SEQ ID NO:5) and m is 5,
- X' is PLP (SEQ ID NO:6) and m is 8,
- X' is MBP13-32 (SEQ ID NO:7) and m is 1,
- X' is MBP83-99 (SEQ ID NO:8) and m is 1,
- X' is MBP111-129 (SEQ ID NO:9) and m is 1,
- X' is MBP146-170 (SEQ ID NO:10) and m is 2,
- X' is MOG1-20 (SEQ ID NO:11) and m is 1,
- X' is MOG35-55 (SEQ ID NO:12) and m is 2,
- X' is PLP139-154 (SEQ ID NO:13) and m is 3,
- X' is MART1 (SEQ ID NO:14) and m is 4,
- X' is Tyrosinase (SEQ ID NO:15) and m is 8,
- X' is PMEL (SEQ ID NO:16) and m is 5,
- X' is Aquaporin-4 (SEQ ID NO:17) and m is 4,
- X' is S-arrestin (SEQ ID NO:18) and m is 12,
- X' is IRBP (SEQ ID NO:19) and m is 21,
- X' is Conarachin and m is 21,
- X' is Alpha-gliadin "33-mer" native (SEQ ID NO:20) and m is 1,
- X' is Alpha-gliadin "33-mer" deamidated (SEQ ID NO:21) and m is 1,
- X' is Alpha-gliadin (SEQ ID NO:22) and m is 1,
- X' is Omega-gliadin (SEQ ID NO:23) and m is 1,
- X' is Fel d 1 and m is 4,
- X' is Cat albumin and m is 16,
- X' is Can f 1 and m is 6,
- X' is Dog albumin and m is 23, and
- X' is RhCE and m is 10.

### 18E. Compounds of Formulae 1h',1i', 1j', 1k', 1L', and 1n'

By following the procedures described in Example 18B, 18C and 18D and substituting Formula 1109 with the following:
- Formula 802 will afford the corresponding random copolymers of Formula 1h',
- Formula 902 will afford the corresponding random copolymers of Formula 1j',
- Formula 902 made with a compound of Formula 103' will afford the corresponding random copolymers of Formula 1j',
- Formula 1002 will afford the corresponding random copolymers of Formula 1k',
- Formula 1002 made with a compound of Formula 103' will afford the corresponding random copolymers of Formula 1L', and
- Formula 1202 will afford the corresponding random copolymers of Formula 1n'.

### 18F. Other Compounds of Formulae 1h',1i', 1j', 1k', 1L', 1m' and 1n'

By following the procedures described in Example 18B, 18C, 18D and 18E, and substituting Formula 1507 with the compounds prepared as described in Examples 17G, 17H and 171, there are obtained the corresponding compounds of Formulae 1h',1i', 1j', 1k', 1L', 1m' and 1n' where t is other than 1, having a plurality of t groups, and having a mixture of glucosyl and galactosyl moieties.

### Example 19

### Formula 1c' where X" is Insulin-B, m is 1, n is 4, p is 90 (30 W¹ + 60 W²), t is 1, R³ is NHAc, R⁴ is OH, R⁸ is CMP, and R¹⁰ is 2-hydroxypropyl

### 19A. Formula 1602 where n is 4, p is 90, t is 1, R³ is NHAc, R⁴ is OH, R⁸ is CMP, and R¹⁰ is 2-hydroxypropyl

A 25 ml Schlenk flask was charged with ((2S,3S,4S,5R,6S)-4,5-dihydroxy-6-(hydroxymethyl)-2-(2-(2-methacrylamidoethoxy)ethoxy)tetrahydro-2H-pyran-3-yl)carbamic acid (272 mg, 0.72 mmol) (Formula 1505, prepared, for example, as described in Example 17D), HPMA (211 mg, 1.47 mmol) (Formula 1506), a dithio-pyridyl functionalized uRAFT agent of Formula 1601 where n is 4 and R⁸ is CMP (12.5 mg, 0.0217 mmol), azobis(isobutyronitril) (0.98 mg, 0.005 mmol), and 1.2 ml dimethylformamide. The reaction mixture was subjected to four freeze-pump-thaw degassing cycles then stirred at 70°C for 20 hours. The corresponding random polymeric product of Formula 1602 (having about 30 W¹ groups and about 60 W² groups) was recovered by precipitating the reaction mixture in acetone. Excess acetone was removed at reduced pressure to provide the random polymeric product, which was used without further purification.

### 19B. Formula 1c' where X" is Insulin-B, m is 1, n is 4, p is 90 (30 W¹ + 60 W²), t is 1, R³ is NHAc, R⁴ is OH, R⁸ is CMP, and R¹⁰ is 2-hydroxypropyl

The Formula 1602 solution obtained in Example 19A (20 mg) was suspended in 200 µl of dimethylformamide and added to an endotoxin-free tube containing Insulin-B (1 mg) and stirred at room temperature for 3 hours to afford the corresponding product of Formula 1c' ("F1c'-Insulin-B-m₁-n₄-p₉₀-CMP-poly-(EtPEG₁AcN-1NAcGLU₃₀-ran-HPMA₆₀"). The reaction mixture was then precipitated in acetone and purified from the reaction mixture via fast protein liquid chromatography (FPLC) using a Superdex 200 prep grade column and used without further purification.

### 19C. Formula 1c' where X" is Insulin-B, m is 1, n is 4, p is 90 (30 W¹ + 60 W²), t is 1, R³ is NHAc, R⁴ is OH, R⁸ is CMP, and R¹⁰ is 2-hydroxypropyl, using N-acetyl-D-galactosamine

By following the procedure of Examples 19A and 19B and substituting ((2S,3S,4S,5S,6S)-4,5-dihydroxy-6-(hydroxymethyl)-2-(2-(2-methacrylamidoethoxy)ethoxy)tetrahydro-2H pyran-3-yl)carbamic acid for Formula 1505, there was obtained the corresponding galactosyl compound of Formula 1c' ("F1c'-Insulin-B-m₁-n₄-p₉₀-CMP-poly-(EtPEG₁AcN-1NAcGAL₃₀-ran-HPMA₆₀").

### 19D. Formula 1c' where X" is P31, m is 1, n is 4, p is 90 (30 W¹ + 60 W²), t is 1, R³ is NHAc, R⁴ is OH, R⁸ is CMP, and R¹⁰ is 2-hydroxypropyl

By following the procedure of Examples 19B and 19C and substituting 20 mg of P31 for Insulin-B, there were obtained the corresponding glucosyl and galactosyl compounds of Formula 1c' where X" is P31.

### 19E. Compounds of Formulae 1f' and 1g'

By following the procedures of Examples 19A and 19B and substituting the uRAFT agent of Formula 1601 with a uRAFT agent of Formulae 600' or 700' there are obtained the corresponding compounds of Formulae 601' or 701', which are in turn contacted with a compound of Formula 101' to afford the corresponding compound of Formula 1f' or Formula 1g', respectively.

### Example 20

### OT-1 challenge-to-tolerance model

20A. As discussed above in Example 14, F1aA-OVA-m₄-n₈ and F1b-OVA-m₁-n₄-p₃₄ mitigated an OVA-specific immune response after adjuvented OVA challenge.

20B. A total of 3 × 10⁵ CFSE-labeled OTI CD8+ T cells and 3 × 10⁵ CFSE-labeled OTII CD4+ T cells were injected into CD45.1+ recipient mice. At 1 and 6 days following adoptive transfer, mice were i.v. administered saline solutions containing OVA, F1m'-OVA-m₁₋₃-n₇₉-p₉₀-q₄-CMP-poly-(EtPEG₁AcN-1NAcGAL₃₀-ran-HPMA₆₀ ["OVA-p(Gal-HPMA)"], F1m'-OVA-m₁₋₃-n₇₉-p₉₀-q₄-CMP-poly-(EtPEG₁AcN-1NAcGLU₃₀-ran-HPMA₆₀ ["OVA-p(Glu-HPMA)"], or saline alone. Each mouse treated with formulations containing OVA in its free or conjugated form, received the molar equivalent of 20 µg OVA. At 15 d following adoptive transfer, mice were challenged with 5 µg of OVA and 25 ng of ultrapure E. coli LPS (InvivoGen) in 25 µL of saline injected intradermally into each rear leg pad (Hock method: total dose of 10 µg of OVA and 50 ng of LPS). Mice were sacrificed 4 days following challenge, and spleen and draining lymph node cells were isolated for restimulation. For flow cytometry analysis of intracellular cytokines, cells were restimulated in the presence of 1 mg/mL OVA or 1 µg/mL SIINFEKL peptide (Genscript) for 3 h. Brefeldin-A (5 µg/mL; Sigma) was added, and restimulation was resumed for an additional 3 h before staining and flow cytometry analysis.

As shown in Figs. 8A-8B, the administration of OVA-p(Gal-HPMA) and OVA-p(Glu-HPMA) resulted in significant reduction in the percentages of OT-I cells (out of the total CD8+ T-cell population) and OT-II cells (out of the total CD4+ T-cell population). Figure 8A shows that OVA-p(Gal-HPMA) and OVA-p(Glu-HPMA) administration significantly reduced OT-I cells as compared to mice receiving repeat administrations of OVA alone (e.g., unconjugated). Reduction was even greater when compared to mice receiving only OVA and LPS challenge (e.g., that received saline injections). Notably, the reduction resulting from treatment with OVA-p(Gal-HPMA) and OVA-p(Glu-HPMA) reduced OT-I cell levels to levels not significantly different from naïve mice. Similarly, as shown in Fig. 8B, OVA-p(Gal-HPMA) and OVA-p(Glu-HPMA) administration resulted in significant reduction in OT-II cells as compared to mice receiving unconjugated OVA or challenge alone. These data indicate that the production of cells that are specifically designed to react when encountering OVA as an antigen decreases, indicative of a reduction in immune response to OVA.

Additionally, the administration of OVA-p(Gal-HPMA) and OVA-p(Glu-HPMA) resulted in significant increases in antigen-specific regulatory T-cells in the lymph node and spleen of mice. As shown in Fig. 9A, treatment with either of these conjugates induced significant increases in CD25+/FoxP3+ cells in the lymph node. Likewise, Fig. 9B shows significant increases (vs. naïve, challenge (saline alone), and OVA treated animals) in CD25+/FoxP3+ OT-II cells. These data indicate that regulatory T cell production is upregulated, which in turn, indicates that the immune system is negatively modulated with respect to its response to OVA (e.g., less responsive, or more tolerant).

Further building on the above data showing the increased tolerance to an antigen after delivery of that antigen complex with a liver targeting moiety is the data shown in Figure 10. In this experiment, the percentage of cells expressing interferon gamma (IFNγ) was measured. IFNy is produced by CD4 and CD8 T cells after antigen-specific immunity develops. As shown in Fig. 10, mice receiving only saline pre-challenge have approximately 60% of the total OTI cells expressing IFNy. In contrast, OVA-treated mice have about 40% IFNy-expressing cells. Nearly the same as naïve mice, the OTI cells of OVA-p(Gal-HPMA) and OVA-p(Glu-HPMA)-treated mice have less than 20% IFNy positive cells. This significant reduction in IFNy indicates a reduction in the mechanisms that drive antigen-specific immunity. Collectively, and in view of the additional disclosure herein, these data demonstrate that targeting an antigen to the liver can reduce the antigen-specific immune response to that antigen. In particular, targeting with glucose or galactose results in significant shifts in the cell populations responsible for antigen-specific immunity, that shift demonstrating a tolerance to the specific antigen.

20C. By following the procedures described in Example 20A or 20B and substituting the tested OVA compositions with other compositions of Formula 1 followed by challenge with the unconjugated antigen X, the treated animals demonstrate a tolerance to the specific antigen X.

### Example 21

### OTI / OTII challenge to tolerance model

Using the model of Example 20, additionally with OTII cells (which are CD4⁺ T cells from CD45.2⁺ mice, analogous to the CD8⁺ T cell OTI cells), the ability of F1m'-OVA-m₁₋₃-n₇₉-p₉₀-q₄-CMP-poly-(EtPEG₁AcN-1NAcGAL₃₀-ran-HPMA₆₀ ["OVA-p(Gal-HPMA)"] and F1m'-OVA-m₁₋₃-n₇₉-p₉₀-q₄-CMP-poly-(EtPEG₁AcN-1NAcGLU₃₀-ran-HPMA₆₀ ["OVA-p(Glu-HPMA)"] to induce T regulatory responses and prevent subsequent responses to vaccine-mediated antigen challenge were demonstrated, moreover using different dosing regimens. 3 × 10⁵ CFSE-labeled OTI and 3 × 10⁵ CFSE-labeled OTII cells were adoptively transferred to CD45.1⁺ mice (n = 8 mice per group) on day 0. On days 1, 4 and 7, tolerogenic doses or control doses were administered. In one regimen, OVA was provided at a dose of 2.5 µg at day 1, 2.5 µg at day 4, and 16 µg at day 7. In another, OVA was provided at a dose of 7 µg at day 1, 7 µg at day 4, and 7 µg at day 7, for the same total dose. Likewise, pGal-OVA and pGlu-OVA were each administered in other groups at the same dosings of 2.5 µg at day 1, 2.5 µg at day 4, and 16 µg at day 7 or 7 µg at day 1, 7 µg at day 4, and 7 µg at day 7, all doses being on an OVA equivalent dose basis. In a final group, saline was administered on the same days. On day 14, the recipient mice were then challenged with OVA (10 µg) adjuvanted with lipopolysaccharide (50 ng) by intradermal injection. Characterization of the draining lymph nodes was done on day 19, to allow determination as to whether or not deletion actually took place and whether regulatory T cells were induced from the adoptively transferred cells.

Profound tolerance was induced in the CD4+ T cell compartment, as shown in Figs. 11A-11B. In terms of total cell frequencies, both dosing regimens of both OVA-p(Gal-HPMA) and OVA-p(Glu-HPMA) resulted in equivalent low levels of OTII cells after challenge, statistically lower than by treatment of OVA (* and # indicate p<0.05, ** and ## indicate p<0.01), as shown in Fig. 11A. When the cells that remained were analyzed by flow cytometry for the presence of the transcription factor FoxP3 and the receptor CD25, the numbers of FoxP3+CD25+ cells (markers of T regulatory cells) was statistically significantly elevated compared to treatment with OVA alone, as shown in Fig. 11B. Here, the number of T regulatory cells was statistically higher with the 2.5 µg / 2.5 µg / 16 µg dosing regimen compared to the 7 µg / 7 µg / 7 µg dosing regimen, with both OVA-p(Gal-HPMA) and OVA-p(Glu-HPMA) treatment.

Profound tolerance was also induced in the CD8+ T cell compartment, as shown in Figs. 12A-12B. In terms of total cell frequencies, both dosing regimens of both OVA-p(Gal-HPMA) and OVA-p(Glu-HPMA) resulted in equivalent low levels of OTI cells after challenge, statistically lower than by treatment of OVA (* and # indicate p<0.05, ** and ## indicate p<0.01), as shown in Fig. 12A. When the cells that remained were analyzed by flow cytometry for the expression of IFN-γ after re-exposure to OVA antigen, the frequency of cells expressing this inflammatory cytokine was decreased in the groups receiving the 2.5 µg / 2.5 µg / 16 µg dosing regimen compared to the 7 µg / 7 µg / 7 µg dosing regimen, with both OVA-p(Gal-HPMA) and OVA-p(Glu-HPMA) treatment, as shown in Fig. 12B.

### Example 22

### BDC2.5 Study

22A. CD4+ T-cells of the transgenic NOD-BDC2.5 mice express the diabetogenic BDC-2.5 specific regulatory T-cell receptor (TCR). BDC2.5 T-cells specifically target the islet beta-cell autoantigen, chromogranin-A. T-cells were isolated from the spleens of transgenic NOD-BDC2.5 mice and cultured for 4 days in DMEM supplemented with 10% (vol/vol) FBS, 0.05 mM β-mercaptoethanol, 1% puromycin/streptomycin, and 0.5 µM P31 peptide, a mimotope of islet beta-cell autoantigen chromogranin-A that stimulates T-cells expressing the BDC2.5 T-cell receptor. Following stimulation with P31, cells were washed with basal DMEM and analyzed for purity by flow cytometry, and 5 × 10⁶ T-cells were i.v. injected into normoglycemic NOD/ShiLtJ mice. At 8 h and 3 days after adoptive transfer, mice were i.v. administered saline, 10 µg F1c'-P31-m₁-n₄-p₉₀-CMP-poly-(EtPEG₁AcN-1NAcGLU₃₀-ran-HPMA₆₀, 10 µg F1c'-P31-m₁-n₄-p₉₀-CMP-poly-(EtPEG₁AcN-1NAcGAL₃₀-ran-HPMA₆₀, or an equimolar dose of P31 peptide. Starting on day 4, diabetes onset was monitored by measuring nonfasting blood glucose levels using an AccuCheck Aviva glucometer (Roche). Mice were considered diabetic at blood glucose readings ≥300 mg/dL. After two hyperglycemic readings, mice were euthanized. The data resulting from this experiment is shown in the time course of Fig. 13. As shown, the mice receiving saline developed diabetic blood glucose levels within 4-8 days of adoptive transfer. Similarly, mice receiving P31 (unconjugated) developed diabetic blood glucose levels within about 7-10 days after transfer. In stark contrast, mice receiving F1c'-P31-m₁-n₄-p₉₀-CMP-poly-(EtPEG₁AcN-1NAcGLU₃₀-ran-HPMA₆₀ or F1c'-P31-m₁-n₄-p₉₀-CMP-poly-(EtPEG₁AcN-1NAcGAL₃₀-ran-HPMA₆₀ maintained relatively steady blood glucose values (<200 mg/dl) for over 40 days.

22B. By following the procedures described in Example 21A and substituting the tested compositions with other compositions of Formula 1 where X is Insulin-B or proinsulin, preproinsulin, glutamic acid decarboxylase-65 (GAD-65 or glutamate decarboxylase 2), GAD-67, glucose-6 phosphatase 2 (IGRP or islet-specific glucose 6 phosphatase catalytic subunit related protein), insulinoma-associated protein 2 (IA-2), and insulinoma-associated protein 2β (IA-2β), ICA69, ICA12 (SOX-13), carboxypeptidase H, Imogen 38, GLIMA 38, chromogranin-A, HSP-60, caboxypeptidase E, peripherin, glucose transporter 2, hepatocarcinoma-intestine-pancreas/pancreatic associated protein, SLOOP, glial fibrillary acidic protein, regenerating gene II, pancreatic duodenal homeobox 1, dystrophia myotonica kinase, islet-specific glucose-6-phosphatase catalytic subunit-related protein and SST G-protein coupled receptors 1-5, such as F1aA-Insulin-m₂-n₈₀, F1aA-Insulin-m₂-n₁₂, F1aA-Insulin-m₂-n₃₃, F1aA-Insulin-m₂-n₈₀, F1aA-Insulin-m₂-n₄₃, F1aA-Insulin-m₂-n₅₀, F1aA-Insulin-m₂-n₆₀, F1aA-Insulin-m₂-n₇₅, F1aA-Insulin-m₂-n₈₄, F1b-Insulin-m₂-n₄-p₃₄-2NAcGAL, F1m-Insulin-m₂-n₈₀-p₃₀-q₄-CMP-2NHAc, F1m-Insulin-m₂-n₆₂-p₃₀-q₈-CMP-2OH, F1n-insulin-m₂-n₁-p₃₀-q₄-CMP-2NHAc, F1c'-Insulin-B-m₁-n₄-p₉₀-CMP-poly-(EtPEG₁AcN-1NAcGLU₃₀-ran-HPMA₆₀ or F1c'-Insulin-B-m₁-n₄-p₉₀-CMP-poly-(EtPEG₁AcN-1NAcGAL₃₀-ran-HPMA₆₀ the blood glucose values in the treated NOD mice remain steady as compared to animals that receive saline.

### Example 23

### NOD Mouse

23A. Non-obese diabetic (NOD) mice, such as NOD/ShiLt mice are susceptible to the spontaneous onset of autoimmune diabetes mellitus, which is the result of an autoimmune response to various pancreatic auto-antigens. Diabetes develops in NOD mice as a result of insulitis, characterized by the infiltration of various leukocytes into the pancreatic islets. As diabetes develops, there is a leukocytic infiltration of the pancreatic islets followed by significant decreases in insulin production, and corresponding increases in blood glucose levels.

In order to evaluate the efficacy of a treatment for diabetes mellitus, compositions and methods for the treatment being provided in the present disclosure, starting at 5 weeks of age diabetes onset in a cohort of NOD/ShiLt mice was monitored on a weekly basis by measuring nonfasting blood glucose levels using an AccuCheck Aviva glucometer (Roche). Starting at 6 weeks of age, the mice were divided into control and test groups (n = 15 for each group) and treated, respectively, with weekly intravenous injections of saline, 10 µg of F1c'-Insulin-B-m₁-n₄-p₉₀-CMP-poly-(EtPEG₁AcN-1NAcGLU₃₀-ran-HPMA₆₀, or 10 µg of F1c'-Insulin-B-m₁-n₄-p₉₀-CMP-poly-(EtPEG₁AcN-1NAcGLU₃₀-ran-HPMA₆₀ (10 µg). The injections continued for 10 consecutive weeks. The percentage of diabetes free animals was measured over time. Mice were considered diabetic at two consecutive blood glucose readings ≥300 mg/dL or one blood glucose readings ≥450 mg/dL. Mice deemed diabetic were euthanized.

Fig. 14 depicts the data obtained as described above as the percentage of diabetes free animals as measured over time. Mice treated with F1c'-Insulin-B-m₁-n₄-p₉₀-CMP-poly-(EtPEG₁AcN-1NAcGLU₃₀-ran-HPMA₆₀ are shown as filled squares. Mice treated with F1c'-Insulin-B-m₁-n₄-p₉₀-CMP-poly-(EtPEG₁AcN-1NAcGAL₃₀-ran-HPMA₆₀ are shown as filled triangles. Mice treated with saline are shown as filled diamonds. As can readily be appreciated from the data collected from the saline treated animals over time as early as 11 weeks of age, spontaneous diabetes was present. Prevalence increased over time (shown by the downward trend in the graph) with 60% of the tested animals developing diabetes by week 20. As shown in Fig. 14, treating NOD mice with either F1c'-Insulin-B-m₁-n₄-p₉₀-CMP-poly-(EtPEG₁AcN-1NAcGLU₃₀-ran-HPMA₆₀ or F1c'-Insulin-B-m₁-n₄-p₉₀-CMP-poly-(EtPEG₁AcN-1NAcGAL₃₀-ran-HPMA₆₀ reduced the incidences of diabetes onset in NOD mice as compared to animals that received saline. The data demonstrate that administration of insulin coupled with linkers and liver targeting moieties as disclosed herein can successfully reduce the development of type I diabetes mellitus by reducing the autoimmune response to the various pancreatic autoantigens produced.

23B. By following the procedures described in Example 22A and substituting the tested compositions with other compositions of Formula 1 where X is Insulin-B or proinsulin, preproinsulin, glutamic acid decarboxylase-65 (GAD-65 or glutamate decarboxylase 2), GAD-67, glucose-6 phosphatase 2 (IGRP or islet-specific glucose 6 phosphatase catalytic subunit related protein), insulinoma-associated protein 2 (IA-2), and insulinoma-associated protein 2β (IA-2β), ICA69, ICA12 (SOX-13), carboxypeptidase H, Imogen 38, GLIMA 38, chromogranin-A, HSP-60, caboxypeptidase E, peripherin, glucose transporter 2, hepatocarcinoma-intestine-pancreas/pancreatic associated protein, S100β, glial fibrillary acidic protein, regenerating gene II, pancreatic duodenal homeobox 1, dystrophia myotonica kinase, islet-specific glucose-6-phosphatase catalytic subunit-related protein and SST G-protein coupled receptors 1-5, such as F1aA-Insulin-m₂-n₈₀, F1aA-Insulin-m₂-n₁₂, F1aA-Insulin-m₂-n₃₃, F1aA-Insulin-m₂-n₈₀, F1aA-Insulin-m₂-n₄₃, F1aA-Insulin-m₂-n₆₀, F1aA-Insulin-m₂-n₆₀, F1aA-Insulin-m₂-n₇₅, F1aA-Insulin-m₂-n₈₄, F1b-Insulin-m₂-n₄-p₃₄-2NAcGAL, F1m-Insulin-m₂-n₈₀-p₃₀-q₄-CMP-2NHAc, F1m-Insulin-m₂-n₆₂-p₃₀-q₈-CMP-2OH, F1n-insulin-m₂-n₁-p₃₀-q₄-CMP-2NHAc, F1c'-P31-m₁-n₄-p₉₀-CMP-poly-(EtPEG₁AcN-1NAcGLU₃₀-ran-HPMA₆₀ or F1c'-P31-m₁-n₄-p₉₀-CMP-poly-(EtPEG₁AcN-1NAcGAL₃₀-ran-HPMA₆₀ the incidences of diabetes onset in the treated NOD mice are reduced as compared to animals that receive saline.

### Example 24

### Biodistribution

In order to examine the biodistribution of antigen glycopolymer conjugates we treated BALB/c mice with fluorescently labeled OVA or fluorescently-labeled OVA conjugated to either p(Gal-HPMA), p(Glu-HPMA), p(Galβ-HPMA), or p(Gluβ-HPMA). The sugar moieties attached to the backbone of p(Gal-HPMA) and p(Glu-HPMA) are attached to the polymer in the α-conformation at the C1 position, whereas the sugars attached to the backbone of p(Galβ-HPMA) and p(Gluβ-HPMA) are attached to the polymer in the β-conformation at the C1 position. OVA was labeled with Dy750. All treatments were given via tail vein injection in 140 µl. Each animal was treated with an equal amount of fluorescent conjugate on a fluorescence unit basis. After 3 hours, the animals were euthanized and the livers of each animal were perfused with saline, then both the livers and spleens were harvested and imaged via an IVIS Spectrum system with appropriate filter set.

Fig. 15 depicts representative images of the fluorescent signals of livers (A) and spleens (B) from animals treated with OVA or OVA glycopolymer conjugates. The formulations are as follows: 1. OVA, 2. F1m'-OVA750-m₁₋₃-n₇₉-p₉₀-q₄-CMP-poly-(EtPEG₁AcN-1NAcGALβ₃₀-ran-HPMA₆₀) ["OVA-p(Galβ-HPMA)"], 3. F1m'-OVA750-m₁₋₃-n₇₉-p₉₀-q₄-CMP-poly-(EtPEG₁AcN-1NAcGAL₃₀-ran-HPMA₆₀) ["OVA-p(Gal-HPMA)"], 4. F1m'-OVA750-m₁₋₃-n₇₉-p₉₀-q₄-CMP-poly-(EtPEG₁AcN-1NAcGLUβ₃₀-ran-HPMA₆₀) ["OVA-p(Gluβ-HPMA)"], 5. F1m'-OVA750-m₁₋₃-n₇₉-p₉₀-q₄-CMP-poly-(EtPEG₁AcN-1NAcGLU₃₀-ran-HPMA₆₀) ["OVA-p(Glu-HPMA)"]. Images of the livers from animals treated as described above show that glycopolymer conjugates significantly enhance the delivery of their conjugated antigen to the liver (or spleen) as compared to the uptake of unconjugated antigens. Livers from animals treated with unconjugated OVA have less fluorescent signal as compared to livers from animals treated with OVA conjugated to either p(Gal-HPMA), p(Glu-HPMA), p(Galβ-HPMA), or p(Gluβ-HPMA). Additionally, images of the spleens taken from animals treated as described above show that conjugating antigens to glycopolymers reduces the delivery of antigens to the spleen. Spleens from animals treated with unconjugated OVA have significantly more fluorescent signal as compared to spleens from animals treated with OVA conjugated to either p(Gal-HPMA), p(Glu-HPMA), p(Galβ-HPMA), or p(Gluβ-HPMA). These data are significant in that they demonstrate enhanced targeting of an antigen to which tolerance is desired to the liver and/or spleen, which , as demonstrated by the experimental data presented herein results in reduced immune response (i.e., induced tolerance) to the antigen. In accordance with several embodiments disclosed herein, this induced tolerance can treat, reduce, prevent, or otherwise ameliorate an unwanted immune response that would have otherwise been associated with exposure to the antigen.

### Example 25

### 7-Dav OTI / OTII Phenotype Analysis

In order to compare the ability of various glycopolymer-antigen conjugates to induce antigen-specific T cell proliferation as well as upregulate the expression and presentation of various markers of T cell anergy and deletion, mice that had received an infusion of 400,000 carboxyfluorescein succinimidyl ester (CSFE)-labeled OTI cells were treated with an intravenous injection of either OVA or OVA conjugated to either p(Gal-HPMA), p(Glu-HPMA), p(Galβ-HPMA), or p(Gluβ-HPMA) (with formulations as follows: F1m'-OVA-m₁₋₃-n₇₉-p₉₀-q₄-CMP-poly-(EtPEG₁AcN-1NAcGAL₃₀-ran-HPMA₆₀) ["OVA-p(Gal-HPMA)"]; F1m'-OVA-m₁₋₃-n₇₉-p₉₀-q₄-CMP-poly-(EtPEG₁AcN-1NAcGLU₃₀-ran-HPMA₆₀) ["OVA-p(Glu-HPMA)"]; F1m'-OVA-m₁₋₃-n₇₉-p₉₀-q₄-CMP-poly-(EtPEG₁AcN-1NAcGALβ₃₀-ran-HPMA₆₀) ["OVA-p(Galβ-HPMA)"]; F1m'-OVA-m₁₋₃-n₇₉-p₉₀-q₄-CMP-poly-(EtPEG₁AcN-1NAcGLUβ₃₀-ran-HPMA₆₀) ["OVA-p(Gluβ-HPMA)"]. Animals treated with OVA in either its free or conjugated form received 10 µg of OVA on day 1 and day 3 of the experiment. A timeline of the experimental details is shown in Figure 16A. After 7 days, the mice were sacrificed and the splenocytes of the animals were harvested and analyzed via flow cytometry for phenotypical markers characteristic of T cell anergy, deletion, and memory.

Figure 16B shows that OVA-glycopolymer conjugates induce more OTI T cell proliferation as compared to the amount of OTI proliferation seen in animals treated with unconjugated OVA. As discussed above, these data further support that, according to several embodiments disclosed herein, the glyoctargeting moieties disclosed herein result in increased antigen-specific T-cell proliferation - a key step in inducing tolerance to an antigen. Interestingly, animals treated with OVA-glycopolymer conjugates containing β-linked sugars induced significantly more proliferation compared to animals treated with glycopolymers containing the same sugar moiety linked to the polymer via an α-linkage (e.g., p(Galβ-HPMA) vs. p(Gal-HPMA)). Unexpectedly, this conformational change in one element of the overall composition leads to an enhanced efficacy in terms of T-cell proliferation, which Applicant believes (without being bound by theory) results from synergistic interaction of the components of the composition with their respective physiological targets. Additionally, the population of OTI cells taken from animals treated with all OVA-glycopolymer conjugates, with the exception of OVA-p(Gal-HPMA), showed significantly more surface expression of the apoptosis marker Annexin V+ as compared to the cells taken from animals treated with OVA (see Figure 16C). Consistent with data discussed above, this indicates a greater percentage of antigen-specific T cells are being targeted for, or are in, the apoptotic cascade. As shown in Figure 16D, OTI cells taken from animals treated with OVA-glycopolymer conjugates containing β-linked sugars showed an increased expression of the T cell exhaustion marker PD-1 as compared to animals treated with glycopolymers containing the same sugar moiety linked to the polymer via an α-linkage as well as animals treated with free OVA. In order to maintain long-term tolerance, treatments must reduce the number of long-lasting antigen-specific memory T cells. Figures 16E and 16F show that both OVA-p(Galβ-HPMA) and OVA-p(Gluβ-HPMA) induce a significant reduction in OTI cells expressing markers for memory T cells. Both OVA-p(Galβ-HPMA) and OVA-p(Gluβ-HPMA) induce a fivefold decrease in the number of memory T cells compared to animals treated with free OVA. These data further indicate that compositions as disclosed herein can induce tolerance to an antigen (OVA chosen here due to its general acceptance in the field as a "gold standard" antigen), and in several embodiments, can unexpectedly enhance the induction of tolerance (as represented at least in part by antigen-specific T cell proliferation, increased Annexin V expression on antigen-specific T cells, increased exhaustion marker expression on antigen-specific T cells, and reduced expression of memory T cells).

### Example 26

### Glucose-Coupled Antigens - BDC2.5 Study

This experiment was conducted to test the ability of p(Glu)-antigen conjugates to inhibit the development of CD4 T cell driven auto immunity. A mouse model of antigen-specific T cell induced autoimmune diabetes was used. Autoimmune diabetes was induced by transferring activated BDC2.5 T cells, which carry a T cell receptor for the diabetic autoantigen chromogranin A, into immune compromised mice. Recipient mice were then treated with an autoantigen peptide mimotope, termed p31, or with the p31-p(Glu) conjugates.

Protocol: Freshly isolated splenocytes from female BDC2.5 transgenic mice were stimulated for 4d in DMEM supplemented with 10% FBS, 0.05 mM β-mercaptoethanol, 1% puromycin/streptomycin, and 0.5 µM p31 peptide. Following stimulation, cells were washed with DMEM, resuspended in DMEM, and injected intravenously into normoglycemic NOD/Scid mice. At 8 h following adoptive transfer, mice were intravenously administered saline (naive), 2 µg of p31, or 2 µg of p31 as p31-pGlu conjugates (glucose in the beta conformation was used in this experiment, though in several embodiments the alpha conformation can be used). Three days after the initial treatment, mice were treated again with saline, 2 µg of p31, or 2 µg of p31 as p31-pGlu conjugates. Starting on day 1, the nonfasting blood glucose levels of each mouse were measured using an Accucheck Aviva glucometer. Mice were considered diabetic upon receiving a single blood sugar measurement over 450 mg/dL, or two consecutive blood sugar readings of over 350 mg/dL. All groups had 7 mice.

Results: As shown in Figure 17, naïve mice began to develop diabetes (as measured by hyperglycemia) within 5 days of the splenocyte transfer. Within about 7 days, all of the naïve mice developed hyperglycemia. Those mice treated with the control composition (p31; filled circles) began to develop hyperglycemia shortly after the naïve mice. By 8 days post-transfer, all of the p31-treated mice had developed hyperglycemia. In stark contrast, administration of p31-p(Glu) conjugates (filled triangles) results in mice that maintain normal blood glucose levels for a significantly longer period of time as compared to naïve animals and those treated with the p31 peptide alone. At 15 days post-transfer, 100% of the p31-pGlu mice still had normal blood glucose levels.

These results demonstrate that p31-p(Glu) conjugates prevent the development of hyperglycemia in mice. Thus, according to several embodiments disclosed herein, such conjugates are efficacious for use in preventing development of diabetes. In still additional embodiments, p(Glu) conjugates are efficacious for use in reducing advancement of pre-existing diabetes, and in still additional embodiments, efficacious for use in reversing pre-existing diabetes. In additional embodiments, other mimotopes of islet beta-cell autoantigen chromogranin-A can be used. Further, chromogranin-A, as a full-length protein, or fragments thereof can be employed as the antigenic portion of the tolerogenic compositions, according to several embodiments disclosed herein. Additionally, other antigens disclosed herein related to diabetes can be used, in some embodiments, including but not limited to insulin, proinsulin, preproinsulin, GAD-65, GAD-67, IGRP, IA-2, IA-2β, fragments thereof, or mimotopes thereof. Other diabetes related antigens are disclosed herein. Further, in several embodiments, antigens related to other autoimmune diseases can be used, as is disclosed herein. In still additional embodiments, foreign antigens, transplant antigens or other types/classifications may be used, as chromogranin A was employed here, and using this model as a non-limiting example of the ability of compositions as disclosed herein to be used in the induction of tolerance.

### Example 27

### Memory of Tolerance

The following experiment was conducted to demonstrate that tolerogenic compositions as disclosed herein can establish memory of tolerance. In order to determine if regulatory T cells (Tregs) induced by administration of tolerogenic compositions as disclosed herein (p(Glu)-antigen conjugates were used here as a non-limiting example) are able to establish long term tolerance after the initial administration of the therapies, mice were pre-treated with an infusion of OTII T cells followed by administration of p(Glu)-OVA conjugates. These mice were then were challenged three weeks later with an infusion of OVA-specific T cells and antigen challenge. To demonstrate that any tolerance inducing effect was the result of Tregs, mice treated with p(Glu)-OVA were injected with an anti-CD25 antibody, which has been shown to deplete Tregs.

Protocol: On day zero, C57BL/6 mice received an intravenous infusion of 450,000 OTII cells, then were treated on days 1 and 4 with saline or 1.5 µg of OVA as free OVA or p(Glu)-OVA conjugates (OVA is used herein as a non-limiting example of an antigen to which tolerance is desired; glucose in the beta conformation was used in this experiment, though in several embodiments the alpha conformation can be used). On day 7, mice received a final treatment of saline, or 15 µg of OVA as free OVA or p(Glu)-OVA conjugates. On day 15, half of the animals treated with p(Glu)-OVA were treated with an intraperitoneal injection of 300 µg of anti-CD25 antibody, which has been shown to deplete regulatory T cells (Tregs). On day 29, mice received an infusion of 750,000 OTII T cells and 750,000 OTI T cells. The next day, animals were challenged with 5µg of OVA and 50 ng of ultrapure LPS in each of the 4 footpads. Five days after antigen challenge, the mice were sacrificed and the number of OTI and OTII T cells in the draining lymph nodes was assessed by flow cytometry. (Groups n=5: Challenge (i.e. vehicle treated animals), OVA, p(Glu)-OVA, p(Glu)-OVA + αCD25). This protocol is schematically depicted in Fig. 18.

Results: The results of this experiment are shown in Figs. 19A-19B. Fig. 19A depicts the remaining OTI T cells after antigen challenge (as a percentage of total CD8⁺ cells). Fig. 19B depicts the remaining OTII T cells after antigen challenge (as a percentage of total CD4⁺ cells). Those mice receiving LPS challenge and OVA absent a tolerogenic composition showed significantly elevated OTI and OTII cells in draining lymph nodes. Those mice receiving pGlu-OVA showed a significant reduction in both OTI and OTII cells. The abrogation of the tolerance induced by pGlu-OVA by the administration of anti-CD25 antibodies demonstrates that there is a role for Tregs in the pGlu-OVA-induced tolerance. Taken together, these data indicate that the tolerogenic compositions disclosed herein are able to induce long-lasting tolerance through the induction of regulatory T cells. This is advantageous, in several embodiments, because the long-lasting tolerance reduces (or in some embodiments eliminates) the need for ongoing administration of the compositions disclosed herein. That being said, in some embodiments, repeated administration is optionally performed.

### Example 28

### Memory of Tolerance (Endogenous)

The following experiment was conducted to demonstrate that tolerogenic compositions as disclosed herein can establish memory of tolerance from endogenous T cells. This experiment was conducted similarly to Example 27, but the mice did not receive the initial infusion of donor OTII T cells.

In order to determine if Tregs from the endogenous T cell population could be induced by p(Glu)-antigen conjugates and exhibit long-term tolerance, mice were treated with p(Glu)-OVA conjugates. They were then challenged three weeks later with an infusion of OVA-specific T cells and an antigen challenge. To investigate whether the demonstrated tolerance inducing effect was the result of endogenous Tregs, mice were treated with p(Glu)-OVA and later treated with anti-CD25 antibody.

Protocol: On day zero and 3, BL6/C57 mice received an intravenous infusion of saline or 1.5 µg of OVA as free OVA or p(Glu)-OVA conjugates (glucose in the beta conformation was used in this experiment, though in several embodiments the alpha conformation can be used). On day 6, mice received a final treatment of saline, or 15 µg of OVA as free OVA or p(Glu)-OVA conjugates. On day 14, half of the animals treated with p(Glu)-OVA were treated with an intraperitoneal injection of 300 µg of anti-CD25 antibody. On day 28, mice received an infusion of 750,000 OTII and 750,000 OTI T cells. The next day, animals were challenged with 5µg of OVA and 50 ng of ultrapure LPS in each of the 4 footpads. Five days after antigen challenge, the mice were sacrificed and the number of OTI and OTII T cells in the draining lymph nodes was assessed by flow cytometry. (Groups n=5: Challenge (i.e. vehicle treated animals), OVA, p(Glu)-OVA, p(Glu)-OVA + αCD25). This protocol is schematically depicted in Fig. 20.

Results: The results of this experiment are shown in Figs. 21A-21B. Fig. 21A depicts the remaining OTI T cells after antigen challenge (as a percentage of total CD8⁺ cells). Fig. 21B depicts the remaining OTII T cells after antigen challenge (as a percentage of total CD4⁺ cells). Those mice receiving LPS challenge and OVA absent a tolerogenic composition showed significantly elevated OTI and OTII cells in draining lymph nodes. Those mice receiving pGlu-OVA showed a significant reduction in both OTI and OTII cells in comparison. The abrogation of the tolerance induced by pGlu-OVA by the administration of anti-CD25 antibodies demonstrates that Tregs derived from the endogenous T cell population play a significant role in the tolerance induction by the pGlu-antigen composition. Taken together, these data indicate that the tolerogenic compositions disclosed herein are able to induce long-lasting tolerance through the induction of endogenous regulatory T cells. This is advantageous, in several embodiments, because the long-lasting tolerance reduces (or in some embodiments eliminates) the need for ongoing administration of the compositions disclosed herein. That being said, in some embodiments, repeated administration is optionally performed. Moreover, these data indicate that supplementing the T cells of a recipient with exogenous T cells is not necessary for induction of tolerance. Rather, the endogenous pool of T cells is sufficient to provide regulatory T cells in appropriate numbers to result in long-term tolerance.

### Example 29

### Prophylactic Administration of Tolerogenic Compositions Reduces Subsequent Antibody Generation

As disclosed herein, in several embodiments, the tolerogenic compositions are useful for the reduction, treatment, prevention or otherwise amelioration of immune responses against antigens of interest. In some embodiments, therefore the ultimate use of the tolerogenic composition, e.g., prevention versus treatment, is determined by the current state of a subject prior to receiving administration of the composition. The present experiment was performed in order to demonstrate that tolerogenic compositions disclosed herein can be used to reduce the degree of antibody generation to a specific antigen, through the prophylactic administration of a tolerogenic composition.

Protocol: The experimental design is depicted in Fig. 22. For pretreatment, there were two groups, those receiving 15 µg of a tolerogenic composition comprising p-Glu-Asparaginase (high dose) and those receiving 2.5 µg of p-Glu-Asparaginase (low dose) (glucose in the beta conformation was used in this experiment, though in several embodiments the alpha conformation can be used). These pretreatment steps are depicted in Figure 22 as step "A". Note that, asparaginase is used in this experiment as a nonlimiting example of an antigen to which tolerance is desired. As discussed above, numerous other antigens, fragments thereof, or mimotopes thereof can also be used, depending on the embodiment. After the pretreatment, or at the initiation of the experimental protocol for the wild type asparaginase (WT-Asn) and mixed groups, animals were administered either 15 µg of asparaginase or a combination of 12.5 µg of asparaginase along with 2.5 µg of pGlu-Asparaginase. These administrations are depicted as step "B" in Figure 22. For all experimental groups, step "C" represents collection of a 10 µL blood sample.

Results: results of this experiment are shown in Figure 23. The trace that show increases in anti-asparaginase antibody titers beginning at approximately two days and steadily increasing to relative values of between 3 and 4 are the result of the administration of asparaginase alone. In contrast, the pretreatment with either high dose or low dose asparaginase significantly reduced the generation of anti-asparaginase antibodies, and consistently held the antibody titer at relatively lower values through the 59 day testing protocol. The negative control group (antigen naïve) shows no development of antigen-specific antibodies.

These data demonstrate that, in addition to treating or reducing a pre-existing immune response to an antigen of interest, in several embodiments, the tolerogenic compositions disclosed herein can also be used in preventing the initial immune response. As shown in this experiment, both high and low doses of glycotargeting therapeutics coupled to an antigen of interest administered as a pretreatment ameliorated the generation of antibodies against the antigen of interest. In several embodiments, such an approach is particularly effective when an exogenous therapeutic agent is to be administered to a patient. In some such embodiments depending upon the therapeutic agent, the tolerogenic composition need not include the entire therapeutic agent as the antigen of interest (although in some embodiments the entire therapeutic agent is included), but rather can include a fragment of the therapeutic agent a particular epitope of the therapeutic agent, or a mimotope of an antigenic region of the therapeutic agent. In some embodiments, the therapeutic agent is a protein drug. Additionally, in some embodiments longer and/or more frequent pretreatment administrations of the tolerogenic composition may serve to even further reduce the generation of antibodies against the antigen of interest. However, in some embodiments a single pretreatment step may be sufficient.

### Example 30

### Tolerogenic Compositions Ameliorate Multiple Sclerosis

As discussed above, a variety of diseases associated with an immune response, including autoimmune responses, can be treated through the generation and use of tolerogenic compositions as disclosed herein. As a nonlimiting example of such a used in the autoimmune context, the present experiment was designed to determine the efficacy of a tolerogenic composition in the treatment of multiple sclerosis (MS).

The MS-related tolerogenic compositions were tested in a mouse model of multiple sclerosis (experimental autoimmune encephalomyelitis, EAE). The auto-antigen used for vaccination in the model was a peptide derived from myelin oligodendrocyte glycoprotein (MOG), amino acid numbers 35-55 (MOG35-55; SEQ ID NO:24). The auto-antigen utilized for treatment in the model was a slightly longer peptide sequence (MOG30-60; SEQ ID NO:25), which contained the vaccination peptide specified above. The longer therapeutic sequence was used to ensure processing by antigen presenting cells and consequently reduce the tendency of the soluble peptide to bind to cell-surface major histocompatibility complex in the absence of uptake by antigen presenting cells.

Protocol: The experimental protocol is shown in Figure 24A, with the therapeutic tolerogenic composition shown in Figure 24B. EAE was induced by immunizing donor B6.SJL mice with MOG35-55/CFA. 11 days later, their spleen cells are harvested and restimulated in culture with MOG35-55 peptide, anti-IFNy antibodies and IL-12 for 3 days. The resultant encephalitogenic cells were injected into recipient groups of mice, which then develop MS symptoms. Group sizes were 10 mice each, of which half were sacrificed at the peak of disease (Day 11) and half were sacrificed at the end of experiment (Day 20). Body weights were measured 3 times per week and disease state was scored daily, blinded. Control peptide (MOG30-60), glycotargeting peptide (pGlu-MOG30-60; (glucose in the alpha conformation was used in this experiment, though in several embodiments the beta conformation can be used), or vehicle (saline) were given on Days 0, 3, and 6 in either 0.8 µg or 4.0 µg doses. A positive efficacy treatment control (FTY720) was given daily for the duration of the study. FTY720 (fingolimod) is a first-in-class sphingosine 1-phosphate (S1P) receptor modulator deemed effective in Phase II clinical trials for MS.

Figure 25A shows the assessment of the tolerogenic composition's ability to delay disease onset as compared to control animals. As can be seen, at day 11 (the peak of the disease symptoms for the control group), administration of 0.8 µg of pGlu-MOG₃₀₋₆₀ (filled triangles) resulted in a significant decrease in the EAE disease score (as compared to the MOG peptide alone). Figure 25B shows data in relation to reduction of weight loss that is associated with MS. Similar to the EAE disease score, pGlu-MOG₃₀₋₆₀ showed significantly reduced weight loss at day 11 of the experiment (Figure 25B; as compared to MOG alone). In contrast, the peptide alone group and the control group exhibited loss of approximately 25% of body weight at the same time point in the experiment.

Figure 26A corresponds to the assessment of disease onset delay with the higher, 4 µg dose of pGlu-MOG₃₀₋₆₀. Unexpectedly, the delay in disease onset was strikingly improved (significant vs. both MOG alone and FTY720, with none of the mice in the treatment group (filled triangles) exhibiting a nonzero EAE disease score until 14 days into the experiment. In contrast, by day 6, each of the other experimental groups was showing signs of MS, as evidenced by the increased EAE disease score. Again, similar to EAE disease score, the higher, 4 µg dose of pGlu-MOG₃₀₋₆₀ resulted in significant reductions in weight loss. At day 11, the pGlu-MOG₃₀₋₆₀ group (closed triangles) had essentially zero weight loss, while all other groups had lost weight (~2.5 grams in the FTY720 group and ~5 grams in the MOG alone and control groups, see Fig. 26B). Thus, the higher, 4 µg dose of pGlu-MOG₃₀₋₆₀ yielded significantly (vs. both MOG alone and FTY720) improved retention of weight, which lasted throughout substantially all of the experiment.

As discussed above, this particular experiment was a nonlimiting example of how a tolerogenic composition according to several embodiments disclosed herein could be used to reduce the immune response that a subject generates to an antigen of interest, here an antigen associated with the autoimmune disease multiple sclerosis. As mentioned previously, other antigens, autoantigens, therapeutic proteins, fragments or specific epitopes of any of the preceding, or mimotope of any of the preceding can also successfully be used in accordance with the disclosure provided herein in order to induce immune tolerance.

### Example 31

### Biodistribution of pGal and pGlu Beta Conjugates

This experiment was conducted to determine the hepatic cell types targeted by pGal and pGlu conjugates as disclosed herein.

Protocol: mice were treated via tail vein injection with 100µg of OVA fluorescently labeled with the fluorescent dye Dy-649 (OVA649), 100µg of OVA conjugated to pGluβ (OVA649-pGluβ), or 100µg of OVA conjugated to pGalβ (OVA649-pGluβ). After 3 h, these mice were sacrificed and the livers were harvested, processed into single cell suspensions, and separated via density gradient centrifugation. The various hepatic cell types were then analyzed for protein content (e.g., as a measure of OVA649) via flow cytometry.

Results: Results show that both OVA649-pGalβ and OVA649-pGluβ conjugates are more effective at targeting liver sinusoidal endothelial cells (LSECs) as compared to OVA649 (Figure 27A). LSECs taken from animals treated with OVA649-pGalβ had a two-fold increase in mean fluorescent intensity (MFI) and LSECs taken from animal treated with OVA649-pGluβ had a 2.5-fold increase in MFI as compared to LSECs taken from animals treated with OVA649.

Kupffer Cells were also efficiently targeted by OVA649-pGluβ conjugates. The percentage of Kupffer cells that took up OVA649-pGluβ in animals treated with OVA649-pGluβ was significantly greater than the percentage of Kupffer Cells that took up OVA649 (Figure 27B). In several embodiments, pGlu in the beta conformation is therefore used when it may be desirable to target Kupffer cells. That being said, pGal conjugates can also optionally be used in certain embodiments.

In addition to Kupffer cells, CD11c+ cells also efficiently took up OVA649-pGluβ. The percentage of CD11c+ cells that took up OVA649-pGluβ was significantly greater than the percentage of CD11c+ that were targeted by OVA649 (Figure 27C). In several embodiments, pGlu in the beta conformation is therefore used when it may be desirable to target CD11C+ cells. That being said, pGal conjugates can also optionally be used in certain embodiments.

Interestingly, both OVA649-pGluβ and OVA649-pGalβ conjugates were more effective at targeting hepatocytes as compared to OVA649. See Figure 27D. However, the percentage of hepatocytes that took up OVA649-pGalβ was greater than the percentage of hepatocytes targeted by OVA649-pGluβ. Thus, in several embodiments, pGal in the beta conformation is therefore used when it may be desirable to target hepatocytes. That being said, pGlu conjugates can also optionally be used in certain embodiments.

Interestingly, an analysis of the ability of free OVA and OVA-glycopolymer conjugates to target stellate cells showed contrasting results. Figure 27E shows that OVA targets stellate cells more efficiently than either of the OVA-glycopolymer conjugates.

In several embodiments, combinations of pGlu and pGal conjugates may be desirable and the two types of composition interact synergistically to target the liver (and various cell types in the liver). Also, in several embodiments mixtures of conjugates in the alpha and beta configurations can also be used. As with other experiments disclosed herein, the use of OVA is as a non-limiting example of an antigen to which tolerance is desired. Other antigens disclosed herein, fragments thereof, and mimotopes thereof can also be conjugated to glycotargeting moieties, and tolerance thereto can be induced, based on the disclosure provided herein. In accordance with several embodiments disclosed herein, this induced tolerance can treat, reduce, prevent, or otherwise ameliorate an unwanted immune response that would have otherwise been associated with exposure to the antigen.

While the present disclosure has been described with reference to the specific embodiments thereof, it should be understood by those skilled in the art that various changes can be made and equivalents can be substituted without departing from the true spirit and scope of the disclosure. In addition, many modifications can be made to adapt a particular situation, material, composition of matter, process, process step or steps, to the objective, spirit and scope of the present disclosure. All such modifications are intended to be within the scope of the claims appended hereto. All publications, patents, patent applications, internet sites, and accession numbers/database sequences (including both polynucleotide and polypeptide sequences) cited are herein incorporated by reference in their entirety for all purposes to the same extent as if each individual publication, patent, patent application, internet site, or accession number/database sequence were specifically and individually indicated to be so incorporated by reference.

It is contemplated that various combinations or subcombinations of the specific features and aspects of the embodiments disclosed above may be made and still fall within one or more of the inventions. Further, the disclosure herein of any particular feature, aspect, method, property, characteristic, quality, attribute, element, or the like in connection with an embodiment can be used in all other embodiments set forth herein. Accordingly, it should be understood that various features and aspects of the disclosed embodiments can be combined with or substituted for one another in order to form varying modes of the disclosed inventions. Thus, it is intended that the scope of the present inventions herein disclosed should not be limited by the particular disclosed embodiments described above. Moreover, while the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents, and alternatives falling within the spirit and scope of the various embodiments described and the appended claims. Any methods disclosed herein need not be performed in the order recited. The methods disclosed herein include certain actions taken by a practitioner; however, they can also include any third-party instruction of those actions, either expressly or by implication. For example, actions such as "administering a glycotargeting tolerogenic composition" include "instructing the administration of a glycotargeting tolerogenic composition." In addition, where features or aspects of the disclosure are described in terms of Markush groups, those skilled in the art will recognize that the disclosure is also thereby described in terms of any individual member or subgroup of members of the Markush group.

The ranges disclosed herein also encompass any and all overlap, sub-ranges, and combinations thereof. Language such as "up to," "at least," "greater than," "less than," "between," and the like includes the number recited. Numbers preceded by a term such as "about" or "approximately" include the recited numbers. For example, "about 10 nanometers" includes "10 nanometers."

**The invention also pertains to the following:**
1. A compound comprising Formula 1: 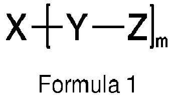 wherein:
   m is an integer from about 1 to 10;
   X comprises a protein or protein fragment comprising an antigenic region;
   Y is of a linker moiety having a formula selected from the group consisting of: 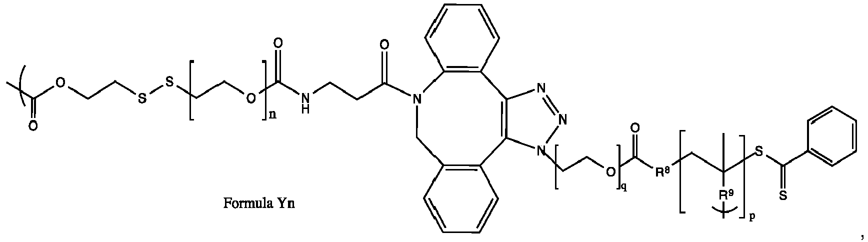 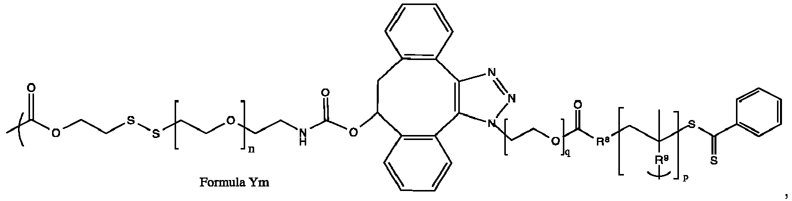 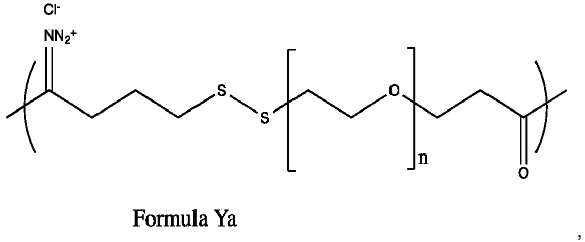 and 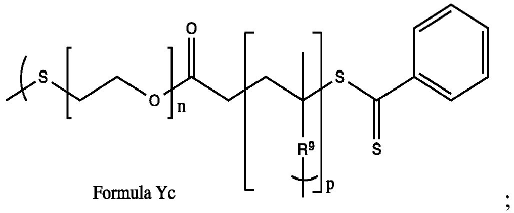
   wherein:
   the left bracket "(" indicates a bond to X;
   the right or bottom bracket and ")" indicates the bond between Y and Z;
   n is an integer from about 1 to 100;
   where present p is an integer from about 2 to 150;
   where present q is an integer from about 1 to 44;
   where present R⁸ is -CH₂- or -CH₂-CH₂-C(CH₃)(CN)-; and
   where present R⁹ is a direct bond or -CH₂-CH₂--NH-C(O)-; and
   Z comprises a liver-targeting moiety.
2. The compound of item 1 where Z is galactose or glucose.
3. The compound of item 1 where Z is galactosamine or glucosamine.
4. The compound of item 1 where Z is N-acetylgalactosamine or N-acetylglucosamine.
5. The compound of item 1, wherein Y is a linker moiety having a formula of: 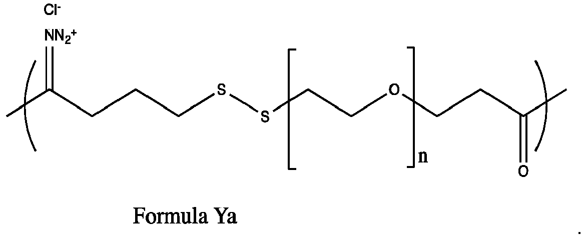
6. The compound of item 1, wherein Y is a linker moiety having a formula of: 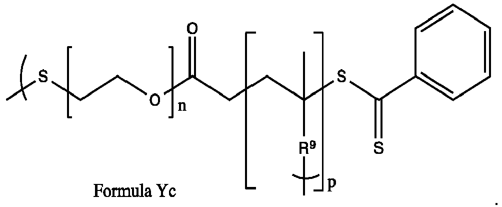
7. The compound of item 1, wherein Y is a linker moiety having a formula of: 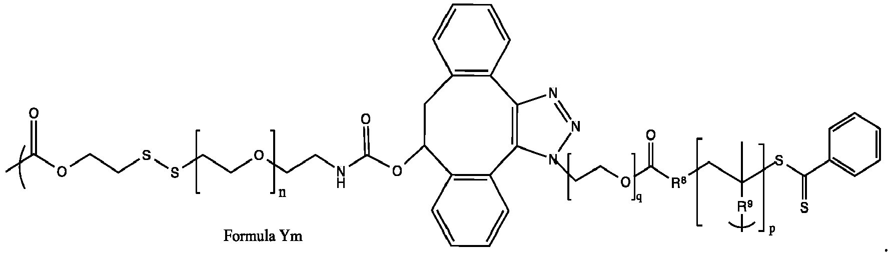
8. The compound of item 1, wherein Y is a linker moiety having a formula of: 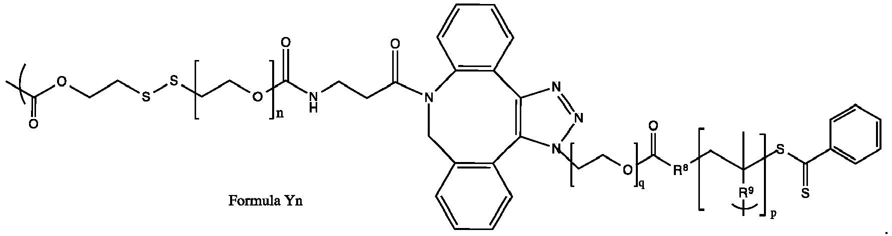
9. The compound of Item 1, wherein X is a is a food antigen selected from the group consisting of: conarachin (Ara h 1), allergen II (Ara h 2), arachis agglutinin, conglutin (Ara h 6), a-lactalbumin (ALA), lactotransferrin, Pen a 1 allergen (Pen a 1), allergen Pen m 2 (Pen m 2), tropomyosin fast isoform, high molecular weight glutenin, low molecular weight glutenin, alpha- gliadin, gamma-gliadin, omega-gliadin, hordein, seclain, and avenin and fragments thereof.
10. The compound of item 9, wherein X is selected from the group consisting of gluten, high molecular weight glutenin, low molecular weight glutenin, alpha- gliadin, gamma-gliadin, omega-gliadin, hordein, seclain, and avenin and fragments thereof.
11. The compound of item 10, wherein X is selected from the group consisting of gluten, high molecular weight glutenin, low molecular weight glutenin, alpha- gliadin, gamma-gliadin, and omega-gliadin and fragments thereof.
12. The compound of item 11, wherein X is gluten or fragment thereof.
13. The compound of item 11, wherein X is gliadin or fragment thereof.
14. The compound of Item 1, wherein X is a therapeutic agent selected from the group consisting of Factor VII, Factor IX, asparaginase, and uricase and fragments thereof.
15. The compound of Item 14, wherein X is a therapeutic agent selected from the group consisting of Factor VII and Factor IX and fragments thereof.
16. The compound of Item 15, wherein X is a therapeutic agent selected from the group consisting of Factor VIII or fragment thereof.
17. The compound of Item 14, wherein X comprises asparaginase or a fragment thereof.
18. The compound of Item 14, wherein X comprises uricase or a fragment thereof.
19. The compound of Item 1, wherein X is associated with an autoimmune disease.
20. The compound of Item 19, wherein the autoimmune disease is selected from the group consisting of Type I diabetes, multiple sclerosis, rheumatoid arthritis, vitiligo, uveitis, pemphis vulgaris and neuromyelitis optica.
21. The compound of Item 20, wherein the autoimmune disease is Type I diabetes and X comprises insulin or a fragment thereof.
22. The compound of Item 20, wherein the autoimmune disease is Type I diabetes and X comprises proinsulin or a fragment thereof.
23. The compound of Item 20, wherein the autoimmune disease is Type I diabetes and X comprises preproinsulin or a fragment thereof.
24. The compound of Item 20, wherein the autoimmune disease is multiple sclerosis and X comprises myelin basic protein or a fragment thereof.
25. The compound of Item 20, wherein the autoimmune disease is multiple sclerosis and X comprises myelin oligodendrocyte glycoprotein or a fragment thereof.
26. The compound of Item 20, wherein the autoimmune disease is multiple sclerosis and X comprises myelin proteolipid protein or a fragment thereof.
27. The compound of Item 20, wherein the autoimmune disease is rheumatoid arthritis and X is selected from the group consisting of fibrinogen, vimentin, collagen type II, alpha enolase and fragments thereof.
28. The compound of Item 20, wherein the autoimmune disease is vitiligo and X is selected from the group consisting of Pmel17, tyrosinase and fragments thereof.
29. The compound of Item 20, wherein the autoimmune disease is uveitis and X is selected from the group consisting of retinal arrestin and interphotoreceptor retinoid-binding protein (IRBP) and fragments thereof.
30. The compound of Item 20, wherein the autoimmune disease is pemphigus vulgaris and X is selected from the group consisting of desmoglein 3, 1 and 4, pemphaxin, desmocollins, plakoglobin, perplakin, desmoplakins, acetylcholine receptor and fragments thereof.
31. The compound of Item 20, wherein the autoimmune disease is neuromyelitis optica and X is aquaporin-4 or a fragment thereof.
32. Use of a compound according to any one of Items 1 to 31, for use in inducing tolerance to X.
33. A composition comprising a compound according to any one of Items 1 to 8.
34. Use of the composition of Item 33, for use in inducing tolerance to X.
35. A method of inducing tolerance to an antigen to which a subject is capable of developing an unwanted immune response, comprising administering a compound according to any one of Items 1 to 31.
36. The method of Item 35, wherein the compound is administered prior to the subject being exposed to the antigen.
37. The method of Item 35, wherein the compound is administered after the subject has been exposed to the antigen.
38. The method of Item 35, wherein the administration comprises at least one intravenous administration of the compound.
39. Use of the compound according to any one of Items 1 to 31 for use in the preparation of a medicament for inducing tolerance to an antigen to which a subject develops an unwanted immune response or a tolerogenic portion thereof.
40. The composition of any one of Items 1 to 8, wherein X comprises a foreign transplant antigen against which transplant recipients develop an unwanted immune response or a tolerogenic portion thereof.
41. The composition of any one of Items 1 to 8, wherein X comprises a foreign food, animal, plant or environmental antigen against which patients develop an unwanted immune response or a tolerogenic portion thereof.
42. The composition of any one of Items 1 to 8, wherein X comprises a foreign therapeutic agent against which patients develop an unwanted immune response or a tolerogenic portion thereof.
43. The composition of any one of Items 1 to 8, wherein X comprises a synthetic self-antigen against the endogenous version of which patients develop an unwanted immune response or a tolerogenic portion thereof.
44. A compound comprising Formula 1: 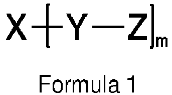 where:
   m is an integer from about 1 to 10;
   X comprises a protein or protein fragment comprising an antigenic region of a therapeutic protein selected from the group consisting of Factor VIII, Factor IX, insulin, uricase, PAL and asparaginase;
   Y is of a linker moiety having a formula selected from the group consisting of: 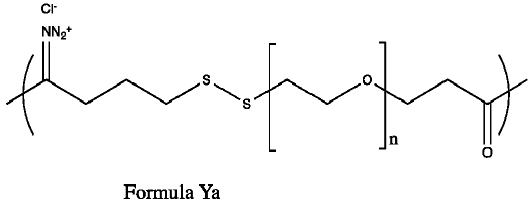 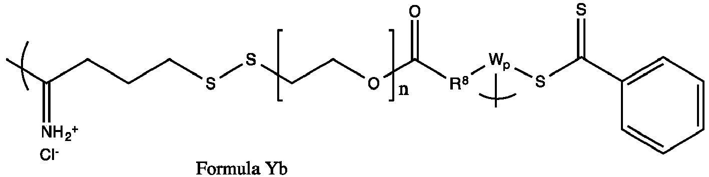 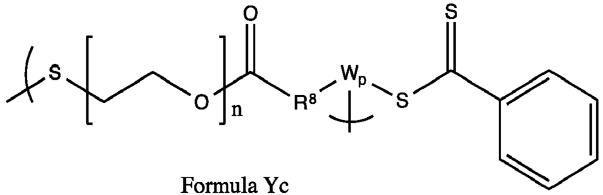 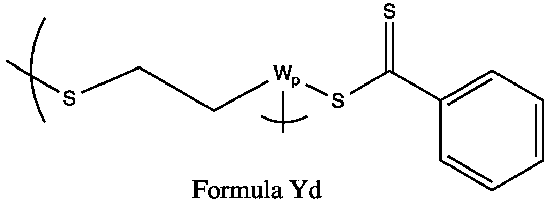 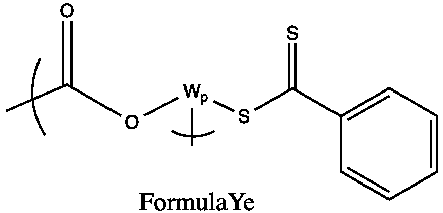 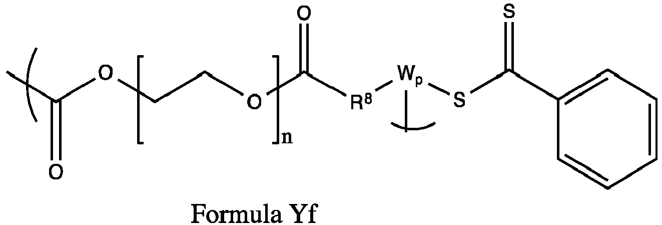 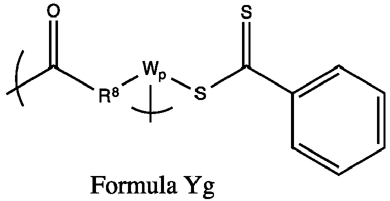 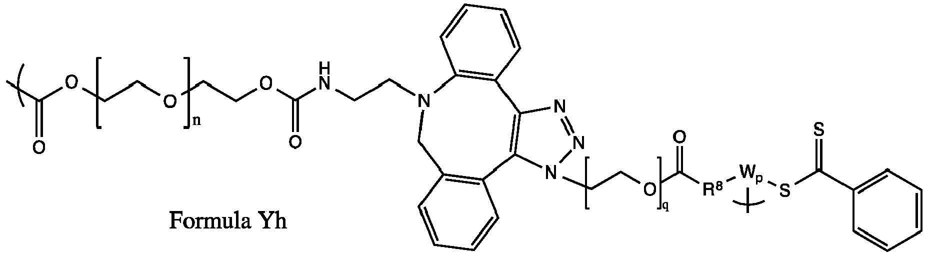 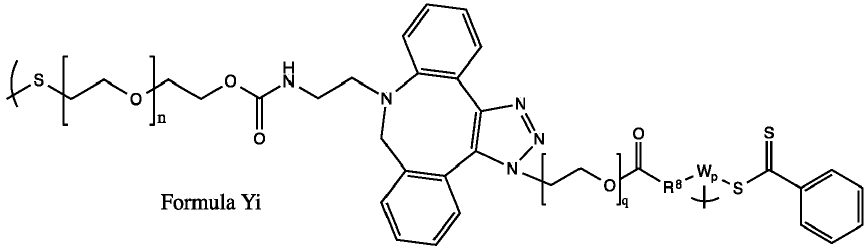 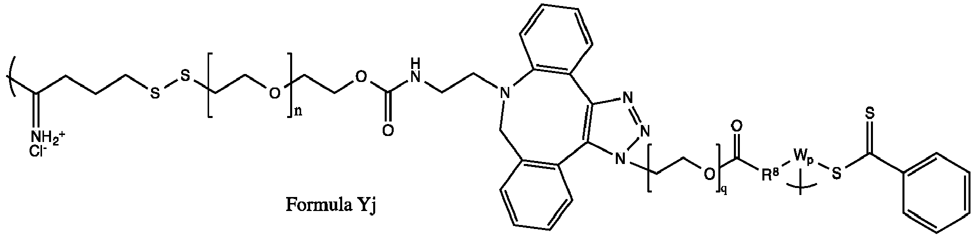 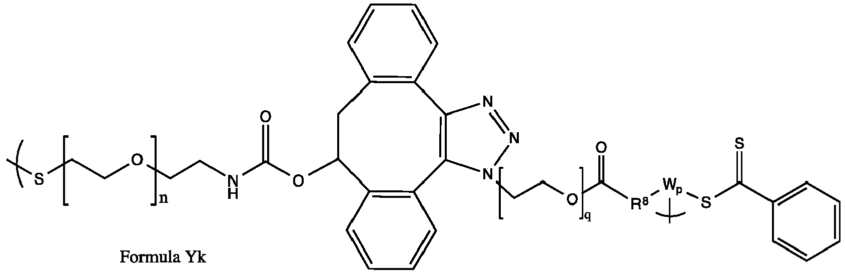 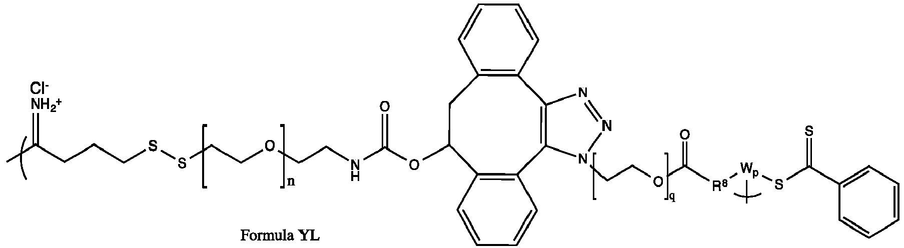 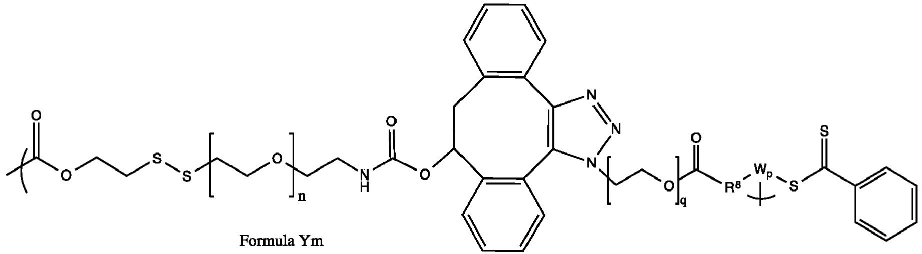 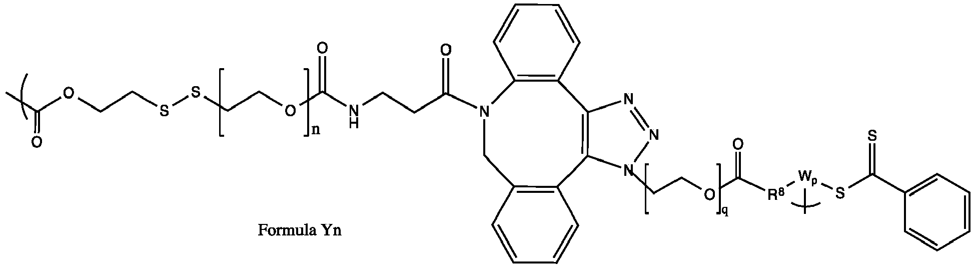 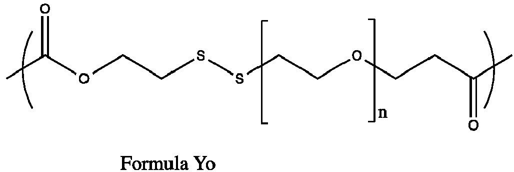 and 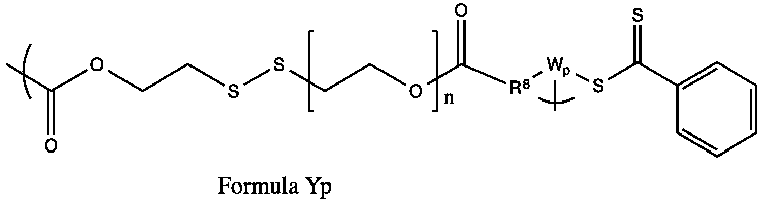
   wherein:
   the left bracket "(" indicates a bond to X;
   where present the right ")" indicates a bond to Z;
   where present the bottom ")" indicates a bond to Z;
   where present n is an integer from about 1 to about 80;
   where present q is an integer from about 1 to about 4;
   where present p is an integer from about 1 to about 90;
   where present R⁸ is -CH₂- or -CH₂-CH₂-C(CH₃)(CN)-; and
   where present W represents a polymer of the Formula W¹ or W² group or
      W is a copolymer of Formula W¹ or W² where: 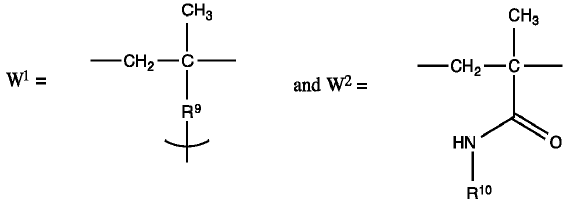
      where:
         R⁹ is a direct bond, -CH₂-CH₂--NH-C(O)- or -CH₂-CH₂-(O-CH₂-CH₂)ₜ-NH-C(O)- ;
         t is an integer from 1 to 5;
         R¹⁰ is an aliphatic group, an alcohol or an aliphatic alcohol; and
   Z comprises one or more liver-targeting moieties.
45. The compound of item 44, wherein the one or more liver targeting moieties comprises one or more of galactose, galactosamine, N-acetylgalactosamine, glucose, glucosamine or N-acetylglucosamine.
46. The compound of item 44 or 45, wherein the one or more liver targeting moieties comprises one or more moieties having a formula selected from the group consisting of: 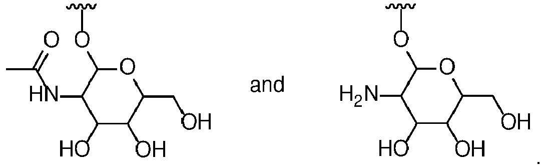
47. The compound of any one of items 44 to 46, wherein Y is selected from the group consisting Formula Ya, Formula Yb, Formula Yc, Formula Ym or Formula Yn.
48. The compound of any one of items 44 to 47, wherein Y is selected from the group consisting of Formula Ya, Formula Yc, and Formula Ym.
49. The compound of any one of items 44 to 48, wherein X comprises an antibody, antibody fragment or ligand that specifically binds a circulating protein or peptide or antibody, which circulating protein or peptide or antibody is causatively involved in transplant rejection, immune response against a therapeutic agent, autoimmune disease, hypersensitivity and/or allergy.
50. The composition of any one of items 44 to 49, wherein Z is conjugated at its C1, C2 or C6 to Y.
51. The composition of any one of items 44 to 50, wherein Y is selected from N-hydroxysuccinamidyl linkers, malaemide linkers, vinylsulfone linkers, pyridyl di-thiol-poly(ethylene glycol) linkers, pyridyl di-thiol linkers, n-nitrophenyl carbonate linkers, NHS-ester linkers, and nitrophenoxy polyethylene glycol)ester linkers.
52. A pharmaceutically acceptable composition for inducing tolerance to a therapeutic protein in a subject having a deficiency in production of a functional analogous native protein, comprising the compound of any one of items 44 to 51.
53. Use of the compound or composition of any one of items 23 to 31 for treating an unwanted immune response against an antigen.
54. The compound or composition of any one of items 44 to 52 for manufacturing a medicament for use in treating an unwanted immune response against an antigen.
55. A compound of Formula 1: 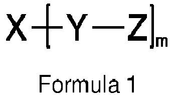 wherein:
   m is an integer from about 1 to 10;
   X comprises a protein or protein fragment comprising an antigenic region;
      Y is of a linker moiety having a formula selected from the group consisting of: 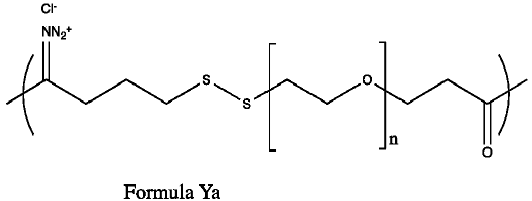 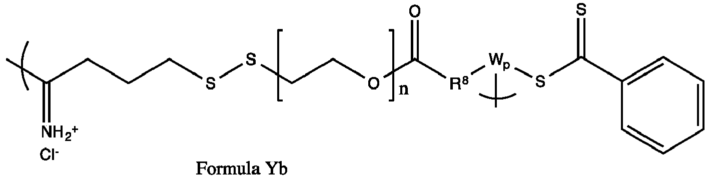 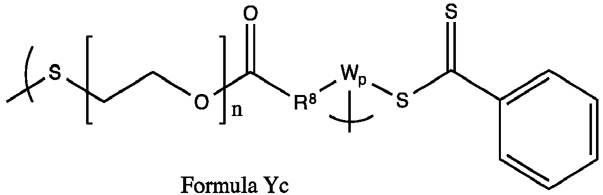 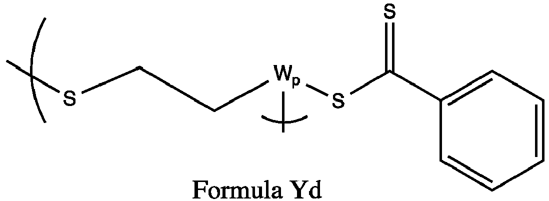 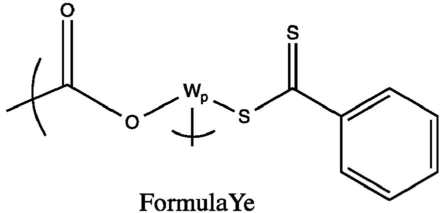 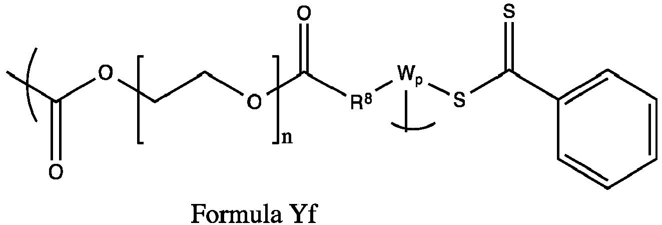 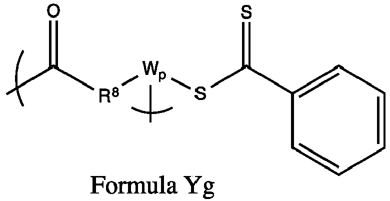 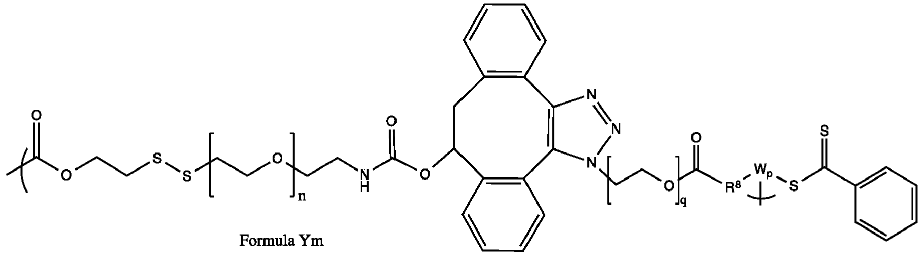 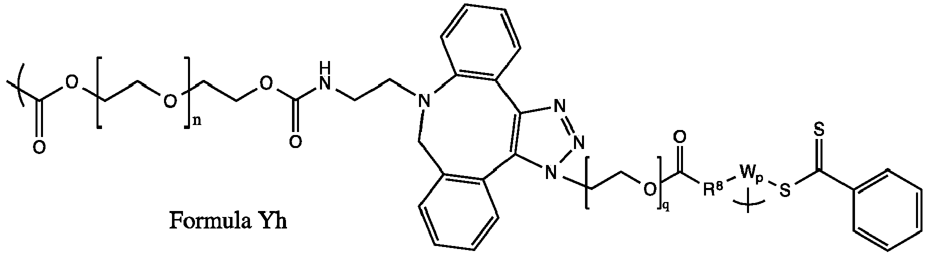 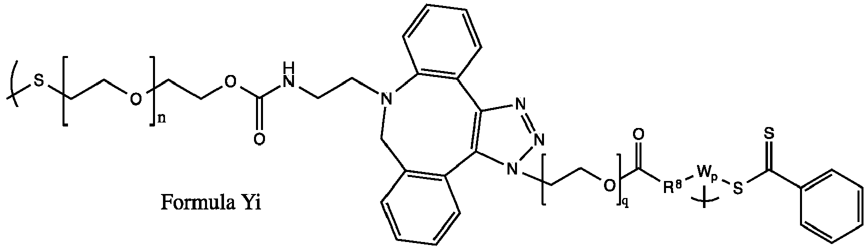 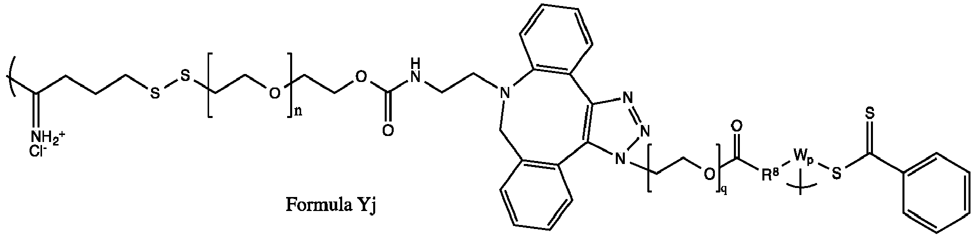 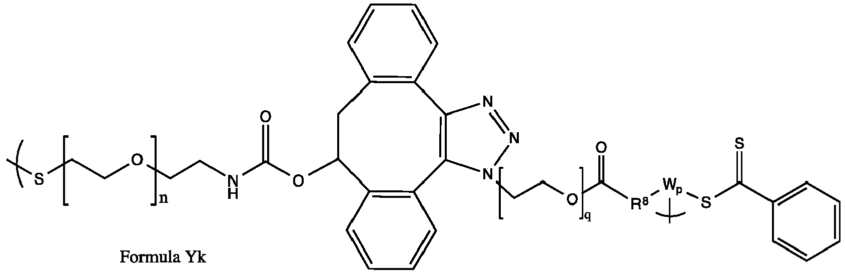 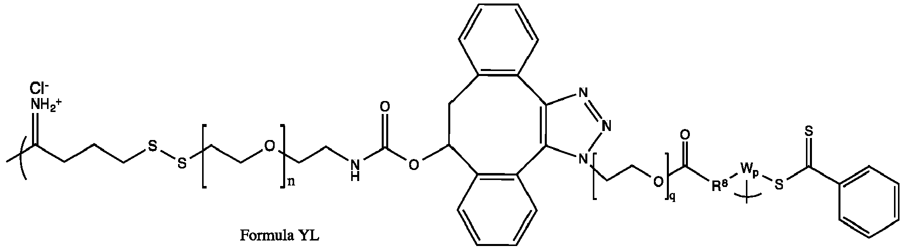 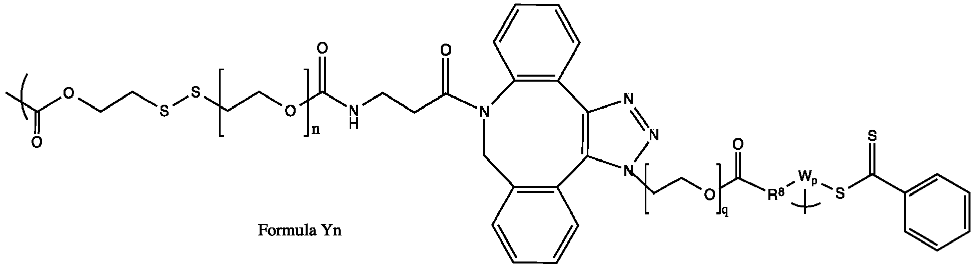 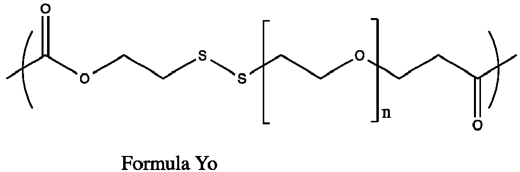 and 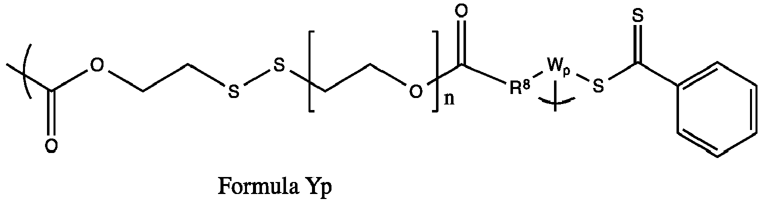 or Y has a portion represented by Formula Y'-CMP: 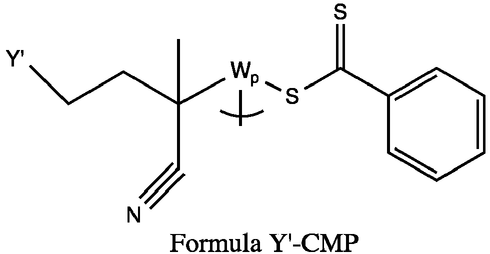
   where:
   the left bracket "(" indicates the bond between X and Y;
   the right or bottom bracket and ")" indicates the bond between Y and Z;
   n is an integer from about 1 to 100;
   q is an integer from about 1 to 44;
   R⁸ is -CH₂- or -CH₂-CH₂-C(CH₃)(CN)-;
   Y' represents the remaining portion of Y; and
   W represents a polymer of the same W¹ group, or W is a copolymer or a random copolymer of the same or different W¹ and W² groups, where: 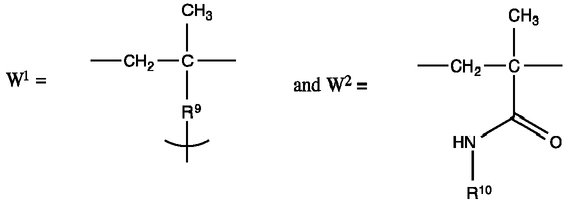 where:
      p is an integer from 2 to about 150;
      R⁹ is a direct bond, -CH₂-CH₂--NH-C(O)- or -CH₂-CH₂-(O-CH₂-CH₂)ₜ-NH-C(O)- ;
      t is an integer from 1 to 5; and
      R¹⁰ is an aliphatic group, an alcohol or an aliphatic alcohol.
56. The compound of Item 54, wherein:
   n is about 40 to 80;
   p is about 10 to 100;
   q is about 3 to 20;
   R⁸ is -CH₂-CH₂-C(CH₃)(CN)-;
   when R⁹ is -CH₂-CH₂--NH-C(O)-, Z one or of galactose, galactosamine, N-acetylgalactosamine, glucose, glucosamine or N-acetylglucosamine; and
   when W is a copolymer, R¹⁰ is 2-hydroxypropyl.
57. The compound of Item 55 or 56, wherein Y comprises Formula Ya, Formula Yb, Formula Yc, Formula Yf, Formula Yg, Formula Yh, Formula Yi, Formula Yk, Formula Ym or Formula Yn.
58. The composition of any one of Items 55 to 57, wherein Y comprises Formula Ya, Formula Yb, Formula Yc, Formula Ym or Formula Yn.
59. The composition of any one of Items 55 to 58, wherein Y comprises Formula Ya, Formula Yb, Formula Yc, Formula Ym or Formula Yn.
60. The composition of any one of Items 55 to 59, wherein X comprises:
   a foreign transplant antigen against which transplant recipients develop an unwanted immune response;
   aforeign food, animal, plant or environmental antigen against which patients develop an unwanted immune response;
   a foreign therapeutic agent against which patients develop an unwanted immune response; or
   a synthetic self-antigen against the endogenous version of which patients develop an unwanted immune response,
   or a tolerogenic portion thereof.
61. A composition comprising the compound of any one of items 55 to 60.
62. The use of the composition or composition of any one of Items 55 to 61, for treatment for an unwanted immune response.
63. Use of a compound of Formula 1: 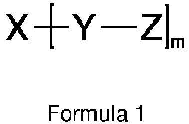 where:
   m is an integer from about 1 to 100;
   X comprises an antigen against which a patient develops an unwanted immune response, or a tolerogenic portion thereof; or
   X comprises an antibody, antibody fragment or ligand that specifically binds a circulating protein or peptide or antibody, which circulating protein or peptide or antibody is causatively involved in transplant rejection, immune response against a therapeutic agent, autoimmune disease, hypersensitivity and/or allergy;
   Y comprises a linker moiety; and
   Z comprises a glucosylated liver-targeting moiety;
   for treatment for an unwanted immune response against an antigen by administering to a mammal in need of such treatment an effective amount of a composition comprising.
64. The use of item 63, wherein:
   X comprises an antigen against which a patient develops an unwanted immune response, or a tolerogenic portion thereof; and
   Y comprises:
      an antibody, antibody fragment, peptide or other ligand that specifically binds X;
      a disulfanyl ethyl ester;
      a structure represented by one of Formulae Ya to Yp: 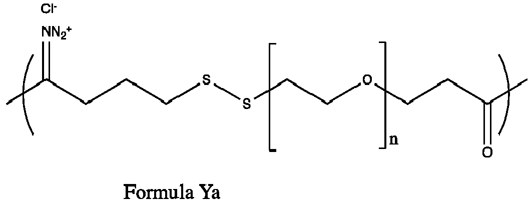 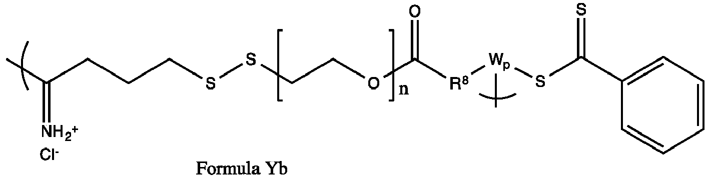 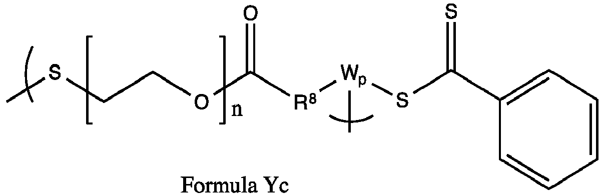 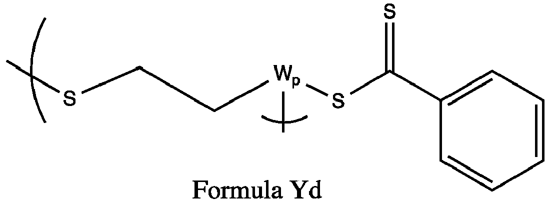 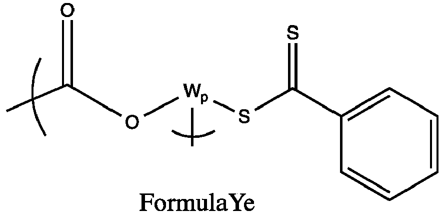 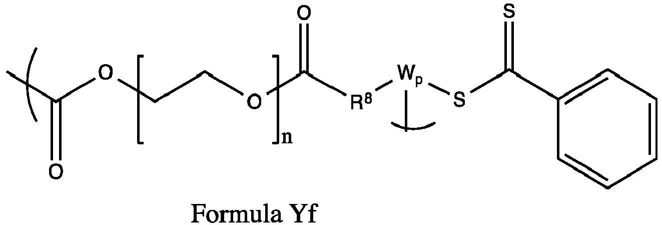 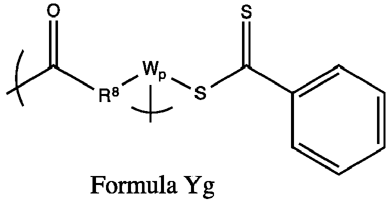 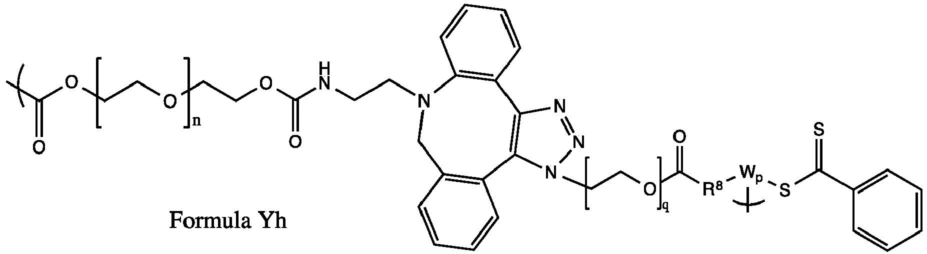 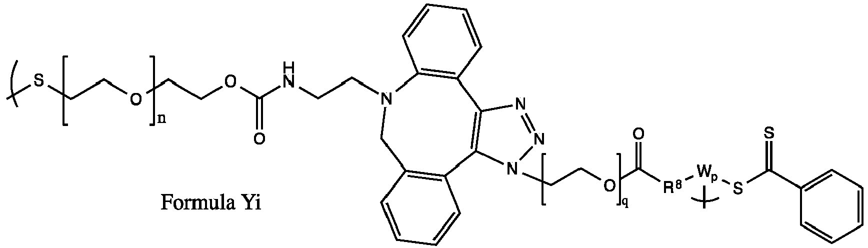 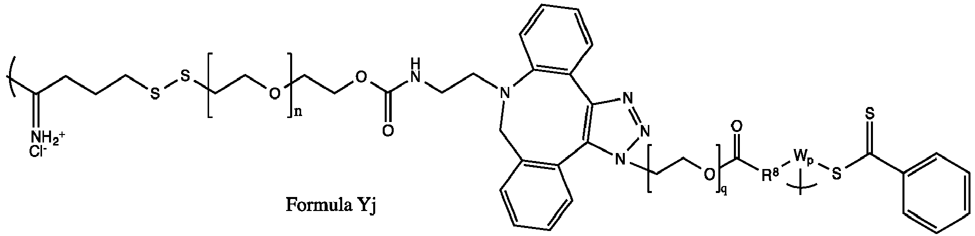 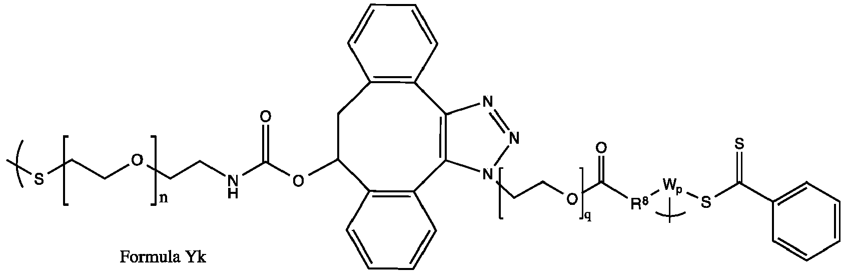 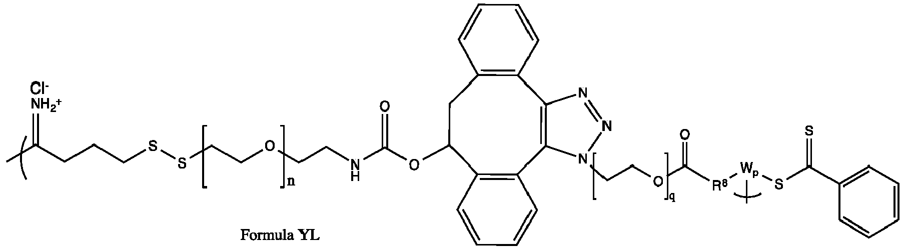 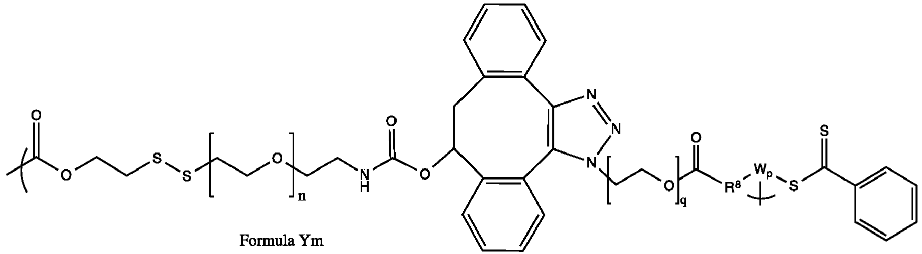 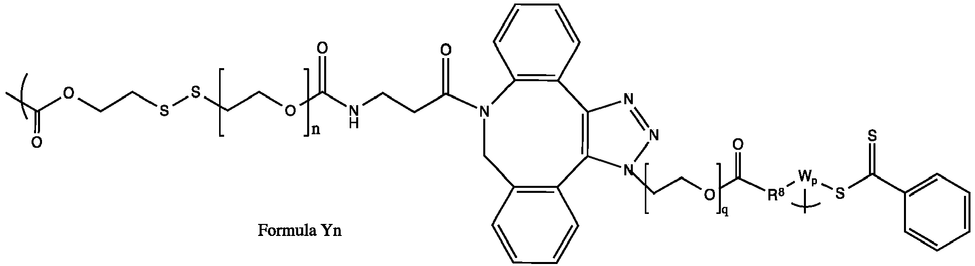 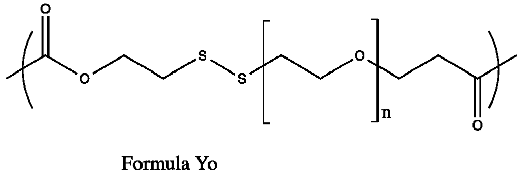 and 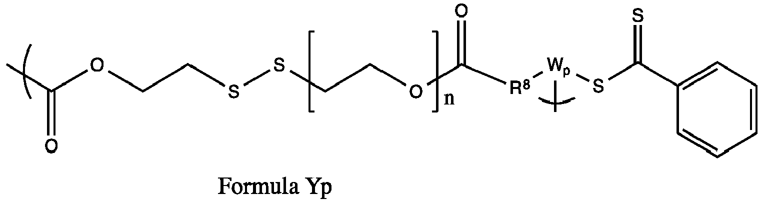
      or Y has a portion represented by Formula Y'-CMP: 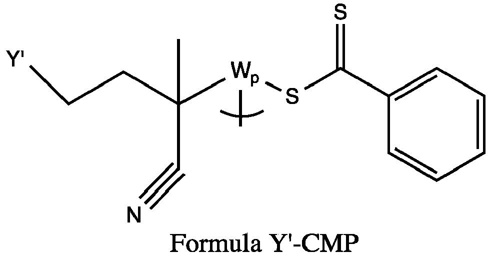
   where:
   the left bracket "(" indicates the bond between X and Y;
   the right or bottom bracket and ")" indicates the bond between Y and Z;
   n is an integer from about 1 to 100;
   q is an integer from about 1 to 44;
   R⁸ is -CH₂- or -CH₂-CH₂-C(CH₃)(CN)-;
   Y' represents the remaining portion of Y; and
   W represents a polymer of the same W¹ group, or W is a copolymer or a random copolymer of the same or different W¹ and W² groups, where: 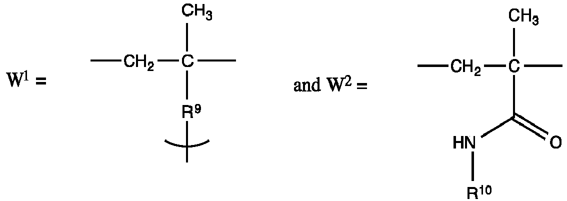 where:
      p is an integer from 2 to about 150;
      R⁹ is a direct bond, -CH₂-CH₂--NH-C(O)- or -CH₂-CH₂-(O-CH₂-CH₂)ₜ-NH-C(O)- ;
      t is an integer from 1 to 5; and
      R¹⁰ is an aliphatic group, an alcohol or an aliphatic alcohol.
65. The use of item 63 or 64, wherein X comprises the antibody, antibody fragment or ligand, and the composition is administered for clearance of a circulating protein or peptide or antibody that specifically binds to X, which circulating protein or peptide or antibody is causatively involved in transplant rejection, immune response against a therapeutic agent, autoimmune disease, hypersensitivity and/or allergy.
66. The use of any one of items 63 to 65, wherein X comprises an antibody, antibody fragment or ligand, and the composition is administered in an amount effective to reduce a concentration of the antibodies that are causatively involved in transplant rejection, immune response against a therapeutic agent, autoimmune disease, hypersensitivity and/or allergy in blood of the patient by at least 50% w/w, as measured at a time between about 12 to about 48 hours after the administration.
67. The use of any one of items 63 to 66, wherein the composition is administered for tolerization of the patient with respect to antigen moiety X.
68. The use of any one of items 63 to 67, wherein X comprises:
   a foreign transplant antigen against which transplant recipients develop an unwanted immune response;
   aforeign food, animal, plant or environmental antigen against which patients develop an unwanted immune response;
   a foreign therapeutic agent against which patients develop an unwanted immune response; or
   a synthetic self-antigen against the endogenous version of which patients develop an unwanted immune response,
   or a tolerogenic portion thereof.
69. A compound comprising Formula 1: 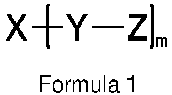 wherein:
   m is an integer from about 1 to 10;
   X comprises a food antigen, a therapeutic agent, a self-antigen, a fragment of any of such antigens, or a mimotope of any of such antigens;
   Y is of a linker moiety having the following formula: 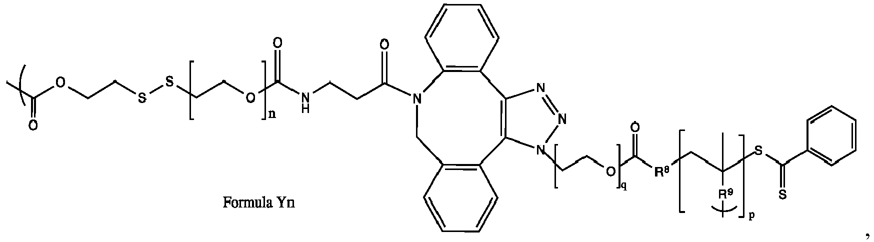
   wherein:
   the left bracket "(" indicates a bond to X;
   the right or bottom bracket and ")" indicates the bond between Y and Z;
   n is an integer from about 70 to 85;
   where present p is an integer from about 85 to 95;
   where present q is an integer from about 1 to 10;
   where present R⁸ is -CH₂- or -CH₂-CH₂-C(CH₃)(CN)-; and
   where present R⁹ is a direct bond or -CH₂-CH₂--NH-C(O)-; and
   Z comprises a liver-targeting moiety comprising glucose or galactose.
70. The compound of item 69, wherein:
   m is between 1 and 3,
   n is 79,
   p is 90, and
   q is 4.
71. The compound of item 70, wherein X is selected from the group consisting of insulin, proinsulin, preproinsulin, gluten, gliadin, myelin basic protein, myelin oligodendrocyte glycoprotein and proteolipid protein, Factor VIII, Factor IX, asparaginase, uricase and fragments of any of the preceding.
72. A compound comprising Formula 1: 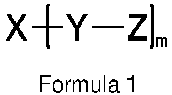 wherein:
   m is an integer from about 1 to 10;
   X is selected from the group consisting of insulin, proinsulin, preproinsulin, gluten, gliadin, myelin basic protein, myelin oligodendrocyte glycoprotein and proteolipid protein, Factor VIII, Factor IX, asparaginase, uricase and fragments of any of the preceding;
   Y is of a linker moiety having the following formula: 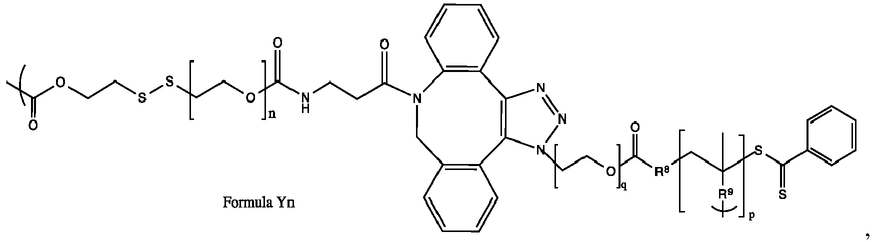
   wherein:
   the left bracket "(" indicates a bond to X;
   the right or bottom bracket and ")" indicates the bond between Y and Z;
   n is an integer from about 70 to 85;
   where present p is an integer from about 85 to 95;
   where present q is an integer from about 1 to 10;
   where present R⁸ is -CH₂- or -CH₂-CH₂-C(CH₃)(CN)-; and
   where present R⁹ is a direct bond or -CH₂-CH₂--NH-C(O)-; and
   Z comprises a liver-targeting moiety comprising a sugar moiety.
73. The compound of item 69, wherein:
   m is between 1 and 3,
   n is 79,
   p is 90, and
   q is 4.
74. The compound of item 73 where Z is selected from the group consisting of glucose, glucosamine, galactose, galactosamine, N-acetylgalactosamine and N-acetylglucosamine.

## Claims

1. A composition comprising a compound of Formula 1 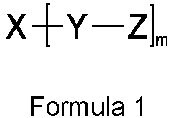 wherein:
m is an integer from about 1 to 100;
X comprises an antigen against which a patient develops an unwanted immune response, or a tolerogenic portion thereof;
Y comprises a linker moiety; and
Z comprises a liver-targeting moiety, wherein the linker moiety comprises N-acetylgalactosamine, galactose, galactosamine, glucose, glucosamine or N-acetylglucosam ine,
wherein the compound is obtainable by a process, which process comprises reacting compounds of formulae 1505 and 1506 in a reversible addition-fragmentation chain transfer (RAFT) polymerization: 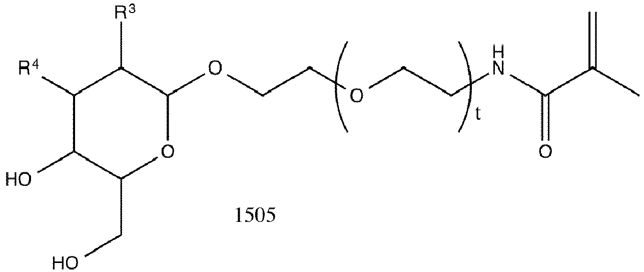 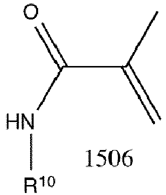
wherein R³ is OH or NHAc, R⁴ is OH, t is 1 to 5, and R¹⁰ is 2-hydroxypropyl.

2. The composition of claim 1, wherein in the RAFT polymerization, an agent of Formula 1106 is used: 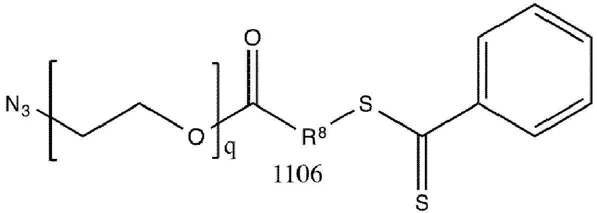 wherein q is 4 and R⁸ is 1-cyano-1-methyl-propyl.

3. The composition of claim 1 or 2, where Z is conjugated to Y at the C1, C2 or C6.

4. The composition of any one of claims 1 t o3, where Y is selected from N-hydroxysuccinamidyl linkers, malaemide linkers, vinylsulfone linkers, pyridyl di-thiol-poly(ethylene glycol) linkers, pyridyl di-thiol linkers, n-nitrophenyl carbonate linkers, NHS-ester linkers, and nitrophenoxy polyethylene glycol)ester linkers.

5. The composition according to any one of claim 1 to 4, wherein X comprises an antigenic region of myelin basic protein, myelin oligodendrocyte glycoprotein, or myelin proteolipid protein.

6. The composition according to any one of claim 1 to 4, wherein X comprises a food antigen against which patients develop an unwanted immune response.

7. The composition according to any one of claim 1 to 6, wherein X comprises
a) a food antigen selected from SEQ ID NO: 20, SEQ ID NO: 21, SEQ ID NO: 22, and SEQ ID NO: 23, to which patients develop an allergic or hypersensitivity response, or
b) an antigen selected from SEQ ID NO: 7, SEQ ID NO: 8, SEQ ID NO: 9, SEQ ID NO: 10, SEQ ID NO: 11, SEQ ID NO: 12, and SEQ ID NO: 13, against which a patient develops an autoimmune response.

8. The composition according to any one of claim 1 to 7 for use in the tolerization of said patient with respect to antigen moiety X.

9. A composition comprising a compound of Formula 1 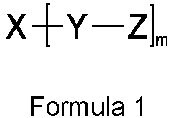 wherein:
m is an integer from about 1 to 100;
X comprises an antigen against which a patient develops an unwanted immune response, or a tolerogenic portion thereof;
Y comprises a linker moiety; and
Z comprises a liver-targeting moiety, wherein the linker moiety comprises glucose, glucosamine or N-acetylglucosamine.

10. The composition of claim 9 where Z is conjugated to Y at the C1, C2 or C6.

11. The composition of claim 9 or 10 where Y is selected from N-hydroxysuccinamidyl linkers, malaemide linkers, vinylsulfone linkers, pyridyl di-thiol-poly(ethylene glycol) linkers, pyridyl di-thiol linkers, n-nitrophenyl carbonate linkers, NHS-ester linkers, and nitrophenoxy polyethylene glycol)ester linkers.

12. The composition according to any one of claims 9 to 11, wherein X comprises an antigenic region of myelin basic protein, myelin oligodendrocyte glycoprotein, or myelin proteolipid protein.

13. The composition according to any one of claims 9 to 11, wherein X comprises a food antigen against which patients develop an unwanted immune response.

14. The composition according to any one of claims 9 to 13, wherein X comprises
a) a food antigen selected from SEQ ID NO: 20, SEQ ID NO: 21, SEQ ID NO: 22, and SEQ ID NO: 23, to which patients develop an allergic or hypersensitivity response, or
b) an antigen selected from SEQ ID NO: 7, SEQ ID NO: 8, SEQ ID NO: 9, SEQ ID NO: 10, SEQ ID NO: 11, SEQ ID NO: 12, and SEQ ID NO: 13, against which a patient develops an autoimmune response.

15. The composition according to any one of claims 9 to 14 for use in the tolerization of said patient with respect to antigen moiety X.
